# EUROPEAN PATENT SPECIFICATION

(11) **EP 4 153 772 B1**
(45) Date of publication and mention of the grant of the patent: **01.07.2026**
(21) Application number: 21807865.7
(22) Date of filing: 18.05.2021
(51) Int. Cl.: C12Q 1/6816

(54) **SYSTEMS AND METHODS OF A NUCLEASE CHAIN REACTION FOR NUCLEIC ACID DETECTION**
SYSTEME UND VERFAHREN EINER NUKLEASEKETTENREAKTION ZUM NUKLEINSÄURENACHWEIS
SYSTÈMES ET PROCÉDÉS DE RÉACTION EN CHAÎNE DE NUCLÉASE POUR LA DÉTECTION D'ACIDE NUCLÉIQUE

(30) Priority: 19.05.2020 US 202063027175 P
(43) Date of publication of application: 29.03.2023
(73) Proprietor: The Regents of the University of California, Oakland, CA 94607 (US)
(72) Inventor: SAVAGE, David, Berkeley, California 94704 (US); HSU, Patrick, Berkeley, California 94704 (US); PRYWES, Noam, Berkeley, California 94704 (US); CHARLES, Emeric J., Berkeley, California 94704 (US); KNOTT, Gavin J., Berkeley, California 94704 (US); DESMARAIS, John James, Berkeley, California 94704 (US); KIM, Shineui, Berkeley, California 94704-1347 (US); DUGAN, Eli, Berkeley, California 94704 (US); LUKARSKA, Maria, Berkeley, California 94704 (US); CHANDRASEKARAN, Sita S., Berkeley, California 94704 (US); LAMMERS, Nicholas C., Berkeley, California 94704 (US); LIU, Tina Y., Berkeley, California 94704 (US); MOK, Amanda, Berkeley, California 94704 (US); LAREAU, Liana, Berkeley, California 94704 (US); THORNTON, Brittney, Wai-Ling, Berkeley, California 94704 (US)
(74) Representative: J A Kemp LLP
(86) International application number: PCT/US2021/032977
(87) International publication number: WO 2021/236651

(56) References cited:
- WO-A1-2017/218573
- US-A- 5 538 848
- US-A1- 2005 048 527
- US-A1- 2009 270 602
- US-A1- 2019 062 724
- KARL PETRI: "SHERLOCK and DETECTR Open a New Frontier in Molecular Diagnostics", vol. 1, no. 3, 1 June 2018 (2018-06-01), pages 209 - 211, XP093162979, ISSN: 2573-1599, Retrieved from the Internet <URL:https://dx.doi.org/10.1089/crispr.2018.29018.kpe> DOI: 10.1089/crispr.2018.29018.kpe
- JAMES P. BROUGHTON ET AL: "CRISPR-Cas12-based detection of SARS-CoV-2", NATURE BIOTECHNOLOGY, 16 April 2020 (2020-04-16), New York, XP055709764, ISSN: 1087-0156, DOI: 10.1038/s41587-020-0513-4
- GOOTENBERG JONATHAN S. ET AL: "Nucleic acid detection with CRISPR-Cas13a/C2c2", SCIENCE, vol. 356, no. 6336, 28 April 2017 (2017-04-28), US, pages 438 - 442, XP055816752, ISSN: 0036-8075, DOI: 10.1126/science.aam9321
- GOOTENBERG JONATHAN S. ET AL: "Nucleic acid detection with CRISPR-Cas13a/C2c2", SCIENCE, vol. 356, no. 6336, 13 April 2017 (2017-04-13), US, pages 438 - 442, XP055781069, ISSN: 0036-8075, Retrieved from the Internet <URL:https://science.sciencemag.org/content/sci/suppl/2017/04/12/science.aam9321.DC1/aam9321_Gootenberg_SM.pdf> DOI: 10.1126/science.aam9321
- YULIA V. GERASIMOVA ET AL: "Enzyme-assisted target recycling (EATR) for nucleic acid detection", CHEMICAL SOCIETY REVIEWS, vol. 43, no. 17, 1 January 2014 (2014-01-01), UK, pages 6405 - 6438, XP055324226, ISSN: 0306-0012, DOI: 10.1039/C4CS00083H

## Description

### CROSS-REFERENCE TO RELATED APPLICATIONS

This application claims the benefit of U.S. Patent Application Serial No. 63/027,175, filed on May 19, 2020.

### TECHNICAL FIELD

The present invention concerns methods and compositions for the detection of a nucleic acid, including detection systems comprising an internal nuclease chair reaction (NCR) for rapid and sensitive detection of any target nucleic acid sequence.

### GOVERNMENT SUPPORT

This invention was made with government support under Grant Number OD021369 awarded by the National Institutes of Health. The government has certain rights in the invention.

### BACKGROUND

Clustered regularly interspaced short palindromic repeats (CRISPR) were discovered in the late 1980s. While the notion that these sequences are involved in bacterial defense systems was suggested over the subsequent decades, it was not until the mid to late 2000s that it became more widely accepted. During that time several papers elucidated the basics of this acquired immunity system: foreign DNA sequences (e.g. from plasmids and viruses) flanked by palindromic repeats are incorporated in into the host genome, and their RNA products direct Cas complexes to cut nucleic acids containing complementary sequences.

Simplified complexes of CRISPR-associated (Cas) proteins in combination with engineered guide RNAs were later shown to be able to locate and cleave specific DNA sequences. This led to an explosion of novel technologies, especially genome editing. Further research has shown that these proteins may be used to edit genomes in vivo. CRISPR systems are found in archaea and a number of bacteria. In addition to their more widely recognized ability to target DNA, some types of Cas proteins also have activity that targets RNA. For example, the Cas13 family of enzymes, such as Cas13a, Cas13b, Cas13c, and Cas13d, have two RNA endonuclease (RNase) domains.

The non-specific ribonuclease (RNase) or deoxyribonuclease (DNase) activity of some CRISPR-associated proteins may be dormant until being activated by the binding of other factors to the protein or protein complex. As such, Cas13 or Cas12 enzymes can be programmed with a guide RNA that recognizes a desired target sequence, activating a non-specific RNase or DNase activity. This can be used to release a detectable label, such as a quenched fluorescent reporter, leading to a detectable signal such as fluorescence. For example, the SHERLOCK (Specific High-sensitivity Enzymatic Report UnLOCKing) uses Cas13 proteins for detection of RNA targets and the DETECTR system uses Cas12 proteins for DNA targets to cleave quenched reporter molecules only in the presence of a specified RNA or DNA target sequence. *See, e.g.,* Li et al. (2019) Trends in Biotech. 37(7):730-743; Petri & Pattanayak (2018) The CRISPR Journal 1(3):209-211; Gootenberg et al. (2017) Science 356(6336):438-442; and U.S. Publication Nos. 20180274017 and 20190241954.

However, current Cas-based detection systems may have limitations in sensitivity, and in addition, rapid testing results are desired in times of wide spread exposure to viral and other pathogens. Testing capable of producing sensitive, specific, and rapid results without the need for specialized equipment such that tests may be performed on a field basis, in a 'point-of care' or POC setting, or in a consumer 'at-home' use are also highly desirable.

Thus, there remains a need for methods and compositions that allow for the direct, sensitive, rapid, and easy-to-use detection of nucleic acids, including for the detection of pathogens in a sample.

### SUMMARY

The invention is defined by the appendant claims.

Disclosed herein are compositions and methods for detecting RNA or DNA. The systems described herein provide a highly sensitive, quantitative, and rapid assay in an all-in-one detection modality that possesses an internal Nuclease Chain Reaction (NCR) which provides an amplifying, feed-forward loop to generate an exponential signal upon detection of a target nucleic acid.

Described herein are systems for detecting a nucleic acid. The systems typically comprise at least two components: (1) a first component that detects the nucleic acid and in addition, is itself capable of generating, or alternatively via one or more other molecules (*e.g*., a reporter), generates a signal upon detection of the target nucleic acid in any sample; and (2) a second component that amplifies the signal generated upon detection by the first component. The system may include a "feed forward" system in which the first component (*e.g*., primary activator complex), when activated, is able to act on a component of an inactive second recognizing complex to activate the second recognizing complex to become an activated signal amplifier, such that the signal (*e.g.,* release of a detectable label) is further amplified. Each of the components may comprise one or more molecules (*e.g*., one or more fusion proteins, one or more enzymes, one or more nucleic acids, one or more fusions of nucleic acids and nucleic acids, etc.). The systems described herein comprising a component that amplifies the detection signal and may increase the detection of the nucleic acid any amount as compared to systems not including an amplifier component, including but not limited to 1 to 10-fold, 1 to 50-fold, 1 to 100-fold, 1 to 500-fold, 1 to 1000-fold (or any value therebetween) or more.

In certain aspects, described herein without being part of the invention is a nucleic acid detection system comprising:
a reporter molecule comprising a detectable label, wherein the detectable label is released for detection upon cleavage of the reporter molecule; a primary activator complex comprising a first recognizing complex ("target sensor"), wherein the first recognizing complex recognizes one or more target sequences in a sample (the "primary activator"), wherein upon recognition of the target sequence the primary activator complex is activated and is able to act on the reporter molecule and release the detectable label; and a secondary amplifier complex comprising a second inactive recognizing complex, wherein following the activity of the activated primary activator complex on a component of the second recognizing complex, the second recognizing complex is activated and recognizes one or more amplifier sequences ("activators"), and acts on the reporter molecule and releases the detectable label and acts on other inactive second recognizing complexes such that they become activated. Thus, the invention relates to a nucleic acid detection system comprising: (i) a reporter molecule comprising a detectable label, wherein the detectable label is released for detection upon cleavage of the reporter molecule; (ii) a primary activator complex comprising a first recognizing complex, and; (iii) an inactive secondary complex wherein; (a) the first recognizing complex recognizes one or more primary activators in a sample, wherein; (b) upon recognition of the primary activator, the primary activator complex is activated and is able to act on the reporter molecule to release the detectable label, and; (c) the activated primary activator complex is able to act on a component of an inactive second recognizing complex to activate the second recognizing complex to become an activated signal amplifier, wherein; (d) said activated signal amplifier is able to act on the reporter molecule to release the detectable label, and; (e) said activated signal amplifier is able to act on a component of an inactive second recognizing complex to activate the second recognizing complex to become an activated signal amplifier such that a feed-forward loop is initiated.

The invention provides a nucleic acid detection system comprising: a reporter molecule comprising a detectable label, wherein the detectable label is released for detection upon cleavage of the reporter molecule; a primary activator complex comprising a first Cas-effector enzyme ("target sensor") programmed with a first guide RNA, wherein the first guide RNA recognizes one or more target sequences in a sample ("primary activator"), wherein upon hybridization of the first guide RNA to the target sequence, the primary activator complex is activated, displaying a non-specific nuclease activity that cleaves the reporter molecule and releases the detectable label; and a signal amplifier complex comprising a second Cas-effector enzyme and a second guide RNA, wherein the second guide RNA recognizes one or more amplifier sequences ("activators"), wherein upon hybridization of the second guide RNA to the amplifier sequence the signal amplifier is activated , displaying a non-specific nuclease activity that cleaves the reporter molecule and releases the detectable label and is able to act on additional second Cas-effector enzymes to activate them to become signal amplifiers. Thus, the invention relates to a nucleic acid detection system comprising: a reporter molecule comprising a detectable label, wherein the detectable label is released for detection upon cleavage of the reporter molecule; a primary activator complex comprising a first Cas-effector enzyme programmed with a first guide RNA, wherein the first guide RNA recognizes one or more primary activators in a sample, wherein upon hybridization of the first guide RNA to the primary activator, the primary activator complex is activated and is a non-specific nuclease that cleaves the reporter molecule and releases the detectable label; and a signal amplifier comprising a second Cas-effector enzyme and a second guide RNA, wherein activation of the primary activation complex results in the activation of one or more activator sequences that are recognized by the second guide RNA, wherein upon hybridization of the second guide RNA to the activator sequence the signal amplifier complex is activated and is a non-specific nuclease that cleaves the reporter molecule and releases the detectable label.

The nucleic acid detection systems described herein preferably involve a "feed forward" loop in which detection of the primary activator triggers activation of the primary activation complex, which in turn causes activation of the secondary activator complex to amplify the detectable signal in the presence of the target (initially detected by the target sensor/primary activator), for example systems in which the activated primary activator complex activates the activator molecule and/or the secondary guide RNA (e.g. through release of a cage structure on the activator molecule and/or secondary guide RNA), such that the second guide RNA of the secondary amplifier complex hybridizes with the released activator molecule such that further quenched reporter molecules are cleaved and further detectable label is released.

In any of the nucleic acid detection systems described herein the first and/or second Cas-effector enzymes can comprise an RNase and/or a DNase, for example one or more Cas13 proteins, one or more Cas12 proteins, one or more Cas14 proteins, one or more Csm6 proteins, and/or one or more Csx1 proteins in any combination(s), optionally one or more proteins listed as shown in any of the appended Tables, Figures or Examples (SEQ ID NO:s 115 - 268). In certain embodiments, the first and/or second Cas-effector enzymes comprise one or more of the same or different Cas13d proteins. The one or more Cas-effector enzymes can be the same or different proteins. Furthermore, the first and/or second guide RNAs of the systems described herein can comprise one or more constant regions, including but not limited to one or more of the same or different RNAs as shown in any of the appended Tables (*e.g*., Table 4), Figures or Examples (SEQ ID NOs:42 - 85).

In any of the nucleic acid detection systems described herein, the reporter may comprise a quencher operably linked to the detectable label, optionally wherein the detectable label comprises one or more fluorescent molecule (*e.g*., fluorescent dyes). The reporter molecule may comprise an oligonucleotide linking the quencher and the fluorophore, wherein the oligonucleotide optinally comprises a caged structure, optionally having a stem-loop structure. The reporter molecule may comprise a sequence as shown in Table 8. In certain embodiments, the reporter molecule is complexed with a trans cage molecule.

**In** any of the nucleic acid detection systems described herein, one or more of the components (*e.g*., activator, one or more guide molecules, amplifier sequences, and/or reporters) may be caged (*e.g.,* by caging structures or molecules). In certain embodiments, the cage comprises or creates a molecule (*e.g*., oligonucleotide sequence) having a stem-loop structure. Oligonucleotide sequences included with the activator may comprise DNA and/or RNA bases and, in addition, one or more of the DNA and/or RNA bases may be modified nucleotide bases, optionally comprising one or more locked nucleic acid (LNA) or moieties and/or 2'-OMe RNA. One or more caging structures may be used, for example wherein one or more of the amplifier sequences comprising caging structures on their 3' and/or 5' ends. One or more trans caging molecules may be also be used in any of the nucleic acid systems described herein.

In any of the nucleic acid detection systems described herein, the interaction as between one or more of the components may be limited to a certain amount of time (*e.g.,* seconds, minutes or more), for example wherein one or both of the first and second guide RNAs and/or one or more of the amplifier sequences are modified to allow conditional interaction with the Cas-effector enzyme during an optimal time frame. In certain embodiments, one or both of the first and second guide RNAs and/or one or more of the amplifier sequences are modified to allow conditional interaction with the Cas-effector enzyme during an optimal time frame. In any of nucleic acid detection systems described herein, the one or more amplifier sequences comprise poly U and/or poly A sequences, optionally A₄-Uₙ, A₅-Uₙ and/or A₆-Uₙ sequences.

The target sequence and/or amplifier sequence of any of the systems described herein may be 100% complementary to first and/or second guide RNAs, or, alternatively, may not be 100% complementary to first and/or second guide RNAs. The target sequence in any of the systems described herein may be DNA and/or RNA from one or more mammals, viruses, bacteria, and/or fungi. In certain embodiments, the target sequence is in an RNA virus, for example a coronavirus, optionally a SARS-Cov-2 coronavirus.

In any of the nucleic acid detection systems described herein, the sample may be a biological or environmental sample. The biological sample may comprise blood, saliva, urine, biopsy, plasma, serum, bronchoalveolar lavage, sputum, a fecal sample, cerebrospinal fluid, a fine needle aspirate, a swab sample (e.g., a buccal swab, a cervical swab, a nasal swab), interstitial fluid, synovial fluid, nasal discharge, tears, buffy coat, a mucous membrane sample, and/or an epithelial cell sample (e.g., epithelial cell scraping), etc.) collected from the individual. The sample may be a liquid sample and may be cell-free or a liquid comprising cells.

Also described are methods of detecting a nucleic acid (*e.g.,* target sequence), the methods comprising: (a) contacting a sample suspected of including the nucleic acid (*e.g*., target sequence) with: (i) a nucleic acid detection system as described herein, and (b) measuring a detectable signal from the detectable label, thereby detecting the target sequence. In certain embodiments, the methods further comprise quantifying the levels of the detectable label. contacting step may be carried out in the presence of divalent metal ions, in an acellular environment or within a cell *in vitro* or *in vivo.* The contacting step may be carried out any length of time, including seconds, minutes or hours or more (or any time therebetween), optionally seconds to 2 hours (or any time therebetween).

### BRIEF DESCRIPTION OF THE DRAWINGS

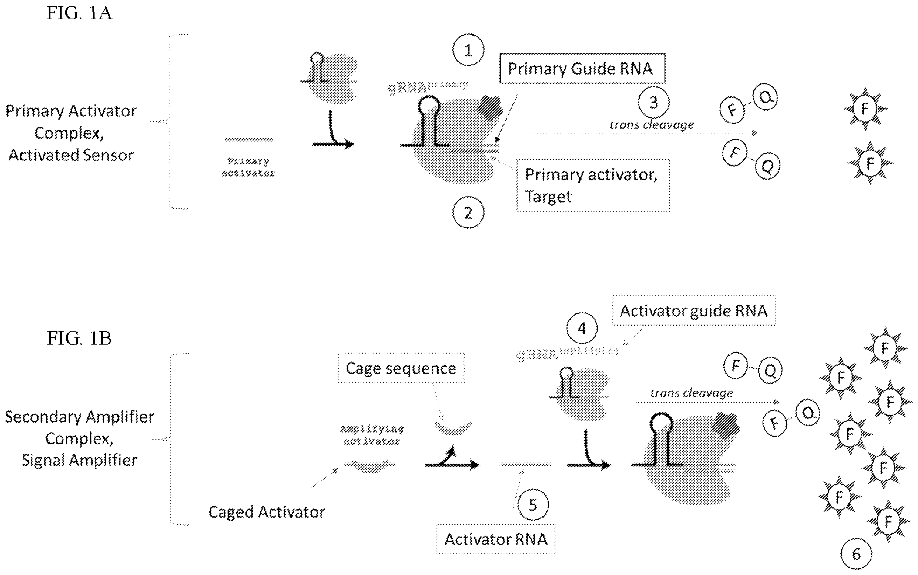

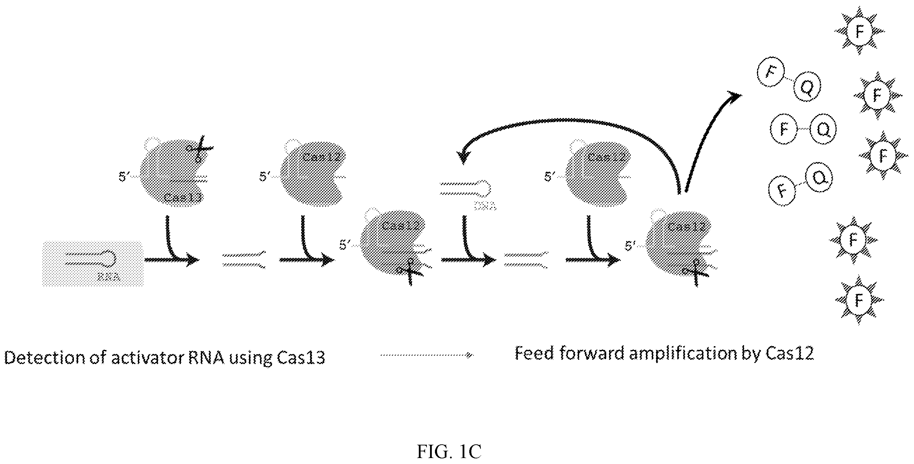

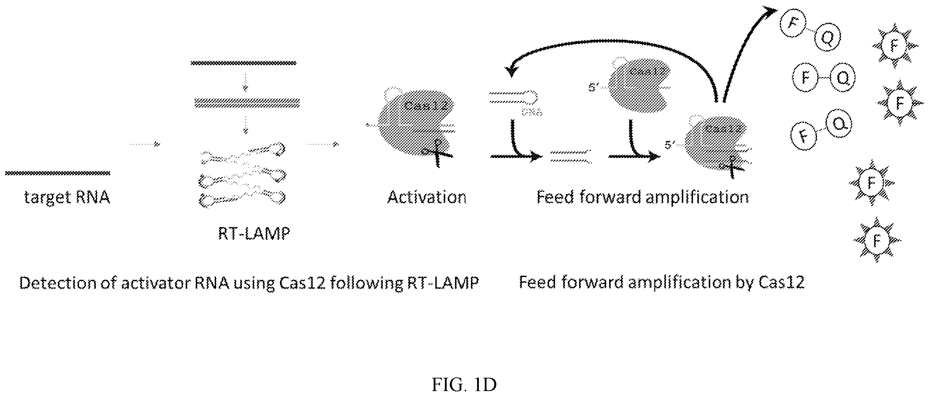

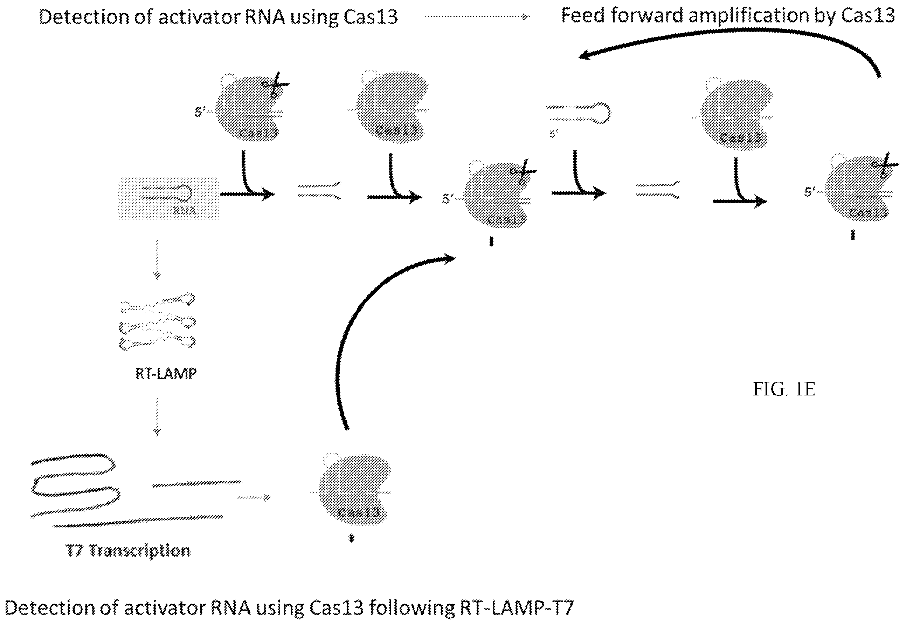

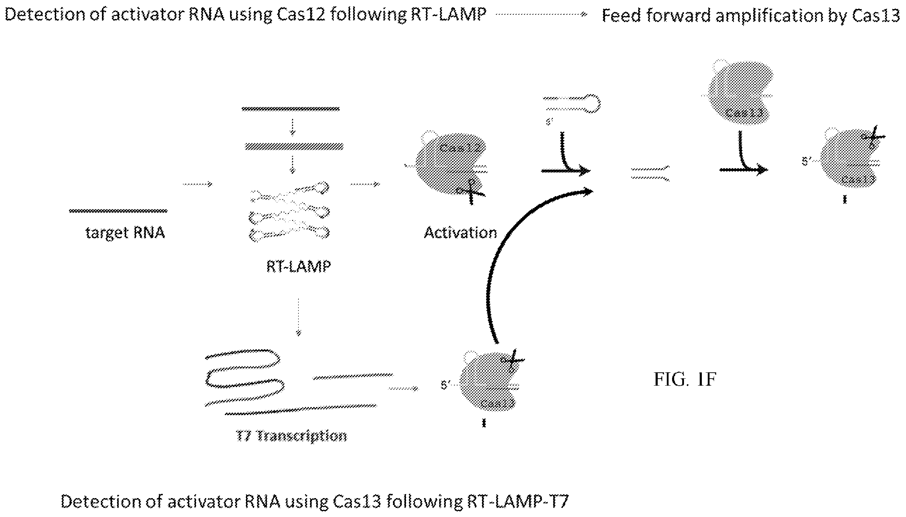

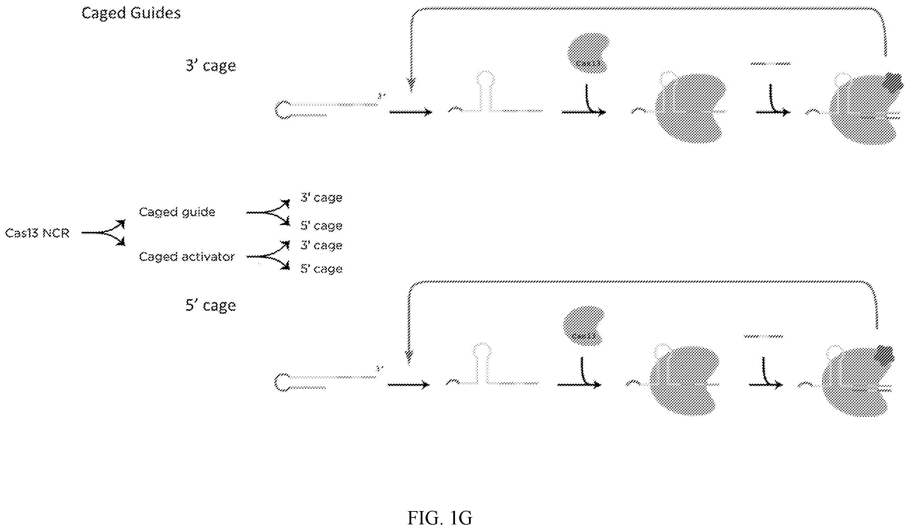

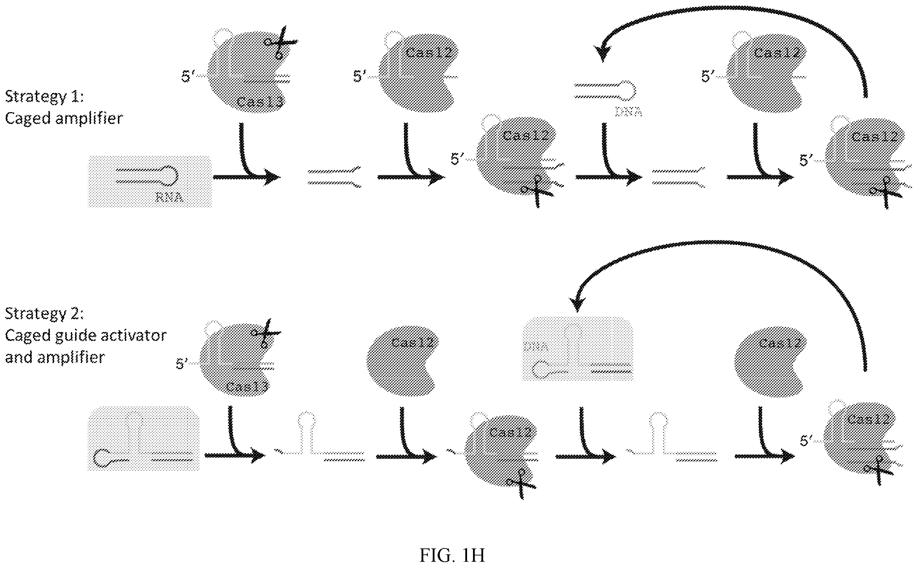

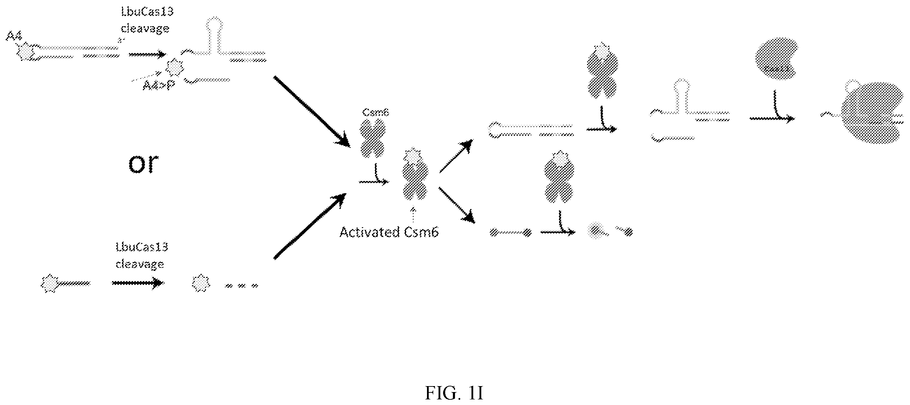

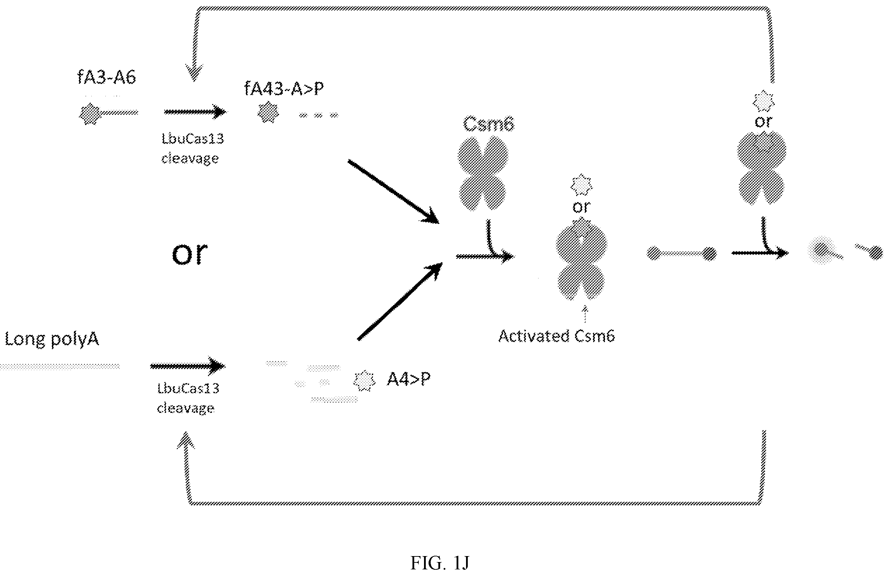

**Figures 1A through 1J** are schematics depicting exemplary NCR systems as described herein. It will be understood that the methods and compositions of the invention include all 'mix and match' variations including any combination of the components of these exemplary NCR systems. Figure 1A shows the Primary Activator complex **(1)** comprises a Cas-effector enzyme programmed with a guide RNA that recognizes a desired target nucleic sequence (the Cas-effector enzyme and guide is the "target sensor", it recognizes the "target" nucleic acid, also known as the "primary activator") (**2**) in the sample (*e.g.*, viral DNA or RNA). Upon hybridization of the target sequence to the guide RNA of the target sensor (also known as "crRNA"), the target sensor is activated as a Cas nuclease and displays non-specific RNase (*e.g*., Cas13 effector protein) or DNase (*e.g*., Cas12 effector protein) activity (the "activated sensor"). The activated sensor cleaves a reporter molecule (**3**) to release a detectable label (*e.g.,* releasing a fluorescent reporter from a quencher), leading to a detectable signal (such as fluorescence). In this primary signaling pathway, signal is generated as a direct response from the sensor detecting the target nucleic acid, which then activates the sensor (the activated sensor). The activated sensor is then able to engage in non-specific cleavage activity of single stranded nucleic acids ("trans cleavage"). As shown in Figure 1B, the NCR systems as described herein include an additional signal amplification pathway comprising a Signal Amplifier (**4**) that, in some embodiments, comprises a second Cas-effector protein programmed with an Activator guide RNA that recognizes an amplifier RNA (**5**) that hybridizes to the guide RNA of the signal amplifier so as to activate the signal amplifier into a non-specific nuclease capable of cleaving the reporter molecule (**3**) and amplifying the signal (**6**) detected from the reporter. As shown, the amplifier RNA (**5**) may be caged such that it will not interact with the amplifier complex. Cleavage of the single stranded loops in the cage complexes by trans cleavage activity of an activated sensor or amplifier complex releases the cage and allows the amplifier RNA to interact with the amplifier complex. In some embodiments, the guide or crRNAs may also comprise cage complexes. In some embodiments, short trans cage molecules are added to interact with an activator or crRNA and serve as a cage. In some embodiments, cages are released by other mechanisms such as through the use of aptamers, ribozymes and the like. Figure 1C depicts the use of Cas13 in the primary activator complex with Cas12 in the signal amplification pathway. Figure 1D shows the use of Cas12 in the primary activator complex with Cas12 in the signal amplification pathway. In this scenario, RT-LAMP (Reverse Transcription Loop-Mediated isothermal amplification, (Fu et al (2011) Appl. Biochem. Biotechnol. 163 (7): 845-50) is used to amplify the target RNA. Other amplification techniques including for example mismatch tolerant LAMP, LAMP-T7, strand displacement amplification (SDA), helicase-dependent amplification (HDA, Recombinase Polymerase Amplification (RPA), Nucleic Acid Sequences Based Amplification (NASBA), transcription mediated amplification (TMA) and the like may be used (see below). Figure 1E shows the use of Cas13 in the primary activator complex with Cas13 in the signal amplification pathway and Figure 1F shows Cas12 in the primary activator complex with Cas13 in the signal amplification pathway. In some embodiments, RT-LAMP-T7 amplification is used to amplify the primary amplifier to increase the number of target molecules for sensing by a Cas13 target sensor complex. This case is illustrated in the context of a Cas13 sensor complex and Cas13 amplification complex (Figure 1E) or in a system with a Cas13 sensor complex and a Cas12 sensor complex (Figure 1F). Figure1G shows the use of exemplary caged guide RNAs where the cages are present on the 5' (top) or 3' (bottom) end of the guide RNA. Figure 1H depicts two strategies for the use of caged amplifiers and caged guides wherein the amplifiers may comprise both RNA and DNA nucleotides. In some embodiments, oligonucleotide sequences may be used that are not covalently linked to the amplifier or guide RNA which are able to interact with the amplifier or guide and act as a cage (trans cage molecules). Figure 1I and Figure 1J show pathways to use Csm6 to amplify the signal. In these figures, the term "NCR" refers to a synthetic nucleic acid, with the numeric code following NCR identifying the particular nucleic acid (*e.g.,* as shown in any of the appended Tables, Examples or Figures). "RNP" refers to a ribonucleotide protein complex, typically a Cas protein and a crRNA or guide RNA.

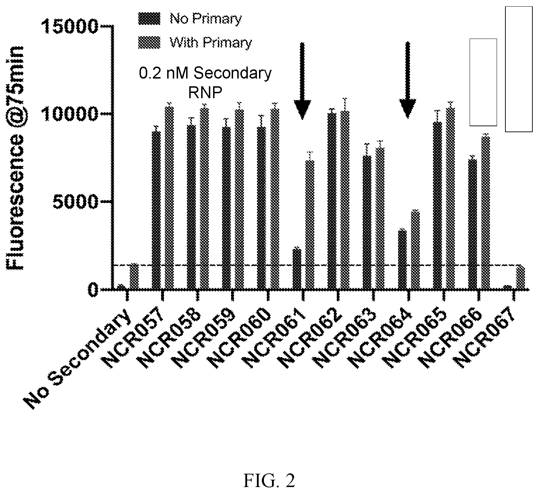

**Figure 2** depicts a graph showing signal detected in the presence of the primary activator RNA ("with primary" shown as the right bars under each condition) and absence of primary ("no primary" shown as the left bars under each condition) for several activator RNAs. "No secondary" shows the signal in the absence of primary or secondary systems. Indicated are two sequences (NCR_061 and NCR_064) that display differential signal in the presence or absence of primary activator RNA.

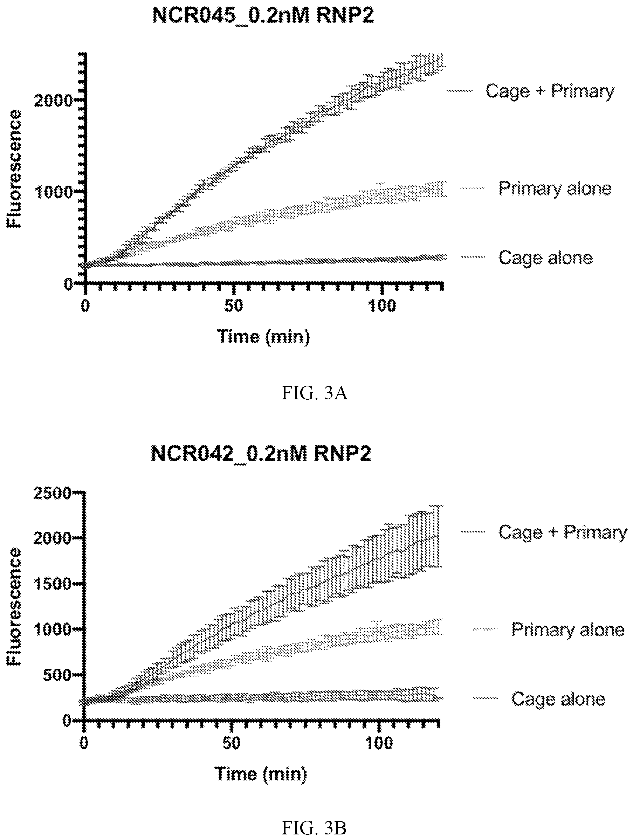

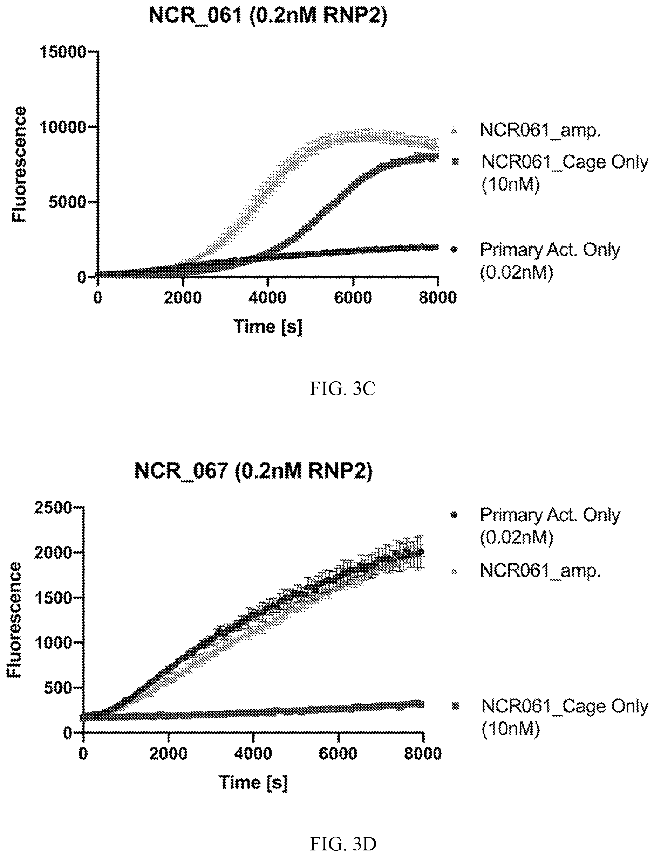

**Figures 3A through 3D** depict four graphs showing signal over time for four activator RNAs, NCR_045 (Figure 3A), NCR_042 (Figure 3B), NCR_061 (Figure 3C) and NCR_067 (Figure 3D). For NCR_045 and NCR_042, "Cage alone" is the signal observed in the presence of the amplifying RNA only; "Primary" indicates the signal observed without any secondary amplification; and "Cage + Primary" indicates the signal observed when all components are present. For NCR_061 and NCR_067, the data curves are as indicated in the graph. "RNP2" means the secondary amplifier complex.

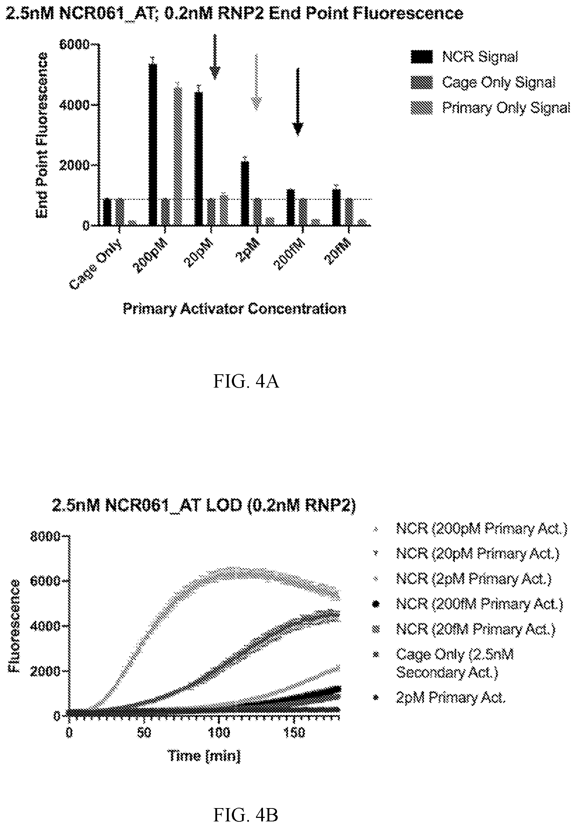

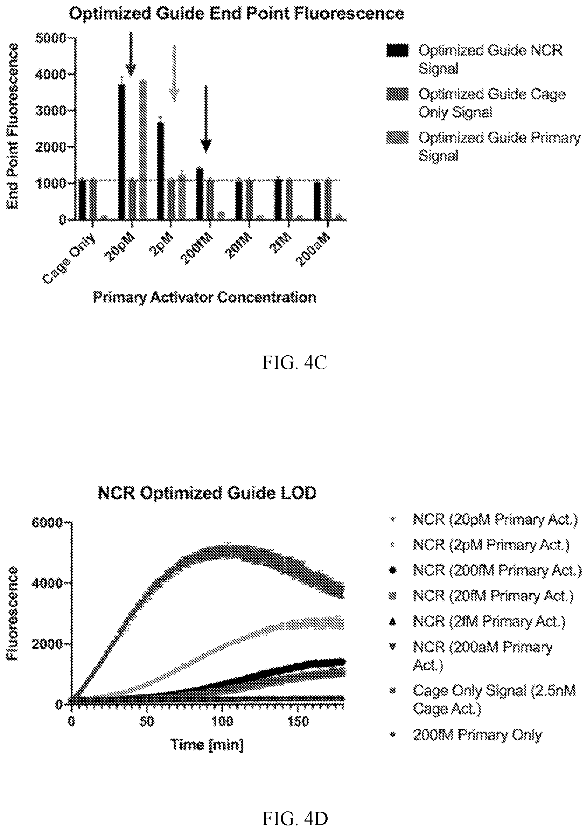

**Figures 4A through 4D** are graphs depicting signal observed in a series of optimization reactions. Figure 4A shows the signal observed as the concentration of primary activator RNA is varied from 200 pM to 20 fM. The signal at 20 pM displays the greatest differential between signal in the absence of the NCR as compared to in the presence of the amplification for NCR_061 in these conditions. Figure 4B depicts the generation of signal over time, demonstrating that at 200 pM the NCR signal develops the fastest. Figure 4C depicts the signal with an optimized guide RNA. Figure 4D shows these results over time and demonstrates that for this guide, the concentration of 20 pM primary activator RNA develops the fastest.

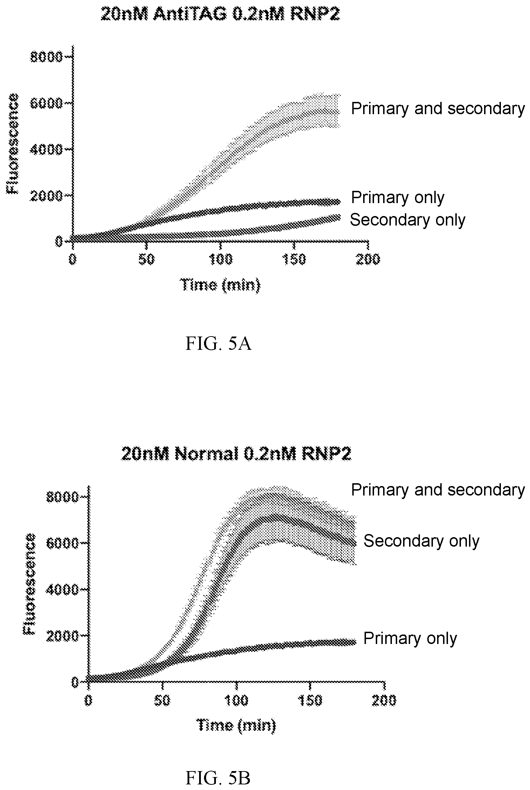

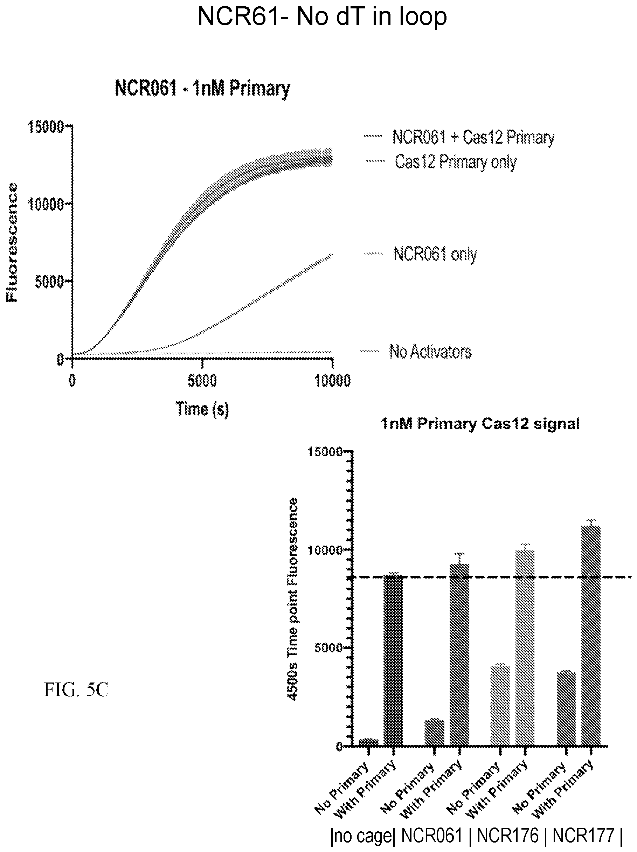

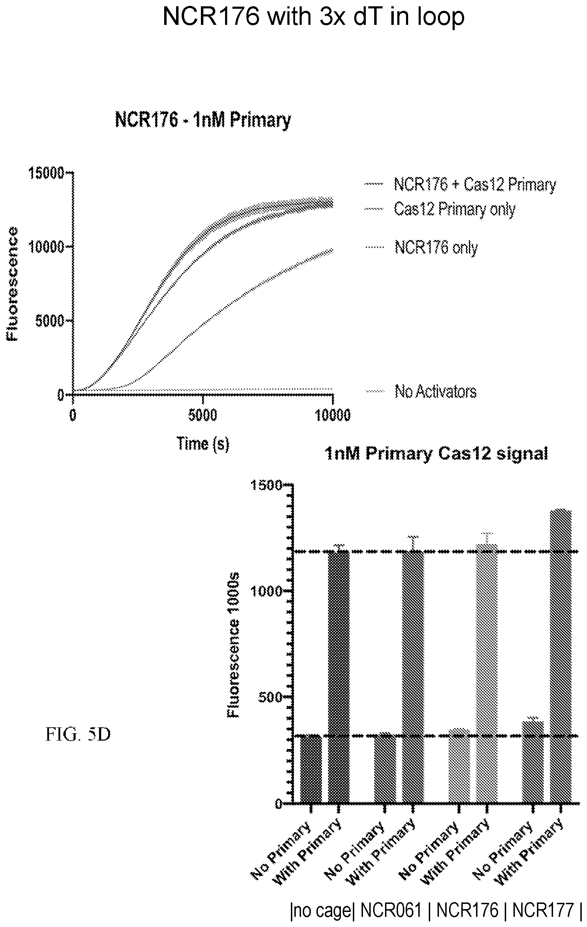

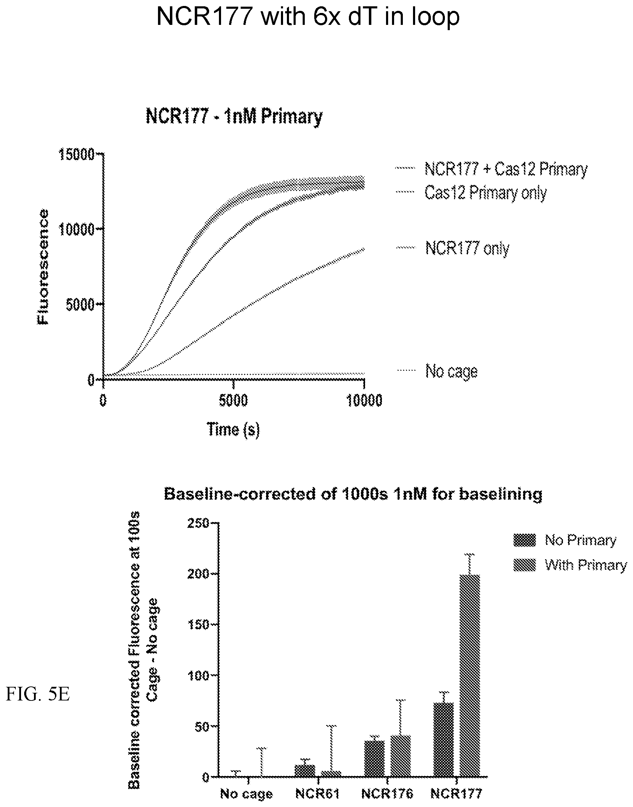

**Figures 5A through 5E** show results from experiments testing modifications of the activator RNA. Figure 5A shows the signal obtained when the activator comprised an anti-TAG sequence and Figure 5B shows the signal when the anti-TAG is absent. "Normal" indicates the data observed when an activator RNA lacking an anti-TAG sequence is used. A differential signal is observed when the anti-TAG sequence is included. Figures 5C through 5E demonstrate the effects on signal amplification from variations in the activator RNA. Figure 5C depicts the signal generation over time without any cage on the activator RNA. Figure 5D and Figure 5E show the effects on signal generation when the activator has more uridines in the cage structure.

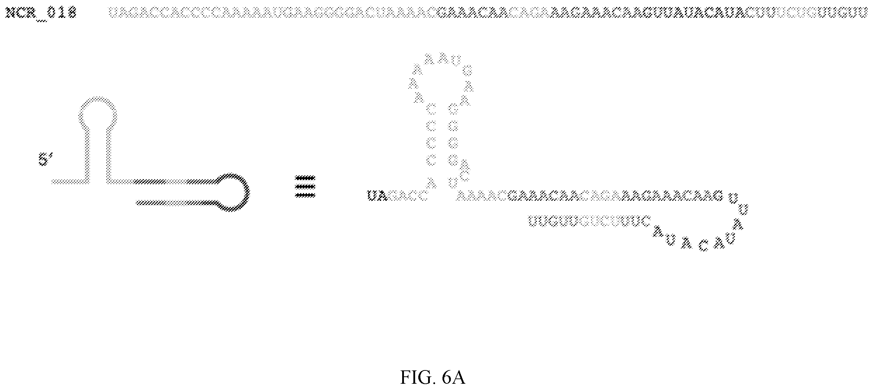

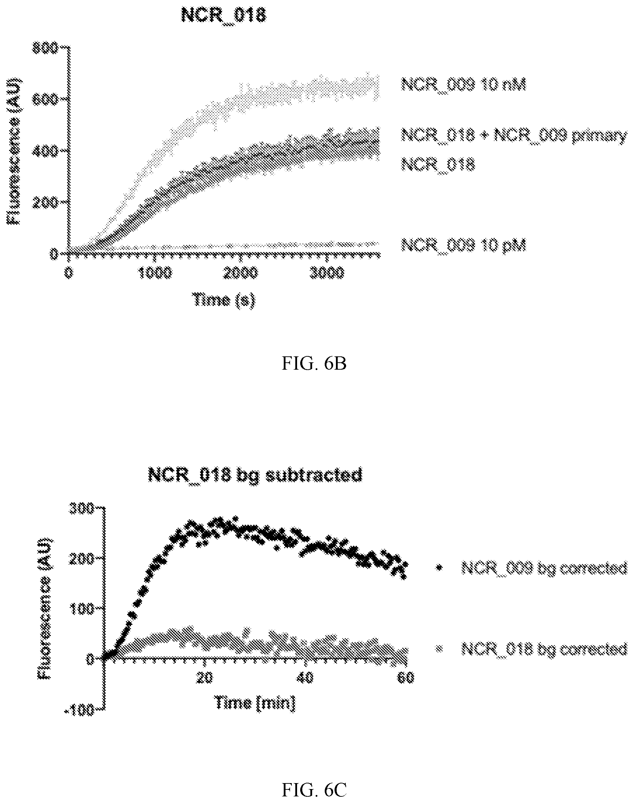

**Figures 6A through 6C** depict sequences and data related to the use of caged guide RNAs. Figure 6A depicts a guide RNA (NCR_018, SEQ ID NO: 21). Figure 6B depicts the data observed when either the caged NCR_018 or its cognate uncaged equivalent (NCR_009) are used. Two concentrations, 10 pM and 10 nM, of NCR_009 were used. Figure 6C depicts the results observed when the background signal is subtracted from the signal in the presence of primary activator and secondary activation.

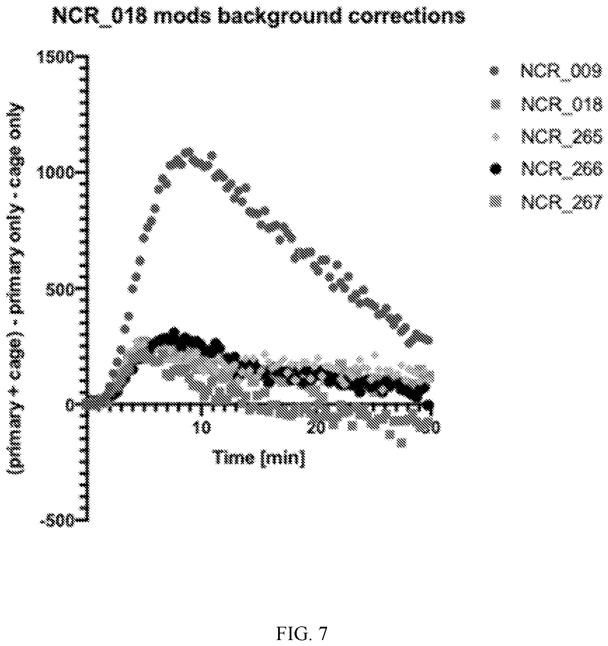

**Figure** 7 depicts a graph showing data obtained with alternate caged guide RNAs. The data depicted is the signal observed when the data obtained in the presence of primary and secondary (cage) has the signal observed from primary signal only and the signal observed from secondary (cage) signal only are subtracted from it.

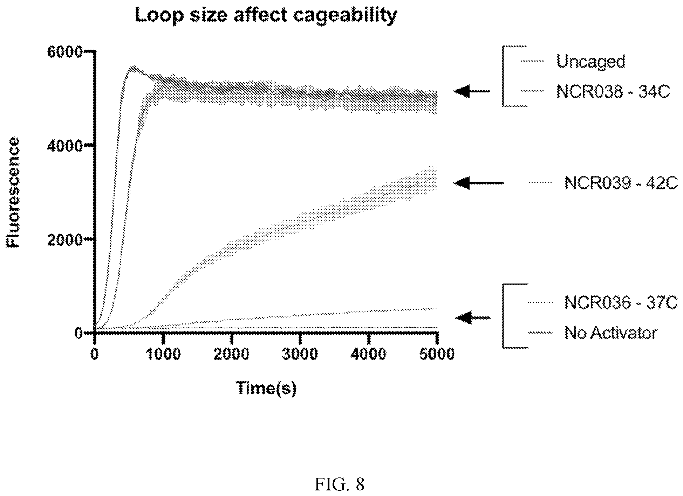

**Figure 8** is a graph showing the signal kinetics observed when caged guide RNAs are used comprising differing single stranded loop structures. The stem-loop sections of the guides are characterized by their melting temperatures which are indicated. Faster signal kinetics are observed using guides comprising stem loops with lower melting temperatures.

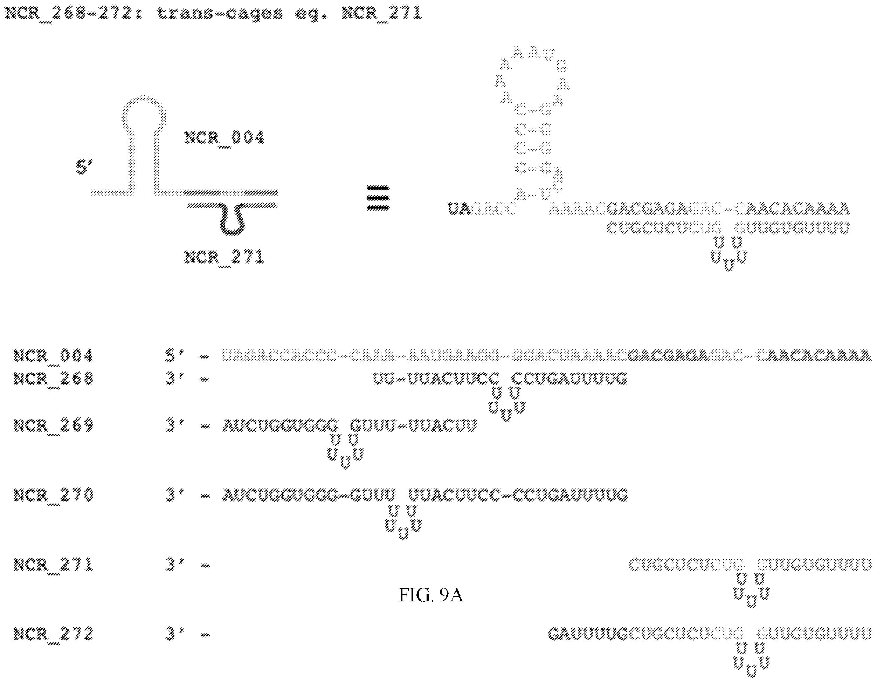

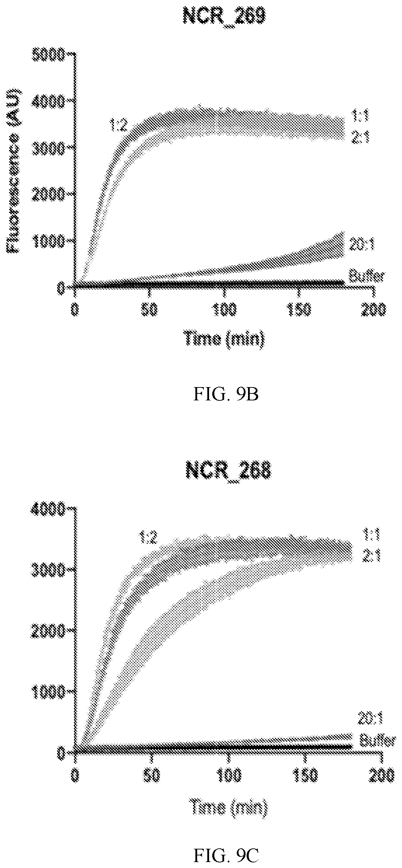

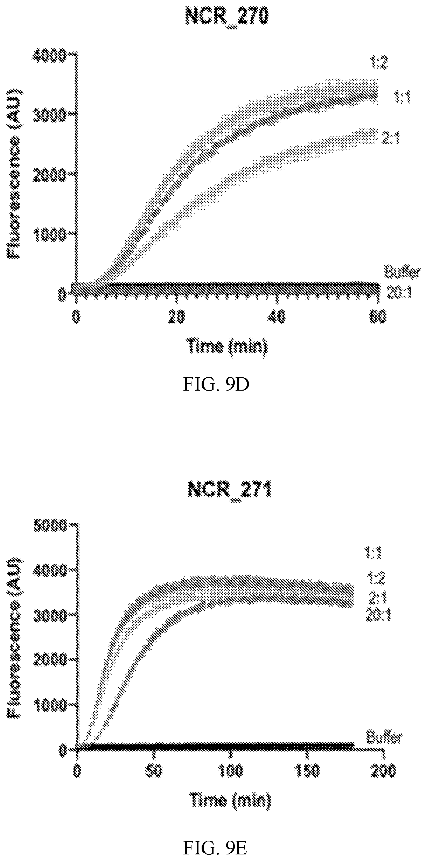

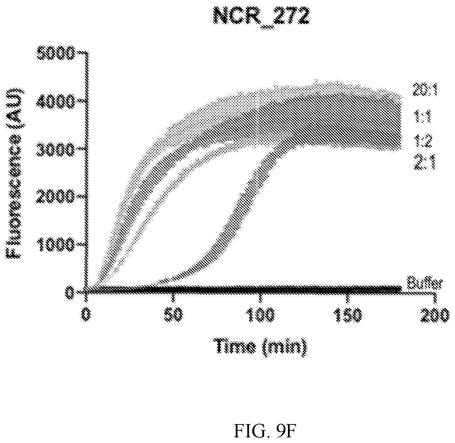

**Figures 9A through 9F** show the results of experiments using trans cage molecules. Figure 9A depicts a guide RNA (NCR_004) which lacks its own cage structure, but is shown on the top complexed with a trans cage molecule NCR_271. Lower down is shown NCR_004 aligned with a series of trans cage molecules including NCR_268. NCR_269, NCR_270. NCR_271 and NCR_272 from top to bottom. Figure 9B (NCR_269), Figure 9C (NCR_268), Figure 9D (NC%_270), Figure 9E (NCR_271) and Figure 9F (NCR_272) depict the signal generated using the different trans cage molecules at varying ratios of trans cage to guide RNA.

### DETAILED DESCRIPTION

Current CRISPR-based nucleic acid detection methods involving Cas proteins exploit the fact that Cas13 or Cas12 enzymes can be programmed with a guide RNA that recognizes a desired target sequence, activating a non-specific RNase or DNase activity. The non-specific nuclease is used to release a detectable label, such as a quenched fluorescent reporter, leading to a detectable signal such as fluorescence. However, current methods can be limited in sensitivity. Current methods may also require specific equipment (e.g. a PCR machine), specialized conditions (e.g. temperatures), repetitive manual techniques such as multiple pipetting steps or other complicated steps that result in long reporting times.

Thus, described herein are nucleic acid detection compositions, systems and methods that overcome the limitations of the current methodology by providing an all-in-one detection modality comprising an internal Nuclease Chain Reaction (NCR) which imparts an amplifying, feed-forward loop to generate an exponential signal upon detection of a target nucleic acid and provide efficient detection of the target sequence. The NCR compositions, systems and methods described herein provide sensitive and rapid detection of any target DNA or RNA, including for detection of transcriptional states, cancers, or pathogens such as bacteria or viruses, including coronaviruses such as SARS-CoV-2 (associated with COVID-19 disease).

### General

Practice of the methods as well as preparation and use of the compositions (not part of the invention) disclosed herein employ, unless otherwise indicated, conventional techniques in molecular biology, biochemistry, chromatin structure and analysis, computational chemistry, cell culture, recombinant DNA and related fields as are within the skill of the art.

### Definitions

"Oligonucleotide," "polynucleotide," and "nucleic acid," are used interchangeably herein. These terms may refer to a polymeric form of nucleic acids of any length, strandedness (double or single), and either ribonucleotides (RNA) or deoxyribonucleotides (DNA), and hybrid molecules (comprising DNA and RNA). The disclosed nucleic acids may also include naturally occurring and synthetic or non-natural nucleobases. Natural nucleobases include adenine (A), thymine (T), cytosine (C), guanine (G), and uracil (U).

"Complementarity" refers to a first nucleic acid having a first sequence that allows it to "base pair," "bind," "anneal", or "hybridize," to a second nucleic acid. Binding may be affected by the amount of complementarity and certain external conditions such as ionic strength of the environment, temperature, etc. Base-pairing rules are well known in the art (A pairs with T in DNA, and with U in RNA; and G pairs with C). In some cases, RNA may include pairings where G may pair with U. Complementarity does not, in all cases, indicate complete or 100% complementarity. For example, complementarity may be less than 100% and more than about 60%.

"Protein," "peptide," "polypeptide" are used interchangeably. The terms refer to a polymeric form of amino acids of any length, which may include natural and non-natural residues. The residues may also be modified prior to, or after incorporation into the polypeptide. In some embodiments, the polypeptides may be branched as well as linear.

"Programmed," in reference to a Cas protein, refers to a Cas protein that includes a guide RNA that contains a sequence complementary to a target sequence. Typically, a programmed Cas protein includes an engineered guide RNA.

"Cas protein" is a CRISPR associated protein. The presently disclosed Cas proteins possess a nuclease activity that may be activated upon binding of a target sequence to a guide RNA bound by the Cas protein. As disclosed in more detail below, the guide RNA may, with other sequences, comprise a crRNA, which may, in some embodiments, be processed from a pre-crRNA sequence. In an embodiment, the guide RNA sequence may include natural or synthetic nucleic acids, for example modified nucleic acids such as, without limitation, locked nucleic acids (LNA), 2'-o-methylated bases, or even ssDNA (single stranded DNA). Cas proteins may be from the Cas12 or Cas13 group, which may be derived from various sources known to those of skill in the art.

The Cas protein may be a "functional derivative" of a naturally occurring Cas protein. A "functional derivative" of a native sequence polypeptide is a compound having a qualitative biological property in common with a native sequence polypeptide. "Functional derivatives" include, but are not limited to, fragments of a native sequence and derivatives of a native sequence polypeptide and its fragments, provided that they have a biological activity in common with a corresponding native sequence polypeptide. A biological activity contemplated herein is the ability of the functional derivative to hydrolyze a DNA substrate into fragments. The term "derivative" encompasses both amino acid sequence variants of polypeptide, covalent modifications, and fusions thereof. Suitable derivatives of a Cas polypeptide or a fragment thereof include but are not limited to mutants, fusions, covalent modifications of Cas protein or a fragment thereof. Cas protein, which includes Cas protein or a fragment thereof, as well as derivatives of Cas protein or a fragment thereof, may be obtainable from a cell or synthesized chemically or by a combination of these two procedures. The cell may be a cell that naturally produces Cas protein, or a cell that naturally produces Cas protein and is genetically engineered to produce the endogenous Cas protein at a higher expression level or to produce a Cas protein from an exogenously introduced nucleic acid, which nucleic acid encodes a Cas that is same or different from the endogenous Cas. In some cases, the cell does not naturally produce Cas protein and is genetically engineered to produce a Cas protein.

"Coding sequences" are DNA sequences that encode polypeptide sequences or RNA sequences, for example guide RNAs. Coding sequences that encode polypeptides are first transcribed into RNA, which, in-turn, may encode the amino acid sequence of the polypeptide. Some RNA sequences, such as guide RNAs may not encode amino acid sequences.

"Native," "naturally-occurring," "unmodified" or "wild-type" describe, among other things, proteins, amino acids, cells, nucleobases, nucleic acids, polynucleotides, and organisms as found in nature. For example, a nucleic acid sequence that is identical to that found in nature, and that has not been modified by man is a native sequence.

By "hybridizable" or "complementary" or "substantially complementary" it is meant that a nucleic acid (e.g. RNA, DNA) comprises a sequence of nucleotides that enables it to non-covalently bind, i.e. form Watson-Crick base pairs and/or G/U base pairs, "anneal", or "hybridize," to another nucleic acid in a sequence-specific, antiparallel, manner (i.e., a nucleic acid specifically binds to a complementary nucleic acid) under the appropriate in vitro and/or in vivo conditions of temperature and solution ionic strength. Standard Watson-Crick base-pairing includes: adenine/adenosine) (A) pairing with thymidine/thymidine (T), A pairing with uracil/uridine (U), and guanine/guanosine) (G) pairing with cytosine/cytidine (C). In addition, for hybridization between two RNA molecules (e.g., dsRNA), and for hybridization of a DNA molecule with an RNA molecule (e.g., when a DNA target nucleic acid base pairs with a guide RNA, etc.): G can also base pair with U. For example, G/U base-pairing is partially responsible for the degeneracy (i.e., redundancy) of the genetic code in the context of tRNA anti-codon base-pairing with codons in mRNA. Thus, in the context of this disclosure, a G (e.g., of a protein-binding segment (e.g., dsRNA duplex) of a guide RNA molecule; of a target nucleic acid (e.g., target DNA) base pairing with a guide RNA) is considered complementary to both a U and to C. For example, when a G/U base-pair can be made at a given nucleotide position of a protein-binding segment (e.g., dsRNA duplex) of a guide RNA molecule, the position is not considered to be non-complementary, but is instead considered to be complementary.

Hybridization requires that the two nucleic acids contain complementary sequences, although mismatches between bases are possible. The conditions appropriate for hybridization between two nucleic acids depend on the length of the nucleic acids and the degree of complementarity, variables well known in the art. The greater the degree of complementarity between two nucleotide sequences, the greater the value of the melting temperature (Tm) for hybrids of nucleic acids having those sequences. Typically, the length for a hybridizable nucleic acid is 8 nucleotides or more (e.g., 10 nucleotides or more, 12 nucleotides or more, 15 nucleotides or more, 20 nucleotides or more, 22 nucleotides or more, 25 nucleotides or more, or 30 nucleotides or more).

It is understood that the sequence of a polynucleotide need not be 100% complementary to that of its target nucleic acid to be specifically hybridizable. Moreover, a polynucleotide may hybridize over one or more segments such that intervening or adjacent segments are not involved in the hybridization event (e.g., a loop structure or hairpin structure, a 'bulge', and the like). A polynucleotide can comprise 60% or more, 65% or more, 70% or more, 75% or more, 80% or more, 85% or more, 90% or more, 95% or more, 98% or more, 99% or more, 99.5% or more, or 100% sequence complementarity to a target region within the target nucleic acid sequence to which it will hybridize. For example, an antisense nucleic acid in which 18 of 20 nucleotides of the antisense compound are complementary to a target region, and would therefore specifically hybridize, would represent 90 percent complementarity. The remaining noncomplementary nucleotides may be clustered or interspersed with complementary nucleotides and need not be contiguous to each other or to complementary nucleotides. Percent complementarity between particular stretches of nucleic acid sequences within nucleic acids can be determined using any convenient method. Example methods include BLAST programs (basic local alignment search tools) and PowerBLAST programs (Altschul et al., J. Mol. Biol., 1990, 215, 403-410; Zhang and Madden, Genome Res., 1997, 7, 649-656) or by using the Gap program (Wisconsin Sequence Analysis Package, Version 8 for Unix, Genetics Computer Group, University Research Park, Madison Wis.), e.g., using default settings, which uses the algorithm of Smith and Waterman (Adv. Appl. Math., 1981, 2, 482-489).

"Binding" as used herein (e.g. with reference to an RNA-binding domain of a polypeptide, binding to a target nucleic acid, and the like) refers to a non-covalent interaction between macromolecules (e.g., between a protein and a nucleic acid; between a guide RNA and a target nucleic acid; and the like). While in a state of non-covalent interaction, the macromolecules are said to be "associated" or "interacting" or "binding" (e.g., when a molecule X is said to interact with a molecule Y, it is meant the molecule X binds to molecule Y in a non-covalent manner). Not all components of a binding interaction need be sequence-specific (e.g., contacts with phosphate residues in a DNA backbone), but some portions of a binding interaction may be sequence-specific. Binding interactions are generally characterized by a dissociation constant (Kd) of less than 10⁻⁶ M, less than 10⁻⁷ M, less than 10⁻⁸ M, less than 10⁻⁹ M, less than 10⁻¹⁰ M, less than 10⁻¹¹ M, less than 10⁻¹² M, less than 10⁻¹³ M, less than 10⁻¹⁴ M, or less than 10⁻¹⁵ M. "Affinity" refers to the strength of binding, increased binding affinity being correlated with a lower Kd.

By "binding domain" it is meant a protein domain that is able to bind non-covalently to another molecule. A binding domain can bind to, for example, an RNA molecule (an RNA-binding domain) and/or a protein molecule (a protein-binding domain). In the case of a protein having a protein-binding domain, it can in some cases bind to itself (to form homodimers, homotrimers, etc.) and/or it can bind to one or more regions of a different protein or proteins.

The term "conservative amino acid substitution" refers to the interchangeability in proteins of amino acid residues having similar side chains. For example, a group of amino acids having aliphatic side chains consists of glycine, alanine, valine, leucine, and isoleucine; a group of amino acids having aliphatic-hydroxyl side chains consists of serine and threonine; a group of amino acids having amide containing side chains consisting of asparagine and glutamine; a group of amino acids having aromatic side chains consists of phenylalanine, tyrosine, and tryptophan; a group of amino acids having basic side chains consists of lysine, arginine, and histidine; a group of amino acids having acidic side chains consists of glutamate and aspartate; and a group of amino acids having sulfur containing side chains consists of cysteine and methionine. Exemplary conservative amino acid substitution groups are: valine-leucine-isoleucine, phenylalanine-tyrosine, lysine-arginine, alanine-valine-glycine, and asparagine-glutamine.

The terms "DNA regulatory sequences," "control elements," and "regulatory elements," used interchangeably herein, refer to transcriptional and translational control sequences, such as promoters, enhancers, polyadenylation signals, terminators, protein degradation signals, and the like, that provide for and/or regulate transcription of a non-coding sequence (e.g., guide RNA) or a coding sequence (e.g., protein coding) and/or regulate translation of an encoded polypeptide.

As used herein, a "promoter sequence" is a DNA regulatory region capable of binding RNA polymerase and initiating transcription of a downstream (3' direction) coding or non-coding sequence. Eukaryotic promoters will often, but not always, contain "TATA" boxes and "CAT" boxes. Various promoters, including inducible promoters, may be used to drive the various nucleic acids (e.g., vectors) of the present disclosure.

The term "naturally-occurring" or "unmodified" or "wild type" as used herein as applied to a nucleic acid, a polypeptide, a cell, or an organism, refers to a nucleic acid, polypeptide, cell, or organism that is found in nature.

"Recombinant," as used herein, means that a particular nucleic acid (DNA or RNA) is the product of various combinations of cloning, restriction, polymerase chain reaction (PCR) and/or ligation steps resulting in a construct having a structural coding or non-coding sequence distinguishable from endogenous nucleic acids found in natural systems. DNA sequences encoding polypeptides can be assembled from cDNA fragments or from a series of synthetic oligonucleotides, to provide a synthetic nucleic acid which is capable of being expressed from a recombinant transcriptional unit contained in a cell or in a cell-free transcription and translation system. Genomic DNA comprising the relevant sequences can also be used in the formation of a recombinant gene or transcriptional unit. Sequences of non-translated DNA may be present 5' or 3' from the open reading frame, where such sequences do not interfere with manipulation or expression of the coding regions, and may indeed act to modulate production of a desired product by various mechanisms (see "DNA regulatory sequences", below). Alternatively, DNA sequences encoding RNA (e.g., guide RNA) that is not translated may also be considered recombinant. Thus, e.g., the term "recombinant" nucleic acid refers to one which is not naturally occurring, e.g., is made by the artificial combination of two otherwise separated segments of sequence through human intervention. This artificial combination is often accomplished by either chemical synthesis means, or by the artificial manipulation of isolated segments of nucleic acids, e.g., by genetic engineering techniques. Such is usually done to replace a codon with a codon encoding the same amino acid, a conservative amino acid, or a non-conservative amino acid. Alternatively, it is performed to join together nucleic acid segments of desired functions to generate a desired combination of functions. This artificial combination is often accomplished by either chemical synthesis means, or by the artificial manipulation of isolated segments of nucleic acids, e.g., by genetic engineering techniques. When a recombinant polynucleotide encodes a polypeptide, the sequence of the encoded polypeptide can be naturally occurring ("wild type") or can be a variant (e.g., a mutant) of the naturally occurring sequence. Thus, the term "recombinant" polypeptide does not necessarily refer to a polypeptide whose sequence does not naturally occur. Instead, a "recombinant" polypeptide is encoded by a recombinant DNA sequence, but the sequence of the polypeptide can be naturally occurring ("wild type") or non-naturally occurring (e.g., a variant, a mutant, etc.). Thus, a "recombinant" polypeptide is the result of human intervention, but may be a naturally occurring amino acid sequence.

A "vector" or "expression vector" is a replicon, such as plasmid, phage, virus, or cosmid, to which another DNA segment, i.e. an "insert", may be attached so as to bring about the replication of the attached segment in a cell.

An "expression cassette" comprises a DNA coding sequence operably linked to a promoter. "Operably linked" refers to a juxtaposition wherein the components so described are in a relationship permitting them to function in their intended manner For instance, a promoter is operably linked to a coding sequence if the promoter affects its transcription or expression.

The terms "recombinant expression vector," or "DNA construct" are used interchangeably herein to refer to a DNA molecule comprising a vector and one insert. Recombinant expression vectors are usually generated for the purpose of expressing and/or propagating the insert(s), or for the construction of other recombinant nucleotide sequences. The insert(s) may or may not be operably linked to a promoter sequence and may or may not be operably linked to DNA regulatory sequences.

Any given component, or combination of components can be unlabeled, or can be detectably labeled with a label moiety. In some cases, when two or more components are labeled, they can be labeled with label moieties that are distinguishable from one another.

"Label" or "labelling" refers to a component with a molecule that renders the component identifiable by one or more techniques. Non-limiting examples of labels include streptavidin and fluorescent molecules. The term "fluorescer" refers to a substance or a portion thereof which is capable of exhibiting fluorescence in the detectable range. The labels may be detected by a binding interaction with a label (e.g. biotin binding streptavidin) or through detection of a fluorescent signal using a fluorimeter. Other detectable labels include enzymatic labels such as luciferase, peroxidase or alkaline phosphatase. A "reporter gene" or "reporter sequence" refers to any sequence that produces a protein product that is easily measured, preferably although not necessarily in a routine assay. Suitable reporter genes include, but are not limited to, sequences encoding colored or fluorescent or luminescent proteins (e.g., green fluorescent protein, enhanced green fluorescent protein, red fluorescent protein). In some embodiments, enzymatic labels are inactivated by way of being split into two or more pieces that are linked by a nucleic acid linker that is targetable by CRISPR enzyme activity (e.g. trans cleavage following activation by the presence of a primary activator). Upon cleavage of the linker, the pieces of the enzymatic reporter would be able to assemble into an active enzyme that could act on a substrate to generate a detectable signal.

The term "sample" is used herein to mean any sample that includes RNA or DNA (e.g., in order to determine whether a target sequence is present among a population of polynucleotide sequences). The sample can be derived from any source, e.g., the sample can be a synthetic combination of purified RNAs/DNAs; the sample can be a cell lysate, an RNA/DNA-enriched cell lysate, or RNA/DNAs isolated and/or purified from a cell lysate. The sample may be an environmental sample, an agricultural sample or a food sample. The sample can be from a patient (e.g., for the purpose of diagnosis). The sample may be selected or derived from one or more of blood, sweat, plasma, serum, sputum, saliva, mucus, cells, excrement, urine, cerebrospinal fluid (CSF), breast milk, semen, vaginal fluid, tissue, etc. The sample can be from permeabilized cells. The sample can be from crosslinked cells. The sample can be in tissue sections. The sample can be from tissues prepared by crosslinking followed by delipidation and adjustment to make a uniform refractive index. Examples of tissue preparation by crosslinking followed by delipidation and adjustment to make a uniform refractive index have been described in, for example, Shah et al., Development (2016) 143, 2862-2867 doi:10.1242/dev.138560.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges, and are also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs.

It must be noted that as used herein and in the appended claims, the singular forms "a," "an," and "the" include plural referents unless the context clearly dictates otherwise. Thus, for example, reference to a "CRISPR/Cas effector protein" includes a plurality of CRISPR/Cas effector proteins (including the same or different Cas effector proteins) and reference to "the guide RNA" includes reference to one or more guide RNAs and equivalents thereof known to those skilled in the art, and so forth. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination. All combinations of the embodiments pertaining to the invention are specifically embraced by the present invention and are disclosed herein just as if each and every combination was individually and explicitly disclosed. In addition, all sub-combinations of the various embodiments and elements thereof are also specifically embraced by the present invention and are disclosed herein just as if each and every such sub-combination was individually and explicitly disclosed herein.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed. The methods, and systems for detecting the presence or absence of specific target nucleic acid sequence (e.g. RNA or DNA) in a sample allow for cost-effectively diagnosing a patient or sample having a viral, bacterial, parasitic, or fungal infection, or a condition, disease, or disorder by identification by the presence of one or more specific nucleic acid sequences. The methods and systems of the invention are also useful in genetic screening, cancer screening, mutational analysis, microRNA analysis, mRNA analysis, single nucleotide polymorphism analysis, etc.

### Compositions and Systems

Provided are compositions (not part of the invention), systems and methods for detecting a target RNA or DNA sequence (double stranded or single stranded) in a sample. In particular, described herein are systems comprising an internal NCR which generates an amplifying, feed-forward loop to provide an exponential increase in the signal upon detection of a target nucleic acid. The systems and methods can comprise any number and type of detection and/or amplification components, for example a system comprising at least a first component comprising a detector that is capable of detecting a nucleic acid of interest and generating a signal following detection and a second component comprising an amplifier that increases (amplifies) the signal generated in the presence of the nucleic acid of interest. The components (*e.g*., detector and/or amplifier) may comprise any number of the same or different molecules, including but not limited to nucleic acid binding molecules such as split-enzymes, Ttago (argonaute) proteins, programmable single guide RNAs, etc.

The (not part of the invention) and systems described herein can include (i) a target sensor comprising a first Cas effector protein (e.g., a Cas13 protein for detection of RNA, a Cas12 protein or Cas14 for detection of DNA) in association with a first guide RNA, which first guide RNA recognizes (hybridizes) to the RNA or DNA target sequence of the sample (the primary activator); (ii) an inactive reporter molecule (complex) comprising a detectable label (*e.g*., fluorescent moiety) linked to a quencher via a sequence not recognized by the first guide RNA, which reporter is activated upon cleavage, for example cleavage (*e.g*. trans cleavage) by a nuclease that releases the detectable label from the complex (*e.g*., cleavage of sequence linking the label to quencher or cage) such that it can be measured; (iii) a signal amplifier comprising a second Cas effector protein in association with the second guide RNA or a signal amplifier comprising a second Cas effector protein in the presence of a caged guide RNA such that the signal amplifier is not active until the cage has been released; and (iv) an activator molecule (also referred to herein an "amplifying activator") comprising a sequence that is recognized by the second guide RNA of the signal amplifier, optionally wherein the activator molecule is part of the inactive reporter complex, in which binding to the activator to the signal amplifier (*e.g*., hybridization of the activator to the guide RNA of the signal amplifier) activates the signal amplifier such that is becomes a non-specific nuclease capable of cleaving the reporter to release the detectable label.

As shown in Figure 1A-1J, upon hybridization (binding) of first Cas effector protein to the primary activator sequence (via the first guide RNA) to the target sensor, an activated primary activator complex comprising a non-specific RNase (Cas13 effector) or DNase (Cas12 effector or Cas14 effector) is formed. This activated primary activator complex displays trans, non-specific nuclease activity, which then activates the reporter (detectable label) by cleaving the inactive reporter complex such that the label is detectable (no longer quenched), where this reaction only occurs in the presence of the target. In addition, the activated primary activator complex may release the activator molecule such that the second guide RNA of the secondary amplifier complex hybridizes with the released activator molecule, or the secondary guide is released to bind with the Cas protein that lacks a complexed guide RNA (apo Cas protein) and free activator. When hybridized to the activator molecule, the secondary amplifier complex becomes an activated non-specific trans RNase or trans DNase capable of cleaving inactive reporter complexes, such that further detectable label is released or the trans cleavage activity acts such that a cage is released allowing a caged activator RNA and/or a caged guide RNA to interact with the secondary amplifier complex. This process is termed "Feed forward amplification". The presence of two activated sensors (the primary activator complex and the secondary amplifier complex) amplifies the signal obtained in the presence of the primary activator sequence for rapid and sensitive detection. In this system, the primary activator complex results in linear amplification of signal from the detection of the primary activator complex while the addition of the secondary amplification system results in exponential amplification. In some cases, the cage comprises one or more stem loop structures. In some cases, the loop structures are caused on an RNA by the addition of complementary sequences that will fold back on the RNA, in some cases leaving a single stranded loop. In this way, the addition of the cage sequences causes the formation of one or more stem loops on an RNA.

Thus, the disclosed systems provide for inexpensive and rapid detection of nucleic acid target sequences from a variety of sources including mammals, viruses, bacteria, fungi, etc. with minimal sample preparation, and specifically without the need to amplify nucleic acids from the sample. The samples may be biological samples from a human or non-human patient, or an environmental sample from water, food, etc.

### Cas and Csm6 Sensors and Signal Amplifiers

Any Cas protein(s) can be used in the Cas-effector molecules (target sensor and/or signal amplifier) of the compositions (not part of the invention) and systems, including but not limited to Cas proteins from any type of CRISPR/Cas system (*e.g*., Type II, Type III, Type V, Type VI ), Csm6 proteins, Csx1 proteins and the like.

The Cas proteins may be derived from any suitable source, including archaea and bacteria. In some embodiments, a native Cas protein may be derived from *Paludibacter, Carnobacterium, Listeria, Herbinix, Rhodobacter, Leptotrichia, Lachnospiraceae, Eubacterium,* or *Clostridium.* In some embodiments, the native Cas protein may be derived from *Paludibacter propionicigenes, Carnobacterium gallinarum, Listeria seeligeri, Listeria newyorkensis, Herbinix hemicellulosilytica, Rhodobacter capsulatus, Leptotrichia wadei, Leptotrichia buccalis, Leptotrichia shahii, Lachnospiraceae* bacterium NK4A179, *Lachnospiraceae* bacterium MA2020, *Eubacterium rectale, Lachnospiraceae* bacterium NK4A144, and *Clostridium aminophilum.*

The Cas protein(s) as described herein may be homologous to a native Cas protein. In some embodiments, the disclosed Cas protein is greater than 75%, 80%, 85%, 90%, 95%, 97%, 98%, or 99%, and less than about 100%, 99%, 98%, 97%, 95%, 90%, 85%, 80%, or 75% identical to a native Cas protein sequence. The disclosed Cas protein may have one or more HEPN domains, and may be able, after activation, to cleave single stranded RNA, including precursor guide RNA and indicator RNA.

Activation of a Cas protein may include contacting one or more target sequences with a guide RNA sequence associated with the Cas protein. In some embodiments, the guide RNA of the Cas protein may help to activate the Cas protein's RNase activity by hybridizing to a complementary target RNA sequence.

The disclosed Cas proteins may be any Cas protein, including but not limited to Type V (*e.g.,* Cas12 and/or Cas14), Type VI (*e.g.,* Cas13), and/or Type III (*e.g.,* Csm6) proteins.

In certain embodiments, the compositions (not part of the invention), systems and methods include one or more Cas13 protein with 4 currently characterized subtypes (Cas13a-d) that each exhibit significant sequence divergence apart from two consensus HEPN (Higher eukaryotes and prokaryotes nucleotide-binding domain) RNase motifs, R-X4-6-H. To defend against viral infection, Cas13 enzymes process precrRNA into mature crRNA guides in a HEPN-independent manner, followed by HEPN-dependent cleavage of a complementary "activator" target RNA in cis. Upon target-dependent activation, Cas13 is also able to cleave bystander RNAs in trans, reflecting a general RNase activity capable of both cis- and trans-cleavage. (*See, e.g.,* U.S. Publication No. 20200032324 and WO2017218573, Konnermann et al (2018) Cell Apr 19;173(3):665-676; Zhang et al (2018) Cell 175 (1), 212-223). The signature protein of Type VI-A CRISPR-Cas systems, Cas13a (formerly C2c2), is a dual nuclease responsible for both crRNA maturation and RNA-activated ssRNA cleavage (East- Seletsky et al., (2016) Nature 538(7624):270-273). Cas13a binds to precursor crRNA (pre-crRNA) transcripts and cleaves them within the repeat region to produce mature crRNAs. When the pre-crRNA is processed to the individual mature crRNAs, an 8 nucleotide piece of the repeat region that separates each of the spacer regions in a CRISPR array remains attached to the mature crRNA and is termed the "tag". Binding to a ssRNA activator (target) sequence with complementarity to the crRNA activates Cas13a for trans-ssRNA cleavage, potentially triggering cell death or dormancy of the host organism. However, if the target or activator RNA comprises a sequence that is complementary to the tag sequence (known as the "anti-tag") the complex is inhibited from being activated. This is thought to be a mechanism involved in preventing autoimmunity (Meeske & Marriffini (2018) Mol Cell 71:791). The Cas13a's trans-ssRNA activity can be exploited for use in releasing cage structures on RNAs; an activity that can be tunes by use of cage sequences that correspond to the preferences for the different Cas13a homologs.

In some embodiments, the Cas13 protein is a Cas13a polypeptide comprising an amino acid sequence having at least 75%, at least 80%, at least 85%, at least 90%, at least 95%, at least 98%, at least 99%, or 100%, amino acid sequence identity to any Cas13a amino acid sequence, for example a Cas13a sequence as shown in Table 1 and/or Example 3.

**Table 1: Exemplary Cas13a proteins**

| **Cas13a abbreviation** | **Organism name** | **Accession number** |
|---|---|---|
| LshCas13a | Leptotrichia shahii | WP_018451595.1 |
| LwaCas13a | Leptotrichia wadei | WP_021746774.1 |
| LseCas13a | Listeria seeligeri | WP_012985477.1 |
| LbmCas13a | Lachnospiraceae bacterium MA2020 | WP_044921188.1 |
| LbnCas13a | Lachnospiraceae bacterium NK4A179 | WP_022785443.1 |
| CamCas13a | [Clostridium] aminophilum DSM 10710 | WP_031473346.1 |
| CgaCas13a | Carnobacterium gallinarum DSM 4847 | WP_034560163.1 |
| Cga2Cas13a | Carnobacterium gallinarum DSM 4847 | WP_034563842.1 |
| Pprcas13a | Paludibacter propionicigenes WB4 | WP_013443710.1 |
| LweCas13a | Listeria weihenstephanensis FSL R9-0317 | WP_036059185.1 |
| LneCas13a | Listeriaceae bacterium FSL M6-0635 (Listeria newyorkensis) | WP_036091002.1 |
| Lwa2cas13a | Leptotrichia wadei F0279 | WP_021746774.1 |
| RcsCas13a | Rhodobacter capsulatus SB 1003 | WP_013067728.1 |
| RcrCas13a | Rhodobacter capsulatus R121 | WP_023911507.1 |
| RcdCas13a | Rhodobacter capsulatus DE442 | WP_023911507.1 |
| LbuCas13a | Leptotrichia buccalis | WP_015770004.1 |
| LbaCas13a | Lachnospiraceae bacterium NK4A179 | WP_022785443.1 |
| RcaCas13a | Rhodobacter capsulatus R121 | ETD76934.1 |
| EreCas13a | [Eubacterium] rectale | WP_055061018.1 |
| HheCas13a | Herbinix hemicellulosilytica | CRZ35554.1 |

Additional Cas13 proteins include BzoCas13b (*Bergeyella zoohelcum*; WP_002664492); PinCas13b (*Prevotella intermedia*; WP_036860899); PbuCas13b (*Prevotella buccae*; WP_004343973); AspCas13b (*Alistipes* sp. ZOR0009; WP_047447901); PsmCas13b (*Prevotella* sp.MA2016; WP_036929175); RanCas13b (*Riemerella anatipestifer*; WP_004919755); PauCas13b (*Prevotella aurantiaca*; WP_025000926 ); PsaCas13b (*Prevotella saccharolytica,* WP_051522484); Pin2Cas13b (*Prevotella intermedia*; WP_061868553); CcaCas13b (*Capnocytophaga canimorsus*; WP_013997271); PguCas13b (*Porphyromonas gulae*; WP_039434803); PspCas13b (*Prevotella* sp. P5-125, WP_0440652940); PgiCas13b (*Porphyromonas gingivalis*; WP_053444417); FbrCas13b (*Flavobacterium branchiophilum*; WP_014084666); and Pin3Cas13b (*Prevotella intermedia*; WP_050955369); FnsCas13c (*Fusobacterium necrophorum subsp.funduliforme* ATCC 51357contig00003; WP_005959231.1); FndCas13c (*Fusobacterium necrophorum* DJ-2 contig0065, whole genome shotgun sequence; WP_035906563.1); FnfCas13c (*Fusobacterium necrophorum* subsp. funduliforme 1_1_36S cont1.14; EHO19081.1); FpeCas13c (*Fusobacterium perfoetens* ATCC 29250 T364DRAFT_scaffold00009.9_C; WP_027128616.1); FulCas13c (*Fusobacterium ulcerans* ATCC 49185 cont2.38; WP_040490876.1); AspCas13c (*Anaerosalibacter* sp. ND1 genome assembly Anaerosalibacter massiliensis ND1; WP _042678931.1); Ruminococcus sp Cas13d, (GI: 1690532978); EsCas13d ([Eubacterium] *siraeum* DSM 15702; GI: 1486942132 or GI: 1486942131) and the Cas13d homologs disclosed in U.S. Patent Publication 20190062724.

In certain embodiments, the compositions (not part of the invention), systems and methods include one or more Type V Cas proteins. Non-limiting examples of Type V CRISPR/Cas proteins include Cas12 and Cas14 proteins. *See, e.g.,* U.S. Publication No. 20190241954. In some embodiments, the Cas12 protein is a Cas12 polypeptide comprising an amino acid sequence having at least 75%, at least 80%, at least 85%, at least 90%, at least 95%, at least 98%, at least 99%, or 100%, amino acid sequence identity to any Cas12 amino acid sequence, for example a Cas12 sequence as shown in Figure 6A-6C. See e.g. PCT/US2020/021213, WO2020023529, WO 2019104058 and WO2019089796.

In some embodiments, the Cas14 protein is a Cas14 polypeptide comprising an amino acid sequence having at least 75%, at least 80%, at least 85%, at least 90%, at least 95%, at least 98%, at least 99%, or 100%, amino acid sequence identity to any Cas14 amino acid sequence, for example a Cas14 sequence as shown in Figure 6. See e.g. Harrington et al (Harrington LB, (2018) Science 362(6416):839-842) and PCT/US2020/021214 (Cas14). See e.g. PCT/US2020/021214.

In certain embodiments, the systems, compositions (not part of the invention) and methods described herein comprises a Csm6 protein. Csm6 is a family of single-stranded ribonucleic acid (ssRNA) endonucleases associated with Type III CRISPR-Cas systems. The RNA cleavage activity of Csm6 can be allosterically activated by binding of either cyclic oligoadenylates (cAₙ) or short linear oligoadenylates bearing a terminal 2'-3' cyclic phosphate (Aₙ>P). Csm6 has been used in the SHERLOCK system to amplify the detection of viral RNAs. In some embodiments, EiCasm6 (*Enterococcus italicus*; WP_007208953.1), LsCsm6 (*Lactobacillus salivarius*; WP_081509150.1) and/or TtCsm6 (*Thermus thermophilus*; WP_011229148.1) is used. In one embodiment, the TtCsm6 is activated specifically by oligoadenylates with a length of four adenosines (cA₄ or A₄>P), and exhibits a cleavage preference for RNA sequences with A's and C's. In one embodiment, EiCsm6 and/or LsCsm6 is used with a guide comprising an A₆ length to amplify the signal following cleavage of a protected (caged) amplifier RNA. Accordingly, Csm6 variants can be used for amplification of detectable signal in the presence of the target, for example by inclusion of a suitable substrate (*e.g*., trigger substrate) comprising such preferred sequences. For example, in some embodiments, an A₆U₅ comprising RNA is added to a reaction mixture comprising Cas13 and EiCsm6 such that upon activation of Cas13 following interaction with its primary RNA activator, trans cleavage by Cas13 of the A₆U₆ RNA will create an activator for the EiCsm6 leading EiCsm6 to cleave the reporter molecule, thus amplifying the signal initiated by the original interaction of the Cas13 with its primary activator. In another embodiment, an A₅Uₓ RNA is used in the reaction which comprises TtCsm6 rather than EiCsm6 (Gootenberg et al, (2018) Science 360(6387): 439).

In an embodiment, the Cas protein is a modified protein that is modified, or engineered or mutated, to alter its interaction with guide or target sequences and/or to alter its nuclease activity, for example specificity, turn-over, nucleotide preferences, etc. In other embodiments, the Cas protein may be fused to another protein, peptide, or marker to aid in isolation, identification, separation, nuclease activity, target sequence binding, etc.

In some cases, RNA (viral RNA, mRNA, small RNAs, etc.) is directly detected (without the need for reverse transcriptase) using systems comprising wild-type and/or modified Cas13 proteins, while DNA (viral DNA, etc.) is directly detected using systems comprising wild-type and/or engineered Cas12 or Cas14 proteins. In other cases, RNA can be reverse transcribed into DNA detected using Cas12 or Cas14 effector proteins.

One or more of the same or different Cas effector proteins can be used in the systems described herein. In some cases, the target sensor and the signal amplifier both comprise one or more Cas12 proteins and/or Cas14 proteins, for example for the detection of DNA target sequences. The one or more Cas12 and/or Cas14 proteins may themselves be the same or different (modified or engineered) proteins. In other cases, the target sensor and the signal amplifier both comprise one or more Cas13 proteins, for example for the detection of RNA target sequences. The Cas13 proteins may themselves be the same proteins may be different (modified or engineered) Cas13 proteins. In other embodiments, the target sensor and the signal amplifier may comprise different types of Cas-effector proteins, including Cas12, Cas13, Cas14 and Csm6 proteins, for example wherein the target sensor comprises one or more Cas13 proteins for detection of an RNA target sequence and the signal amplifier comprises one or more Cas12 and/or Cas14 proteins. In some cases, the signal amplifier also comprises a Csm6 protein (enzyme). In some embodiments, the target sensor comprises one or more Cas12 and/or Cas14 proteins for detection of a nucleic acid target sequence and the signal amplifier comprises one or more Cas13 proteins. In some cases, the signal amplifier also comprises a Csm6 protein (enzyme).

### Guide RNA Sequences

The NCR systems and compositions (not part of the invention) as described herein also include a molecule, typically a guide RNA, used to program the one or more Cas proteins such that they are activated into nucleases upon binding of the guide RNA to a cognate (target, activator, etc.) sequence.

A nucleic acid molecule associates with (binds to) a Cas effector protein (e.g., a Cas13 or Cas12 protein), forming a ribonucleoprotein complex (RNP), and targets the complex to a specific target sequence within the polynucleotide is referred to herein as a "guide RNA." It is to be understood that in some cases, a hybrid DNA/RNA can be made such that a guide RNA includes DNA bases in addition to RNA bases--but the term "guide RNA" is still used herein to encompass such hybrid molecules. A subject guide RNA may include a guide sequence (also referred to as a "spacer") (that hybridizes to target sequence of a target RNA or DNA) and a constant region (e.g., a region that is adjacent to the guide sequence and binds to the Cas effector protein). A "constant region" can also be referred to herein as a "protein-binding segment." In some cases, the constant region is 5' of the guide sequence.

Guide RNAs include at least one sequence complementary to a target RNA sequence. In some embodiments, this target-complementary sequence may be referred to as a spacer sequence, additional sequences may be referred to as scaffold sequences. In some embodiments, the spacer sequence is derived from a human (e.g. genomic DNA or reverse transcribed RNA) or non-human source (for example a pathogen). In some embodiments, the pathogen selected may be from bacteria, viruses, fungi, and parasites. In some embodiments, the pathogen may be a virus (*e.g., Orthocoronavirinae, Dependovirus, Picornaviridae, Poxviridae, Flaviviridae, Rhabdoviridae, Togaviridae, Filoviridae, Herpesviridae, Bunyaviridae, Hepadnaviridae, Adenoviridae, Retroviridae, Papillomaviridae, Pneumoviridae, Orthomyxoviridae, Arenaviridae, and Paramyxoviridae, Caliciviridae)* or a bacterium (*e.g., Mycobacterium, Streptococcus Pseudomonas, Shigella, Campylobacter, Salmonella, Clostridium, Corynebacterium*, and *Treponema*)*.* In some embodiments the virus may be selected from DNA or RNA viruses including *Orthocoronavirinae, Adenoviridae, Picornaviridae, Herpesviridae, Hepadnaviridae, Flaviviridae, Retroviridae, Orthomyxoviridae, Paramyxoviridae, Papovaviridae, Polyomavirus, Rhabdoviridae*, and *Togaviridae.* In some embodiments, pathogenic fungi include Candida, Aspergillus, Cryptococcus, Histoplasma, Pneumosystis, and Stachybotrys.

In other embodiments, the spacer RNA sequence is complementary to a non-pathogen. For example, the spacer RNA sequence may be engineered to hybridize to any nucleic acid sequence of interest. In some embodiments, the guide RNA sequence may be engineered to be complementary to a mammalian sequence of interest, for example a genomic sequence, or transcribed sequence (mRNA, microRNA, etc.). In various embodiments, the guide RNA may include a sequence complementary to a sequence associated with a mammalian biological state, condition, disease, or disorder, such as sepsis, cancer, viral infection, bacterial infection, fungal infection. In some embodiments, the guide RNAs may be complementary to a mRNA or micro RNA, for example a microRNA sequence in a microRNA signature. In some embodiments, the guide RNA sequence may be within a precursor RNA, which may, in turn be part of an array with a plurality of guide RNA sequences. In some embodiments, precursor RNA sequences may be processed by the Cas protein to provide guide RNA sequences.

Guide RNA sequences include the spacer sequence, which is complementary to the target sequence, and a more constant sequence that is 5' of the spacer sequence. This constant sequence may be referred to as a scaffold sequence, repeat, handle, or constant region and aids in binding the guide RNA to the Cas protein. In some embodiments, the constant sequence can be replaced with that of an evolutionarily related constant sequence. As is known in the art, Cas proteins may be grouped into different families comprising functional groups that recognize orthogonal sets of crRNAs and possess different ssRNA cleavage specificity. In some embodiments, the constant sequence can be modified to improve affinity and stability by including naturally occurring and synthetic or non-natural nucleobases or backbone modifications. In some embodiments, the constant sequence may include a precursor sequence. In an embodiment, a pre-crRNA sequence may be processed to form a crRNA sequence, which includes the guide sequence.

The guide sequence having complementarity with (hybridizes to) a target sequence of the target RNA or DNA sequence can be of any suitable length. In some cases, the guide sequence is 15-28 nucleotides (nt) in length (e.g., 15-26, 15-24, 15-22, 15-20, 15-18, 16-28, 16-26, 16-24, 16-22, 16-20, 16-18, 17-26, 17-24, 17-22, 17-20, 17-18, 18-26, 18-24, or 18-22 nt in length). In some cases, the guide sequence is 18-24 nucleotides (nt) in length. In some cases, the guide sequence is at least 15 nt long (e.g., at least 16, 18, 20, or 22 nt long). In some cases, the guide sequence is at least 17 nt long. In some cases, the guide sequence is at least 18 nt long. In some cases, the guide sequence is at least 20 nt long.

**In** some cases, the guide sequence has 80% or more (e.g., 85% or more, 90% or more, 95% or more, or 100% complementarity) with the target sequence of the target sequence. In some cases, the guide sequence is 100% complementary to the target sequence. In some cases, the target sequence includes at least 15 nucleotides (nt) of complementarity with the guide sequence of the guide RNA.

The guide RNA can be provided as RNA or as a nucleic acid encoding the guide RNA (e.g., a DNA such as a recombinant expression vector). The Cas effector protein (e.g., a Cas 13 protein such as LshCas13a, LwaCas13a, LseCas13a, LbmCas13a, LbnCas13a, CamCas13a, CgaCas13a, Cga2Cas13a, Pprcas13a, LweCas13a, LneCas13a, Lwa2cas13a, RcsCas13a, RcrCas13a, RcdCas13a, LbuCas13a, RcaCas13a, EreCas13a, BzoCas13b, PinCas13b, PbuCas13b, AspCas13b, PsmCas13b, RanCas13b, PauCas13b, PsaCas13b, Pin2Cas13b, CcaCas13b, PguCas13b, PigCas13b, Pin3Cas13b and HheCas13a , EsCas13d ([Eubacterium] *siraeum* DSM 15702, UrCas13d, Cas13d isolated from *Ruminococcus* species, Cas13d isolated from gut metagenomes, Cas13d from new_flavefaciens,_strain_XPD3002 (see e.g. U.S. Patent Application 20190062724), and/or a Cas12 protein such as Cas12a, Cas12b, Cas12c, Cas12d, Cas12e and/or a Cas14 protein such as Cas14a, Cas14b, Cas14c, Cas14i, Cas14j, Cas14k, Cas14u) can be provided as a protein or as a nucleic acid encoding the protein (e.g., an mRNA, a DNA such as a recombinant expression vector) (Harrington et al (2018) Science 362(6416):839-842). In some cases, two or more (e.g., 3 or more, 4 or more, 5 or more, or 6 or more) guide RNAs can be provided by (e.g., using a precursor guide RNA array, which can be cleaved by the Cas effector protein into individual ("mature") guide RNAs).

A Cas protein comprising a guide RNA may be referred to as a "programmed" Cas protein. Guide RNA sequences may be introduced to and bound by a Cas protein. For example, the guide RNA may contact the Cas protein in a cell or outside a cell. Various methods may be used to contact the guide RNA with the Cas protein to produce a programmed Cas protein. In some embodiments, contacting requires less than about 2 hours, for example less than about 90 min., 60 min., 40 min., 30 min., 20 min., 10 min., 5 min., 4 min., 3 min., 2 min., or 1 min.

### Constant Region

Any constant region can be used in the guide RNAs of the invention. Non-limiting examples of constant regions are disclosed in U.S. Publication No. 20190241954.

In some cases, the guide RNA includes a double stranded RNA duplex (dsRNA duplex). In some cases, a guide RNA includes a dsRNA duplex with a length of from 2 to 12 bp (e.g., from 2 to 10 bp, 2 to 8 bp, 2 to 6 bp, 2 to 5 bp, 2 to 4 bp, 3 to 12 bp, 3 to 10 bp, 3 to 8 bp, 3 to 6 bp, 3 to 5 bp, 3 to 4 bp, 4 to 12 bp, 4 to 10 bp, 4 to 8 bp, 4 to 6 bp, or 4 to 5 bp). In some cases, a guide RNA includes a dsRNA duplex that is 2 or more bp in length (e.g., 3 or more, 4 or more, 5 or more, 6 or more, or 7 or more bp in length). In some cases, a guide RNA includes a dsRNA duplex that is longer than the dsRNA duplex of a corresponding wild type guide RNA. In some cases, a guide RNA includes a dsRNA duplex that is shorter than the dsRNA duplex of a corresponding wild type guide RNA.

In some cases, the constant region of a guide RNA is 15 or more nucleotides (nt) in length (e.g., 18 or more, 20 or more, 21 or more, 22 or more, 23 or more, 24 or more, 25 or more, 26 or more, 27 or more, 28 or more, 29 or more, 30 or more, 31 or more nt, 32 or more, 33 or more, 34 or more, or 35 or more nt in length). In some cases, the constant region of a guide RNA is 18 or more nt in length.

In some cases, the constant region of a guide RNA has a length in a range of from 12 to 100 nt (e.g., from 12 to 90, 12 to 80, 12 to 70, 12 to 60, 12 to 50, 12 to 40, 15 to 100, 15 to 90, 15 to 80, 15 to 70, 15 to 60, 15 to 50, 15 to 40, 20 to 100, 20 to 90, 20 to 80, 20 to 70, 20 to 60, 20 to 50, 20 to 40, 25 to 100, 25 to 90, 25 to 80, 25 to 70, 25 to 60, 25 to 50, 25 to 40, 28 to 100, 28 to 90, 28 to 80, 28 to 70, 28 to 60, 28 to 50, 28 to 40, 29 to 100, 29 to 90, 29 to 80, 29 to 70, 29 to 60, 29 to 50, or 29 to 40 nt). In some cases, the constant region of a guide RNA has a length in a range of from 28 to 100 nt. In some cases, the region of a guide RNA that is 5' of the guide sequence has a length in a range of from 28 to 40 nt.

In some cases, the constant region of a guide RNA is truncated relative to (shorter than) the corresponding region of a corresponding wild type guide RNA. In some cases, the constant region of a guide RNA is extended relative to (longer than) the corresponding region of a corresponding wild type guide RNA. In some cases, a subject guide RNA is 30 or more nucleotides (nt) in length (e.g., 34 or more, 40 or more, 45 or more, 50 or more, 55 or more, 60 or more, 65 or more, 70 or more, or 80 or more nt in length). In some cases, the guide RNA is 35 or more nt in length.

### Precursor Guide RNA Array

The Cas effector protein can cleave a precursor guide RNA into a mature guide RNA, e.g., by endoribonucleolytic cleavage of the precursor, for example by cleaving a precursor guide RNA array (that includes more than one guide RNA arrayed in tandem) into two or more individual guide RNAs. Thus, in some cases a precursor guide RNA array comprises two or more (e.g., 3 or more, 4 or more, 5 or more, 2, 3, 4, or 5) guide RNAs (e.g., arrayed in tandem as precursor molecules). In other words, in some cases, two or more guide RNAs can be present on an array (a precursor guide RNA array). A Cas effector protein as described herein can cleave the precursor guide RNA array into individual guide RNAs.

In some cases, a subject guide RNA array includes 2 or more guide RNAs (e.g., 3 or more, 4 or more, 5 or more, 6 or more, or 7 or more, guide RNAs). The guide RNAs of a given array can target (i.e., can include guide sequences that hybridize to) different target sites of the same target RNA/DNA (e.g., which can increase sensitivity of detection) and/or can target different target RNA/DNA molecules (e.g., single nucleotide polymorphisms (SNPs), different strains of a particular virus, etc.), and such could be used for example to detect multiple strains of a virus. In some cases, each guide RNA of a precursor guide RNA array has a different guide sequence. In some cases, two or more guide RNAs of a precursor guide RNA array have the same guide sequence.

In some cases, the precursor guide RNA array comprises two or more guide RNAs that target different target sites within the same target sequence. In some cases, the precursor guide RNA array comprises two or more guide RNAs that target different target sequences. For example, such a scenario can result in a positive signal when any one of a family of potential target RNAs/DNAs is present. Such an array could be used for targeting a family of transcripts, e.g., based on variation such as single nucleotide polymorphisms (SNPs) (e.g., for diagnostic purposes). Such could also be useful for detecting whether any one of a number of different strains of virus is present. Such could also be useful for detecting whether any one of a number of different species, strains, isolates, or variants of a virus or bacterium is present. As such, in some cases as subject composition (not part of the invention; e.g. kit) or method includes two or more guide RNAs (in the context of a precursor guide RNA array, or not in the context of a precursor guide RNA array, e.g., the guide RNAs can be mature guide RNAs).

### Protospacer Adjacent Motif (PAM)

In cases where the target sequence is a dsDNA, identification of a PAM sequence in the target may be required. A Type V CRISPR/Cas effector protein binds to target DNA at a target sequence defined by the region of complementarity between the DNA-targeting RNA and the target DNA. As is the case for many CRISPR/Cas endonucleases, site-specific binding (and/or cleavage) of a double stranded target DNA occurs at locations determined by both (i) base-pairing complementarity between the guide RNA and the target DNA; and (ii) a short motif [referred to as the protospacer adjacent motif (PAM)] in the target DNA.

In some cases, the PAM for a Type V CRISPR/Cas effector protein is immediately 5' of the target sequence (e.g., of the non-complementary strand of the target DNA--the complementary strand hybridizes to the guide sequence of the guide RNA while the non-complementary strand does not directly hybridize with the guide RNA and is the reverse complement of the non-complementary strand). In some cases (e.g., when Cas12a or Cas12b as described herein is used), the PAM sequence is 5'-TTN-3'. In some cases, the PAM sequence is 5'-TTTN-3'.

In some cases, different Type V CRISPR/Cas effector proteins (i.e., Type V CRISPR/Cas effector proteins from various species) may be advantageous to use in the various provided methods in order to capitalize on a desired feature (e.g., specific enzymatic characteristics of different Type V CRISPR/Cas effector proteins). Type V CRISPR/Cas effector proteins from different species may require different PAM sequences in the target DNA. Thus, for a particular Type V CRISPR/Cas effector protein of choice, the PAM sequence requirement may be different than the 5'-TTN-3' or 5'-TTTN-3' sequence described above. Various methods (including in silico and/or wet lab methods) for identification of the appropriate PAM sequence are known in the art and are routine, and any convenient method can be used.

Members of the CRISPR-Cas13 system work as dual-component systems, in which a crRNA forms a complex with the Cas13 protein without involving any tracrRNA. The flanking regions of protospacers comprise a 3' protospacer flanking site (PFS) that affects the efficacy of Cas13a-mediated targeting (Abudayyeh et al (2016) Science. 353(6299)) Although the PFS is adjacent to the protospacer target, the commonly used protospacer adjacent motif (PAM) nomenclature is not used as it has come to connote a sequence used in self vs. non-self differentiation, which is irrelevant in a RNA-targeting system. Thus, in some cases, identification of a PFS sequence in a target may be required.

### Reporters

Any reporter molecule (also referred to as a detector sequence) can be used in the systems described herein.

The reporter molecules (complexes) comprise a detectable label (also referred to a signal moiety). Non-limiting examples of detectable labels include fluorescent labels, enzymatic labels and/or bioluminescent labels. The reporter typically further comprises a molecule (also referred to as a "quencher" or "quencher molecule" or "quencher moiety"), which when in close proximity (linked) to the detectable label, prevents the label from being detected, for example by emitting a signal.

In some cases, a detectable signal is produced when the reporter molecule is cleaved (e.g., a quencher/fluor pair also referred to as an F/Q reporter). One signal partner of a signal quenching pair produces a detectable signal and the other signal partner is a quencher moiety that quenches the detectable signal of the first signal partner (i.e., the quencher moiety quenches the signal of the signal moiety such that the signal from the signal moiety is reduced (quenched) when the signal partners are in proximity to one another, e.g., when the signal partners of the signal pair are in close proximity).

For example, in some cases, an amount of detectable signal increases when the labeled reporter is cleaved. For example, in some cases, the signal exhibited by one signal partner (a signal moiety) is quenched by the other signal partner (a quencher signal moiety), e.g., when both are present on the same molecule prior to cleavage by a by the activated Cas non-specific nucleases (*e.g*., activated sensor and signal amplifier). Such a signal pair is referred to herein as a "quencher/fluor pair", "quenching pair", or "signal quenching pair." For example, in some cases, one signal partner (e.g., the first signal partner) is a signal moiety that produces a detectable signal that is quenched by the second signal partner (e.g., a quencher moiety). The signal partners of such a quencher/fluor pair will thus produce a detectable signal when the partners are separated (e.g., after cleavage of the reporter molecule by the activated sensor or signal amplifier), but the signal will be quenched when the partners are in close proximity (e.g., prior to cleavage of the reporter).

The reporter typically includes amplifier activators substrates as described herein. In some cases, the amplifier activator sequence is positioned between the F/Q pair and may comprises A's or C's, but also accommodate additional nucleotides necessary for Cas13 (U), Cas12 (deoxyribonucleotides), Csm6, etc. activity, namely sequences bound by the Cas-effector protein of the signal amplifier. For example, reporters comprising Csm6 homologs that respond to A₆>P, like *S. epidermidis* Csm6 (SeCsm6) can generated by changing the number of A's from four to six in the hairpin loop of the caged crRNA.

The detectable label can include one or more modifications to reduce background activity and/or improve sensitivity. In some embodiments, the detectable label is comprised of a fluorophore and quencher molecule linked by an oligonucleotide sequence. Stem-loop structures can be exploited to increase the proximity of fluorophore to quencher, and loop length and sequence (such as U and A bases) can be incorporated to modulate the efficiency of their release by Cas nuclease activity. In some cases, the sequence of an oligo linking the fluorophore to the quencher comprises caged structures sensitive to release by specific trans nuclease activity. In some cases, the reporter molecule is associated with a trans caging molecule. A "trans caging molecule" can comprise any nucleic acid that binds to another nucleic acid such that a duplex structure is formed or created. In some cases, the duplex structure comprises one or more loops (e.g. stem loops).

A quencher moiety can quench a signal from the signal moiety (e.g., prior to cleavage) to various degrees. In some cases, a quencher moiety quenches the signal from the signal moiety where the signal detected in the presence of the quencher moiety (when the signal partners are in proximity to one another) is 95% or less of the signal detected in the absence of the quencher moiety (when the signal partners are separated). For example, in some cases, the signal detected in the presence of the quencher moiety can be 90% or less, 80% or less, 70% or less, 60% or less, 50% or less, 40% or less, 30% or less, 20% or less, 15% or less, 10% or less, or 5% or less of the signal detected in the absence of the quencher moiety. In some cases, no signal (e.g., above background) is detected in the presence of the quencher moiety.

In some cases, the signal detected in the absence of the quencher moiety (when the signal partners are separated) is at least 1.2 fold greater (e.g., at least 1.3 fold, at least 1.5 fold, at least 1.7 fold, at least 2 fold, at least 2.5 fold, at least 3 fold, at least 3.5 fold, at least 4 fold, at least 5 fold, at least 7 fold, at least 10 fold, at least 20 fold, or at least 50 fold greater) than the signal detected in the presence of the quencher moiety (when the signal partners are in proximity to one another).

In some cases, the signal moiety is a fluorescent label. In some such cases, the quencher moiety quenches the signal (the light signal) from the fluorescent label (e.g., by absorbing energy in the emission spectra of the label). Thus, when the quencher moiety is not in proximity with the signal moiety, the emission (the signal) from the fluorescent label is detectable because the signal is not absorbed by the quencher moiety. Any convenient donor acceptor pair (signal moiety/quencher moiety pair) can be used and many suitable pairs are known in the art.

In some cases, the quencher moiety absorbs energy from the signal moiety (also referred to herein as a "detectable label") and then emits a signal (e.g., light at a different wavelength). Thus, in some cases, the quencher moiety is itself a signal moiety (e.g., a signal moiety can be 6-carboxyfluorescein while the quencher moiety can be 6-carboxy-tetramethylrhodamine), and in some such cases, the pair could also be a FRET pair. In some cases, a quencher moiety is a dark quencher. A dark quencher can absorb excitation energy and dissipate the energy in a different way (e.g., as heat). Thus, a dark quencher has minimal to no fluorescence of its own (does not emit fluorescence). Examples of dark quenchers are further described in U.S. Pate. Nos. 8,822,673 and 8,586,718; U.S. patent publications 20140378330, 20140349295, and 20140194611; and international patent applications: WO200142505 and WO200186001.

Examples of fluorescent labels include, but are not limited to: an Alexa Fluor^{™}. dye, an ATTO dye (e.g., ATTO 390, ATTO 425, ATTO 465, ATTO 488, ATTO 495, ATTO 514, ATTO 520, ATTO 532, ATTO Rho6G, ATTO 542, ATTO 550, ATTO 565, ATTO Rho3B, ATTO Rho11, ATTO Rho12, ATTO Thio12, ATTO Rho101, ATTO 590, ATTO 594, ATTO Rho13, ATTO 610, ATTO 620, ATTO Rho14, ATTO 633, ATTO 647, ATTO 647N, ATTO 655, ATTO Oxa12, ATTO 665, ATTO 680, ATTO 700, ATTO 725, ATTO 740), a DyLight dye, a cyanine dye (e.g., Cy2, Cy3, Cy3.5, Cy3b, Cy5, Cy5.5, Cy7, Cy7.5), a FluoProbes dye, a Sulfo Cy dye, a Seta dye, an IRIS Dye, a SeTau dye, an SRfluor dye, a Square dye, fluorescein isothiocyanate (FITC), tetramethylrhodamine (TRITC), Texas Red, Oregon Green, Pacific Blue, Pacific Green, Pacific Orange, quantum dots, and a tethered fluorescent protein.

In some cases, a detectable label is a fluorescent label selected from: an Alexa Fluor^{™}. dye, an ATTO dye (e.g., ATTO 390, ATTO 425, ATTO 465, ATTO 488, ATTO 495, ATTO 514, ATTO 520, ATTO 532, ATTO Rho6G, ATTO 542, ATTO 550, ATTO 565, ATTO Rho3B, ATTO Rho11, ATTO Rho12, ATTO Thio12, ATTO Rho101, ATTO 590, ATTO 594, ATTO Rho13, ATTO 610, ATTO 620, ATTO Rho14, ATTO 633, ATTO 647, ATTO 647N, ATTO 655, ATTO Oxa12, ATTO 665, ATTO 680, ATTO 700, ATTO 725, ATTO 740), a DyLight dye, a cyanine dye (e.g., Cy2, Cy3, Cy3.5, Cy3b, Cy5, Cy5.5, Cy7, Cy7.5), a FluoProbes dye, a Sulfo Cy dye, a Seta dye, an IRIS Dye, a SeTau dye, an SRfluor dye, a Square dye, fluorescein (FITC), tetramethylrhodamine (TRITC), Texas Red, Oregon Green, Pacific Blue, Pacific Green, and Pacific Orange.

In some cases, a detectable label is a fluorescent label selected from: an Alexa Fluor^{™} dye, an ATTO dye (e.g., ATTO 390, ATTO 425, ATTO 465, ATTO 488, ATTO 495, ATTO 514, ATTO 520, ATTO 532, ATTO Rho6G, ATTO 542, ATTO 550, ATTO 565, ATTO Rho3B, ATTO Rho11, ATTO Rho12, ATTO Thio12, ATTO Rho101, ATTO 590, ATTO 594, ATTO Rho13, ATTO 610, ATTO 620, ATTO Rho14, ATTO 633, ATTO 647, ATTO 647N, ATTO 655, ATTO Oxa12, ATTO 665, ATTO 680, ATTO 700, ATTO 725, ATTO 740), a DyLight dye, a cyanine dye (e.g., Cy2, Cy3, Cy3.5, Cy3b, Cy5, Cy5.5, Cy7, Cy7.5), a FluoProbes dye, a Sulfo Cy dye, a Seta dye, an IRIS Dye, a SeTau dye, an SRfluor dye, a Square dye, fluorescein (FITC), tetramethylrhodamine (TRITC), Texas Red, Oregon Green, Pacific Blue, Pacific Green, Pacific Orange, a quantum dot, and a tethered fluorescent protein.

Examples of ATTO dyes include, but are not limited to: ATTO 390, ATTO 425, ATTO 465, ATTO 488, ATTO 495, ATTO 514, ATTO 520, ATTO 532, ATTO Rho6G, ATTO 542, ATTO 550, ATTO 565, ATTO Rho3B, ATTO Rho11, ATTO Rho12, ATTO Thio12, ATTO Rho101, ATTO 590, ATTO 594, ATTO Rho13, ATTO 610, ATTO 620, ATTO Rho14, ATTO 633, ATTO 647, ATTO 647N, ATTO 655, ATTO Oxa12, ATTO 665, ATTO 680, ATTO 700, ATTO 725, and ATTO 740.

Examples of AlexaFluor dyes include, but are not limited to: Alexa Fluor^{™} 350, Alexa Fluor^{™} 405, Alexa Fluor^{™} 430, Alexa Fluor^{™} 488, Alexa Fluor^{™} 500, Alexa Fluor^{™} 514, Alexa Fluor^{™} 532, Alexa Fluor^{™} 546, Alexa Fluor^{™} 555, Alexa Fluor^{™} 568, Alexa Fluor^{™} 594, Alexa Fluor^{™} 610, Alexa Fluor^{™} 633, Alexa Fluor^{™} 635, Alexa Fluor^{™} 647, Alexa Fluor^{™} 660, Alexa Fluor^{™} 680, Alexa Fluor^{™} 700, Alexa Fluor^{™} 750, Alexa Fluor^{™} 790, and the like.

Examples of quencher moieties include, but are not limited to: a dark quencher, a Black Hole Quencher^{™} (BHQ^{™}) (e.g., BHQ-0, BHQ-1, BHQ-2, BHQ-3), a Qx1 quencher, an ATTO quencher (e.g., ATTO 540Q, ATTO 580Q, and ATTO 612Q), dimethylaminoazobenzenesulfonic acid (Dabsyl), Iowa Black RQ, Iowa Black FQ, IRDye QC-1, a QSY dye (e.g., QSY 7, QSY 9, QSY 21), AbsoluteQuencher, Eclipse, and metal clusters such as gold nanoparticles, and the like.

In some cases, a quencher moiety is selected from: a dark quencher, a Black Hole Quencher^{™} (BHQ^{™}) (e.g., BHQ-0, BHQ-1, BHQ-2, BHQ-3), a Qx1 quencher, an ATTO quencher (e.g., ATTO 540Q, ATTO 580Q, and ATTO 612Q), dimethylaminoazobenzenesulfonic acid (Dabsyl), Iowa Black RQ, Iowa Black FQ, IRDye QC-1, a QSY dye (e.g., QSY 7, QSY 9, QSY 21), AbsoluteQuencher, Eclipse, and a metal cluster.

Examples of an ATTO quencher include, but are not limited to: ATTO 540Q, ATTO 580Q, and ATTO 612Q. Examples of a Black Hole Quencher^{™}. (BHQ^{™}) include, but are not limited to: BHQ-0 (493 nm), BHQ-1 (534 nm), BHQ-2 (579 nm) and BHQ-3 (672 nm).

For examples of some detectable labels (e.g., fluorescent dyes) and/or quencher moieties, see, e.g., Bao et al., Annu Rev Biomed Eng. 2009;11:25-47; as well as U.S. Pat. Nos. 8,822,673 and 8,586,718; U.S. patent publications 20140378330, 20140349295, 20140194611, 20130323851, 20130224871, 20110223677, 20110190486,20110172420, 20060179585 and 20030003486; and international patent applications: WO200142505 and WO200186001 .

In some cases, cleavage of a labeled detector can be detected by measuring a colorimetric read-out. For example, the liberation of a fluorophore (e.g., liberation from a FRET pair, liberation from a quencher/fluor pair, and the like) can result in a wavelength shift (and thus color shift) of a detectable signal. Thus, in some cases, cleavage of a subject labeled detector ssDNA can be detected by a color-shift. Such a shift can be expressed as a loss of an amount of signal of one color (wavelength), a gain in the amount of another color, a change in the ration of one color to another, and the like.

In some cases, signal is detected using lateral flow chromatography. In a simple sandwich type of system, the sample is applied to a pad in the lateral flow device that acts as the first stage of the absorption process, and in some cases contains a filter, to ensure the accurate and controlled flow of the sample. The conjugate pad, which stores the conjugated labels and antibodies, will receive the sample. If the target is present, the immobilized conjugated antibodies and labels will bind to the target and continue to migrate along the test. As the sample moves along the device the binding reagents situated on the nitrocellulose membrane will bind to the target at the test line. A colored line will form and the density of the line will vary depending on the quantity of the target present. Some targets may require quantification to determine target concentration. This is where a rapid test can be combined with a reader to provide quantitative results.

In some cases, the methods are carried out with a reporter molecule that is detected via lateral flow. If the primary activator is present, the system is activated and an NCR occurs. The activated Cas proteins exhibit trans cleavage of a reporter that comprises a detectable signal. The reaction mixture is loaded onto a lateral flow device, and uncleaved reporter molecule flows to the control line whereas the part of any cleaved reporter comprising the detector flows past the control to the zone comprising the capture molecule to bind to the detector. In some cases, Milenia Genline HybriDetect 1 (TwistDx^{™}) dipsticks are used. For example, in some cases the step of measuring can include one or more of: gold nanoparticle-based detection (e.g., see Xu et al., (2017) Angew Chem Int Ed Engl.;46(19):3468-70; and Xia et al., (2010) Proc Natl Acad Sci U S A. Jun 15;107(24):10837-41), fluorescence polarization, colloid phase transition/dispersion (e.g., Baksh et al., (2004) Nature. Jan 8;427(6970): 139-41), electrochemical detection, semiconductor- based sensing (e.g., Rothberg et al., (2011) Nature Jul 20;475(7356):348-52; e.g., one could use a phosphatase to generate a pH change after ssDNA cleavage reactions, by opening 2' -3' cyclic phosphates, and by releasing inorganic phosphate into solution), and detection of a labeled detector ssDNA. The readout of such detection methods can be any convenient readout. Examples of possible readouts include but are not limited to: a measured amount of detectable fluorescent signal; a visual analysis of bands on a gel (e.g., bands that represent cleaved product versus uncleaved substrate), a visual or sensor-based detection of the presence or absence of a color (i.e., color detection method), and the presence or absence of (or a particular amount of) an electrical signal.

In some cases, the detectable signal that is measured is produced by the fluorescence -emitting dye pair. For example, in some cases, a subject method includes contacting a sample with a labeled detector ssDNA comprising a fluorescence resonance energy transfer (FRET) pair or a quencher/fluor pair, or both.

In some cases, a subject method includes contacting a sample with a labeled detector ssDNA comprising a FRET pair. In some cases, a subject method includes contacting a sample with a labeled detector ssDNA comprising a fluor/quencher pair. Fluorescence-emitting dye pairs comprise a FRET pair or a quencher/fluor pair. In both cases of a FRET pair and a quencher/fluor pair, the emission spectrum of one of the dyes overlaps a region of the absorption spectrum of the other dye in the pair. As used herein, the term "fluorescence-emitting dye pair" is a generic term used to encompass both a "fluorescence resonance energy transfer (FRET) pair" and a "quencher/fluor pair," both of which terms are discussed in more detail below. The term "fluorescence-emitting dye pair" is used interchangeably with the phrase "a FRET pair and/or a quencher/fluor pair." In some cases (e.g., when the detector ssDNA includes a FRET pair) the labeled detector ssDNA produces an amount of detectable signal prior to being cleaved, and the amount of detectable signal that is measured is reduced when the labeled detector ssDNA is cleaved. In some cases, the labeled detector ssDNA produces a first detectable signal prior to being cleaved (e.g., from a FRET pair) and a second detectable signal when the labeled detector ssDNA is cleaved (e.g., from a quencher/fluor pair). As such, in some cases, the labeled detector ssDNA comprises a FRET pair and a quencher/fluor pair. In some cases, the labeled detector ssDNA comprises a FRET pair. FRET is a process by which radiationless transfer of energy occurs from an excited state fluorophore to a second chromophore in close proximity. The range over which the energy transfer can take place is limited to approximately 10 nanometers (100 angstroms), and the efficiency of transfer is extremely sensitive to the separation distance between fluorophores. Thus, as used herein, the term "FRET" ("fluorescence resonance energy transfer"; also known as "Forster resonance energy transfer") refers to a physical phenomenon involving a donor fluorophore and a matching acceptor fluorophore selected so that the emission spectrum of the donor overlaps the excitation spectrum of the acceptor, and further selected so that when donor and acceptor are in close proximity (usually 10 nm or less) to one another, excitation of the donor will cause excitation of and emission from the acceptor, as some of the energy passes from donor to acceptor via a quantum coupling effect. Thus, a FRET signal serves as a proximity gauge of the donor and acceptor; only when they are in close proximity to one another is a signal generated. The FRET donor moiety (e.g., donor fluorophore) and FRET acceptor moiety (e.g., acceptor fluorophore) are collectively referred to herein as a "FRET pair". The donor-acceptor pair (a FRET donor moiety and a FRET acceptor moiety) is referred to herein as a "FRET pair" or a "signal FRET pair." Thus, in some cases, a subject labeled detector ssDNA includes two signal partners (a signal pair), when one signal partner is a FRET donor moiety and the other signal partner is a FRET acceptor moiety. A subject labeled detector ssDNA that includes such a FRET pair (a FRET donor moiety and a FRET acceptor moiety) will thus exhibit a detectable signal (a FRET signal) when the signal partners are in close proximity (e.g., while on the same RNA molecule), but the signal will be reduced (or absent) when the partners are separated. FRET donor and acceptor moieties (FRET pairs) will be known to one of ordinary skill in the art and any convenient FRET pair (e.g., any convenient donor and acceptor moiety pair) can be used. See: Bajar et al. (2016) Sensors (Basel). Sep 14; 16(9); and Abraham et al. (2015) PLoS One. Aug 3; 10(8):e0134436.

### Nucleic acid signal Amplifier

Any nucleic acid amplifier activator (also referred to as an "RNA-amplifier" (if RNA), "activator" or a "substrate") may be used in the NCR compositions (not part of the invention), systems and methods described herein. Prior to the detection by Cas12 or Cas13 of a target nucleic acid (*e.g.,* a coronavirus viral sequence), the activator of the systems described herein is not capable of binding to (activating the signal amplifier), for example, it is caged such that it cannot hybridize to (or activate) the signal amplifier. However, upon the activation of the target sensor in the presence of target sequence to be detected, the amplifier substrate is released and becomes available to hybridize to (and activate) the signal amplifier. Thus, the released substrate activates the signal amplifier and unleashes a second cascade of Cas enzyme activity to generate more detectable label, thereby generating an exponential signal in the same reaction.

Thus, the activator substrate comprises any sequence which activates the signal amplifier to become a non-specific nuclease capable of cleaving the reporter complex and releasing the detectable label. Non-limiting examples of suitable sequences include guide RNAs or their cognate activator nucleic acids (*e.g.,* RNA in the case of Cas13, and DNA in the case of Cas12).

Any of the nucleotide sequences described herein may comprise one or more modifications, e.g., a base modification, a backbone modification, a sugar modification, etc., to provide the nucleic acid with a new or enhanced feature (e.g., improved stability). As is known in the art, a nucleoside is a base-sugar combination. The base portion of the nucleoside is normally a heterocyclic base. The two most common classes of such heterocyclic bases are the purines and the pyrimidines. Nucleotides are nucleosides that further include a phosphate group covalently linked to the sugar portion of the nucleoside. For those nucleosides that include a pentofuranosyl sugar, the phosphate group can be linked to the 2', the 3', or the 5' hydroxyl moiety of the sugar. In forming oligonucleotides, the phosphate groups covalently link adjacent nucleosides to one another to form a linear polymeric compound. In turn, the respective ends of this linear polymeric compound can be further joined to form a circular compound, however, linear compounds are generally suitable. In addition, linear compounds may have internal nucleotide base complementarity and may therefore fold in a manner as to produce a fully or partially double-stranded compound. Within oligonucleotides, the phosphate groups are commonly referred to as forming the internucleoside backbone of the oligonucleotide. The normal linkage or backbone of RNA and DNA is a 3' to 5' phosphodiester linkage. Additional nucleotide modifications, including modifications of backbones, internucleoside linkages, the use of mimetics, the use of locked nucleic acids (LNAs), and/or base modifications of substitutions such as the inclusion of one or more nucleobases are described in U.S. Publication No. 20190241954.

One or more of the nucleic acids described herein may also include one or more substituted sugar moieties.

One or more RNA amplifiers may be included in the reporter molecule, for example as part of the sequence linking the detectable label and quenching moiety of the reporter complex and/or distal to one or both of the detectable label and/or quencher. In some cases, one or more amplifier activators are included between the detectable label and quencher. Alternatively, and or in addition to embodiments in which the reporter molecule comprises trigger substrate sequences, the triggers may be a separate component in the compositions (not part of the invention), systems and methods described herein.

The amplifier activators may include poly U or poly A sequences to allow for selective cleavage. Cas13 enzymes produce cleaved RNA fragments with 2',3'-cyclic phosphates, and a subset of them prefer to cleave at U. Thus, by using a substrate with the sequence, A₄-Uₙ, Cas13 generates A₄>P that activates the ribonuclease (RNase) activity of TtCsm6. Sequences with A₅-Uₙ and A₆-Uₙ have previously been used to couple Csm6 to Cas13 (Gootenberg et al. (2017) Science 356(6336):438-442), but these do not produce optimal activation of Csm6 enzymes which recognize A₄, like TtCsm6. In addition, no Csm6 enzyme has been demonstrated to work in a feed-forward loop that provide higher detection speed and sensitivity than existing nucleic acid detection methods.

In some embodiments, the guide RNAs and/or activator RNAs may be modified to allow conditional interaction with a Cas protein, such that interaction only occurs in the optimal time frame such as after detection of a target nucleic acid. There are many different mechanisms to enable conditional interaction including using cleavable antisense-DNA as a protector for gRNA activity (Jain et al, (2016) Angew Chem Int Ed Engl 55 (40) 12440-4); ligand-dependent RNA cleavage and deprotection (Ferry et al (2017) Nat Commun 8:14633) ligand-dependent recruitment of transcriptional activators to dCas (Maji et al (2017) Nat Chem Biol 13 (1):9-11) and small molecule-induced reassembly of the Cas:guide RNP complex (Kunert et al (2019) Nat Commun. 10(1):2127) and photocaged gRNA designs for the direct regulation of the interaction between RNP and dsDNA using light (Zhou et al (2020) Angew Chem Int Ed Engl doi: 10.1002/anie.201914575). Another approach relies on the unique cleavage preferences of different Cas enzymes. In some embodiments, activators are not perfect anti-sense matches to their cognate guide RNAs. In some embodiments, non-matching nucleotides are used to de-tune the interaction with the guide to make it less sensitive, and less likely to result in non-specific signal. In some embodiments, the non-matching nucleotides are introduced into 'wobble' positions within the complementary region.

In some embodiments, activator molecules comprise both RNA and DNA sequences.

In some cases, the activator RNA molecules are caged. Caging may be accomplished by any physical or chemical means. For example, the triggers may be caged using 5' and 3' sequence extensions that can modulate critical features for Cas enzyme binding or cleavage. In this regard, the selectivity of Cas12 and Cas13 non-specific nuclease activity can be exploited for single-stranded nucleic acids by employing sequence extensions that can base-pair with the direct repeat of the guide RNA, the guide RNA spacer, the seed sequence (the sequence within the guide RNA known to be sensitive to mismatch with the activating sequence, see Abudayyeh et al (2016) Science 353: aaf5573) within the guide RNA spacer, and/or the cognate of the seed sequence in the activator nucleic acid. Such caging sequences may be linear or may be in loop or other formations. These 5' and 3' sequence extensions can be combined on the same caged substrate. Different combinations of sequence extensions can be used across the guide RNA and activator nucleic acid pair.

In some cases, the substrate cage can also be a separate molecule that base pairs with the guide RNA or activator. This cage can be released via endonuclease or exonuclease activity, such as Xrn1 or Csm6. In some embodiments, the cage can contain chemical modifications such as locked nucleic acid (LNA) moieties or 2'-OMe RNA as described herein. *See, also,* U.S. Publication No. 20190241954.

The substrates may be activated (uncaged) in any way. In some cases, the caging sequences (*e.g*., exposed loop regions) in the sequence extensions are designed to be specifically cleavable by distinct subtypes of the Cas protein (Cas13 or Cas12) causing the release of the cage and production of a functional guide RNA or activator. Profiling cleavage preferences of Cas12 and Cas13 proteins on homopolymer reporter substrates demonstrated that most orthologs preferred either uridine, a combination of bases, or adenine (East-Seletsky (2017) Mol Cell 66(3); 373-383; Gootenberg et al (2018) Science 360(6387): 439-444). For example, LmaCas13a, LbuCas13a and PprCas13a exhibited a strong preference for polyU while CcaCas13b exhibited a medium preference for polyU. Pin2Cas13b demonstrated a medium preference for polyU and for polyC. PbuCas13b and PguCas13b both showed a slight preference for poly U while LbaCas13a has a strong preference for polyA. Finally, BzoCas13b demonstrated a slight preference for polyU and polyA. Furthermore, some of the Cas13 orthologs tested showed differential activity dependent on ion concentration and/or guide RNA length. Thus, a cage structure may be added to a guide RNA such that when intact, the cage prevents the guide from interacting with its cognate target. When the cage is released, for example following trans cleavage by a Cas13a complex, the guide is free to interact with target, causing activation of the RNP complex. In some embodiments, the activator nucleic acid is a DNA wherein the DNA sequence comprises an RNA cage. In some embodiments, RNA and DNA activator nucleic acids are used, e.g. when using secondary amplifier complexes with Cas13 and Cas12 RNP complex amplifiers. The activated RNP complexes then cleave more caged substrates. Each additional activated Cas complex can activate more caged substrates, in addition to producing a fluorescent signal, leading to a feed-forward cycle of Cas-directed nuclease activity. As the reaction progresses, these enzymes are capable of cleaving still more detectable label, leading to an exponential amplification of signal.

In some embodiments, Csm6 is used to further amplify the detection reaction. In this system, protected guide RNA amplifiers comprising a poly-A stretch followed by a protecting poly-U stretch that could be cleaved by a uracil preferring Cas13 enzyme, such that the Cas13 would degrade all the uridines down to the homopolymeric A stretch in the guide. In some embodiments, a protected Csm6 activator RNA is used, comprising for example, a poly-A stretch followed by a protecting poly-U stretch that could be cleaved by a uracil preferring Cas13 enzyme, such that the Cas13 would degrade all the uridines down to the homoploymeric A stretch in guide. Protected guide RNA amplifiers and protected activator RNAs will have 2'3' cyclic phosphates following Cas13 cleavage and thus will activate Csm6. (A)₆ polynucleotides are known to activate Csm6, and both EiCsm6 and LsCsm6 were shown to amplify the detection signal following the initial binding of the target nucleic acid (Gootenberg et al (2018) Science 360(6387): 439-444).

Csm6 can also be used as the second enzyme that is activated by Cas13 to start a feed-forward loop. A caged activator for Csm6 would include four, five or six adenosines (A4, A5 or A6) at the 5' end of the sequence that may be modified to prevent premature cleavage (e.g. 2'-OMe, 2'-H, 2'-F) by either itself or Cas13. It would also have additional cleavable A's or U's at its 3' end that cage the substrate. Uncaging would be accomplished by trimming away the 3' RNA nucleotides to generate either A4>P, A5>P or A6>P by Cas13 to activate Csm6. The identity of the 3'-nucleotides used to cage the substrate can vary, depending on the nucleotide preference of the Cas enzyme used for uncaging. For example, one potential substrate could be 5'-fA-fA-fA-AAAAAAA-3'(SEQ ID NO:1), in which three fA nucleotides have the 2'-F modification, and the 5 terminal A's could be cleaved off by either Csm6 or LbaCas13.

In one embodiment of NCR amplification with Csm6, a Cas enzyme, LbaCas13, would sense complementary RNA, and become activated for trans RNA cleavage. It would then trim away adenosines at the 3' end of the caged Csm6 activator, leaving a modified A4>P, A5>P or A6>P that would bind to and activate a Csm6 enzyme. The activated Csm6 would then uncage additional caged activators, which would then lead to further activation of Csm6 molecules, triggering a feed-forward loop following the initial detection. The activated Csm6 molecules would also cleave the fluorescent reporter substrate, leading to a detectable signal. This strategy is different from previous uses of Csm6 with Cas13 (Gootenberg et al., 2018, Science*,* ibid), since the modified substrate enables a feed-forward loop with Csm6 that provides greater sensitivity.

This strategy can also be adapted to amplify primary RNA detection by U-cleaving Cas13 orthologs as well, by caging the activator with U's, instead of A's, at its 3' end (e.g. 5'-AAAAUUUUUU-3', SEQ ID NO:2). For example, LbuCas13 would cleave an A4-U6 caged activator to generate an A4>P. This uncaged substrate would activate TtCsm6, which would then uncage a second activator (e.g. 5'-fA-fA-fA-AAAAAAA-3', SEQ ID NO: 1) as well as cleave the reporter. This would initiate a feed-forward loop that amplifies the primary detection event. Modification of the 2'-OH of nucleotides comprising the activator sequence would be used to prevent degradation of the activator by Csm6.

Various versions of the activator may be tested, including non-modified versions or different numbers or sequences of caging nucleotides following the A4, A5 or A6 activator sequence. All approaches described could potentially apply to any Csm6 or Csx1 enzyme. This strategy could also be used to multiplex with other NCR pathways involving Cas13 or Cas12 to detect multiple RNA targets simultaneously, based on differences in the cleavage preference of the Cas enzymes.

In some embodiments, a Csm6 activator sequence is included within a cage sequence, for example, on a guide RNA (Figure 1I, top). Upon trans cleavage of the sequences on either side of the Csm6 activator sequence by Cas13 (e.g. LbuCas13a) that has been activated by its interaction with the primary activator RNA, the Csm6 sequence is released and able to interact with and activate Csm6. The activated Csm6 then is able to act on the caged guide RNA, making more available to complex with a Cas13 enzyme and then the primary activator to continue more detection of the primary activator. At the same time, the activated Csm6 also will cleave the reporter molecule and amplify the signal. In some embodiments, a caged activator RNA is used comprising the Csm6 activator sequence such that following cleavage by the activated Csm6, the uncaged RNA can interact with a Cas12-based amplification system. In some embodiments, both a caged guide comprising a Csm6 activator and a caged activator RNA comprising a Csm6 activator is used. All signal that is detected is dependent upon the initial interaction of the Cas13 with the primary activator.

In some embodiments, a protected Csm6-specific activator RNA is included in the system (Figure 1I, bottom). In this embodiment, the protected Csm6 activator is protected by nucleotides that are trimmed by Cas13 that has been activated through its interaction with the primary activator. The trimmed Csm6-specific activator RNA can now interact with and activate the Csm6 enzyme which can now act on molecules such as caged guide RNAs, caged activator RNAs and RNA reporter molecules to amplify the detection such that detection is dependent upon initial interaction of the Cas13 with the primary activator.

In some embodiments, a protected Csm6-specific activator RNA is included in the system (Figure 1J, top) and the Csm6 amplification serves as the sole amplifier (e.g. no additional Cas12 amplification step). In this embodiment, a protected Csm6-specific activator RNA is included in the system, where the protection comprises the use of poly A sequences where some of the As at the 5' end of the Csm6-specific activator comprise a 2'-F (fluorine). Cas13 enzymes likely use a classic metal-independent cleavage mechanism, and thus are not able to cleave As comprising the 2'-F (Gootenberg et al., (2018) *ibid;* Yang, W. (2011) Q. Rev. Biophys. 44, 1-93). The Cas13 that has been activated by its interaction with the primary activator then trims all the A nucleotides that comprise a 2'-OH except the As immediately 3' to the stretch of As comprising 2'F. This resultant trimmed sequence will have the 2'3' cyclic phosphate required for activation of Csm6. The activated Csm6 can now act on molecules such as caged guide RNAs and reporter molecules to amplify the detection such that detection is dependent upon initial interaction of the Cas13 with the primary activator. In some embodiments, the Csm6-specific activator RNA comprising the 5' As with 2'-F is used in systems comprising a secondary Cas12 amplification system.

In some embodiments, the activation of Csm6 is accomplished through the use of long polyA RNAs (Figure 1J, bottom). In this embodiment, activation of Csm6 following Cas13 cleavage of long polyA RNAs. These long polyA molecules are supplied in the reaction and cleaved by Cas13 that has been activated following interaction with the primary activator RNA such that Cas13's trans cleavage activity is active. The polyA RNAs will be cleaved at random lengths and will have 2'3' cyclic phosphates on the 3' end of the lengths. Some of those lengths will be the correct size (e.g. A4>P , A5>P or A6>P) to activate Csm6. The activated Csm6 enzyme can now act on molecules such as caged guide RNAs, caged activator RNAs and reporter molecules to amplify the detection such that detection is dependent upon initial interaction of the Cas13 with the primary activator. In some embodiments, a Csm6-specific protected activator as discussed above is also included such that once the correct length of polyA has stimulated Csm6, the activated Csm6 could then release a protected Csm6-specific activator RNA (described above) resulting in an amplification of signal.

In some cases, for example when using Cas13, the anti-tag sequence is included in the activator RNA, such as in the cleavable loop on the 3' end, to suppress background nuclease activity if Cas enzymes become activated without cleavage of the cage.

Signals generated from orthogonal targeting systems, such as Cas12 and Cas13, or distinct subfamilies of U-specific and A-specific cleaving Cas13 may be combined for signal transduction cascades. Logic gates, in which multiple distinct sequences need to be detected for amplification, can also be incorporated. Distinct Cas enzymes can also exhibit different nucleotide motif preferences, such as dinucleotide motifs, which can be incorporated for multiplexed signal readout.

In some cases, the activity of a Cas enzyme may be dampened, for example in order to lower background signal (which may lead to a false positive amplification). This may be accomplished by any suitable means, for example via the use of mutations that lower enzyme activity. Suitable mutations can be experimentally obtained using known techniques, may be commercially obtained or identified from the literature.

Loop Mediated Isothermal Amplification (LAMP) methodologies and/or nucleic acid amplification may also be employed. *See, e.g.,* Gadkar et al. (2018) Scientific Methods 8:5548; Notomi et al. (2006) Nucleic Acids Research 28(12):e63; Piepenburg et al. (2000) PLoS Biology 4(7):1115-1121. LAMP is a simple and accurate isothermal nucleic acid amplification technique that has found wide spread use in laboratory and point of care settings (Gadkar et al (2018) Sci Reports 8 (5548)). The technique is carried out at a single temperature (60-65°C) and is capable of producing approximately 50 times the amount of amplified product in a short amount of time (15 minutes). LAMP typically utilizes four primers (FIP (forward inner primer), BIP (backward inner primer), F3 (forward primer) and B3 (backward primer) to recognize six different regions of the target sequences. LAMP may be combined with a reverse transcriptase step for detecting RNA molecules (see Shen (2020) J Pharm Anal 10(2):97).

In some cases, the mismatch tolerant LAMP technique is used. Mismatch tolerant LAMP comprises the addition of a high-fidelity DNA polymerase to the reaction mixture which removes mismatches at the 3' end of the LAMP primers during amplification. Use of mismatch tolerant LAMP may be helpful when amplifying sequences from genetically diverse viral strains (Zhou et al (2019) Front Micro 10, art 1056).

Nucleic Acid Sequenced Based Amplification and Transcription Mediated Amplification (NASBA and TMA, respectively), are similar isothermal amplification techniques that proceed through RNA and may be used. NASBA utilizes two RNA target-specific primers and three enzymes (i.e., avian myeloblastosis virus reverse transcriptase, T7 DNA-dependent RNA polymerase (DdRp) and RNase H). The standard NASBA protocol for RNA amplification requires a 65 °C RNA incubation step to denature the target prior to the addition of enzymes. In the initiation phase, a specific forward primer (P1), that possesses a 5' sequence corresponding to the promoter of the T7 DdRp, hybridizes to any target RNA present in the sample and is extended by the reverse transcriptase. Subsequently, the RNA portion of the resulting RNA:DNA heteroduplex is degraded by RNase H, while a specific reverse primer (P2) hybridizes to the complementary sequence and is extended by the reverse transcriptase, leading to the formation of a dsDNA with the target sequence and a T7 promoter. Then, the T7 DdRp produces many RNA molecules that are complementary to the original target RNA. In the amplification phase, each newly synthesized RNA can be copied, resulting in an exponential amplification of RNA complimentary to the target.

Primers are designed to target a region of interest, but importantly, one primer includes the promoter sequence for T7 RNA polymerase at the 5' end. This enables production of single-stranded RNA species, which are reverse transcribed to cDNA by a reverse transcriptase included in the reaction. The RNA in the DNA-RNA hybrids is destroyed by RNase H activity (from an exogenous protein in NASBA, or by an RNase H+ RT in TMA) and dsDNA is produced by the RT. This template then gets transcribed to RNA by T7 RNAP and exponential amplification results (Compton (1991) Nature. 350 (6313): 91-2; United States Patent 5480784).

Strand Displacement Amplification (SDA) or Nicking Enzyme Amplification Reaction (NEAR) are two similar approaches that can be used for DNA amplification and may be used with the methods of the invention. Both techniques rely on a strand-displacing DNA polymerase, typically Bst DNA Polymerase, Large Fragment or Klenow Fragment (3'-5' exo-), to initiate at nicks created by a strand-limited restriction endonuclease or nicking enzyme at a site contained in a primer. The nicking site is regenerated with each polymerase displacement step, resulting in exponential amplification. NEAR is extremely rapid and sensitive, enabling detection of small target amounts in minutes (Van Ness et al (2003) Proc Natl Acad Sci USA 100(8):4504-9). SDA and NEAR are typically utilized in clinical and biosafety applications.

In some cases, Helicase-dependent amplification (HDA) is used. HDA exploits the activity of a DNA helicase to separate complementary strands of double strand (ds) DNAs thus avoiding the temperature cycling to produce single-stranded templates for primer hybridization and subsequent primer extension by a DNA polymerase. It mimics the replication fork and enables DNA synthesis in the presence of ATP when it loads on to the dsDNA template and traverses along the target DNA, disrupting the hydrogen bonds linking the two strands. Two sequence-specific primers hybridize to the 3'-end of each ssDNA template and DNA polymerases extend primers annealed to the target by producing dsDNA. The two newly synthesized dsDNA products act then as substrates for DNA helicases in the next round of the reaction, resulting in an exponential amplification of the selected target sequence (Jeong, Y-J. et al; (2009) Cell. Mol. Life Sci. 66, 3325-3336).

Recombinase polymerase amplification (RPA) is the isothermal amplification of specific DNA fragments achieved by the binding of opposing oligonucleotide primers to template or target DNA and their extension by a DNA polymerase. Global melting of the amplification template is not required for the primers to be directed to their complementary target sequences. Instead, RPA employs recombinase-primer complexes to scan the double-stranded DNA and facilitate strand exchange at cognate sites. The resulting structures are stabilized by single-stranded DNA binding proteins interacting with the displaced template strand, thus preventing the ejection of the primer by branch migration. Recombinase disassembly leaves the 3' end of the oligonucleotide accessible to a strand displacing DNA polymerase, in this case the large fragment of Bacillus subtilis Pol I (Bsu), and primer extension ensues. Exponential amplification is accomplished by the cyclic repetition of this process. Thus, in some embodiments, a preamplification of the target nucleic acid may be performed using LAMP, RPA or other isothermal amplification techniques including strand displacement amplification (SDA, Walker et al (1992) PNAS USA 89:392-396) and helicase-dependent amplification (Vincent et al (2004) EMBO Rep 5: 795-800).

Amplification techniques may be performed in microfluidic devices (Zanoli and Spoto (2013) Biosensors 3(1), 18-43).

In some embodiments, the system detection utilizes multiplexed guide RNAs to detect a target primary activator. Multiple guides that recognize different sections of a target nucleic acid primary activator may be used. In some embodiments, multiplexing can be done to recognize different target primary activators. Different Cas orthologs, Csm6 or Csx1 enzymes that cleave different nucleic acid substrates and/or multiplexed guides may be used with different fluorophore-quencher reporters for detection. This would allow identification of one or more primary activators in a single reaction. In some cases, the system is designed to detect multiple primary activators such that the presence or absence of multiple targets can be assayed. For example, one Cas/fluorophore combination may be used to detect one type of virus (e.g. influenza) while another may detect another type of virus (e.g. coronavirus), or the system may be used to detect the presence of a virus (e.g. coronavirus) as well as any co-infecting pathogens such as influenza A and B, respiratory syncytial virus (RSV), non-COVID-19 coronaviruses, adenovirus, parainfluenza 1 through 4, human metapneumovirus, rhinovirus/enterovirus, Chlamydia pneumoniae, and Mycoplasma pneumoniae (Kim et al (2020) JAMA doi:10.1001/jama.2020.6266).

### Target (primary activator) sequences

The source of the primary target sequence can be any source, including mammals, viruses, bacteria, and fungi. In some embodiments, the target sequence is a microbial or viral sequence, for example a coronavirus sequence such as COVID-19. In still other embodiments the target sequence is a mammalian genomic or transcribed sequence. In some embodiments, the source may be a human, non-human, or animal. In some embodiments, an animal source may be a domesticated or non-domestic animal, for example wild game. In some embodiments, the domesticated animal is a service or companion animal (e.g. a dog, cat, bird, fish, or reptile), or a domesticated farm animal.

For primary target sequences from pathogenic sources, the pathogen may have significant public health relevance, such as bacteria, fungus, or protozoan, and the target sequence may be found, without limitation, in one or more of coronavirus (*e.g*., severe acute respiratory syndrome-related coronavirus (SARS), Middle East respiratory syndrome-related coronavirus (MERS), COVID-19, etc.), Hepatitis C virus, Japanese Encephalitis, Dengue fever, or Zika virus. Any pathogen (*e.g.,* virus, bacteria, etc.) can be detected.

A primary target sequence can be single stranded (ss) or double stranded (ds) DNA or RNA (*e.g.,* viral RNA, mRNA, tRNA, rRNA, iRNA, miRNA, etc.). When the target sequence is single stranded, there is no preference or requirement for a PAM sequence in the target. However, when the target DNA is dsDNA, a PAM is usually present adjacent to the target sequence of the target DNA (e.g., see discussion of the PAM elsewhere herein). The source of the target DNA can be the same as the source of the sample, e.g., as described below.

In some cases, the primary target sequence is a viral sequence (e.g., a genomic RNA of an RNA virus or DNA of a DNA virus). As such, subject method can be for detecting the presence of a viral sequence amongst a population of nucleic acids (e.g., in a sample).

Non-limiting examples of possible primary RNA targets include viral RNAs such as coronavirus (SARS, MERS, SARS-CoV-2), Orthomyxoviruses, Hepatitis C Virus (HCV), Ebola disease, influenza, polio measles and retrovirus including adult Human T-cell lymphotropic virus type 1 (HTLV-1) and human immunodeficiency virus (HIV).

Non-limiting examples of possible target DNAs include, but are not limited to, viral DNAs such as: a papovavirus (e.g., human papillomavirus (HPV), polyomavirus); a hepadnavirus (e.g., Hepatitis B Virus (HBV)); a herpesvirus (e.g., herpes simplex virus (HSV), varicella zoster virus (VZV), epstein-barr virus (EBV), cytomegalovirus (CMV), herpes lymphotropic virus, Pityriasis Rosea, kaposi's sarcoma-associated herpesvirus); an adenovirus (e.g., atadenovirus, aviadenovirus, ichtadenovirus, mastadenovirus, siadenovirus); a poxvirus (e.g., smallpox, vaccinia virus, cowpox virus, monkeypox virus, orf virus, pseudocowpox, bovine papular stomatitis virus; tanapox virus, yaba monkey tumor virus; molluscum contagiosum virus (MCV)); a parvovirus (e.g., adeno-associated virus (AAV), Parvovirus B19, human bocavirus, bufavirus, human parv4 G1); Geminiviridae; Nanoviridae; Phycodnaviridae; and the like. In some cases, the target DNA is parasite DNA. In some cases, the target DNA is bacterial DNA, e.g., DNA of a pathogenic bacterium.

In some embodiments, the target nucleic acid is a DNA or RNA sequence associated with cancer. These can include genes that play a role in DNA methylation, histone modification, message splicing, and microRNA expression. Along with well known examples such as the so-called Philadelphia chromosome associated with chronic myeloid leukemia, in some embodiments, the target is a DNA associated with a translocation such as t(8;14)(q24:q32), t(2;8)(p12;q24), t(8;22)(q24;q11), t(8;14)(q24;q11), and t(8;12)(q24;q22), each associated with an alteration of C-Myc and associated with acute lymphocytic leukemia. Other examples include t(10;14)(q24;q32) which effects the LYT10 gene and is associated with B cell lymphoma (see Nambiar (2008) Biochim Biophys Acta 1786: 139-152). Other targets include mutant genes associated with cancers such as *BRCA2* (ovarian cancer), *BMP2,* 3, 4, 7 (endometrial cancer), *CAGE* (cervical cancer), *HOXA10* (ovarian cancer) and more (see Jeong et al (2014) Front Oncol 4(12*)).*

In some cases, the methods of the invention are used to examine other disorders that display an altered transcriptional state. Examples include diabetes, metabolic syndrome (Hawkins et al (2018) Peer J 6; e5062), Huntington syndrome and other neurological diseases (Xiang et al, (2018) Front Mol Neurosci 11: 153) and cancer. In some cases, the methods and compositions (not part of the invention) are used to monitor response to a therapy administered for the treatment of a disorder characterized by an altered transcriptional state. In some cases, the methods and compositions (not part of the invention) are used to monitor altered transcriptional activity in a non-disease condition such as the onset of puberty, pregnancy or menopause.

### Samples

Any sample that includes nucleic acid (e.g., a plurality of nucleic acids) can be used in the compositions (not part of the invention), systems and methods described herein. The term "plurality" is used herein to mean two or more. Thus, in some cases a sample includes two or more (e.g., 3 or more, 5 or more, 10 or more, 20 or more, 50 or more, 100 or more, 500 or more, 1,000 or more, or 5,000 or more) nucleic acids (e.g., RNAs or DNAs). A subject method can be used as a very sensitive way to detect a target sequence present in a sample (e.g., in a complex mixture of nucleic acids such as RNAs or DNAs). In some cases, the sample includes 5 or more RNAs or DNAs (e.g., 10 or more, 20 or more, 50 or more, 100 or more, 500 or more, 1,000 or more, or 5,000 or more RNAs or DNAs) that differ from one another in sequence. In some cases, the sample includes 10 or more, 20 or more, 50 or more, 100 or more, 500 or more, 10³ or more, 5x10³ or more, 10⁴ or more, 5x10⁴ or more, 10⁵ or more, 5x10⁵ or more, 10⁶ or more 5x10⁶ or more, or 10⁷ or more, RNAs or DNAs. In some cases, the sample comprises from 10 to 20, from 20 to 50, from 50 to 100, from 100 to 500, from 500 to 10³, from 10³ to 5x10³, from 5x10³ to 10⁴, from 10⁴ to 5x10⁴, from 5x10⁴ to 10⁵, from 10⁵ to 5x10⁵, from 5x10⁵ to 10⁶, from 10⁶ to 5x10⁶, or from 5x10⁶ to 10⁷, or more than 10⁷, RNAs or DNAs. In some cases, the sample comprises from 5 to 10⁷ RNAs or DNAs (e.g., that differ from one another in sequence)(e.g., from 5 to 10⁶, from 5 to 10⁵, from 5 to 50,000, from 5 to 30,000, from 10 to 10⁶, from 10 to 10.sup.5, from 10 to 50,000, from 10 to 30,000, from 20 to 10⁶, from 20 to 10⁵, from 20 to 50,000, or from 20 to 30,000 RNAs or DNAs). In some cases, the sample includes 20 or more RNAs or DNAs that differ from one another in sequence. In some cases, the sample includes RNAs or DNAs from a cell lysate (e.g., a eukaryotic cell lysate, a mammalian cell lysate, a human cell lysate, a prokaryotic cell lysate, a plant cell lysate, and the like). For example, in some cases the sample includes RNA or DNA from a cell such as a eukaryotic cell, e.g., a mammalian cell such as a human cell.

Suitable samples include but are not limited to saliva, blood, serum, plasma, urine, aspirate, and biopsy samples. Thus, the term "sample" with respect to a patient encompasses blood and other liquid samples of biological origin, solid tissue samples such as a biopsy specimen or tissue cultures or cells derived therefrom and the progeny thereof. The definition also includes samples that have been manipulated in any way after their procurement, such as by treatment with reagents; washed; or enrichment for certain cell populations, such as cancer cells. The definition also includes sample that have been enriched for particular types of molecules, e.g., DNAs. The term "sample" encompasses biological samples such as a clinical sample such as blood, plasma, serum, aspirate, cerebral spinal fluid (CSF), and also includes tissue obtained by surgical resection, tissue obtained by biopsy, cells in culture, cell supernatants, cell lysates, tissue samples, organs, bone marrow, and the like. A "biological sample" includes biological fluids derived therefrom (e.g., cancerous cell, infected cell, etc.), e.g., a sample comprising DNAs that is obtained from such cells (e.g., a cell lysate or other cell extract comprising DNAs).

A sample can comprise, or can be obtained from, any of a variety of cells, tissues, organs, or acellular fluids. Suitable sample sources include eukaryotic cells, bacterial cells, and archaeal cells. Suitable sample sources include single-celled organisms and multi-cellular organisms. Suitable sample sources include single-cell eukaryotic organisms; a plant or a plant cell; an algal cell, e.g., *Botryococcus braunii, Chlamydomonas reinhardtii, Nannochloropsis gaditana, Chlorella pyrenoidosa, Sargassum patens, C. agardh,* and the like; a fungal cell (e.g., a yeast cell); an animal cell, tissue, or organ; a cell, tissue, or organ from an invertebrate animal (e.g. fruit fly, cnidarian, echinoderm, nematode, an insect, an arachnid, etc.); a cell, tissue, fluid, or organ from a vertebrate animal (e.g., fish, amphibian, reptile, bird, mammal); a cell, tissue, fluid, or organ from a mammal (e.g., a human; a non-human primate; an ungulate; a feline; a bovine; an ovine; a caprine; etc.). Suitable sample sources include nematodes, protozoans, and the like. Suitable sample sources include parasites such as helminths, malarial parasites, etc.

Suitable sample sources include a cell, tissue, or organism of any of the six kingdoms, e.g., Bacteria (e.g., Eubacteria); Archaebacteria; Protista; Fungi; Plantae; and Animalia. Suitable sample sources include plant-like members of the kingdom Protista, including, but not limited to, algae (e.g., green algae, red algae, glaucophytes, cyanobacteria); fungus-like members of Protista, e.g., slime molds, water molds, etc; animal-like members of Protista, e.g., flagellates (e.g., Euglena), amoeboids (e.g., amoeba), sporozoans (e.g., Apicomplexa, Myxozoa, Microsporidia), and ciliates (e.g., Paramecium). Suitable sample sources include members of the kingdom Fungi, including, but not limited to, members of any of the phyla: Basidiomycota (club fungi; e.g., members of Agaricus, Amanita, Boletus, Cantherellus, etc.); Ascomycota (sac fungi, including, e.g., Saccharomyces); Mycophycophyta (lichens); Zygomycota (conjugation fungi); and Deuteromycota. Suitable sample sources include members of the kingdom Plantae, including, but not limited to, members of any of the following divisions: Bryophyta (e.g., mosses), Anthocerotophyta (e.g., hornworts), Hepaticophyta (e.g., liverworts), Lycophyta (e.g., club mosses), Sphenophyta (e.g., horsetails), Psilophyta (e.g., whisk ferns), Ophioglossophyta, Pterophyta (e.g., ferns), Cycadophyta, Gingkophyta, Pinophyta, Gnetophyta, and Magnoliophyta (e.g., flowering plants). Suitable sample sources include members of the kingdom Animalia, including, but not limited to, members of any of the following phyla: Porifera (sponges); Placozoa; Orthonectida (parasites of marine invertebrates); Rhombozoa; Cnidaria (corals, anemones, jellyfish, sea pens, sea pansies, sea wasps); Ctenophora (comb jellies); Platyhelminthes (flatworms); Nemertina (ribbon worms); Ngathostomulida (jawed worms)p Gastrotricha; Rotifera; Priapulida; Kinorhyncha; Loricifera; Acanthocephala; Entoprocta; Nemotoda; Nematomorpha; Cycliophora; Mollusca (mollusks); Sipuncula (peanut worms); Annelida (segmented worms); Tardigrada (water bears); Onychophora (velvet worms); Arthropoda (including the subphyla: Chelicerata, Myriapoda, Hexapoda, and Crustacea, where the Chelicerata include, e.g., arachnids, Merostomata, and Pycnogonida, where the Myriapoda include, e.g., Chilopoda (centipedes), Diplopoda (millipedes), Paropoda, and Symphyla, where the Hexapoda include insects, and where the Crustacea include shrimp, krill, barnacles, etc.; Phoronida; Ectoprocta (moss animals); Brachiopoda; Echinodermata (e.g. starfish, sea daisies, feather stars, sea urchins, sea cucumbers, brittle stars, brittle baskets, etc.); Chaetognatha (arrow worms); Hemichordata (acorn worms); and Chordata. Suitable members of Chordata include any member of the following subphyla: Urochordata (sea squirts; including Ascidiacea, Thaliacea, and Larvacea); Cephalochordata (lancelets); Myxini (hagfish); and Vertebrata, where members of Vertebrata include, e.g., members of Petromyzontida (lampreys), Chondrichthyces (cartilaginous fish), Actinopterygii (ray-finned fish), Actinista (coelocanths), Dipnoi (lungfish), Reptilia (reptiles, e.g., snakes, alligators, crocodiles, lizards, etc.), Ayes (birds); and Mammalian (mammals) Suitable plants include any monocotyledon and any dicotyledon.

Suitable sources of a sample include cells, fluid, tissue, or organ taken from an organism; from a particular cell or group of cells isolated from an organism; etc. For example, where the organism is a plant, suitable sources include xylem, the phloem, the cambium layer, leaves, roots, etc. Where the organism is an animal, suitable sources include particular tissues (e.g., lung, liver, heart, kidney, brain, spleen, skin, fetal tissue, etc.), or a particular cell type (e.g., neuronal cells, epithelial cells, endothelial cells, astrocytes, macrophages, glial cells, islet cells, T lymphocytes, B lymphocytes, etc.).

In some cases, the source of the sample is a (or is suspected of being a diseased cell, fluid, tissue, or organ, for example of a human subject. In some cases, the source of the sample is a normal (non-diseased) cell, fluid, tissue, or organ. In some cases, the source of the sample is a (or is suspected of being a pathogen-infected cell, tissue, or organ. For example, the source of a sample can be an individual who may or may not be infected--and the sample could be any biological sample (e.g., blood, saliva, biopsy, plasma, serum, bronchoalveolar lavage, sputum, a fecal sample, cerebrospinal fluid, a fine needle aspirate, a swab sample (e.g., a buccal swab, a cervical swab, a nasal swab), interstitial fluid, synovial fluid, nasal discharge, tears, buffy coat, a mucous membrane sample, an epithelial cell sample (e.g., epithelial cell scraping), etc.) collected from the individual. In some cases, the sample is a cell-free liquid sample. In some cases, the sample is a liquid sample that can comprise cells.

Pathogens to be detected in samples include viruses, bacteria, fungi, helminths, protozoa, malarial parasites, Plasmodium parasites, Toxoplasma parasites, Schistosoma parasites, and the like. "Helminths" include roundworms, heartworms, and phytophagous nematodes (Nematoda), flukes (Tematoda), Acanthocephala, and tapeworms (Cestoda). Protozoan infections include infections from Giardia spp., Trichomonas spp., African trypanosomiasis, amoebic dysentery, babesiosis, balantidial dysentery, Chaga's disease, coccidiosis, malaria and toxoplasmosis. Examples of pathogens such as parasitic/protozoan pathogens include, but are not limited to: *Plasmodium falciparum, Plasmodium vivax, Trypanosoma cruzi and Toxoplasma gondii.* Fungal pathogens include, but are not limited to: Cryptococcus neoformans, Histoplasma capsulatum, Coccidioides immitis, Blastomyces dermatitidis, Chlamydia trachomatis, and Candida albicans. Pathogenic viruses include, e.g., coronaviruses (e.g., COVID-19, MERS, SARS, etc.); immunodeficiency virus (e.g., HIV); influenza virus; dengue; West Nile virus; herpes virus; yellow fever virus; Hepatitis Virus C; Hepatitis Virus A; Hepatitis Virus B; papillomavirus; and the like. Pathogenic viruses can include DNA viruses such as: a papovavirus (e.g., human papillomavirus (HPV), polyomavirus); a hepadnavirus (e.g., Hepatitis B Virus (HBV)); a herpesvirus (e.g., herpes simplex virus (HSV), varicella zoster virus (VZV), epstein-barr virus (EBV), cytomegalovirus (CMV), herpes lymphotropic virus, Pityriasis Rosea, kaposi's sarcoma-associated herpesvirus); an adenovirus (e.g., atadenovirus, aviadenovirus, ichtadenovirus, mastadenovirus, siadenovirus); a poxvirus (e.g., smallpox, vaccinia virus, cowpox virus, monkeypox virus, orf virus, pseudocowpox, bovine papular stomatitis virus; tanapox virus, yaba monkey tumor virus; molluscum contagiosum virus (MCV)); a parvovirus (e.g., adeno-associated virus (AAV), Parvovirus B19, human bocavirus, bufavirus, human parv4 G1); Geminiviridae; Nanoviridae; Phycodnaviridae; and the like. Pathogens can include, e.g., DNAviruses [e.g.: a papovavirus (e.g., human papillomavirus (HPV), polyomavirus); a hepadnavirus (e.g., Hepatitis B Virus (HBV)); a herpesvirus (e.g., herpes simplex virus (HSV), varicella zoster virus (VZV), epstein-barr virus (EBV), cytomegalovirus (CMV), herpes lymphotropic virus, Pityriasis Rosea, kaposi's sarcoma-associated herpesvirus); an adenovirus (e.g., atadenovirus, aviadenovirus, ichtadenovirus, mastadenovirus, siadenovirus); a poxvirus (e.g., smallpox, vaccinia virus, cowpox virus, monkeypox virus, orf virus, pseudocowpox, bovine papular stomatitis virus; tanapox virus, yaba monkey tumor virus; molluscum contagiosum virus (MCV)); a parvovirus (e.g., adeno-associated virus (AAV), Parvovirus B19, human bocavirus, bufavirus, human parv4 G1); Geminiviridae; Nanoviridae; Phycodnaviridae; and the like], Mycobacterium tuberculosis, Streptococcus agalactiae, methicillin-resistant Staphylococcus aureus, Legionella pneumophila, Streptococcus pyogenes, Escherichia coli, Neisseria gonorrhoeae, Neisseria meningitidis, Pneumococcus, Cryptococcus neoformans, Histoplasma capsulatum, Hemophilus influenzae B, Treponema pallidum, Lyme disease spirochetes, Pseudomonas aeruginosa, Mycobacterium leprae, Brucella abortus, rabies virus, influenza virus, cytomegalovirus, herpes simplex virus I, herpes simplex virus II, human serum parvo-like virus, respiratory syncytial virus, varicella-zoster virus, hepatitis B virus, hepatitis C virus, measles virus, adenovirus, human T-cell leukemia viruses, Epstein-Barr virus, murine leukemia virus, mumps virus, vesicular stomatitis virus, Sindbis virus, lymphocytic choriomeningitis virus, wart virus, blue tongue virus, Sendai virus, feline leukemia virus, Reovirus, polio virus, simian virus 40, mouse mammary tumor virus, dengue virus, rubella virus, West Nile virus, Plasmodium falciparum, Plasmodium vivax, Toxoplasma gondii, Trypanosoma rangeli, *Trypanosoma cruzi, Trypanosoma rhodesiense, Trypanosoma brucei, Schistosoma mansoni, Schistosoma japonicum, Babesia bovis, Eimeria tenella, Onchocerca volvulus, Leishmania tropica, Mycobacterium tuberculosis, Trichinella spiralis, Theileria parva, Taenia hydatigena, Taenia ovis, Taenia saginata, Echinococcus granulosus, Mesocestoides corti, Mycoplasma arthritidis, M. hyorhinis, M. orale, M. arginini, Acholeplasma laidlawii, M. salivarium* and *M. pneumoniae.*

### Methods

Thus, methods of the invention include (a) contacting a sample potentially including the target sequence with: (i) any of the compositions (not part of the invention) or systems as described herein and (b) measuring a detectable signal, thereby detecting the target sequence (DNA or RNA).

In some cases, the methods comprise contacting a target sensor comprising one or more Cas-effector enzymes programmed with one or more guide RNAs that recognize the desired target nucleic sequence(s) in the sample (e.g., viral DNA or RNA) such that the target sensor is activated into a non-specific nuclease (e.g., non-specific RNase when the target sensor comprises a Cas13 effector protein or non-specific DNase when the target sensor comprises a Cas12 effector protein). In certain cases, the target sensor comprises one Cas-effector protein and one guide RNA. The methods also comprise contacting the activated target sensor (non-specific nuclease) with a reporter molecule, which comprises a detectable label and an amplifier activator sequence, in which the detectable label is masked (quenched) and the amplifier activator molecule is caged (unavailable for hybridization) prior to cleavage by the non-specific nuclease. Upon cleavage, both the detectable label (e.g., fluorescent label) and amplifier activator molecules are released. Subsequently, the released amplifier activator binds to (hybridizes to) a guide RNA of a signal amplifier comprising a Cas-effector protein programmed and the guide RNA, activating an additional non-specific nuclease capable of cleaving the reporter molecule and releasing the detectable label and the amplifier activator molecule from the reporter complex. The methods also comprise measuring the detectable label and, optionally quantifying the levels.

The contacting steps and measuring steps may be performed in the same or different containers and in liquid and/or solid supports. For example, the contacting may be performed in the same container and transferred for detection or, alternatively, the contacting and measuring steps may be performed in the same container.

The assay mixture may be incubated under various conditions to allow a target nucleic acid sequence, if present in the sample, to hybridize to the guide RNA. In some embodiments, the conditions are designed to aid in hybridization of RNA sequences, wherein the sequences are 100% complementary. In other embodiments, the conditions for incubation of the assay mixture may be varied to allow for less than 100% complementarity between the guide RNA sequence and the target sequence, for example 1 mismatch between target nucleic acid and guide RNA, or less than about 2 mismatches, 3 mismatches, 4 mismatches, or 5 mismatches. In some embodiments, hybridization between a target RNA and a guide RNA may activate non-specific RNase activity of a Cas-effector protein, when complementarity is greater than about 80%.

The contacting step of a subject methods can be carried out in a composition comprising divalent metal ions. The contacting step can be carried out in an acellular environment, e.g., outside of a cell. The contacting step can be carried out inside a cell. The contacting step can be carried out in a cell in vitro. The contacting step can be carried out in a cell ex vivo. The contacting step can be carried out in a cell in vivo.

The contacting step may be for any length of time, including but not limited to 2 hours or less (e.g., 1.5 hours or less, 1 hour or less, 40 minutes or less, 30 minutes or less, 20 minutes or less, 10 minutes or less, or 5 minutes or less, or 1 minute or less) prior to the measuring step. For example, in some cases the sample is contacted for 40 minutes or less prior to the measuring step. In some cases, the sample is contacted for 20 minutes or less prior to the measuring step. In some cases, the sample is contacted for 10 minutes or less prior to the measuring step. In some cases, the sample is contacted for 5 minutes or less prior to the measuring step. In some cases, the sample is contacted for 1 minute or less prior to the measuring step. In some cases, the sample is contacted for from 50 seconds to 60 seconds prior to the measuring step. In some cases, the sample is contacted for from 40 seconds to 50 seconds prior to the measuring step. In some cases, the sample is contacted for from 30 seconds to 40 seconds prior to the measuring step. In some cases, the sample is contacted for from 20 seconds to 30 seconds prior to the measuring step. In some cases, the sample is contacted for from 10 seconds to 20 seconds prior to the measuring step. In some embodiments, the sample is incubated with the Cas protein for less than about 2 hrs., 90 min., 60 min., 40 min., 30 min., 20 min., 10 min., 5 min., 4 min., 3 min., 2 min., 1 min., 55 sec., 50 sec., 40 sec., 30 sec., 20 sec., or 10 sec., and more than about 5 sec., 10 sec., 20 sec., 30 sec., 40 sec., 50 sec., 60 sec., 2 min., 3 min., 4 min., 5 min., 10 min., 20 min., 30 min., 40 min., 50 min., 60 min., or 90 min.

The method may be conducted at any temperature, including from about 30°C to about 30°C (or any temperature therebetween). In some embodiments, the assays (methods) are conducted at a physiological temperature, for example about 37°C. This allows the methods to be readily practiced in any location, including a doctor's office or home (for example by performing the assay using body temperature (*e.g.,* holding the assay contained under the arm, against the skin, etc.). In some embodiments, the assays (methods) are conducted at 60-65°C.

The methods described herein can detect the target sequence (RNA or DNA) with a high degree of sensitivity. In some cases, a method of the present disclosure can be used to detect a target sequence present in a sample comprising a plurality of nucleotides (including the target sequence and a plurality of non-target sequences), where the target sequence is present at one or more copies per 10⁷ non-target sequences (e.g., one or more copies per 10⁶ non-target sequences, one or more copies per 10⁵ non-target sequences, one or more copies per 10⁴ non-target sequences, one or more copies per 10³ non-target sequences, one or more copies per 10² non-target sequences, one or more copies per 50 non-target sequences, one or more copies per 20 non-target sequences, one or more copies per 10 non-target sequences, or one or more copies per 5 non-target sequences). In some cases, a method of the present disclosure can be used to detect a target sequences present in a sample comprising a plurality of sequences (including the target sequences and a plurality of non-target sequences), where the target sequence is present at one or more copies per 10¹⁸ non-target sequences (e.g., one or more copies per 10¹⁵ non-target sequences, one or more copies per 10¹² non-target sequences, one or more copies per 10⁹ non-target sequences, one or more copies per 10⁶ non-target sequences, one or more copies per 10⁵ non-target sequences, one or more copies per 10⁴ non-target sequences, one or more copies per 10³ non-target sequences, one or more copies per 10² non-target sequences, one or more copies per 50 non-target sequences, one or more copies per 20 non-target sequences, one or more copies per 10 non-target sequences, or one or more copies per 5 non-target sequences).

In some cases, a method of the present disclosure can detect a target sequence (DNA or RNA) present in a sample, where the target sequences is present at from one copy per 10⁷ non-target sequences to one copy per 10 non-target sequences (e.g., from 1 copy per 10⁷ non-target sequences to 1 copy per 10² non-target sequences, from 1 copy per 10⁷ non-target sequences to 1 copy per 10³ non-target sequences, from 1 copy per 10⁷ non-target sequences to 1 copy per 10⁴ non-target sequences, from 1 copy per 10⁷ non-target sequences to 1 copy per 10⁵ non-target sequences, from 1 copy per 10⁷ non-target sequences to 1 copy per 10⁶ non-target sequences, from 1 copy per 10⁶ non-target sequences to 1 copy per 10 non-target sequences, from 1 copy per 10.sup.6 non-target sequences to 1 copy per 10² non-target sequences, from 1 copy per 10⁶ non-target sequences to 1 copy per 10³ non-target sequences, from 1 copy per 10⁶ non-target sequences to 1 copy per 10⁴ non-target sequences, from 1 copy per 10⁶ non-target sequences to 1 copy per 10⁵ non-target sequences, from 1 copy per 10⁵ non-target sequences to 1 copy per 10 non-target sequences, from 1 copy per 105 non-target sequences to 1 copy per 10² non-target sequences, from 1 copy per 10⁵ non-target sequences to 1 copy per 10³ non-target sequences, or from 1 copy per 10⁵ non-target sequences to 1 copy per 10⁴ non-target sequences).

In some cases, a method of the present disclosure can detect a target sequence (RNA or DNA) present in a sample, where the target sequences is present at from one copy per 10¹⁸ non-target sequences to one copy per 10 non-target sequences (e.g., from 1 copy per 10¹⁸ non-target sequences to 1 copy per 10² non-target sequences, from 1 copy per 10¹⁵ non-target sequences to 1 copy per 10² non-target sequences, from 1 copy per 10¹² non-target sequences to 1 copy per 10² non-target sequences, from 1 copy per 10⁹ non-target sequences to 1 copy per 10² non-target sequences, from 1 copy per 10⁷ non-target sequences to 1 copy per 10² non-target sequences, from 1 copy per 10⁷ non-target sequences to 1 copy per 10³ non-target sequences, from 1 copy per 10⁷ non-target sequences to 1 copy per 10⁴ non-target sequences, from 1 copy per 10⁷ non-target sequences to 1 copy per 10⁵ non-target sequences, from 1 copy per 10⁷ non-target sequences to 1 copy per 10⁶ non-target sequences, from 1 copy per 10⁶ non-target sequences to 1 copy per 10 non-target sequences, from 1 copy per 10⁶ non-target sequences to 1 copy per 10.sup.2 non-target sequences, from 1 copy per 10.sup.6 non-target sequences to 1 copy per 10³ non-target sequences, from 1 copy per 10⁶ non-target sequences to 1 copy per 10⁴ non-target sequences, from 1 copy per 10⁶ non-target sequences to 1 copy per 10⁵ non-target sequences, from 1 copy per 10⁵ non-target sequences to 1 copy per 10 non-target sequences, from 1 copy per 10⁵ non-target sequences to 1 copy per 10² non-target sequences, from 1 copy per 10⁵ non-target sequences to 1 copy per 10³ non-target sequences, or from 1 copy per 10⁵ non-target sequences to 1 copy per 10⁴ non-target sequences).

In some cases, a method of the present disclosure can detect a target sequence (RNA or DNA) present in a sample, where the target sequence is present at from one copy per 10⁷ non-target sequences to one copy per 100 non-target sequences (e.g., from 1 copy per 10⁷ non-target sequences to 1 copy per 10² non-target sequences, from 1 copy per 10⁷ non-target sequences to 1 copy per 10³ non-target sequences, from 1 copy per 10⁷ non-target sequences to 1 copy per 10⁴ non-target sequences, from 1 copy per 10⁷ non-target sequences to 1 copy per 10⁵ non-target sequences, from 1 copy per 10⁷ non-target sequences to 1 copy per 10⁶ non-target sequences, from 1 copy per 10⁶ non-target sequences to 1 copy per 100 non-target sequences, from 1 copy per 10⁶ non-target sequences to 1 copy per 10² non-target sequences, from 1 copy per 10⁶ non-target sequences to 1 copy per 10³ non-target sequences, from 1 copy per 10⁶ non-target sequences to 1 copy per 10⁴ non-target sequences, from 1 copy per 10⁶ non-target sequences to 1 copy per 10⁵ non-target sequences, from 1 copy per 10⁵ non-target sequences to 1 copy per 100 non-target sequences, from 1 copy per 10⁵ non-target sequences to 1 copy per 10² non-target sequences, from 1 copy per 10⁵ non-target sequences to 1 copy per 10³ non-target sequences, or from 1 copy per 10⁵ non-target sequences to 1 copy per 10⁴ non-target sequences).

In some cases, the threshold of detection, for a subject method of detecting a target sequence (RNA or DNA) in a sample, is 10 nM or less. The term "threshold of detection" is used herein to describe the minimal amount of target sequence that must be present in a sample in order for detection to occur. Thus, as an illustrative example, when a threshold of detection is 10 nM, then a signal can be detected when a target sequence is present in the sample at a concentration of 10 nM or more. In some cases, a method of the present disclosure has a threshold of detection of 5 nM or less. In some cases, a method of the present disclosure has a threshold of detection of 1 nM or less. In some cases, a method of the present disclosure has a threshold of detection of 0.5 nM or less. In some cases, a method of the present disclosure has a threshold of detection of 0.1 nM or less. In some cases, a method of the present disclosure has a threshold of detection of 0.05 nM or less. In some cases, a method of the present disclosure has a threshold of detection of 0.01 nM or less. In some cases, a method of the present disclosure has a threshold of detection of 0.005 nM or less. In some cases, a method of the present disclosure has a threshold of detection of 0.001 nM or less. In some cases, a method of the present disclosure has a threshold of detection of 0.0005 nM or less. In some cases, a method of the present disclosure has a threshold of detection of 0.0001 nM or less. In some cases, a method of the present disclosure has a threshold of detection of 0.00005 nM or less. In some cases, a method of the present disclosure has a threshold of detection of 0.00001 nM or less. In some cases, a method of the present disclosure has a threshold of detection of 10 pM or less. In some cases, a method of the present disclosure has a threshold of detection of 1 pM or less. In some cases, a method of the present disclosure has a threshold of detection of 500 fM or less. In some cases, a method of the present disclosure has a threshold of detection of 250 fM or less. In some cases, a method of the present disclosure has a threshold of detection of 100 fM or less. In some cases, a method of the present disclosure has a threshold of detection of 50 fM or less. In some cases, a method of the present disclosure has a threshold of detection of 500 aM (attomolar) or less. In some cases, a method of the present disclosure has a threshold of detection of 250 aM or less. In some cases, a method of the present disclosure has a threshold of detection of 100 aM or less. In some cases, a method of the present disclosure has a threshold of detection of 50 aM or less. In some cases, a method of the present disclosure has a threshold of detection of 10 aM or less. In some cases, a method of the present disclosure has a threshold of detection of 1 aM or less.

In some cases, the threshold of detection (for detecting the target sequence in a subject method), is in a range of from 500 fM to 1 nM (e.g., from 500 fM to 500 pM, from 500 fM to 200 pM, from 500 fM to 100 pM, from 500 fM to 10 pM, from 500 fM to 1 pM, from 800 fM to 1 nM, from 800 fM to 500 pM, from 800 fM to 200 pM, from 800 fM to 100 pM, from 800 fM to 10 pM, from 800 fM to 1 pM, from 1 pM to 1 nM, from 1 pM to 500 pM, from 1 pM to 200 pM, from 1 pM to 100 pM, or from 1 pM to 10 pM) (where the concentration refers to the threshold concentration of target sequence at which the target sequence can be detected). In some cases, a method of the present disclosure has a threshold of detection in a range of from 800 fM to 100 pM. In some cases, a method of the present disclosure has a threshold of detection in a range of from 1 pM to 10 pM. In some cases, a method of the present disclosure has a threshold of detection in a range of from 10 fM to 500 fM, e.g., from 10 fM to 50 fM, from 50 fM to 100 fM, from 100 fM to 250 fM, or from 250 fM to 500 fM.

In some cases, the minimum concentration at which a target sequence (DNA or RNA) can be detected in a sample is in a range of from 500 fM to 1 nM (e.g., from 500 fM to 500 pM, from 500 fM to 200 pM, from 500 fM to 100 pM, from 500 fM to 10 pM, from 500 fM to 1 pM, from 800 fM to 1 nM, from 800 fM to 500 pM, from 800 fM to 200 pM, from 800 fM to 100 pM, from 800 fM to 10 pM, from 800 fM to 1 pM, from 1 pM to 1 nM, from 1 pM to 500 pM, from 1 pM to 200 pM, from 1 pM to 100 pM, or from 1 pM to 10 pM). In some cases, the minimum concentration at which a target sequence can be detected in a sample is in a range of from 800 fM to 100 pM. In some cases, the minimum concentration at which a target sequence can be detected in a sample is in a range of from 1 pM to 10 pM.

In some cases, the threshold of detection (for detecting the target sequences), is in a range of from 1 aM to 1 nM (e.g., from 1 aM to 500 pM, from 1 aM to 200 pM, from 1 aM to 100 pM, from 1 aM to 10 pM, from 1 aM to 1 pM, from 100 aM to 1 nM, from 100 aM to 500 pM, from 100 aM to 200 pM, from 100 aM to 100 pM, from 100 aM to 10 pM, from 100 aM to 1 pM, from 250 aM to 1 nM, from 250 aM to 500 pM, from 250 aM to 200 pM, from 250 aM to 100 pM, from 250 aM to 10 pM, from 250 aM to 1 pM, from 500 aM to 1 nM, from 500 aM to 500 pM, from 500 aM to 200 pM, from 500 aM to 100 pM, from 500 aM to 10 pM, from 500 aM to 1 pM, from 750 aM to 1 nM, from 750 aM to 500 pM, from 750 aM to 200 pM, from 750 aM to 100 pM, from 750 aM to 10 pM, from 750 aM to 1 pM, from 1 fM to 1 nM, from 1 fM to 500 pM, from 1 fM to 200 pM, from 1 fM to 100 pM, from 1 fM to 10 pM, from 1 fM to 1 pM, from 500 fM to 500 pM, from 500 fM to 200 pM, from 500 fM to 100 pM, from 500 fM to 10 pM, from 500 fM to 1 pM, from 800 fM to 1 nM, from 800 fM to 500 pM, from 800 fM to 200 pM, from 800 fM to 100 pM, from 800 fM to 10 pM, from 800 fM to 1 pM, from 1 pM to 1 nM, from 1 pM to 500 pM, from 1 pM to 200 pM, from 1 pM to 100 pM, or from 1 pM to 10 pM) (where the concentration refers to the threshold concentration of target sequence at which the target sequence can be detected). In some cases, a method of the present disclosure has a threshold of detection in a range of from 1 aM to 800 aM. In some cases, a method of the present disclosure has a threshold of detection in a range of from 50 aM to 1 pM. In some cases, a method of the present disclosure has a threshold of detection in a range of from 50 aM to 500 fM.

In some cases, the minimum concentration at which a target sequence can be detected in a sample is in a range of from 1 aM to 1 nM (e.g., from 1 aM to 500 pM, from 1 aM to 200 pM, from 1 aM to 100 pM, from 1 aM to 10 pM, from 1 aM to 1 pM, from 100 aM to 1 nM, from 100 aM to 500 pM, from 100 aM to 200 pM, from 100 aM to 100 pM, from 100 aM to 10 pM, from 100 aM to 1 pM, from 250 aM to 1 nM, from 250 aM to 500 pM, from 250 aM to 200 pM, from 250 aM to 100 pM, from 250 aM to 10 pM, from 250 aM to 1 pM, from 500 aM to 1 nM, from 500 aM to 500 pM, from 500 aM to 200 pM, from 500 aM to 100 pM, from 500 aM to 10 pM, from 500 aM to 1 pM, from 750 aM to 1 nM, from 750 aM to 500 pM, from 750 aM to 200 pM, from 750 aM to 100 pM, from 750 aM to 10 pM, from 750 aM to 1 pM, from 1 fM to 1 nM, from 1 fM to 500 pM, from 1 fM to 200 pM, from 1 fM to 100 pM, from 1 fM to 10 pM, from 1 fM to 1 pM, from 500 fM to 500 pM, from 500 fM to 200 pM, from 500 fM to 100 pM, from 500 fM to 10 pM, from 500 fM to 1 pM, from 800 fM to 1 nM, from 800 fM to 500 pM, from 800 fM to 200 pM, from 800 fM to 100 pM, from 800 fM to 10 pM, from 800 fM to 1 pM, from 1 pM to 1 nM, from 1 pM to 500 pM, from 1 pM to 200 pM, from 1 pM to 100 pM, or from 1 pM to 10 pM). In some cases, the minimum concentration at which a target sequence can be detected in a sample is in a range of from 1 aM to 500 pM. In some cases, the minimum concentration at which a target sequence can be detected in a sample is in a range of from 100 aM to 500 pM.

In some cases, a subject composition (not part of the invention) or method exhibits an attomolar (aM) sensitivity of detection. In some cases, a subject composition or method exhibits a femtomolar (fM) sensitivity of detection. In some cases, a subject composition (not part of the invention) or method exhibits a picomolar (pM) sensitivity of detection. In some cases, a subject composition (not part of the invention) or method exhibits a nanomolar (nM) sensitivity of detection.

The measuring can in some cases be quantitative, e.g., in the sense that the amount of signal detected can be used to determine the amount of target sequence present in the sample. The measuring can in some cases be qualitative, e.g., in the sense that the presence or absence of detectable signal can indicate the presence or absence of targeted sequence (e.g., virus, SNP, etc.). In some cases, a detectable signal will not be present (e.g., above a given threshold level) unless the targeted sequences(s) (e.g., virus, SNP, etc.) is present above a particular threshold concentration. In some cases, the threshold of detection can be titrated by modifying the amount of Cas effector protein of the system (e.g., sensor or amplifier), guide RNA, sample volume, and/or detector (if one is used). As such, for example, as would be understood by one of ordinary skill in the art, a number of controls can be used if desired in order to set up one or more reactions, each set up to detect a different threshold level of target sequence, and thus such a series of reactions could be used to determine the amount of target sequence present in a sample (e.g., one could use such a series of reactions to determine that a target sequence is present in the sample 'at a concentration of at least X').

In some cases, a method of the present disclosure can be used to determine the amount of a target sequence (RNA or DNA) in a sample (e.g., a sample comprising the target sequence and a plurality of non-target sequences). Determining the amount of a target sequence in a sample can comprise comparing the amount of detectable signal generated from a test sample to the amount of detectable signal generated from a reference sample. Determining the amount of a target sequence in a sample can comprise: measuring the detectable signal to generate a test measurement; measuring a detectable signal produced by a reference sample to generate a reference measurement; and comparing the test measurement to the reference measurement to determine an amount of target sequence present in the sample.

RNase inhibitors may be used in the methods as described herein. In some embodiments, the assay mixture may include one or more molecules that inhibit non-Cas13a-dependent RNase activity, but do not affect RNase activity by activated Cas13a proteins. For example, the inhibitor may inhibit mammalian, bacterial, or viral RNases, such as, without limitation, RNase A and RNase H. In some embodiments, the RNase Inhibitor may be added to the sample to help preserve a target nucleic acid sequence. In these embodiments, the method may include a step of adding one or more RNA preserving compounds to the sample, for example one or more RNase inhibitors.

Detecting the label may be achieved in various ways known in the art. For example, detection of colorimetric, fluorescent, or luminescent labels may be accomplished by measurement of absorbance or emission of light at a particular wavelength. In some embodiments the signal may be detected by visual inspection, microscope, or light detector.

### Kits

The present disclosure not forming part of the invention relates to provides a kit for detecting a target nucleotide sequences, e.g., in a sample comprising a plurality of sequences. In some cases, the kit comprises one or more NCR compositions or systems as described herein. Positive and/or negative controls may also be included and/or instructions for use may also be included.

### EXAMPLES

### Example 1: Nuclease Chain Reaction Detection of Coronavirus DNA

Experiments were conducted to compare detection without nuclease chain reaction (NCR) or with NCR systems as described herein using a synthetic target sequence ("Primary activator"). In particular, assays were conducted with NCR systems as described herein using a primary activator sequence detected by a primary activator complex triggering non-specific cleavage activity which leads to a fluorescent signal (see Figure 1A). In addition to activation of the primary activator complex, activation of a signal amplified complex can also occur when the non-specific cleavage activity releases a cage from a caged activator RNA (Figure 1B). In some cases, additional activation occurs via Csm6. In some cases, the primary activator complex is an RNP comprising a Cas13 protein (Figure 1C), while in other cases, the primary activator complex comprises a Cas12 protein (Figure 1D). When Cas12 is used in the primary activator approach, the primary activator nucleic acid may be subject to RT-LAMP if the primary activator molecule is RNA. Either of these systems may include the use of Csm6 to boost signal. A caged activator for Csm6 would include four or six adenosines (A4 or A6) at the 5' end of the sequence that may be modified to prevent premature cleavage (e.g. 2'-OMe, 2'-H, 2'-F) by either itself or Cas13. The Csm6 activator also has additional cleavable A's or U's at its 3' end that cage the substrate. Uncaging would be accomplished by trimming away the 3' RNA nucleotides to generate either A4>P or A6>P to activate Csm6. The identity of the 3'-nucleotides used to cage the substrate can vary, depending on the nucleotide preference of the Cas enzyme used for uncaging. For example, one potential substrate could be 5'-fA-fA-fA-AAAAAAA-3' (SEQ ID NO: 1), in which fA nucleotides have the 2'-F modification, and the 6 terminal A's could be cleaved by either Csm6 or LbaCas13.

In some cases, the primary activator complex is an RNP comprising a Cas13 protein and a guide specific for the primary activator RNA, while the signal amplification complex also comprises an RNP comprising a Cas13 protein and a guide specific for an activator RNA (Figure 1E). In some cases, the primary activator complex is an RNP comprising a Cas12 protein and a guide specific for the primary activator RNA which is made using RT-LAMP. The signal activation complex also comprises a Cas12 protein and a guide specific for the activator DNA (Figure 1F). In either case, Csm6 amplification may also be utilized.

In some cases, the primary activation complex comprises a Cas13, and caged guide RNAs are used. In some cases, the cage is on the 3' end of the guide molecule, wherein in other cases, the cage is on the 5' end of the guide (Figure 1E). In some cases, the activator RNA is caged (Figure 1F, strategy 1) while in other cases, the activator RNA and/or the amplifier guide is caged (Figure 1F, strategy 2). In all cases, cages may be present on the 5', 3' or 5' and 3' ends of the molecules. Cages may also be used on nucleic acid activators used with Csm6 amplification.

Various activator RNAs were tested in the presence or absence of primary activator. In addition, assays without NCR capability were also conducted in experiments lacking the activator RNA sequences and the primary activator complex ("No secondary" in Figure 2). In these experiments, the primary activator and the activator RNA sequences were the same, sequence R004 below in Table 2A in the control experiments. The activator RNAs tested all contained the same sequence linked to differing caging sequences to determine the cage structure that would result in the best induction of the amplification pathway while preventing activation in the absence of primary activator. Sequences used in this experiment are presented below in Table 2A. Note: the designation "-nc" identifies nucleic acids lacking any cage structure.

**Table 2A: Synthetic target, detector and amplifier nucleic acids**

| Role | Name | Sequence (5'-3') | SEQ ID NO |
|---|---|---|---|
| Primary activator-nc | R004 | AAAAGCCUGAACCACCAGGCUAUAUCUG | 3 |
| Primary guide RNA-nc | R010 | auuuagaccaccccaaaaaugaaggggacuaaaacaCAGAUAUAGCCUGGUGGUUCAGGC | 4 |
| Activator guide RNA-nc | NCR_034 | UAGACCACCCCAAAAAUGAAGGGGACUAAAACUUGUUUCUUUCUGUUGUUUC | 5 |
| Activator RNA | NCR_042 | GAC GAG AGA CCG ACA CAA Aa aUUg GC C GAAA GGC cc CACA cA CaUGU GUC GGU CUC UCG | 6 |
| Activator RNA | NCR_045 | GAC GAG AGA CCG ACA CAA Aa aUUg GGCUUCGGCC cc CACA cA CaUGU GUC GGU CUC UCG | 7 |
| Activator RNA-nc | NCR_057 | GAAACAACAGAAAGAAACAaCUU | 8 |
| Activator RNA | NCR_058 | GAAACAACAGAAAGAAACAaCUU GCA AGA AAC AUC UGU UGU UUC | 9 |
| Activator RNA | NCR_059 | GAAACAACAGAAAGAAACAaCUU GCA AGA UCU UUC UGUUGU | 10 |
| Activator RNA | NCR_060 | GAAACAACAGAAAGAAACAaCUU GCA AUU UCU UUC UGUUGUU | 11 |
| Activator RNA | NCR_061 | GAAACAACAGAAAGAAACAaCUU GCA AUU UCU UUC UGUUGUUUC | 12 |
| Activator RNA | NCR_062 | GAAACAACAGAAAGAAACAa aUUg GC C GAAA GGC cc CACAcA CaUUU CUG UUG | 13 |
| Activator RNA | NCR_063 | GAAACAACAGAAAGAAACAa aUUg GC C GAAA GGC cc CACAcA CaGUU UCU UUC UGU UGU UU | 14 |
| Activator RNA | NCR_064 | GAAACAACAGAAAGAAACAa aUUg GC C GAAA GGC cc CACAcA CaUU GUU UCU UUC UGU UGU UUC | 15 |
| Activator RNA | NCR_065 | GAAACAACAGAAAGAAACAa aUUg GGCUUCGGCC cc CACAcA CaUUU CUG UUG | 16 |
| Activator RNA | NCR_066 | GAAACAACAGAAAGAAACAa aUUg GGCUUCGGCC cc CACAcA CaGUU UCU UUC UGU UGU UU | 17 |
| Activator RNA | NCR_067 | GAAACAACAGAAAGAAACAa aUUg GGCUUCGGCC cc CACAcA CaUU GUU UCU UUC UGU UGU UUC | 18 |
| Activator RNA | NCR_193 | GAA ACA ACA GAA AGA AAC Aa GUU UCA AUU UCU UUC UGU UGU UUC | 19 |

For all reactions indicated, 1x buffer: 20mM HEPES (pH 6.8), 50mM KCL, 100ug/ml BSA, 0.01% Igepal. Integrated Detection Technologies (IDT) synthesized guide RNAs and activator RNAs were assembled in 5µM reactions using 1x buffer, and folded by boiling at 95°C for 3min in a thermocycler, followed by immediate cooling on ice. Cas13-crRNA complexes targeting the primary activator, and amplifier RNA were assembled separately for 10min at room temperature in 1x buffer using a 2:1 protein to crRNA ratio, with 1µM Cas13, and 0.5µM crRNA. RNP complexes targeting caged amplifiers were then mixed with RNP targeting the primary activator, and further diluted using 1x buffer in the presence of 200nM RNase alert substrate (IDT DNA), 20nM amplifier RNA, and varying amounts of primary activator (1nM - 1aM). A final concentration of 50nM RNP targeting the primary activator, and 1nM-50nM RNP targeting the amplifier RNA was used. Reactions were incubated in a fluorescence plate reader for up to 120min at 37°C, with fluorescence measurements taken every 30s (λex: 485nm; λem: 535nm).

As shown in Figure 2, for among the different activator RNAs evaluated, NCR_061 showed the greatest differential between signal in the presence of activator and signal without activator. Furthermore, the NCR systems showed a differential increased the detectable signal from the synthetic target sequence as compared to the signal in the experiments lacking the primary activator. NCR_45 and NCR_42 showed some differential in signal between signal in the absence of amplification ("Primary alone") and when both activation and amplification processes were occurring ("Cage+Primary"). NCR_67 did not display any differential signal. NCR_061 gave a rapid amplification when both primary and amplification were present ("NCR061_amp") that was separated from the data observable from primary signal only ("Primary Act only"). Use of the NCR_061 amplification pathway only ("NCR061 cage only") gave signal which was delayed as compared to activation + amplification.

Figure 3A-3D shows results of experiments using four activator RNAs tested over time, (NCR_42, NCR_45, NCR_61 and NCR_67) showed a range of different results (Figure 3).

Further optimization of signal was done by varying the concentration of primary activation RNP complex added (Figure 4A). Measurements over time showed rapid signal onset when 200 pM of primary activation complex was used in this experiment (Figure 4B). At the end time point, the signal detected for primary activator only and primary plus amplification using the NCR showed similar signal (Figure 4C) but when the measurements were taken over time, the experiment with primary + NCR showed a more rapid rise of signal (Figure 4D).

Activator RNAs comprising an anti-TAG sequence were also tested. An anti-TAG sequence was included in NCR_061 such that signal from the primary activation and activator-RNA- alone driven signal is much less than the primary activation + NCR (Figure 5A-5E).

Activator RNAs based on the NCR_061 sequence comprising alternative caging structures were designed and tested. The NCR_176 activator RNA comprises 3x more polyU sequences and the NCR_177 comprises 6x more polyU sequences. In these experiments, Cas12 was used in the primary sensing complex while Cas13 was used in the amplification complex. LbuCas13a was used for the Cas13 component, and the increased polyU sequences were included for potentially more recognition and cleavage by its trans RNase activity. The results (Figure 5A-5E) show an increase in signal using the caged activators that are more easily released by LbuCas13a.

An NCR reaction testing use of another caged amplifier crRNA was also performed. Sequences for the uncaged and caged guides as well as other sequences tested are shown below in Table 2B.

**Table 2B: Nucleic Acids used**

| Role | Name | Sequence (5'-3') | SEQ ID NO |
|---|---|---|---|
| Amplifier crRNA | NCR_009-nc | UAGACCACCCCAAAAAUGAAGGGGACUAAAACGCGAGAACAGGACGAAGCAG | 20 |
| Amplifier crRNA | NCR_0018 | UAGACCACCCCAAAAUGAAGGGGACUAAAACGAAACAACAGAAAGAAACAAGUUAUACAU ACUUUCUGUUGUU | 21 |
| Amplifier crRNA | NCR_265 | UAGACCACCCCAAAAUGAAGGGGACUAAAACGAAACAACAGAAAGAAACAAGUUAUACAU ACUUUCUGUUGUUU | 22 |
| Amplifier crRNA | NCR_266 | UAGACCACCCCAAAAAUGAAGGGGACUAAAACGAAACAACAGAAAGAAACAAGUUAUACAU ACUUUCUGUUGUUUCGU | 23 |
| Amplifier crRNA | NCR_267 | UAGACCACCCCAAAAUGAAGGGGACUAAAACGAAACAACAGAAAGAAACAAGUUAUACAU ACUUCCUGUUGUCUCGU | 24 |
| Amplifier RNA-nc | NCR_035 | GAC GAG AGA CCA ACA CAA Aa | 25 |
| Amplifier RNA | NCR_036 | GAC GAG AGA CCG ACA CAA AaCUU GGA CUU UUG UGU CGG UC | 26 |
| Amplifier RNA | NCR_038 | GAC GAG AGA CCG ACA CAA AaCUU GGA CAC UGUCGG UCUCU | 27 |
| Amplifier RNA | NCR_039 | GAC GAG AGA CCG ACA CAA AaCUU GGA CAG UGUCGG UCUCUC | 28 |
| Trans cage | NCR_268 | GUUUUAGUCC UUUUU CCUUCAUUUU | 39 |
| Trans cage | NCR_269 | UUCAUUUUUG UUUUU GGGUGGUCUA | 30 |
| Trans cage | NCR_270 | GUUUUAGUCCCCUUCAUU UUUUU UUUGGGGUGGUCUA | 31 |
| Trans cage | NCR_271 | UUUUGUGUUG UUUUU GUCUCUCGUC | 32 |

The use of the caged amplifier crRNA NCR_018 demonstrated a boost in signal as compared with the uncaged amplifier crRNA NCR_009 (see Figure 6A-6C). When background signal (signal in the absence of primary activator and caged amplifier crRNA only signal) is subtracted, a large increase in detection is observed.

Further, in an effort to reduce background signal observed in the presence caged amplifier crRNA and Cas13 alone, various alternative caging structures were tested. Exemplary results are shown in Figure 7, where some improvement of background subtracted signal was observed.

The size of the loop in the caged structure was also tested. Caged crRNAs comprising loops with different melting temperatures were tested (see Figure 8). The results demonstrated that those cage structures with the lowest melting temperature showed the most rapid signal generation. For example, NCR_038 comprises a 14 nucleotide single stranded loop with a melting temperature of 34°C and demonstrated similar signal kinetics as the uncaged activator RNA (NCR_035). NCR_039 (12 nucleotide loop) and NCR_036 (12 nucleotide loop) demonstrated slower signal kinetics in this experiment.

Trans cages were also tested in the system. Several trans cages (see Table 2B) were synthesized for Cas13a crRNAs that comprised different single stranded regions for potential cleavage by trans nuclease activity of an activated Cas complex. The trans cages were constructed to have the single stranded loops occur in different locations when complexed with the crRNAs (see Figure 9A). The trans age molecules were then tested in varying ratios with respect to the crRNA used (e.g. 20:1, 2:1, 1:1, and 1:2 ratios of trans cage: crRNA). The results demonstrated that in ratios of 2:1, 1:1, and 1:2, background signal was still detected, so inhibition of non-specific background signal was only detected at a ratio of 20:1 in this experiment (see Figure 9B).

### Example 2:

A Cas13-guide RNA complex recognizes target ssRNA sequence, activating it for RNA cleavage. The target ssRNA is a sequence specific for SARS-Co-V2. Also included are experiments run with a target ssRNA that is pan-coronavirus specific ("Pan-corona"). Examples of these sequences are shown below in Table 3. Also shown are sequences of the SARS-CoV-2 N and E genes that may be used to identify specific target sequences:

**Table 3: Coronavirus specific target nucleic acids**

| Specificity | Sequence | SEQ ID NO |
|---|---|---|
| SARS-Co-V2 | UGUAUGGAAAAGUUAUGUGC | 33 |
| SARS-Co-V2 | GCAGAUAGUAAAAUUGUUCA | 34 |
| SARS-CoV2 | AGACUUCAUUAAGAUGUGGU | 35 |
| Pan-corona | AUGGGUUGGGAUUAUCCUAA | 36 |
| Pan-corona | UGGGAUUAUCCUAAAUGUGA | 37 |
| Pan-corona | GGGAUUAUCCUAAAUGUGAU | 38 |
| SARS-CoV-2* | gaaatTAATACGACTCACTATAgggGTGAGTTTAAATTGGCTTCACAT ATGTATTGTTCTTTCTACCCTCCAGATGAGGATGAAGAAGAAGGTGAT TGTGAAGAAGAAGAGTTTGAGCCATCAACTCAATATGAGTATGGTACT GAAGATGATTACCAAGGTAAACCTTTGGAATTTGGTGCCACTTCTGCT GCTCTTCAACCTGAAGAAGAGCAAGAAGAAGATTGGTTAGATGATGAT AGTCAACAAACTGTTGGTCAACAAGACGGCAGTGAGGACAATCAGACA ACTACTATTCAAACAATTGTTGAGGTTCAACCTCAATTAGAGATGGAA CTTACACCAGTTGTTCAGACTATTGAAGTGAATAGTTTTAGTGGTTAT | 39 |
| | TTAAAACTTACTGACAATGTATACATTAAAAATGCAGACATTGTGGAA GAAGCTAAAAAGGTAAAACCAACAGTGGTTGTTAATGCAGCCAATGTT TACCTTAAACATGGAGGAGG | |
| SARs-CoV-2 N gene** | CCAAATTGGCTACTACCGAAGAGCTACCAGACGAATTCGTGGTGGTGACGGTAAAATGAAAG ATCTCAGTCCAAGATGGTATTTCTACTACCTAGGAACTGGGCCAGAAGCTGGACTTCCCTATG GTGCTAACAAAGACGGCATCATATGGGTTGCAACTGAGGGAGCCTTGAATACACCAAAAGAT CACATTGGCACCCGCAATCCTGCTAACAATGCTGCAATCGTGCTACAACTTCCTCAAGGAACA ACATTGCCAAAAGGCTTCTACGCAGAAGGGAGCAGAGGCGGCAGTCAAGCCTCTTCTCGTTC CTCATCACGTAGTCGCAACAGTTCAAGAAATTCAACTCCAGGCAGCAGTAGGGGAACTTCTC CTGCTAGAATGGCTGGCAATGGCGGTGATGCTGCTCTTGCTTTGCTGCTGCTTGACAGATTGA ACCAGCTTGAGAGCAAAATGTCTGGTAAAGGCCAACAACAACAAGGCCAAACTGTCACTAAGA AATCTGCTGCTGAGGCTTCTAAGAAGCCTCGGCAAAAACGTACTGCCACTAAAGCATACAATG TAACACAAGCTTTCGGCAGACGTGGTCCAGAACAAACCCAAGGAAATTTTGGGGACCAGGAAC TAATCAGACAAGGAACTGATTACAAACATTGGCCGCAAATTGCACAATTTGCCCCCAGCGCTT CAGCGTTCTTCGGAATGTCGCGCATTGGCATGGAAGTCACACCTTCGGGAACGTGGTTGACCT ACACAGGTGCCATCAAATTGGATGACAAAGATCCAAATTTCAAAGATCAAGTCATTTTGCTGA ATAAGCATATTGACGCATACAAAACATTCCCACCAACAGAGCCTAAAAAGGACAAAAAGAAGA AGGCTGATGAAACTCAAGCCTTACCGCAGAGACAGAAGAAACAGCAAACTGTG | 40 |
| SARS CoV-2 E gene ** | ACTATTACCAGCTGTACTCAACTCAATTGAGTACAGACACTGGTGTTGAACATGTTACCTTC TTCATCTACAATAAAATTGTTGATGAGCCTGAAGAACATGTCCAAATTCACACAATCGACGG TTCATCCGGAGTTGTTAATCCAGTAATGGAACCAATTTATGATGAACCGACGACGACTACTA GCGTGCCTTTGTAAGCACAAGCTGATGAGTACGAACTTATGTACTCATTCGTTTCGGAAGAG ACAGGTACGTTAATAGTTAATAGCGTACTTCTTTTTCTTGCTTTCGTGGTATTCTTGCTAGT TACACTAGCCATCCTTACTGCGCTTCGATTGTGTGCGTACTGCTGCAATATTGTTAACGTGA GTCTTGTAAAACCTTCTTTTTACGTTTACTCTCGTGTTAAAAATCTGAATTCTTCTAGAGTT CCTGATCTTCTGGTCTAAACGAACTAAATATTATATTAGTTTTTCTGTTTGGAACTTTAATT TTAGCCATGGCAGATTCCAACGGTACTATTACCGTTGAAGAGCTTAAAAAGCTCCTTGAACA ATGGAACCTAGTAATAGGTTTCCTATTCCTTACATGGATT | 41 |

| | | |
|---|---|---|
| * from Metsky et al (2020) BioRxiv doi.org/10.1101/2020.02.26.967026. ** from Broughton et al (2020) Nature doi.org/10.1038/s41587-020-0513-4 For other SARS Co-V-2 target sequences, see Abbott et al (2020) BioRxiv doi.org/10.1101/2020.03.13.991307; Rauch et al (2020) BioRxiv doi.org/10.1101/2020.04.20.052159. | | |

Guide RNAs (crRNAs) are prepared for use with Cas13a that target the SARS-CoV-2 genome. Exemplary guide sequences are shows below in Table 4.

**Table 4: Guide RNAs for use with Cas13a**

| Use | Name | Sequence 5'-3' | SEQ ID NO |
|---|---|---|---|
| Cas13a crRNA targeting SARS-Cov-2 | NCR_273 | UAGACCACCCCAAAAAUGAAGGGGACUAAAACAAUUUGA UGGCACCUGUGUA | 42 |
| Cas13a crRNA targeting SARS-Cov-2 | NCR_274 | UAGACCACCCCAAAAUGAAGGGACUAAAACUAGAUCU GUGUGGCCAACCU | 43 |
| Cas13a crRNA targeting SARS-Cov-2 | NCR_275 | UAGACCACCCCAAAAAUGAAGGGGACUAAAACGCACCAGC UGUCCAACCUGA | 44 |
| Cas13a crRNA targeting SARS-Cov-2 | NCR_276 | UAGACCACCCCAAAAAUGAAGGGGACUAAAACAUAAUAA GCUGCAGCACCAG | 45 |
| Cas13a crRNA targeting SARS-Cov-2 | NCR_277 | UAGACCACCCCAAAAAUGAAGGGGACUAAAACUAACCCAC AUAAUAAGCUGC | 46 |
| Cas13a crRNA targeting SARS-Cov-2 | NCR_278 | UAGACCACCCCAAAAAUGAAGGGGACUAAAACAGAUAAC CCACAUAAUAAGC | 47 |
| Cas13a crRNA targeting SARS-Cov-2 | NCR_279 | UAGACCACCCCAAAAUGAAGGGACUAAAACGAAGAUA ACCCACAUAAUAA | 48 |
| Cas13a crRNA targeting SARS-Cov-2 | NCR_280 | UAGACCACCCCAAAAAUGAAGGGACUAAAACUGAAGAU AACCCACAUAAUA | 49 |
| Cas13a crRNA targeting SARS-Cov-2 | NCR_281 | UAGACCACCCCAAAAAUGAAGGGACUAAAACUUGAAGA UAACCCACAUAAU | 50 |
| Cas13a crRNA targeting SARS-Cov-2 | NCR_282 | UAGACCACCCCAAAAUGAAGGGACUAAAACGUUGAAG AUAACCCACAUAA | 51 |
| Cas13a crRNA targeting SARS-Cov-2 | NCR_283 | UAGACCACCCCAAAAAUGAAGGGACUAAAACCUCACCAC UACGACCGUACU | 52 |
| Cas13a crRNA targeting SARS-Cov-2 | NCR_284 | UAGACCACCCCAAAAAUGAAGGGACUAAAACACAUGAG GGACAAGGACACC | 53 |
| Cas13a crRNA targeting SARS-Cov-2 | NCR_285 | UAGACCACCCCAAAAAUGAAGGGACUAAAACAUCAUCU UCAGUACCAUACU | 54 |
| Cas13a crRNA targeting SARS-Cov-2 | NCR_286 | UAGACCACCCCAAAAAUGAAGGGGACUAAAACAAGUGGC ACCAAAUUCCAAA | 55 |
| Cas13a crRNA targeting SARS-Cov-2 | NCR_287 | UAGACCACCCCAAAAAUGAAGGGGACUAAAACUAUCAUC AUCUAACCAAUCU | 56 |
| Cas13a crRNA targeting SARS-Cov-2 | NCR_288 | UAGACCACCCCAAAAAUGAAGGGACUAAAACUCCUCACU GCCGUCUUGUUG | 57 |
| Cas13a crRNA targeting SARS-Cov-2 | NCR_289 | UAGACCACCCCAAAAAUGAAGGGACUAAAACUAAGUUC CAUCUCUAAUUGA | 58 |
| Cas13a crRNA targeting SARS-Cov-2 | NCR _290 | UAGACCACCCCAAAAAUGAAGGGACUAAAACACUUCAG UACAUCAAACGAA | 59 |
| Cas13a crRNA targeting SARS-Cov-2 | NCR_291 | UAGACCACCCCAAAAUGAAGGGACUAAAACCAUUCCCU GCGCGUCCUCUG | 60 |
| Cas13a crRNA targeting SARS-Cov-2 | NCR_292 | UAGACCACCCCAAAAAUGAAGGGACUAAAACCGCAGGCA AGAUUAUCCAUU | 61 |
| Cas13a crRNA targeting SARS-Cov-2 | NCR_293 | UAGACCACCCCAAAAAUGAAGGGACUAAAACAAGUGAG GCCAUAAUUCUAA | 62 |
| Cas13a crRNA targeting SARS-Cov-2 | NCR_294 | UAGACCACCCCAAAAAUGAAGGGACUAAAACACCAUAG GGAAGUCCAGCUU | 63 |
| Cas13a crRNA targeting SARS-Cov-2 | NCR_295 | UAGACCACCCCAAAAAUGAAGGGACUAAAACCGUCUU UGUUAGCACCAUA | 64 |
| Cas13a crRNA targeting SARS-Cov-2 | NCR_296 | UAGACCACCCCAAAAAUGAAGGGACUAAAACACAUUCCG AAGAACGCUGAA | 65 |
| Cas13a crRNA targeting SARS-Cov-2 | NCR_297 | UAGACCACCCCAAAAAUGAAGGGGACUAAAACAUUUCUU GAACUGUUGCGAC | 66 |
| Cas13a crRNA targeting SARS-Cov-2 | NCR_298 | UAGACCACCCCAAAAAUGAAGGGGACUAAAACCCAUUGCC AGCCAUUCUAGC | 67 |
| Cas13a crRNA targeting SARS-Cov-2 | NCR_299 | UAGACCACCCCAAAAAUGAAGGGGACUAAAACGAGCAGCA UCACCGCCAUUG | 68 |
| Cas13a crRNA targeting SARS-Cov-2 | NCR_300 | UAGACCACCCCAAAAAUGAAGGGGACUAAAACUCAAGCA GCAGCAAAGCAAG | 69 |
| Cas13a crRNA targeting SARS-Cov-2 | NCR_301 | UAGACCACCCCAAAAAUGAAGGGGACUAAAACUUUGCCG AGGCUUCUUAGAA | 70 |
| Cas13a crRNA targeting SARS-Cov-2 | NCR_302 | UAGACCACCCCAAAAAUGAAGGGGACUAAAACGCUUGUG UUACAUUGUAUGC | 71 |
| Cas13a crRNA targeting SARS-Cov-2 | NCR_303 | UAGACCACCCCAAAAAUGAAGGGGACUAAAACACUUGAU CUUUGAAAUUUGG | 72 |
| Cas13a crRNA targeting SARS-Cov-2 | NCR_304 | UAGACCACCCCAAAAAUGAAGGGGACUAAAACCAGCAAA AUGACUUGAUCUU | 73 |
| Cas13a crRNA targeting SARS-Cov-2 | NCR_305 | UAGACCACCCCAAAAAUGAAGGGGACUAAAACGUUUCAU CAGCCUUCUUCUU | 74 |
| Cas13a crRNA targeting SARS-Cov-2 | NCR_306 | UAGACCACCCCAAAAAUGAAGGGGACUAAAACGCGGUAA GGCUUGAGUUUCA | 75 |
| Cas13a crRNA targeting SARS-Cov-2 | NCR_307 | UAGACCACCCCAAAAAUGAAGGGGACUAAAACGAAAUCA UCCAAAUCUGCAG | 76 |
| Cas13a crRNA targeting SARS-Cov-2 | NCR_308 | UAGACCACCCCAAAAAUGAAGGGGACUAAAACUAUAUCG UAAACGGAAAAGC | 77 |
| Cas13a crRNA targeting SARS-Cov-2 | NCR_309 | UAGACCACCCCAAAAAUGAAGGGGACUAAAACUAGAUCG GCGCCGUAACUAU | 78 |
| Cas13a crRNA targeting SARS-Cov-2 | NCR_310 | UAGACCACCCCAAAAAUGAAGGGGACUAAAACUCGUCGCC UAAGUCAAAUGA | 79 |
| Cas13a crRNA targeting SARS-Cov-2 | NCR_311 | UAGACCACCCCAAAAAUGAAGGGGACUAAAACCCAGUUU UCUUGAAAAUCUU | 80 |
| Cas13a crRNA targeting SARS-Cov-2 | NCR_312 | UAGACCACCCCAAAAAUGAAGGGGACUAAAACUAACACCA CUGCUAUGUUUA | 81 |
| Cas13a crRNA targeting SARS-Cov-2 | NCR_313 | UAGACCACCCCAAAAAUGAAGGGGACUAAAACCCCCUCCG UUAAGCUCACGC | 82 |
| Cas13a crRNA targeting SARS-Cov-2 | NCR_314 | UAGACCACCCCAAAAAUGAAGGGGACUAAAACCAUAGCG AGUGUAUGCCCCU | 83 |
| Cas13a crRNA targeting SARS-Cov-2 | NCR_315 | UAGACCACCCCAAAAAUGAAGGGGACUAAAACGUCUUUA AUGCACUCAAGAG | 84 |
| Cas13a crRNA targeting SARS-Cov-2 | NCR_316 | UAGACCACCCCAAAAAUGAAGGGGACUAAAACGUUCGGA CAAAGUGCAUGAA | 85 |

To test a subset (NCR_273 through NCR_282) of the guide sequences from Table 4, a series of guide RNAs are made by in vitro transcription according to standard protocols. These sequences also comprise the T7 promoter sequence to allow for RPA and RT-LAMP-T7 amplification. Exemplary sequences (NCR_317 through NCR_326) are shown in Table 5 where the underlined portion identifies the T7 promoter sequence. In addition, synthetic primary activator or target sequences are also generated to test the system. Shown in Table 6 are exemplary sequences (NCR_327 through NCR_336) used to test the guide RNAs from Table 5.

**Table 5: Guide sequences for use with RPA/RT-LAMP-T7**

| Name | Sequence 5'-3' | SEQ ID NO |
|---|---|---|
| NCR_317 | TACACAGGTGCCATCAAATTGTTTAGTCCCCTTCATTTTTGGGGTGGT CTACCTATAGTGAGTCGTATTAATTTCGAC | 86 |
| NCR_318 | AGGTTGGCCACACAGATCTAGTTTTAGTCCCCTTCATTTTTGGGGTGGT CTACCTATAGTGAGTCGTATTAATTTCGAC | 87 |
| NCR_319 | TCAGGTTGGACAGCTGGTGCGTTTTAGTCCCCTTCATTTTTGGGGTGGT CTACCTATAGTGAGTCGTATTAATTTCGAC | 88 |
| NCR 320 | CTGGTGCTGCAGCTTATTATGTTTAGTCCCCTTCATTTTGGGGTGGT CTACCTATAGTGAGTCGTATTAATTTCGAC | 89 |
| NCR_321 | GCAGCTTATTATGTGGGTTAGTTTAGTCCCCTTCATTTTTGGGGTGGT CTACCTATAGTGAGTCGTATTAATTTCGAC | 90 |
| NCR_322 | GCTTATTATGTGGGTTATCTGTTTTAGTCCCCTTCATTTTTGGGGTGGTC TACCTATAGTGAGTCGTATTAATTTCGAC | 91 |
| NCR_323 | TTATTATGTGGGTTATCTTCGTTTAGTCCCCTTCATTTTTGGGGTGGTC TACCTATAGTGAGTCGTATTAATTTCGAC | 92 |
| NCR_324 | TATTATGTGGGTTATCTTCAGTTTTAGTCCCCTTCATTTTTGGGGTGGTC TACCTATAGTGAGTCGTATTAATTTCGAC | 93 |
| NCR_325 | ATTATGTGGGTTATCTTCAAGTTTTAGTCCCCTTCATTTTTGGGGTGGT CTACCTATAGTGAGTCGTATTAATTTCGAC | 94 |
| NCR_326 | TTATGTGGGTTATCTTCAACGTTTTAGTCCCCTTCATTTTTGGGGTGGTC TACCTATAGTGAGTCGTATTAATTTCGAC | 95 |

**Table 6: Synthetic primary activator sequences**

| Name | Sequence 5'-3' | SEQ ID NO |
|---|---|---|
| NCR_327 | TCATCCAATTTGATGGCACCTGTGTAGGTCAACCTATAGTGAGTCGTAT TAATTTCGAC | 96 |
| NCR_328 | AGCCATTAGATCTGTGTGGCCAACCTCTTCTGCCTATAGTGAGTCGTAT TAATTTCGAC | 97 |
| NCR_329 | GCTGCAGCACCAGCTGTCCAACCTGAAGAAGACCTATAGTGAGTCGTA TTAATTTCGAC | 98 |
| NCR_330 | ACCCACATAATAAGCTGCAGCACCAGCTGTCCCCTATAGTGAGTCGTA TTAATTTCGAC | 99 |
| NCR_331 | TGAAGATAACCCACATAATAAGCTGCAGCACCCCTATAGTGAGTCGTA TTAATTTCGAC | 100 |
| NCR_332 | GGTTGAAGATAACCCACATAATAAGCTGCAGCCCTATAGTGAGTCGTA TTAATTTCGAC | 101 |
| NCR_333 | TAGGTTGAAGATAACCCACATAATAAGCTGCACCTATAGTGAGTCGTA TTAATTTCGAC | 102 |
| NCR_334 | CTAGGTTGAAGATAACCCACATAATAAGCTGCCCTATAGTGAGTCGTA TTAATTTCGAC | 103 |
| NCR_335 | CCTAGGTTGAAGATAACCCACATAATAAGCTGCCTATAGTGAGTCGTA TTAATTTCGAC | 104 |
| NCR_336 | TCCTAGGTTGAAGATAACCCACATAATAAGCTCCTATAGTGAGTCGTA TTAATTTCGAC | 105 |

Experiments are performed in vitro using the synthetic primary activator sequences and the synthetic crRNAs under conditions described in Example 1 and demonstrate that the NCR system detects the primary activator sequences.

In some cases, modified reporter molecules are used comprising caging structures to protect the reporter oligo prior to cleavage by a specific activator complexes (e.g. comprising Cas13). Exemplary sequences are shown below in Table 7. "/56-FAM" means the 5' 6-FAM (Fluorescein) molecule and "/3IABkFQ" is the 3' Iowa Black^{®} FQ quencher (IDT). The sequences are used with the methods and compositions of the invention and display reduced background signal.

**Table 7: Reporter sequences**

| Category | Name | Sequence 5'-3' | SEQ ID NO |
|---|---|---|---|
| Cas12 Reporter | dGJK_273 | /56-FAM/TTTTTTT /3IABkFQ/ | 106 |
| Cas13 Reporter | NCR_137 | /56-FAM/CGCTCrUrUrUrUrUGAGCG /3IABkFQ/ | 107 |
| Cas13 Reporter | NCR_138 | /56-FAM/CGCTCrUrUrUrUrUrUrUGAGCG /3IABkFQ/ | 108 |
| Cas13 Reporter | NCR_139 | /56-FAM/CGCTCrUrUrUrUrUrUrUrUrtUGAGCG /3IABkFQ/ | 109 |
| Cas13 Reporter | NCR_140 | /56-FAM/rCrCrCrCrC /3IABkFQ/ | 110 |
| Cas13 Reporter | NCR_141 | /56-FAM/rUrUrUrArA /3IABkFQ/ | 111 |
| Cas12 Reporter | NCR_231 | /56-FAM/TTATTATT /3IABkFQ/ | 112 |
| Cas13 Reporter | rGJK_086 | /56-FAM/rUrUrUrUrU /3IABkFQ/ | 113 |
| Cas13 Reporter | rGJK_087 | /56-FAM/rArArArArA /3IABkFQ/ | 114 |

Following demonstration of the system using the synthetic reagents, patient samples are treated per standard protocols. For example, nasopharyngeal swabs are acquired from healthy donors and patients. In some embodiments, saliva samples are obtained. Sample RNA of SARS-CoV-2 is extracted following instructions as described in the CDC EUA-approved
protocol (Centers for Disease Control and Prevention. Real-time RT-PCR Panel for Detection 2019-nCoV (US Centers for Disease Control and Prevention, 2020); input 120 µl, elution of 120 µl) using Qiagen DSP Viral RNA Mini kit (Qiagen) and the MagNA Pure 24 instrument (Roche Life Science). In some cases, patient swabs are inserted into specific viral inactivation buffers (e.g.DNA/RNA Shield (Zymo Research) or QuickExtract^{™} (Lucigen)) or transport buffers (e.g. PBS-Azide). The initial interaction of the Cas13 sensor complex leads to a cleavage event of a caged activator or caged guide molecule which initiates an NCR event. Here, an A₄ activator sequence for Csm6 is included in the hairpin loop of the caged crRNA of the amplifier Cas enzyme, flanked by uracils.

Additional cleavage events on the loop by Cas13 liberates A₄>P, which bind and activate the RNase activity of TtCsm6. Following activation of the Csm6, it cleaves both the detectable label, as well as the single-stranded hairpin loops of any remaining uncaged crRNA, further activating the NCR.

Similar experiments are performed using in which the A₄ sequence with flanking U's are be present in the caged crRNA of Cas12, rather than of Cas13 for detection of DNA targets.

The detectable label may be an F/Q reporter. The fluorescent reporter used could include A's or C's, but also accommodate additional nucleotides necessary for Cas13 (U) or Cas12 (deoxyribonucleotides) activity. This strategy could also be used with Csm6 homologs that respond to A₆>P, like *S. epidermidis* Csm6 (SeCsm6), by changing the number of A's from four to six in the hairpin loop of the caged crRNA.

### Example 3

Sequences of proteins that may be used with the methods and compositions of the invention.
Cas13a sequences 
LshCas13a, WP_018451595.1 (Abudayyeh,O.O., (2016) Science 353 (6299), aaf5573)
1 mgnlfghkrw yevrdkkdfk ikrkvkvkrn ydgnkyilni nennnkekid nnkfirkyin
61 ykkndnilke ftrkfhagni lfklkgkegi irienndd fl eteevvlyie aygksekl ka
121 lgitkkkiid eairqgitkd dkkieikrqe neeeieidir deytnktlnd csiilriien
181 deletkksiy eifkninmsl ykiiekiien etekvfenry yeehlrekll kddkidvilt
241 nfmeirekik snleilgfvk fylnvggdkk ksknkkmlve kilninvdlt vediadfvik
301 elefwnitkr iekvkkvnne flekrrnrty iksyvlldkh ekfkierenk kdkivkffve
361 niknnsikek iekilaefki delikkleke lkkgncdtei fgifkkhykv nfdskkfskk
421 sdeekelyki iyrylkgrie kilvneqkvr lkkmekieie kilnesilse kilkrvkqyt
481 lehimylgkl rhndidmttv ntddfsrlha keeldlelit ffastnmeln kifsreninn
541 denidffggd reknyvldkk ilnskikiir dldfidnknn itnnfirkft kigtnernri
601 lhaiskerdl qgtqddynkv iniiqnlkis deevskalnl dvvfkdkkni itkindikis
661 eennndikyl psfskvlpei lnlyrnnpkn epfdtietek ivlnaliyvn kelykklile
721 ddleeneskn iflqelkktl gnideideni ienyyknaqi saskgnnkai kkyqkkviec
781 yigylrknye elfdfsdfkm niqeikkqik dindnktyer itvktsdkti vinddfeyii
841 sifallnsna vinkirnrff atsvwlntse yqniidilde imqlntlrne citenwnlnl
901 eefiqkmkei ekdfddfkiq tkkeifnnyy ediknnilte fkddingcdv lekklekivi
961 fddetkfeid kksnilqdeq rklsninkkd lkkkvdqyik dkdqeikski lcriifnsdf
1021 lkkykkeidn liedmesene nkfqeiyypk erknelyiyk knlflnignp nfdkiyglis
1081 ndikmadakf lfnidgknir knkiseidai lknlndklng yskeykekyi kklkenddff
1141 akniqnknyk sfekdynrvs eykkirdlve fnylnkiesy lidinwklai qmarferdmh
1201 yivnglrelg iiklsgyntg israypkrng sdgfytttay ykffdeesyk kfekicygfg
1261 idlsensein kpenesirny ishfyivrnp fadysiaeqi drvsnllsys trynnstyas
1321 vfevfkkdvn ldydelkkkf klignndile rlmkpkkvsv lelesynsdy iknliiellt
1381 kientndtl (SEQ ID NO:115)
LwaCas13a, WP_021746774.1, hypothetical protein 
1 mkvtkvdgis hkkyieegkl vkstseenrt serlsellsi rldiyiknpd naseeenrir
61 renlkkffsn kvlhlkdsvl ylknrkekna vqdknyseed iseydlknkn sfsvlkkill
121 nedvnseele ifrkdveakl nkinslkysf eenkanyqki nennvekvgg kskrniiydy
 
181 yresakrndy innvqeafdk lykkediekl fflienskkh ekykireyyh kiigrkndke
241 nfakiiyeei qnvnnikeli ekipdmselk ksqvfykyyl dkeelndkni kyafchfvei
301 emsqllknyv ykrlsnisnd kikrifeyqn lkklienkll nkldtyvrnc gkynyylqvg
361 eiatsdfiar nrqneaflrn iigvssvayf slrnileten enditgrmrg ktvknnkgee
421 kyvsgevdki ynenkqnevk enlkmfysyd fnmdnkneie dffanideai ssirhgivhf
481 nlelegkdif afkniapsei skkmfqnein ekklklkifk qlnsanvfny yekdviikyl
541 kntkfnfvnk nipfvpsftk lynkiedlrn tlkffwsvpk dkeekdaqiy llkniyygef
601 lnkfvknskv ffkitnevik inkqrnqktg hykyqkfeni ektvpveyla iiqsreminn
661 qdkeekntyi dfiqqiflkg fidylnknnl kyiesnnnnd nndifskiki kkdnkekydk
721 ilknyekhnr nkeipheine fvreiklgki lkytenlnmf ylilkllnhk eltnlkgsle
781 kyqsankeet fsdelelinl lnldnnrvte dfeleaneig kfldfnenki kdrkelkkfd
841 tnkiyfdgen iikhrafyni kkygmlnlle kiadkakyki slkelkeysn kkneieknyt
901 mqqnlhrkya rpkkdekfnd edykeyekai gniqkythlk nkvefnelnl lqglllkilh
961 rlvgytsiwe rdlrfrlkge fpenhyieei fnfdnsknvk yksgqiveky infykelykd
1021 nvekrsiysd kkvkklkqek kdlyirnyia hfnyiphaei sllevlenlr kllsydrklk
1081 naimksivdi lkeygfvatf kigadkkiei qtlesekivh lknlkkkklm tdrnseelce
1141 lvkvmfeyka le (SEQ ID NO:116)
LseCas13a, WP 012985477.1. (Liu,L. et al (2017) Cell 170 (4), 714-726) 
1 mwisiktlih hlgvlffcdy mynrrekkii evktmritkv evdrkkvlis rdknggklvy
61 enemqdnteq imhhkkssfy ksvvnkticr peqkqmkklv hgllqensqe kikvsdvtkl
121 nisnflnhrf kkslyyfpen spdkseeyri einlsqlled slkkqqgtfi cwesfskdme
181 lyinwaenyi ssktklikks irnnriqste srsgqlmdry mkdilnknkp fdiqsvseky
241 qlekltsalk atfkeakknd keinyklkst lqnherqiie elkenselnq fnieirkhle
301 tyfpikktnr kvgdirnlei geiqkivnhr lknkivqril qegklasyei estvnsnslq
361 kikieeafal kfinaclfas nnlrnmvypv ckkdilmige fknsfkeikh kkfirqwsqf
421 fsqeitvddi elaswglrga iapirneiih lkkhswkkff nnptfkvkks kiingktkdv
481 tseflyketl fkdyfyseld svpeliinkm esskildyys sdqlnqvfti pnfelsllts
541 avpfapsfkr vylkgfdyqn qdeaqpdynl klniynekaf nseafqaqys lfkmvyyqvf
601 lpqfttnndl fkssvdfilt lnkerkgyak afqdirkmnk dekpseymsy iqsqlmlyqk
661 kqeekekinh fekfinqvfi kgfnsfiekn rltyichptk ntvpendnie ipfhtdmdds
721 niafwlmckl ldakqlselr nemikfscsl qsteeistft karevigla1 lngekgcndw
781 kelfddkeaw kknmslyvse ellqslpytq edgqtpvinr sidlvkkygt etileklfss
841 sddykvsakd iaklheydvt ekiaqqeslh kqwiekpgla rdsawtkkyq nvindisnyq
901 waktkveltq vrhlhqltid llsrlagyms iadrdfqfss nyilerense yrvtswills
961 enknknkynd yelynlknas ikvssknqpq lkvdlkqlrl tleylelfdn rlkekrnnis
1021 hfnylngqlg nsilelfdda rdvlsydrkl knavskslke ilsshgmevt fkplyqtnhh
1081 lkidklqpkk ihhlgekstv ssnqvsneyc qlvrtlltmk (SEQ ID NO:117)
LbmCas13a, WP 044921188.1 (hypothetical protein) 1 mqiskvnhkh vavgqkdrer itgfiyncpv gdeksledvv akrandtkvl fnvfntkdly 
61 dsqesdksek dkeiiskgak fvaksfnsai tilkkqnkiy stltsqqvik elkdkfggar
121 iydddieeal tetlkksfrk envrnsikvl ienaagirss lskdeeeliq eyfvkqlvee
181 ytktklqknv vksiknqnmv iqpdsdsqvl slsesrrekq ssavssdtlv nckekdvlka
241 fltdyavlde dernsllwkl rnlvnlyfyg sesirdysyt keksvwkehd eqkanktlfi
301 deichitkig kngkeqkvld yeenrsrcrk qninyyrsal nyaknntsgi fenedsnhfw
361 ihlienever lyngiengee fkfetgyise kvwkavinhl sikyialgka vynyamkels
421 spgdiepgki ddsyingits fdyeiikaee slqrdismnv vfatnylaca tvdtdkdfll
481 fskedirsct kkdgnlckni mqfwggystw knfceeylkd dkdalellys lksmlysmrn
541 ssfhfstenv dngswdteli gklfeedcnr aariekekfy nnnlhmfyss sllekvlerl
601 ysshherasq vpsfnrvfvr knfpsslseq ritpkftdsk deqiwqsavy ylckeiyynd
661 flqskeaykl fregvknldk ndinnqkaad sfkqavvyyg kaignatlsq vcqaimteyn
721 rqnndglkkk sayaekqnsn kykhyplflk qvlqsafwey ldenkeiygf isaqihksnv
781 eikaedfian yssqqykklv dkvkktpelq kwytlgrlin prqanqflgs irnyvqfvkd
841 iqrrakengn pirnyyevle sdsiikilem ctklngttsn dihdyfrded eyaeyisqfv
901 nfgdvhsgaa lnafcnsese gkkngiyydg inpivnrnwv lcklygspdl iskiisrvne
961 nmihdfhkqe dlireyqikg icsnkkeqqd lrtfqvlknr velrdiveys eiinelygql
1021 ikwcylrerd lmyfqlgfhy lclnnasske adyikinvdd rnisgailyq iaamyinglp
1081 vyykkddmyv alksgkkasd elnsneqtsk kinyflkygn nilgdkkdql ylaglelfen
1141 vaeheniiif rneidhfhyf ydrdrsmldl ysevfdrfft ydmklrknvv nmlynilldh
1201 nivssfvfet gekkvgrgds evikpsakir lranngvssd vftykvgskd elkiatlpak
1261 neefllnvar liyypdmeav senmvregvv kveksndkkg kisrgsntrs snqskynnks
1321 knrmnysmgs ifekmdlkfd (SEQ ID NO:118)
LbnCas13a, WP 022785443.1 (Liu, L. et al, Cell 170 (4), 714-726) 
1 mkiskvreen rgakltvnak tavvsenrsq egilyndpsr ygksrknded rdryiesrlk
61 ssgklyrifn edknkretde lqwflseivk kinrrnglvl sdmlsvddra fekafekyae
121 lsytnrrnkv sgspafetcg vdaataerlk giisetnfin riknnidnkv sediidriia
181 kylkkslcre rvkrglkkll mnafdlpysd pdidvqrdfi dyvledfyhv raksqvsrsi
241 knmnmpvqpe gdgkfaitvs kggtesgnkr saekeafkkf lsdyaslder vrddmlrrmr
301 rlvvlyfygs ddsklsdvne kfdvwedhaa rrvdnrefik lplenklang ktdkdaerir
361 kntvkelyrn qnigcyrqav kaveednngr yfddkmlnmf fihrieygve kiyanlkqvt
421 efkartgyls ekiwkdliny isikyiamgk avynyamdel nasdkkeiel gkiseeylsg
481 issfdyelik aeemlqreta vyvafaarhl ssqtveldse nsdflllkpk gtmdkndknk
541 lasnnilnfl kdketlrdti lqyfgghslw tdfpfdkyla ggkddvdflt dlkdviysmr
601 ndsfhyaten hnngkwnkel isamfehete rmtvvmkdkf ysnnlpmfyk nddlkkllid
661 lykdnveras qvpsfnkvfv rknfpalvrd kdnlgieldl kadadkgene lkfynalyym
721 fkeiyynafl ndknvrerfi tkatkvacny drnkernlkd riksagsdek kklreqlqny
781 iaendfgqri knivqvnpdy tlaqicqlim teynqqnngc mqkksaarkd inkdsyqhyk
841 mlllvnlrka flefikenya fvlkpykhdl cdkadfvpdf akyvkpyagl isrvagssel
901 qkwyivsrfl spaqanhmlg flhsykqyvw diyrrasetg teinhsiaed kiagvditdv
961 davidlsvkl cgtisseisd yfkddevyae yissyldfey dggnykdsln rfcnsdavnd
 
1021 qkvalyydge hpklnrniil sklygerrfl ekitdrvsrs diveyyklkk etsqyqtkgi
1081 fdsedeqkni kkfqemkniv efrdlmdyse iadelqgqli nwiylrerdl mnfqlgyhya
1141 clnndsnkqa tyvtldyqgk knrkingail yqicamying lplyyvdkds sewtvsdgke
1201 stgakigefy ryaksfents dcyasgleif enisehdnit elrnyiehfr yyssfdrsfl
1261 giysevfdrf ftydlkyrkn vptilynill qhfvnvrfef vsgkkmigid kkdrkiakek
1321 ecaritirek ngvyseqfty klkngtvyvd ardkrylqsi irllfypekv nmdemievke
1381 kkkpsdnntg kgyskrdrqq drkeydkyke kkkkegnfls gmggninwde inaqlkn (SEQ
ID NO:119)
CamCas13a, WP 031473346.1 (hypothetical protein) 
1 mkfskvdhtr savgiqkatd svhgmlytdp kkqevndldk rfdqlnvkak rlynvfnqsk
61 aeedddekrf gkvvkklnre lkdllfhrev srynsignak ynyygiksnp eeivsnlgmv
121 eslkgerdpq kvisklllyy lrkglkpgtd glrmileasc glrklsgdek elkvflqtld
181 edfekktfkk nlirsienqn mavqpsnegd piigitqgrf nsqkneeksa iermmsmyad
241 lnedhredvl rklrrlnvly fnvdtektee ptlpgevdtn pvfevwhdhe kgkendrqfa
301 tfakiltedr etrkkeklav kealndlksa irdhnimayr csikvteqdk dglffedqri
361 nrfwihhies averilasin peklyklrig ylgekvwkdl lnylsikyia vgkavfhfam
421 edlgktgqdi elgklsnsvs ggltsfdyeq iradetlqrq lsvevafaan nlfravvgqt
481 gkkieqskse eneedfllwk aekiaesikk egegntlksi lqffggassw dlnhfcaayg
541 nessalgyet kfaddlrkai yslrnetfhf ttlnkgsfdw nakligdmfs heaatgiave
601 rtrfysnnlp mfyresdlkr imdhlyntyh prasqvpsfn svfvrknfrl flsntlntnt
661 sfdtevyqkw esgvyylfke iyynsflpsg dahhlffegl rrirkeadnl pivgkeakkr
721 navqdfgrrc delknlslsa icqmimteyn eqnngnrkvk stredkrkpd ifqhykmlll
781 rtlqeafaiy irreefkfif dlpktlyvmk pveeflpnwk sgmfdslver vkqspdlqrw
841 yvlckflngr llnqlsgvir syiqfagdiq rrakanhnrl ymdntqrvey ysnvlevvdf
901 cikgtsrfsn vfsdyfrded ayadyldnyl qfkdekiaev ssfaalktfc neeevkagiy
961 mdgenpvmqr nivmaklfgp devlknvvpk vtreeieeyy qlekqiapyr qngyckseed
1021 qkkllrfqri knrvefqtit efseiinell gqliswsflr erdllyfqlg fhylclhndt
1081 ekpaeykeis redgtvirna ilhqvaamyv gglpvytlad kklaafekge adcklsiskd
1141 tagagkkikd ffryskyvli kdrmltdqnq kytiylagle lfentdehdn itdvrkyvdh
1201 fkyyatsden amsildlyse ihdrfftydm kyqknvanml enillrhfvl irpefftgsk
1261 kvgegkkitc karaqieiae ngmrsedfty klsdgkknis tcmiaardqk ylntvarlly
1321 ypheakksiv dtrekknnkk tnrgdgtfnk qkgtarkekd ngprefndtg fsntpfagfd
1381 pfrns (SEQ ID NO:120)
CgaCas13a, WP 034560163.1 (hypothetical protein) 
  1 mritkvkikl dnklyqvtmq keekygtlkl neesrkstae ilrlkkasfn ksfhsktins
 61 qkenknatik kngdyisqif eklvgvdtnk nirkpkmslt dlkdlpkkdl alfikrkfkn
121 ddiveiknld lislfynalq kvpgehftde swadfcqemm pyreyknkfi erkiillans
181 ieqnkgfsin petfskrkrv lhqwaievqe rgdfsildek lsklaeiynf kkmckrvqde
241 lndleksmkk gknpekekea ykkqknfkik tiwkdypykt higliekike neelnqfnie
 
301 igkyfehyfp ikkerctede pyylnsetia ttvnyqlkna lisylmqigk ykqfglenqv
361 ldskklqeig iyegfqtkfm dacvfatssl kniiepmrsg dilgkrefke aiatssfvny
421 hhffpyfpfe lkgmkdrese lipfgeqtea kqmqniwalr gsvqqirnei fhsfdknqkf
481 nlpqldksnf efdasenstg ksqsyietdy kflfeaeknq leqffierik ssgaleyypl
541 ksleklfakk emkfslgsqv vafapsykkl vkkghsyqta tegtanylgl syynryelke
601 esfqaqyyll kliyqyvflp nfsqgnspaf retvkailri nkdearkkmk knkkflrkya
661 feqvremefk etpdqymsyl qsemreekvr kaekndkgfe knitmnfekl lmqifvkgfd
721 vflttfagke lllsseekvi keteislskk inerektlka siqvehqlva tnsaisywlf
781 cklldsrhln elrnemikfk qsrikfnhtq haeliqnllp iveltilsnd ydekndsqnv
841 dvsayfedks lyetapyvqt ddrtrvsfrp ilklekyhtk slieallkdn pqfrvaatdi
901 qewmhkreei gelvekrknl htewaegcqt lgaekreeyr dyckkidrfn wkankvtlty
961 lsqlhylitd llgrmvgfsa lferdlvyfs rsfselgget yhisdyknls gvlrlnaevk
1021 pikiknikvi dneenpykgn epevkpfldr lhaylenvig ikavhgkirn qtahlsvlql
1081 elsmiesmnn lrdlmaydrk lknavtksmi kildkhgmil klkidenhkn feieslipke
1141 iihlkdkaik tnqvseeycq lvlallttnp gnqln (SEQ ID NO:121)
Cga2Cas13a, WP 034563842.1 (hypothetical protein) 
1 mrmtkvking spvsmnrskl nghlvwngtt ntvniltkke qsfaasflnk tlvkadqvkg
61 ykvlaenifi ifeqleksns ekpsvylnni rrlkeaglkr ffksykheei kytseknqsv
121 ptklnliplf fnavdriqed kfdeknwsyf ckemspyldy kksylnrkke ilansiqqnr
181 gfsmptaeep nllskrkqlf qqwamkfces pliqqnnfav eqfnkefank inelaavynv
241 delctaitek lmnfdkdksn ktrnfeikkl wkqhphnkdk aliklfnqeg nealnqfnie
301 lgkyfehyfp ktgkkesaes yylnpqtiik tvgyqlrnaf vqyllqvgkl hqynkgvlds
361 qtlqeigmye gfqtkfmdac vfassslrni iqattnedil trekfkkele knvelkhdlf
421 fkteiveerd enpakkiamt pneldlwair gavqrvrnqi fhqqinkrhe pnqlkvgsfe
481 ngdlgnvsyq ktiyqklfda eikdieiyfa ekikssgale qysmkdlekl fsnkeltlsl
541 ggqvvafaps ykklykqgyf yqnektiele qftdydfsnd vfkanyylik liyhyvflpq
601 fsqannklfk dtvhyviqqn kelnttekdk knnkkirkya feqvklmkne spekymqylq
661 remqeertik eakktneekp nynfeklliq ifikgfdtfl rnfdlnlnpa eelvgtvkek
721 aeglrkrker iakilnvdeq iktgdeeiaf wifaklldar hlselrnemi kfkqssvkkg
781 liknqdlieq mqpilelcil sndsesmeke sfdkievfle kvelaknepy mqedkltpvk
841 frfmkqleky qtrnfienlv ienpefkvse kivlnwheek ekiadlvdkr tklheewask
901 areieeynek ikknkskkld kpaefakfae ykiiceaien fnrldhkvrl tylknlhylm
961 idlmgrmvgf svlferdfvy mgrsysalkk qsiylndydt fanirdwevn enkhlfgtss
1021 sdltfqetae fknlkkpmen qlkallgvtn hsfeirnnia hlhvlrndgk gegvsllscm
1081 ndlrklmsyd rklknavtka iikildkhgm ilkltnndht kpfeieslkp kkiihleksn
1141 hsfpmdqvsq eycdlvkkml vftn (SEQ ID NO:122)
Pprcas13a, WP 013443710.1 (Liu, L. et al, Cell 170 (4), 714-726) 
1 mrvskvkvkd ggkdkmvlvh rkttgaqlvy sgqpvsnets nilpekkrqs fdlstlnkti
61 ikfdtakkqk lnvdqykive kifkypkcel pkqikaeeil pflnhkfqep vkywkngkee
 
121 sfnltllive avqaqdkrkl qpyydwktwy iqtksdllkk siennridlt enlskrkkal
181 laweteftas gsidlthyhk vymtdvlckm lqdvkpltdd kgkintnayh rglkkalqnh
241 qpaifgtrev pneanradnq lsiyhlevvk ylehyfpikt skrrntaddi ahylkaqtlk
301 ttiekqlvna iraniiqqgk tnhhelkadt tsndlirikt neafvlnltg tcafaannir
361 nmvdneqtnd ilgkgdfiks llkdntnsql ysfffgegls tnkaeketql wgirgavqqi
421 rnnvnhykkd alktvfnisn fenptitcpk qqtnyadtiy karfinelek ipeafaqqlk
481 tggavsyyti enlksllttf qfslcrstip fapgfkkvfn gginyqnakq desfyelmle
541 qylrkenfae esynaryfml kliynnlflp gfttdrkafa dsvgfvqmqn kkqaekvnpr
601 kkeayafeav rpmtaadsia dymayvqsel mqeqnkkeek vaeetrinfe kfvlqvfikg
661 fdsflrakef dfvqmpqpql tatasnqcka dklnqleasi tadckltpqy akaddathia
721 fyvfckllda ahlsnlrnel ikfresvnef kfhhlleiie icllsadvvp tdyrdlysse
781 adclarlrpf ieqgaditnw sdlfvqsckh spvihaniel svkygttkll eqiinkdtqf
841 ktteanftaw ntaqksieql ikqredhheq wvkaknaddk ekqerkreks nfaqkfiekh
901 gddyldicdy intynwldnk mhfvhlnrlh gltiellgrm agfvalfdrd fqffdeqqia
961 defklhgfvn lhsidkklne vptkkikeiy dirnkiiqin gnkinesvra nliqfisskr
1021 nyynnaflhv sndeikekqm ydirnhiahf nyltkdaadf slidlinelr ellhydrklk
1081 navskafidl fdkhgmilkl klnadhklkv eslepkkiyh lgssakdkpe yqyctnqvmm
1141 aycnmcrsll emkk (SEQ ID NO:123)
LweCas13a, WP 036059185.1 (hypothetical protein) 
1 mlallhqevp sqklhnlksl ntesltklfk pkfqnmisyp pskgaehvqf cltdiavpai
61 rdldeikpdw giffeklkpy tdwaesyihy kqttiqksie qnkiqspdsp rklvlqkyvt
121 aflngeplgl dlvakkykla dlaesfkvvd lnedksanyk ikaclqqhqr nildelkedp
181 elnqygievk kyiqryfpik rapnrskhar adflkkelie stveqqfkna vyhyvleqgk
241 meayeltdpk tkdlqdirsg eafsfkfina cafasnnlkm ilnpecekdi lgkgdfkknl
301 pnsttqsdvv kkmipffsde iqnvnfdeai wairgsiqqi rnevyhckkh swksilkikg
361 fefepnnmky tdsdmqklmd kdiakipdfi eeklkssgii rfyshdklqs iwemkqgfsl
421 lttnapfvps fkrvyakghd yqtsknryyd lglttfdile ygeedfrary fltklvyyqq
481 fmpwftadnn afrdaanfvl rlnknrqqda kafinireve egemprdymg yvqgqiaihe
541 dstedtpnhf ekfisqvfik gfdshmrsad lkfiknprnq gleqseieem sfdikvepsf
601 lknkddyiaf wtfckmldar hlselrnemi kydghltgeq eiiglallgv dsrendwkqf
661 fssereyeki mkgyvgeely qrepyrqsdg ktpilfrgve qarkygtetv iqrlfdaspe
721 fkvskcnite werqketiee tierrkelhn eweknpkkpq nnaffkeyke ccdaidaynw
781 hknkttlvyv nelhhlliei lgryvgyvai adrdfqcman qyfkhsgite rveywgdnrl
841 ksikkldtfl kkeglfvsek narnhiahln ylslksectl lylserlrei fkydrklkna
901 vskslidild rhgmsvvfan lkenkhrlvi kslepkklrh lgekkidngy ietnqvseey
961 cgivkrllei (SEQ ID NO:124)
LneCas13a, WP 036091002.1 (hypothetical protein) 
1 mkitkmrvdg rtivmertsk egqlgyegid gnktteiifd kkkesfyksi lnktvrkpde
61 keknrrkqai nkainkeite lmlavlhcev psqklhnlks lntesltklf kpkfqnmisy
 
121 ppskgaehvq fcltdiavpa irdldeikpd wgiffeklkp ytdwaesyih ykqttiqksi
181 eqnkiqspds prklvlqkyv taflngeplg ldlvakkykl adlaesfklv dlnedksany
241 kikaclqqhq rnildelked pelnqygiev kkyiqryfpi krapnrskha radflkkeli
301 estveqqfkn avyhyvleqg kmeayeltdp ktkdlqdirs geafsfkfin acafasnnlk
361 milnpecekd ilgkgnfkkn lpnsttrsdv vkkmipffsd elqnvnfdea iwairgsiqq
421 irnevyhckk hswksilkik gfefepnnmk yadsdmqklm dkdiakipef ieeklkssgv
481 vrfyrhdelq siwemkqgfs llttnapfvp sfkrvyakgh dyqtsknryy nldlttfdil
541 eygeedfrar yfltklvyyq qfmpwftadn nafrdaanfv lrlnknrqqd akafinirev
601 eegemprdym gyvqgqiaih edsiedtpnh fekfisqvfi kgfdrhmrsa nlkfiknprn
661 qgleqseiee msfdikveps flknkddyia fwifckmlda rhlselrnem ikydghltge
721 qeiiglallg vdsrendwkq ffssereyek imkgyvveel yqrepyrqsd gktpilfrgv
781 eqarkygtet viqrlfdanp efkvskcnla ewerqketie etikrrkelh newaknpkkp
841 qnnaffkeyk eccdaidayn whknkttlay vnelhhllie ilgryvgyva iadrdfqcma
901 nqyfkhsgit erveywgdnr lksikkldtf lkkeglfvse knarnhiahl nylslksect
961 llylserlre ifkydrklkn avskslidil drhgmsvvfa nlkenkhrlv ikslepkklr
1021 hlggkkidgg yietnqvsee ycgivkrlle m (SEQ ID NO:125)
Lwa2cas13a, WP 021746774.1 (hypothetical protein) 
1 mkvtkvdgis hkkyieegkl vkstseenrt serlsellsi rldiyiknpd naseeenrir
61 renlkkffsn kvlhlkdsvl ylknrkekna vqdknyseed iseydlknkn sfsvlkkill
121 nedvnseele ifrkdveakl nkinslkysf eenkanyqki nennvekvgg kskrniiydy
181 yresakrndy innvqeafdk lykkediekl fflienskkh ekykireyyh kiigrkndke
241 nfakiiyeei qnvnnikeli ekipdmselk ksqvfykyyl dkeelndkni kyafchfvei
301 emsqllknyv ykrlsnisnd kikrifeyqn lkklienkll nkldtyvrnc gkynyylqvg
361 eiatsdfiar nrqneaflrn iigvssvayf slrnileten enditgrmrg ktvknnkgee
421 kyvsgevdki ynenkqnevk enlkmfysyd fnmdnkneie dffanideai ssirhgivhf
481 nlelegkdif afkniapsei skkmfqnein ekklklkifk qlnsanvfny yekdviikyl
541 kntkfnfvnk nipfvpsftk lynkiedlrn tlkffwsvpk dkeekdaqiy llkniyygef
601 lnkfvknskv ffkitnevik inkqrnqktg hykyqkfeni ektvpveyla iiqsreminn
661 qdkeekntyi dfiqqiflkg fidylnknnl kyiesnnnnd nndifskiki kkdnkekydk
721 ilknyekhnr nkeipheine fvreiklgki lkytenlnmf ylilkllnhk eltnlkgsle
781 kyqsankeet fsdelelinl lnldnnrvte dfeleaneig kfldfnenki kdrkelkkfd
841 tnkiyfdgen iikhrafyni kkygmlnlle kiadkakyki slkelkeysn kkneieknyt
901 mqqnlhrkya rpkkdekfnd edykeyekai gniqkythlk nkvefnelnl lqglllkilh
961 rlvgytsiwe rdlrfrlkge fpenhyieei fnfdnsknvk yksgqiveky infykelykd
1021 nvekrsiysd kkvkklkqek kdlyirnyia hfnyiphaei sllevlenlr kllsydrklk
1081 naimksivdi lkeygfvatf kigadkkiei qtlesekivh lknlkkkklm tdrnseelce
1141 lvkvmfeyka le (SEQ ID NO:126)
RcsCas13a, WP 013067728.1 (hypothetical protein) 1 mqigkvqgrt isefgdpagg lkrkistdgk nrkelpahls sdpkaliqqw isgidkiyrk 
61 pdsrksdgka ihsptpskmq fdarddlgea fwklvseagl aqdsdydqfk rrlhpygdkf
121 qpadsgaklk feadppepqa fhgrwygams krgndakela aalyehlhvd ekridgqpkr
181 npktdkfapg lvvaralgie ssvlprgmar larnwgeeei qtyfvvdvaa svkevakaav
241 saaqafdppr qvsgrslspk vgfalaehle rvtgskrcsf dpaagpsvla lhdevkktyk
301 rlcargknaa rafpadktel lalmrhthen rvrnqmvrmg rvseyrgqqa gdlaqshywt
361 sagqteikes eifvrlwvga falagrsmka widpmgkivn tekndrdlta avnirqvisn
421 kemvaeamar rgiyfgetpe ldrlgaegne gfvfallryl rgcrnqtfhl garagflkei
481 rkelektrwg kakeaehvvl tdktvaaira iidndakalg arlladlsga fvahyaskeh
541 fstlyseivk avkdapevss glprlklllk radgvrgyvh glrdtrkhaf atklppppap
601 relddpatka ryiallrlyd gpfrayasgi tgtalagpaa rakeaatala qsvnvtkays
661 dvmegrtsrl rppndgetlr eylsaltget atefrvqigy esdsenarkq aefienyrrd
721 mlafmfedyi rakgfdwilk iepgatamtr apvlpepidt rgqyehwqaa lylvmhfvpa
781 sdvsnllhql rkwealqgky elvqdgdatd qadarreald lvkrfrdvlv lflktgearf
841 egraapfdlk pfralfanpa tfdrlfmatp ttarpaeddp egdgasepel rvartlrglr
901 qiarynhmav lsdlfakhkv rdeevarlae iedetqeksq ivaaqelrtd lhdkvmkchp
961 ktispeerqs yaaaiktiee hrflvgrvyl gdhlrlhrlm mdvigrlidy agayerdtgt
1021 flinaskqlg agadwavtia gaantdartq trkdlahfnv ldradgtpdl talvnrarem
1081 maydrkrkna vprsildmla rlgltlkwqm kdhllqdati tqaaikhldk vrltvggpaa
1141 vtearfsqdy lqmvaavfng svqnpkprrr ddgdawhkpp kpataqsqpd qkppnkapsa
1201 gsrlpppqvg evyegvvvkv idtgslgfla vegvagnigl hisrlrrire daiivgrryr
1261 frveiyvppk sntsklnaad lvrid (SEQ ID NO:127)
RcrCas13a, WP 023911507.1 (hypothetical protein) 
1 mqigkvqgrt isefgdpagg lkrkistdgk nrkelpahls sdpkaligqw isgidkiyrk
61 pdsrksdgka ihsptpskmq fdarddlgea fwklvseagl aqdsdydqfk rrlhpygdkf
121 qpadsgaklk feadppepqa fhgrwygams krgndakela aalyehlhvd ekridgqpkr
181 npktdkfapg lvvaralgie ssvlprgmar larnwgeeei qtyfvvdvaa svkevakaav
241 saaqafdppr qvsgrslspk vgfalaehle rvtgskrcsf dpaagpsvla lhdevkktyk
301 rlcargknaa rafpadktel lalmrhthen rvrnqmvrmg rvseyrgqqa gdlaqshywt
361 sagqteikes eifvrlwvga falagrsmka widpmgkivn tekndrdlta avnirqvisn
421 kemvaeamar rgiyfgetpe ldrlgaegne gfvfallryl rgcrnqtfhl garagflkei
481 rkelektrwg kakeaehvvl tdktvaaira iidndakalg arlladlsga fvahyaskeh
541 fstlyseivk avkdapevss glprlklllk radgvrgyvh glrdtrkhaf atklppppap
601 relddpatka ryiallrlyd gpfrayasgi tgtalagpaa rakeaatala qsvnvtkays
661 dvmegrssrl rppndgetlr eylsaltget atefrvqigy esdsenarkq aefienyrrd
721 mlafmfedyi rakgfdwilk iepgatamtr apvlpepidt rgqyehwqaa lylvmhfvpa
781 sdvsnllhql rkwealqgky elvqdgdatd qadarreald lvkrfrdvlv lflktgearf
841 egraapfdlk pfralfanpa tfdrlfmatp ttarpaeddp egdgasepel rvartlrglr
901 qiarynhmav lsdlfakhkv rdeevarlae iedetqeksq ivaaqelrtd lhdkvmkchp
961 ktispeerqs yaaaiktiee hrflvgrvyl gdhlrlhrlm mdvigrlidy agayerdtgt
1021 flinaskqlg agadwavtia gaantdartq trkdlahfnv ldradgtpdl talvnrarem
 
1081 maydrkrkna vprsildmla rlgltlkwqm kdhllqdati tqaaikhldk vrltvggpaa
1141 vtearfsqdy lqmvaavfng svqnpkprrr ddgdawhkpp kpataqsqpd qkppnkapsa
1201 gsrlpppqvg evyegvvvkv idtgslgfla vegvagnigl hisrlrrire daiivgrryr
1261 frveiyvppk sntsklnaad lvrid (SEQ ID NO:128)
RcdCas13a, WP 023911507.1 (hypothetical protein) 
1 mqigkvqgrt isefgdpagg lkrkistdgk nrkelpahls sdpkaligqw isgidkiyrk
61 pdsrksdgka ihsptpskmq fdarddlgea fwklvseagl aqdsdydqfk rrlhpygdkf
121 qpadsgaklk feadppepqa fhgrwygams krgndakela aalyehlhvd ekridgqpkr
181 npktdkfapg lvvaralgie ssvlprgmar larnwgeeei qtyfvvdvaa svkevakaav
241 saaqafdppr qvsgrslspk vgfalaehle rvtgskrcsf dpaagpsvla lhdevkktyk
301 rlcargknaa rafpadktel lalmrhthen rvrnqmvrmg rvseyrgqqa gdlaqshywt
361 sagqteikes eifvrlwvga falagrsmka widpmgkivn tekndrdlta avnirqvisn
421 kemvaeamar rgiyfgetpe ldrlgaegne gfvfallryl rgcrnqtfhl garagflkei
481 rkelektrwg kakeaehvvl tdktvaaira iidndakalg arlladlsga fvahyaskeh
541 fstlyseivk avkdapevss glprlklllk radgvrgyvh glrdtrkhaf atklppppap
601 relddpatka ryiallrlyd gpfrayasgi tgtalagpaa rakeaatala qsvnvtkays
661 dvmegrssrl rppndgetlr eylsaltget atefrvqigy esdsenarkq aefienyrrd
721 mlafmfedyi rakgfdwilk iepgatamtr apvlpepidt rgqyehwqaa lylvmhfvpa
781 sdvsnllhql rkwealqgky elvqdgdatd qadarreald lvkrfrdvlv lflktgearf
841 egraapfdlk pfralfanpa tfdrlfmatp ttarpaeddp egdgasepel rvartlrglr
901 qiarynhmav lsdlfakhkv rdeevarlae iedetqeksq ivaaqelrtd lhdkvmkchp
961 ktispeerqs yaaaiktiee hrflvgrvyl gdhlrlhrlm mdvigrlidy agayerdtgt
1021 flinaskqlg agadwavtia gaantdartq trkdlahfnv ldradgtpdl talvnrarem
1081 maydrkrkna vprsildmla rlgltlkwqm kdhllqdati tqaaikhldk vrltvggpaa
1141 vtearfsqdy lqmvaavfng svqnpkprrr ddgdawhkpp kpataqsqpd qkppnkapsa
1201 gsrlpppqvg evyegvvvkv idtgslgfla vegvagnigl hisrlrrire daiivgrryr
1261 frveiyvppk sntsklnaad lvrid (SEQ ID NO:129)
LbuCas13a, WP_015770004, (Liu, L. et al, Cell 170 (4), 714-726) 
1 mkvtkvggis hkkytsegrl vkseseenrt derlsallnm rldmyiknps stetkenqkr
61 igklkkffsn kmvylkdntl slkngkkeni dreysetdil esdvrdkknf avlkkiylne
121 nvnseelevf rndikkklnk inslkysfek nkanyqkine nniekvegks krniiydyyr
181 esakrdayvs nvkeafdkly keediaklvl eienltklek ykirefyhei igrkndkenf
241 akiiyeeiqn vnnmkeliek vpdmselkks qvfykyyldk eelndkniky afchfveiem
301 sqllknyvyk rlsnisndki krifeyqnlk klienkllnk ldtyvrncgk ynyylqdgei
361 atsdfiarnr qneaflrnii gvssvayfsl rniletenen ditgrmrgkt vknnkgeeky
421 vsgevdkiyn enkknevken lkmfysydfn mdnkneiedf fanideaiss irhgivhfnl
481 elegkdifaf kniapseisk kmfqneinek klklkifrql nsanvfryle kykilnylkr
541 trfefvnkni pfvpsftkly sriddlknsl giywktpktn ddnktkeiid aqiyllkniy
601 ygeflnyfms nngnffeisk eiielnkndk rnlktgfykl qkfediqeki pkeylaniqs
 
 661 lyminagnqd eeekdtyidf iqkiflkgfm tylanngrls liyigsdeet ntslaekkqe
 721 fdkflkkyeq nnnikipyei neflreiklg nilkyterln mfylilklln hkeltnlkgs
 781 lekyqsanke eafsdqleli nllnldnnrv tedfeleade igkfldfngn kvkdnkelkk
 841 fdtnkiyfdg eniikhrafy nikkygmlnl lekiadkagy kisieelkky snkkneiekn
 901 hkmqenlhrk yarprkdekf tdedyesykq aienieeyth lknkvefnel nllqglllri
 961 lhrlvgytsi werdlrfrlk gefpenqyie eifnfenkkn vkykggqive kyikfykelh
1021 qndevkinky ssanikvlkq ekkdlyirny iahfnyipha eisllevlen lrkllsydrk
1081 lknavmksvv dilkeygfva tfkigadkki giqtleseki vhlknlkkkk lmtdrnseel
1141 cklvkimfey kmeekksen (SEQ ID NO:130)
RcaCas13a, ETD76934.1 (Ding, H. et al (2014) Genome Announc 2 (1), e00050-14) 
1 mqigkvqgrt isefgdpagg lkrkistdgk nrkelpahls sdpkaligqw isgidkiyrk
61 pdsrksdgka ihsptpskmq fdarddlgea fwklvseagl aqdsdydqfk rrlhpygdkf
121 qpadsgaklk feadppepqa fhgrwygams krgndakela aalyehlhvd ekridgqpkr
181 npktdkfapg lvvaralgie ssvlprgmar larnwgeeei qtyfvvdvaa svkevakaav
241 saaqafdppr qvsgrslspk vgfalaehle rvtgskrcsf dpaagpsvla lhdevkktyk
301 rlcargknaa rafpadktel lalmrhthen rvrnqmvrmg rvseyrgqqa gdlaqshywt
361 sagqteikes eifvrlwvga falagrsmka widpmgkivn tekndrdlta avnirqvisn
421 kemvaeamar rgiyfgetpe ldrlgaegne gfvfallryl rgcrnqtfhl garagflkei
481 rkelektrwg kakeaehvvl tdktvaaira iidndakalg arlladlsga fvahyaskeh
541 fstlyseivk avkdapevss glprlklllk radgvrgyvh glrdtrkhaf atklppppap
601 relddpatka ryiallrlyd gpfrayasgi tgtalagpaa rakeaatala qsvnvtkays
661 dvmegrssrl rppndgetlr eylsaltget atefrvqigy esdsenarkq aefienyrrd
721 mlafmfedyi rakgfdwilk iepgatamtr apvlpepidt rgqyehwqaa lylvmhfvpa
781 sdvsnllhql rkwealqgky elvqdgdatd qadarreald lvkrfrdvlv lflktgearf
841 egraapfdlk pfralfanpa tfdrlfmatp ttarpaeddp egdgasepel rvartlrglr
901 qiarynhmav lsdlfakhkv rdeevarlae iedetqeksq ivaaqelrtd lhdkvmkchp
961 ktispeerqs yaaaiktiee hrflvgrvyl gdhlrlhrlm mdvigrlidy agayerdtgt
1021 flinaskqlg agadwavtia gaantdartq trkdlahfnv ldradgtpdl talvnrarem
1081 maydrkrkna vprsildmla rlgltlkwqm kdhllqdati tqaaikhldk vrltvggpaa
1141 vtearfsqdy lqmvaavfng svqnpkprrr ddgdawhkpp kpataqsqpd qkppnkapsa
1201 gsrlpppqvg evyegvvvkv idtgslgfla vegvagnigl hisrlrrire daiivgrryr
1261 frveiyvppk snntsklnaad lvrid (SEQ ID NO:131)
EreCas13a, WP 055061018.1 (hypothetical protein) 
1 mlrrdkevkk lynvfnqiqv gtkpkkwnnd eklspeener raqqknikmk nykwreacsk
61 yvessqriin dvifysyrka knklrymrkn edilkkmqea eklskfsggk ledfvaytlr
121 kslvvskydt qefdslaamv vflecigknn isdhereivc kllelirkdf skldpnvkgs
181 qganivrsvr nqnmivqpqg drflfpqvya kenetvtnkn vekeglnefl lnyanlddek
241 raeslrklrr ildvyfsapn hyekdmditl sdniekekfn vwekhecgkk etglfvdipd
301 vlmeaeaeni kldavvekre rkvlndrvrk qniicyrytr avvekynsne plffennain
 
361 qywihhiena verilkncka gklfklrkgy laekvwkdai nlisikyial gkavynfald
421 diwkdkknke lgivderirn gitsfdyemi kahenlqrel avdiafsvnn laravcdmsn
481 lgnkesdfll wkrndiadkl knkddmasvs avlqffggks swdinifkda ykgkkkynye
541 vrfiddlrka iycarnenfh fktalvncek wntelfgkif eretefclnv ekdrfysnnl
601 ymfyqvselr nmldhlysrs vsraaqvpsy nsvivrtafp eyitnvlgyq kpsydadtlg
661 kwysacyyll keiyynsflq sdralqlfek svktlswddk kqqravdnfk dhfsdiksac
721 tslaqvcqiy mteynqqnnq ikkvrssnds ifdqpvyqhy kvllkkaian afadylknnk
781 dlfgfigkpf kaneireidk eqflpdwtsr kyealcievs gsqelqkwyi vgkflnarsl
841 nlmvgsmrsy iqyvtdikrr aasignelhv svhdvekvek wvqvievcsl lasrtsnqfe
901 dyfndkddya rylksyvdfs nvdmpseysa lvdfsneeqs dlyvdpknpk vnrnivhskl
961 faadhilrdi vepvskdnie efysqkaeia yckikgkeit aeeqkavlky qklknrvelr
1021 diveygeiin ellgqlinws fmrerdllyf qlgfhydclr ndskkpegyk nikvdensik
1081 dailyqiigm yvngvtvyap ekdgdklkeq cvkggvgvkv safhryskyl glnektlyna
1141 gleifevvae hediinlrng idhfkyylgd yrsmlsiyse vfdrfftydi kyqknvlnll
1201 qnillrhnvi vepilesgfk tigeqtkpga klsirsiksd tfqykvkggt litdakdery
1261 letirkilyy aeneednlkk svvvtnacky eknkesddqn kqkekknkdn kgkkneetks
1321 daeknnnerl synpfanlnf klsn (SEQ ID NO:132)
HheCas13a, CRZ35554.1 (Wibberg, Daniel, direct submission) 
1 mkltrrrisg nsvdqkitaa fyrdmsqgll yydsedndct dkviesmdfe rswrgrilkn
61 geddknpfym fvkglvgsnd kivcepidvd sdpdnldili nknltgfgrn lkapdsndtl
121 enlirkiqag ipeeevlpel kkikemiqkd ivnrkeqllk siknnripfs legsklvpst
181 kkmkwlfkli dvpnktfnek mlekyweiyd ydklkanitn rldktdkkar sisravseel
241 reyhknlrtn ynrfvsgdrp aagldnggsa kynpdkeefl lflkeveqyf kkyfpvkskh
301 snkskdkslv dkyknycsyk vvkkevnrsi inqlvagliq qgkllyyfyy ndtwqedfln
361 syglsyiqve eafkksvmts lswginrlts ffiddsntvk fddittkkak eaiesnyfnk
421 lrtcsrmqdh fkeklaffyp vyvkdkkdrp dddienlivl vknaiesvsy lrnrtfhfke
481 ssllellkel ddknsgqnki dysvaaefik rdienlydvf reqirslgia eyykadmisd
541 cfktcglefa lyspknslmp afknvykrga nlnkayirdk gpketgdqgq nsykaleeyr
601 eltwyievkn ndqsynaykn llqliyyhaf lpevreneal itdfinrtke wnrketeerl
661 ntknnkkhkn fdendditvn tyryesipdy qgesldd ylk vlqrkqmara kevnekeegn
721 nnyiqfirdv vvwafgayle nklknykne l qpplskenig lndtlkelfp eekvkspfni
781 kcrfsistfi dnkgkstdnt saeavktdgk edekdkknik rkdllcfylf lrlldeneic
841 klqhqfikyr cslkerrfpg nrtklekete llaeleelme lvrftmpsip eisakaesgy
901 dtmikkyfkd fiekkvfknp ktsnlyyhsd sktpvtrkym allmrsaplh lykdifkgyy
961 litkkecley iklsniikdy qnslnelheq leriklksek qngkdslyld kkdfykvkey
1021 venleqvary khlqhkinfe slyrifrihv diaarmvgyt qdwerdmhfl fkalvyngvl
1081 eerrfeaifn nnddnndgri vkkiqnnlnn knrelvsmlc wnkklnknef gaiiwkrnpi
1141 ahlnhftqte qnsksslesl inslrillay drkrqnavtk tindlllndy hirikwegrv
1201 degqiyfnik ekedienepi ihlkhlhkkd cyiyknsymf dkqkewicng ikeevydksi
1261 lkcignlfkf dyedknkssa npkht (SEQ ID NO:133)
LbaCas13a, WP 022785443.1 ((Liu, L. et al, Cell 170 (4), 714-726 
1 mkiskvreen rgakltvnak tavvsenrsq egilyndpsr ygksrknded rdryiesrlk
61 ssgklyrifn edknkretde lqwflseivk kinrrnglvl sdmlsvddra fekafekyae
121 lsytnrrnkv sgspafetcg vdaataerlk giisetnfin riknnidnkv sediidriia
181 kylkkslcre rvkrglkkll mnafdlpysd pdidvqrdfi dyvledfyhv raksqvsrsi
241 knmnmpvqpe gdgkfaitvs kggtesgnkr saekeafkkf lsdyaslder vrddmlrrmr
301 rlvvlyfygs ddsklsdvne kfdvwedhaa rrvdnrefik lplenklang ktdkdaerir
361 kntvkelyrn qnigcyrqav kaveednngr yfddkmlnmf fihrieygve kiyanlkqvt
421 efkartgyls ekiwkdliny isikyiamgk avynyamdel nasdkkeiel gkiseeylsg
481 issfdyelik aeemlqreta vyvafaarhl ssqtveldse nsdflllkpk gtmdkndknk
541 lasnnilnfl kdketlrdti lqyfgghslw tdfpfdkyla ggkddvdflt dlkdviysmr
601 ndsfhyaten hnngkwnkel isamfehete rmtvvmkdkf ysnnlpmfyk nddlkkllid
661 lykdnveras qvpsfnkvfv rknfpalvrd kdnlgieldl kadadkgene lkfynalyym
721 fkeiyynafl ndknvrerfi tkatkvacny drnkernlkd riksagsdek kklreqlqny
781 iaendfgqri knivqvnpdy tlaqicqlim teynqqnngc mqkksaarkd inkdsyqhyk
841 mlllvnlrka flefikenya fvlkpykhdl cdkadfvpdf akyvkpyagl isrvagssel
901 qkwyivsrfl spaqanhmlg flhsykqyvw diyrrasetg teinhsiaed kiagvditdv
961 davidlsvkl cgtisseisd yfkddevyae yissyldfey dggnykdsln rfcnsdavnd
1021 qkvalyydge hpklnrniil sklygerrfl ekitdrvsrs diveyyklkk etsqyqtkgi
1081 fdsedeqkni kkfqemkniv efrdlmdyse iadelqgqli nwiylrerdl mnfqlgyhya
1141 clnndsnkqa tyvtldyqgk knrkingail yqicamying lplyyvdkds sewtvsdgke
1201 stgakigefy ryaksfents dcyasgleif enisehdnit elrnyiehfr yyssfdrsfl
1261 giysevfdrf ftydlkyrkn vptilynill qhfvnvrfef vsgkkmigid kkdrkiakek
1321 ecaritirek ngvyseqfty klkngtvyvd ardkrylqsi irllfypekv nmdemievke
1381 kkkpsdnntg kgyskrdrqq drkeydkyke kkkkegnfls gmggninwde inaqlkn (SEQ
ID NO:**)
Cas13d
   [Eubacterium] siraeum DSM 15702 ESCas13d 
1 mgkkihardl reqrktdrte kfadqnkkre aeravpkkda avsvksvssv sskkdnvtks
61 makaagvksv favgntvymt sfgrgndavl eqkivdtshe plniddpayq lnvvtmngys
121 vtghrgetvs avtdnplrrf ngrkkdepeq svptdmlclk ptlekkffgk efddnihiql
181 iynildieki lavystnaiy alnnmsacen iensdffmkr ttdetfddfe kkkestnsre
241 kadfdafekf ignyrlayfa dafyvnkknp kgkaknvlre dkelysvltl igklahwcva
301 seegraefwl ykldelkddf knvldvvynr pveeinnrfi ennkvniqil gsvykntdia
361 elvrsyyefl itkkyknmgf sikklresml egkgyadkey dsvrnklyqm tdfilytgyi
421 nedsdraddl vntlrsslke ddkttvycke adylwkkyre sirevadald gdnikklsks
481 nieiqedklr kcfisyadsv seftkliyll trflsgkein dlvttlinkf dnirsfleim
541 delgldrtft aeysffegst kylaelveln sfvkscsfdi nakrtmyrda ldilgiesdk
 
601 teediekmid nilqidangd kklkknnglr nfiasnvids nrfkylvryg npkkiretak
661 ckpavrfvln eipdaqiery yeaccpknta lcsankrrek ladmiaeikf enfsdagnyq
721 kanvtsrtse aeikrknqai irlyltvmyi mlknlvnvna ryviafhcve rdtklyaesg
781 levgnieknk tnltmavmgv klengiikte fdksfaenaa nrylrnarwy klildnlkks
841 eravvnefan tvcalnairn ininikeike venyfalyhy liqkhlenrf adkkverdtg
901 dfiskleehk tyckdfvkay ctpfgynlvr yknltidglf dknypgkdds deqk. (SEQ ID
NO:134)
uncultured Ruminococcus sp. URCas13d (PDB: 6IV9_A) 
1 makknkmkpr elreaqkkar qlkaaeinnn aapaiaampa aeviapvaek kkssvkaagm
61 ksilvsenkm yitsfgkgns avleyevcnn dynktqlssk dnsnielgdv nevnitfssk
121 hgfgsgvein tsnpthrsge sspvrgdmlg lkselekrff gktfddnihi qliynildie
181 kilavyvtni vyalnnmlgi kdsesyddfm gylsarntye vfthpdksnl sdkvkgnikk
241 slskfndllk tkrlgyfgle epktkdtras eaykkrvyhm laivgqiaqc vfhdksgakr
301 fdlysfinni dpeyrdtldy lveerlksin kdfiegnkvn isllidmmkg yeaddiirly
361 ydfivlksqk nlgfsikklr ekmleeygfr fkdkqydsvr skmyklmdfl lfcnyyrndv
421 aagealvrkl rfsmtddeke giyadeaakl wgkfrndfen iadhmngdvi kelgkadmdf
481 dekildsekk nasdllyfsk miymltyfld gkeindlltt liskfdnike flkimkssav
541 dveceltagy klfndsqrit nelfivknia smrkpaasak ltmfrdalti lgiddnitdd
601 riseilklke kgkgihglrn fitnnviess rfvylikyan aqkirevakn ekvvmfvlgg
661 ipdtqieryy kscvefpdmn ssleakrsel armiknisfd dfknvkqqak grenvakera
721 kaviglyltv myllvknlvn vnaryviaih clerdfglyk eiipelaskn lkndyrilsq
781 tlcelcddrn essnlflkkn krlrkcvevd innadssmtr kyanciahlt vvrelkeyig
841 dirtvdsyfs iyhyvmqrci tkrgddtkqe ekikyeddll knhgytkdfv kalnspfgyn
901 iprfknlsie qlfdrneylt eklehhhhhh (SEQ ID NO:135)
Cas12a sequences
   Lachnospiraceae bacterium (LbCas12a) 
MSKLEKFTNCYSLSKTLRFKAIPVGKTQENIDNKRLLVEDEKRAEDYKGVKKLLDRYYLSFINDVLHSIKLKNLNNYIS
LFRKKTRTEKENKELENLEINLRKEIAKAFKGNEGYKSLFKKDIIETILPEFLDDKDEIALVNSFNGFTTAFTGFFDNR
ENMFSEEAKSTSIAFRCINENLTRYISNMDIFEKVDAIFDKHEVQEIKEKILNSDYDVEDFFEGEFFNFVLTQEGIDVY
NAIIGGFVTESGEKIKGLNEYINLYNQKTKQKLPKFKPLYKQVLSDRESLSFYGEGYTSDEEVLEVFRNTLNKNSEIFS
SIKKLEKLFKNFDEYSSAGIFVKNGPAISTISKDIFGEWNVIRDKWNAEYDDIHLKKKAVVTEKYEDDRRKSFKKIGSF
SLEQLQEYADADLSVVEKLKEIIIQKVDEIYKVYGSSEKLFDADFVLEKSLKKNDAVVAIMKDLLDSVKSFENYIKAFF
GEGKETNRDESFYGDFVLAYDILLKVDHIYDAIRNYVTQKPYSKDKFKLYFQNPQFMGGWDKDKETDYRATILRYGSKY
YLAIMDKKYAKCLQKIDKDDVNGNYEKINYKLLPGPNKMLPKVFFSKKWMAYYNPSEDIQKIYKNGTFKKGDMFNLNDC
HKLIDFFKDSISRYPKWSNAYDFNFSETEKYKDIAGFYREVEEQGYKVSFESASKKEVDKLVEEGKLYMFQIYNKDFSD
KSHGTPNLHTMYFKLLFDENNHGQIRLSGGAELFMRRASLKKEELVVHPANSPIANKNPDNPKKTTTLSYDVYKDKRFS
EDQYELHIPIAINKCPKNIFKINTEVRVLLKHDDNPYVIGIDRGERNLLYIVVVDGKGNIVEQYSLNEIINNFNGIRIK
TDYHSLLDKKEKERFEARQNWTSIENIKELKAGYISQVVHKICELVEKYDAVIALEDLNSGFKNSRVKVEKQVYQKFEK
 
MLIDKLNYMVDKKSNPCATGGALKGYQITNKFESFKSMSTQNGFIFYIPAWLTSKIDPSTGFVNLLKTKYTSIADSKKF
ISSFDRIMYVPEEDLFEFALDYKNFSRTDADYIKKWKLYSYGNRIRIFRNPKKNNVFDWEEVCLTSAYKELFNKYGINY
QQGDIRALLCEQSDKAFYSSFMALMSLMLQMRNSITGRTDVDFLISPVKNSDGIFYDSRNYEAQENAILPKNADANGAY
NIARKVLWAIGQFKKAEDEKLDKVKIAISNKEWLEYAQTSVKH (SEQ ID NO:136)
Acidaminococcus sp.BV3L6 (Cas12a) 
MTQFEGFTNLYQVSKTLRFELIPQGKTLKHIQEQGFIEEDKARNDHYKELKPIIDRIYKTYADQCLQLVQLDWENLSAA

IDSYRKEKTEETRNALIEEQATYRNAIHDYFIGRTDNLTDAINKRHAEIYKGLFKAELFNGKVLKQLGTVTTTEHENAL

LRSFDKFTTYFSGFYENRKNVFSAEDISTAIPHRIVQDNFPKFKENCHIFTRLITAVPSLREHFENVKKAIGIFVSTSI

EEVFSFPFYNQLLTQTQIDLYNQLLGGISREAGTEKIKGLNEVLNLAIQKNDETAHIIASLPHRFIPLFKQILSDRNTL

SFILEEFKSDEEVIQSFCKYKTLLRNENVLETAEALFNELNSIDLTHIFISHKKLETISSALCDHWDTLRNALYERRIS

ELTGKITKSAKEKVQRSLKHEDINLQEIISAAGKELSEAFKQKTSEILSHAHAALDQPLPTTLKKQEEKEILKSQLDSL

LGLYHLLDWFAVDESNEVDPEFSARLTGIKLEMEPSLSFYNKARNYATKKPYSVEKFKLNFQMPTLASGWDVNKEKNNG

AILFVKNGLYYLGIMPKQKGRYKALSFEPTEKTSEGFDKMYYDYFPDAAKMIPKCSTQLKAVTAHFQTHTTPILLSNNF

IEPLEITKEIYDLNNPEKEPKKFQTAYAKKTGDQKGYREALCKWIDFTRDFLSKYTKTTSIDLSSLRPSSQYKDLGEYY

AELNPLLYHISFQRIAEKEIMDAVETGKLYLFQIYNKDFAKGHHGKPNLHTLYWTGLFSPENLAKTSIKLNGQAELFYR

PKSRMKRMAHRLGEKMLNKKLKDQKTPIPDTLYQELYDYVNHRLSHDLSDEARALLPNVITKEVSHEIIKDRRFTSDKF

FFHVPITLNYQAANSPSKFNQRVNAYLKEHPETPIIGIDRGERNLIYITVIDSTGKILEQRSLNTIQQFDYQKKLDNRE

KERVAARQAWSVVGTIKDLKQGYLSQVIHEIVDLMIHYQAVVVLENLNFGFKSKRTGIAEKAVYQQFEKMLIDKLNCLV

LKDYPAEKVGGVLNPYQLTDQFTSFAKMGTQSGFLFYVPAPYTSKIDPLTGFVDPFVWKTIKNHESRKHFLEGFDFLHY

DVKTGDFILHFKMNRNLSFQRGLPGFMPAWDIVFEKNETQFDAKGTPFIAGKRIVPVIENHRFTGRYRDLYPANELIAL

LEEKGIVFRDGSNILPKLLENDDSHAIDTMVALIRSVLQMRNSNAATGEDYINSPVRDLNGVCFDSRFQNPEWPMDADA

NGAYHIALKGQLLLNHLKESKDLKLQNGISNQDWLAYIQELRN (SEQ ID NO:137)
Francisella novicida (FnCas12a) 
MSIYQEFVNKYSLSKTLRFELIPQGKTLENIKARGLILDDEKRAKDYKKAKQIIDKYHQFFIEEILSSVCISEDLLQNY
SDVYFKLKKSDDDNLQKDFKSAKDTIKKQISEYIKDSEKFKNLFNQNLIDAKKGQESDLILWLKQSKDNGIELFKANSD
ITDIDEALEIIKSFKGWTTYFKGFHENRKNVYSSNDIPTSIIYRIVDDNLPKFLENKAKYESLKDKAPEAINYEQIKKD
LAEELTFDIDYKTSEVNQRVFSLDEVFEIANFNNYLNQSGITKFNTIIGGKFVNGENTKRKGINEYINLYSQQINDKTL
KKYKMSVLFKQILSDTESKSFVIDKLEDDSDVVTTMQSFYEQIAAFKTVEEKSIKETLSLLFDDLKAQKLDLSKIYFKN
DKSLTDLSQQVFDDYSVIGTAVLEYITQQIAPKNLDNPSKKEQELIAKKTEKAKYLSLETIKLALEEFNKHRDIDKQCR
FEEILANFAAIPMIFDEIAQNKDNLAQISIKYQNQGKKDLLQASAEDDVKAIKDLLDQTNNLLHKLKIFHISQSEDKAN
ILDKDEHFYLVFEECYFELANIVPLYNKIRNYITQKPYSDEKFKLNFENSTLANGWDKNKEPDNTAILFIKDDKYYLGV
MNKKNNKIFDDKAIKENKGEGYKKIVYKLLPGANKMLPKVFFSAKSIKFYNPSEDILRIRNHSTHTKNGSPQKGYEKFE
FNIEDCRKFIDFYKQSISKHPEWKDFGFRFSDTQRYNSIDEFYREVENQGYKLTFENISESYIDSVVNQGKLYLFQIYN
KDFSAYSKGRPNLHTLYWKALFDERNLQDVVYKLNGEAELFYRKQSIPKKITHPAKEAIANKNKDNPKKESVFEYDLIK
DKRFTEDKFFFHCPITINFKSSGANKFNDEINLLLKEKANDVHILSIDRGERHLAYYTLVDGKGNIIKQDTFNIIGNDR
MKTNYHDKLAAIEKDRDSARKDWKKINNIKEMKEGYLSQVVHEIAKLVIEYNAIVVFEDLNFGFKRGRFKVEKQVYQKL
EKMLIEKLNYLVFKDNEFDKTGGVLRAYQLTAPFETFKKMGKQTGIIYYVPAGFTSKICPVTGFVNQLYPKYESVSKSQ
EFFSKFDKICYNLDKGYFEFSFDYKNFGDKAAKGKWTIASFGSRLINFRNSDKNHNWDTREVYPTKELEKLLKDYSIEY
 
GHGECIKAAICGESDKKFFAKLTSVLNTILQMRNSKTGTELDYLISPVADVNGNFFDSRQAPKNMPQDADANGAYHIGL
KGLMLLGRIKNNQEGKKLNLVIKNEEYFEFVQNRNN (SEQ ID NO:138)
Porphyromonas macacae Cas12a 
MKTQHFFEDFTSLYSLSKTIRFELKPIGKTLENIKKNGLIRRDEQRLDDYEKLKKVIDEYHEDFIANILSSFSFSEEIL
QSYIQNLSESEARAKIEKTMRDTLAKAFSEDERYKSIFKKELVKKDIPVWCPAYKSLCKKFDNFTTSLVPFHENRKNLY
TSNEITASIPYRIVHVNLPKFIQNIEALCELQKKMGADLYLEMMENLRNVWPSFVKTPDDLCNLKTYNHLMVQSSISEY
NRFVGGYSTEDGTKHQGINEWINIYRQRNKEMRLPGLVFLHKQILAKVDSSSFISDTLENDDQVFCVLRQFRKLFWNTV
SSKEDDAASLKDLFCGLSGYDPEAIYVSDAHLATISKNIFDRWNYISDAIRRKTEVLMPRKKESVERYAEKISKQIKKR
QSYSLAELDDLLAHYSEESLPAGFSLLSYFTSLGGQKYLVSDGEVILYEEGSNIWDEVLIAFRDLQVILDKDFTEKKLG
KDEEAVSVIKKALDSALRLRKFFDLLSGTGAEIRRDSSFYALYTDRMDKLKGLLKMYDKVRNYLTKKPYSIEKFKLHFD
NPSLLSGWDKNKELNNLSVIFRQNGYYYLGIMTPKGKNLFKTLPKLGAEEMFYEKMEYKQIAEPMLMLPKVFFPKKTKP
AFAPDQSVVDIYNKKTFKTGQKGFNKKDLYRLIDFYKEALTVHEWKLFNFSFSPTEQYRNIGEFFDEVREQAYKVSMVN
VPASYIDEAVENGKLYLFQIYNKDFSPYSKGIPNLHTLYWKALFSEQNQSRVYKLCGGGELFYRKASLHMQDTTVHPKG
ISIHKKNLNKKGETSLFNYDLVKDKRFTEDKFFFHVPISINYKNKKITNVNQMVRDYIAQNDDLQIIGIDRGERNLLYI
SRIDTRGNLLEQFSLNVIESDKGDLRTDYQKILGDREQERLRRRQEWKSIESIKDLKDGYMSQVVHKICNMVVEHKAIV
VLENLNLSFMKGRKKVEKSVYEKFERMLVDKLNYLVVDKKNLSNEPGGLYAAYQLTNPLFSFEELHRYPQSGILFFVDP
WNTSLTDPSTGFVNLLGRINYTNVGDARKFFDRFNAIRYDGKGNILFDLDLSRFDVRVETQRKLWTLTTFGSRIAKSKK
SGKWMVERIENLSLCFLELFEQFNIGYRVEKDLKKAILSQDRKEFYVRLIYLFNLMMQIRNSDGEEDYILSPALNEKNL
QFDSRLIEAKDLPVDADANGAYNVARKGLMVVQRIKRGDHESIHRIGRAQWLRY (SEQ ID NO:139)
Moraxella bovoculi 237 Cas12a 
MLFQDFTHLYPLSKTVRFELKPIDRTLEHIHAKNFLSQDETMADMHQKVKVILDDYHRDFIADMMGEVKLTKLAEFYDV
YLKFRKNPKDDELQKQLKDLQAVLRKEIVKPIGNGGKYKAGYDRLFGAKLFKDGKELGDLAKFVIAQEGESSPKLAHLA
HFEKFSTYFTGFHDNRKNMYSDEDKHTAIAYRLIHENLPRFIDNLQILTTIKQKHSALYDQIINELTASGLDVSLASHL
DGYHKLLTQEGITAYNTLLGGISGEAGSPKIQGINELINSHHNQHCHKSERIAKLRPLHKQILSDGMSVSFLPSKFADD
SEMCQAVNEFYRHYADVFAKVQSLFDGFDDHQKDGIYVEHKNLNELSKQAFGDFALLGRVLDGYYVDVVNPEFNERFAK
AKTDNAKAKLTKEKDKFIKGVHSLASLEQAIEHYTARHDDESVQAGKLGQYFKHGLAGVDNPIQKIHNNHSTIKGFLER
ERPAGERALPKIKSGKNPEMTQLRQLKELLDNALNVAHFAKLLTTKTTLDNQDGNFYGEFGVLYDELAKIPTLYNKVRD
YLSQKPFSTEKYKLNFGNPTLLNGWDLNKEKDNFGVILQKDGCYYLALLDKAHKKVFDNAPNTGKSIYQKMIYKYLEVR
KQFPKVFFSKEAIAINYHPSKELVEIKDKGRQRSDDERLKLYRFILECLKIHPKYDKKFEGAIGDIQLFKKDKKGREVP
ISEKDLFDKINGIFSSKPKLEMEDFFIGEFKRYNPSQDLVDQYNIYKKIDSNDNRKKENFYNNHPKFKKDLVRYYYESM
CKHEEWEESFEFSKKLQDIGCYVDVNELFTEIETRRLNYKISFCNINADYIDELVEQGQLYLFQIYNKDFSPKAHGKPN
LHTLYFKALFSEDNLADPIYKLNGEAQIFYRKASLDMNETTIHRAGEVLENKNPDNPKKRQFVYDIIKDKRYTQDKFML
HVPITMNFGVQGMTIKEFNKKVNQSIQQYDEVNVIGIDRGERHLLYLTVINSKGEILEQCSLNDITTASANGTQMTTPY
HKILDKREIERLNARVGWGEIETIKELKSGYLSHVVHQISQLMLKYNAIVVLEDLNFGFKRGRFKVEKQIYQNFENALI
KKLNHLVLKDKADDEIGSYKNALQLTNNFTDLKSIGKQTGFLFYVPAWNTSKIDPETGFVDLLKPRYENIAQSQAFFGK
FDKICYNADKDYFEFHIDYAKFTDKAKNSRQIWTICSHGDKRYVYDKTANQNKGAAKGINVNDELKSLFARHHINEKQP
NLVMDICQNNDKEFHKSLMYLLKTLLALRYSNASSDEDFILSPVANDEGVFFNSALADDTQPQNADANGAYHIALKGLW
LLNELKNSDDLNKVKLAIDNQTWLNFAQNR (SEQ ID NO:140)
Thiomicrospira sp.XS5 Cas12a 
MGIHGVPAATKTFDSEFFNLYSLQKTVRFELKPVGETASFVEDFKNEGLKRVVSEDERRAVDYQKVKEIIDDYHRDFIE
ESLNYFPEQVSKDALEQAFHLYQKLKAAKVEEREKALKEWEALQKKLREKVVKCFSDSNKARFSRIDKKELIKEDLINW
LVAQNREDDIPTVETFNNFTTYFTGFHENRKNIYSKDDHATAISFRLIHENLPKFFDNVISFNKLKEGFPELKFDKVKE
DLEVDYDLKHAFEIEYFVNFVTQAGIDQYNYLLGGKTLEDGTKKQGMNEQINLFKQQQTRDKARQIPKLIPLFKQILSE
RTESQSFIPKQFESDQELFDSLQKLHNNCQDKFTVLQQAILGLAEADLKKVFIKTSDLNALSNTIFGNYSVFSDALNLY
KESLKTKKAQEAFEKLPAHSIHDLIQYLEQFNSSLDAEKQQSTDTVLNYFIKTDELYSRFIKSTSEAFTQVQPLFELEA
LSSKRRPPESEDEGAKGQEGFEQIKRIKAYLDTLMEAVHFAKPLYLVKGRKMIEGLDKDQSFYEAFEMAYQELESLIIP
IYNKARSYLSRKPFKADKFKINFDNNTLLSGWDANKETANASILFKKDGLYYLGIMPKGKTFLFDYFVSSEDSEKLKQR
RQKTAEEALAQDGESYFEKIRYKLLPGASKMLPKVFFSNKNIGFYNPSDDILRIRNTASHTKNGTPQKGHSKVEFNLND
CHKMIDFFKSSIQKHPEWGSFGFTFSDTSDFEDMSAFYREVENQGYVISFDKIKETYIQSQVEQGNLYLFQIYNKDFSP
YSKGKPNLHTLYWKALFEEANLNNVVAKLNGEAEIFFRRHSIKASDKVVHPANQAIDNKNPHTEKTQSTFEYDLVKDKR
YTQDKFFFHVPISLNFKAQGVSKFNDKVNGFLKGNPDVNIIGIDRGERHLLYFTVVNQKGEILVQESLNTLMSDKGHVN
DYQQKLDKKEQERDAARKSWTTVENIKELKEGYLSHVVHKLAHLIIKYNAIVCLEDLNFGFKRGRFKVEKQVYQKFEKA
LIDKLNYLVFKEKELGEVGHYLTAYQLTAPFESFKKLGKQSGILFYVPADYTSKIDPTTGFVNFLDLRYQSVEKAKQLL
SDFNAIRFNSVQNYFEFEIDYKKLTPKRKVGTQSKWVICTYGDVRYQNRRNQKGHWETEEVNVTEKLKALFASDSKTTT
VIDYANDDNLIDVILEQDKASFFKELLWLLKLTMTLRHSKIKSEDDFILSPVKNEQGEFYDSRKAGEVWPKDADANGAY
HIALKGLWNLQQINQWEKGKTLNLAIKNQDWFSFIQEKPYQE (SEQ ID NO:141)
Butyrivibrio sp. NC3005 Cas12a 
MGIHGVPAAYYQNLTKKYPVSKTIRNELIPIGKTLENIRKNNILESDVKRKQDYEHVKGIMDEYHKQLINEALDNYMLP

SLNQAAEIYLKKHVDVEDREEFKKTQDLLRREVTGRLKEHENYTKIGKKDILDLLEKLPSISEEDYNALESFRNFYTYF

TSYNKVRENLYSDEEKSSTVAYRLINENLPKFLDNIKSYAFVKAAGVLADCIEEEEQDALFMVETFNMTLTQEGIDMYN

YQIGKVNSAINLYNQKNHKVEEFKKIPKMKVLYKQILSDREEVFIGEFKDDETLLSSIGAYGNVLMTYLKSEKINIFFD

ALRESEGKNVYVKNDLSKTTMSNIVFGSWSAFDELLNQEYDLANENKKKDDKYFEKRQKELKKNKSYTLEQMSNLSKED

ISPIENYIERISEDIEKICIYNGEFEKIVVNEHDSSRKLSKNIKAVKVIKDYLDSIKELEHDIKLINGSGQELEKNLVV

YVGQEEALEQLRPVDSLYNLTRNYLTKKPFSTEKVKLNFNKSTLLNGWDKNKETDNLGILFFKDGKYYLGIMNTTANKA

FVNPPAAKTENVFKKVDYKLLPGSNKMLPKVFFAKSNIGYYNPSTELYSNYKKGTHKKGPSFSIDDCHNLIDFFKESIK

KHEDWSKFGFEFSDTADYRDISEFYREVEKQGYKLTFTDIDESYINDLIEKNELYLFQIYNKDFSEYSKGKLNLHTLYF

MMLFDQRNLDNVVYKLNGEAEVFYRPASIAENELVIHKAGEGIKNKNPNRAKVKETSTFSYDIVKDKRYSKYKFTLHIP

ITMNFGVDEVRRFNDVINNALRTDDNVNVIGIDRGERNLLYVVVINSEGKILEQISLNSIINKEYDIETNYHALLDERE

DDRNKARKDWNTIENIKELKTGYLSQVVNVVAKLVLKYNAIICLEDLNFGFKRGRQKVEKQVYQKFEKMLIEKLNYLVI

DKSREQVSPEKMGGALNALQLTSKFKSFAELGKQSGIIYYVPAYLTSKIDPTTGFVNLFYIKYENIEKAKQFFDGFDFI

RFNKKDDMFEFSFDYKSFTQKACGIRSKWIVYTNGERIIKYPNPEKNNLFDEKVINVTDEIKGLFKQYRIPYENGEDIK

EIIISKAEADFYKRLFRLLHQTLQMRNSTSDGTRDYIISPVKNDRGEFFCSEFSEGTMPKDADANGAYNIARKGLWVLE

QIRQKDEGEKVNLSM[?]NAEWLKYAQLHLL (SEQ ID NO:142)
Brumimicrobium aurantiacum Cas12a 
1 MKNQINLFTN KFQLSKTLRF ELKPQGKTLE HINSKGFIKN DEKRADSYKK MKATIDAFHR
61 DFIDLAMSNV KLTNLIDFEE IYNASNADKK DEKYKTKLSK IQEILRKEIA KGFKGEEVKD
121 IFSKIDKKDL ITKLLEEWII ENKIEDIHFD PEFKNFTTYF SGFHQNRKNM YTDQEQSTAI
 
181 AYRLIHENLP RFIDNINIFQ KINKVPDLEE NLKKLYQEIE EYLGINAINE AFELEYFNET
241 LSQKGIDIYN LILGGRTAEE GKQKIQGLNE YINLYNQKQD KKNRVPKLKV LYKQILSDRT
301 RTSFLPDTFE DDEESSASQK VLDSINNFYL ENLIDYLPND KNSTINVLEN LKLLLAELIN
361 FELDKVYIKN DTSITNISMK IFKNYSVIRE ALNYFYENKI DPNFAHNFNN ANTDKKREKL
421 EKEKAKITKQ TYLSISFIEE AIHLYINENS NGNQYKNTYK PNCIANYFKD FFIAENKEGS
481 NKEFDFISKI KARYNTIKGV LNTPFPDNKR LHQEKNNIDN IKHFLDSIME YLHFAKPLVL
541 SGSFAFEKDE QFYTNFDELY NQLELIIPLY NKVRNYATQK PYSTEKFKLN FENSTLLNGW
601 DVNKEEANTS ILFIKNGFYY LGIMDKNHNK IFRNTPKSTN TDIYKKVNYK LLPGASKMLP
661 KVFFGKKNLD YYKPSKDILR IRNHGTHTKG GKPQSGFDKL DFNLNDCHKL IDFFKDSIQK
721 HPDWSKFKFK FSDTQIYESI DQFYRELEPQ AYSITYTNID SSFIEEQINE GKLYLFQIYN
781 KDFSKFSNGK PNLHTLYWKA LFDEQNLKDV TYKLNGEAEI FYRKKSIQHD RQIIHKRNQP
841 IINKNPNNEK KESIFKYNII KDKRYTIDKF QFHVPITLNF KAKGTDYINY DVLDYLKENP
901 DVKIIGLDRG ERHLIYLTLI DQKGKILEQI SLNEIVNKKH NITTSYHNLL ETKEIERDKA
961 RKNWGTVETI KELKEGYISQ VVHKISKMMI EHNAIVVMED LNMGFKRGRF KVEKQVYQKL
1021 EKMLIDKLNY LVLKDRQPNE PAGIYNALQL TNKFESFQKL GKQSGFLFYV PAWNTSKIDP
1081 TTGFVNLFHV KYESVRKSQE FFNKFNSIKY NPKEAIFEFD FDYNEFTTRA EGTKTNWTVC
1141 TYGDRIKTFR NPEKLNQWDN KEINITTAFE DFFGRHNITY GNGSDIKSQL ISREEKDFFS
1201 ELIHLFRLTL QMRNSKTNSE IDYLISPVKN ENGFFYDSRH ADKNLPKDAD ANGAYHIAKK
1261 GLQWIKEIQS (SEQ ID NO:143)
Porphyromonas crevioricanis Cas12a 
1 MDSLKDFTNL YPVSKTLRFE LKPVGKTLEN IEKAGILKED EHRAESYRRV KKIIDTYHKV
61 FIDSSLENMA KMGIENEIKA MLQSFCELYK KDHRTEGEDK ALDKIRAVLR GLIVGAFTGV
121 CGRRENTVQN EKYESLFKEK LIKEILPDFV LSTEAESLPF SVEEATRSLK EFDSFTSYFA
181 GFYENRKNIY STKPQSTAIA YRLIHENLPK FIDNILVFQK IKEPIAKELE HIRADFSAGG
241 YIKKDERLED IFSLNYYIHV LSQAGIEKYN ALIGKIVTEG DGEMKGLNEH INLYNQQRGR
301 EDRLPLFRPL YKQILSDREQ LSYLPESFEK DEELLRALKE FYDHIAEDIL GRTQQLMTSI
361 SEYDLSRIYV RNDSQLTDIS KKMLGDWNAI YMARERAYDH EQAPKRITAK YERDRIKALK
421 GEESISLANL NSCIAFLDNV RDCRVDTYLS TLGQKEGPHG LSNLVENVFA SYHEAEQLLS
481 FPYPEENNLI QDKDNVVLIK NLLDNISDLQ RFLKPLWGMG DEPDKDERFY GEYNYIRGAL
541 DQVIPLYNKV RNYLTRKPYS TRKVKLNFGN SQLLSGWDRN KEKDNSCVIL RKGQNFYLAI
601 MNNRHKRSFE NKVLPEYKEG EPYFEKMDYK FLPDPNKMLP KVFLSKKGIE IYKPSPKLLE
661 QYGHGTHKKG DTFSMDDLHE LIDFFKHSIE AHEDWKQFGF KFSDTATYEN VSSFYREVED
721 QGYKLSFRKV SESYVYSLID QGKLYLFQIY NKDFSPCSKG TPNLHTLYWR MLFDERNLAD
781 VIYKLDGKAE IFFREKSLKN DHPTHPAGKP IKKKSRQKKG EESLFEYDLV KDRRYTMDKF
841 QFHVPITMNF KCSAGSKVND MVNAHIREAK DMHVIGIDRG ERNLLYICVI DSRGTILDQI
901 SLNTINDIDY HDLLESRDKD RQQERRNWQT IEGIKELKQG YLSQAVHRIA ELMVAYKAVV
961 ALEDLNMGFK RGRQKVESSV YQQFEKQLID KLNYLVDKKK RPEDIGGLLR AYQFTAPFKS
1021 FKEMGKQNGF LFYIPAWNTS NIDPTTGFVN LFHAQYENVD KAKSFFQKFD SISYNPKKDW
1081 FEFAFDYKNF TKKAEGSRSM WILCTHGSRI KNFRNSQKNG QWDSEEFALT EAFKSLFVRY
 
1141 EIDYTADLKT AIVDEKQKDF FVDLLKLFKL TVQMRNSWKE KDLDYLISPV AGADGRFFDT
(SEQ ID NO:144)
Francisella tularensis Cas12a (Ft Cas12a) 
1 MSIYQEFVNK YSLSKTLRFE LIPQGKTLEN IKARGLILDD EKRAKDYKKA KQIIDKYHQF
61 FIEEILSSVC ISEDLLQNYS DVYFKLKKSD DDNLQKDFKS AKDTIKKQIS KYINDSEKFK
121 NLFNQNLIDA KKGQESDLIL WLKQSKDNGI ELFKANSDIT DIDEALEIIK SFKGWTTYFK
181 GFHENRKNVY SSNDIPTSII YRIVDDNLPK FLENKAKYES LKDKAPEAIN YEQIKKDLAE
241 ELTFDIDYKT SEVNQRVFSL DEVFEIANFN NYLNQSGITK FNTIIGGKFV NGENTKRKGI
301 NEYINLYSQQ INDKTLKKYK MSVLFKQILS DTESKSFVID KLEDDSDVVT TMQSFYEQIA
361 AFKTVEEKSI KETLSLLFDD LKAQKLDLSK IYFKNDKSLT DLSQQVFDDY SVISTAVLEY
421 ITQQVAPKNL DNPSKKEQEL IAKKTEKAKY LSLETIKLAL EEFNKHRDID KQCRFEEILA
481 NFAAIPMIFD EIAQNKDNLA QISIKYQNQG KKDLLQASAE DDVKAIKDLL DQTNNLLHRL
541 KIFHISQSED KANILDKDEH FYLVFEECYF ELANIVPLYN KIRNYITQKP YSDEKFKLNF
601 ENSTLANGWD KNKEPDNTAI LFIKDDKYYL GVMNKKNNKI FDDKAIKENK GEGYKKIVYK
661 LLPGANKMLP KVFFSAKSIK FYNPSEDILR IRNHSTHTKN GSPQKGYEKF EFNIEDCRKF
721 IDFYKQSISK HPEWKDFGFR FSDTQRYNSI DEFYREVENQ GYKLTFENIS ESYIDSVVNQ
781 GKLYLFQIYN KDFSAYSKGR PNLHTLYWKA LFDERNLQDV VYKLNGEAEL FYRKQSIPKK
841 ITHPAKETIA NKNKDNPKKE SVFEYDLIKD KRFTEDKFFF HCPITINFKS SGANKFNDEI
901 NLLLKEKAND VHILSIDRGE RHLAYYTLVD GKGNIIKQDT FNIIGNDRMK TNYHDKLAAI
961 EKDRDSARKD WKKINNIKEM KEGYLSQVVH EIAKLVIEHN AIVVFEDLNF GFKRGRFKVE
1021 KQVYQKLEKM LIEKLNYLVF KDNEFDKTGG VLRAYQLTAP FETFKKMGKQ TGIIYYVPAG
1081 FTSKICPVTG FVNQLYPKYE SVSKSQEFFS KFDKICYNLD KGYFEFSFDY KNFGDKAAKG
1141 KWTIASFGSR LINFRNSDKN HNWDTREVYP TKELEKLLKD YSIEYGHGEC IKAAICGESD
1201 KKFFAKLTSV LNTILQMRNS KTGTELDYLI SPVADVNGNF FDSRQAPKNM PQDADANGAY
1261 HIGLKGLMLL DRIKNNQEGK KLNLVIKNEE YFEFVQNRNN (SEQ ID NO:145)
Eubacterium ventriosum Cas12a 
1 MESNNKIFTE TIGTSSIAKT MRNSLVPTES TKRNIEKNGI IIDDQLRAEK RQQLKEIMDE
61 YYRAYIDSKL SNVALTRTID WKELFQAIEN NYKQNTTKTK NELEKKQKEK RTEIYKILSD
121 DEEFKQLFNA KLLTNILPEF IKNQNIDNEE KQEKISTVEL FQRFTSSFTD FFKNRKNVFS
181 KDEISTSICY RVVQENAWIF YQNLLAFEEI KKTAEQEIKK IEAENRDSIS DYSLKEIFDF
241 DFYGLLLNQG GIRFYNDVCG KINYHMNLYG QKHNIKSNKF KMKRMHKQIL SIDESTFEVP
301 TMFENDKEVY QVLNEFLSDL ASKKILERVE KIGENVSEYE INKIYIQSKN FENFSSFMCG
361 NWQIINDSLK TYYNEKIKSK GKAKVEKVKK AIKAIEYKSL ADINQLVERY NHDELNRKAE
421 EYISAINEKI KDLYVNEIEF DEKTNLIENE TKSEEIKSKL DSIMEIMHWT KMFIIEEEIE
481 KDVNFYNEIE EIYDELQPLV TIYNRIRNYV TQKPYSEEKI KLNFGIPTLA NGWSKTKEYD
541 NNAIIMIRDG KYYLGIFNAK NKPDKKIMEG HQSEENGDYK KMIYRLLPGP NKMLPKVFMS
601 KTGIAEYKPS QYILECYEQN KHIKSDKNFD IKFCRDLIDF FKTSINRHPE WSKFNFKFSE
661 TSEYEDISTF YREVEKQGYK IEWTYISEKE IKELDENGQL YLFQIYNKDF SEKSKGKENL
721 HTMYLKNLFS EENLKNIVLK LNGEAEVFFR KSSIKKPIIH KKGSVLVNKT YNENGERKSI
 
781 PEEQYTEIYK YLNSIGTNEL SEKSKKLMEE GKVEYYKANY DIVKDYRYSV DKFFIHLPMT
841 INFKAAGFSP INNIALKSIA LKEDMHIIGI DRGERNLIYV SVIDTKGNIV EQRNFNIVNG
901 IDYKEKLKQK ELDRDNARKN WKEIGKIKDL KEGYLSLVVH EIAKLVVKYN AIITMEDLNQ
961 GFKRGRFKVE RQVYQKFETM LINKLNYLVD KDLAVDQEGG LLRGYQLTYI PESLKVLGRQ
1021 CGYIFYVPAA YTSKIDPTTG FVAIFNYKGM TDKDFVTSFD SIKYDDERGL FAFEFDYENF
1081 VTHKVEMARN KWTVYTYGER IKRKFKNGLW DTAEKVDLTY QMRSILEKYE IEYNKGQDIL
1141 EQIEELDEKA QNGICKEIKY LVKDIVQMRN SLPDNAVEDY DAIISPVINN NGEFFDSTRG
1201 DEDKPLDADA NGAYCIALKG LYEVMQIKKN WNEETEFPRK ELKIRHQDWF DFIQNKRYL
(SEQ ID NO:146)
Cas12b sequences
   Alicyclobacillus acidoterrestris Cas12b 
1 MAVKSIKVKL RLDDMPEIRA GLWKLHKEVN AGVRYYTEWL SLLRQENLYR RSPNGDGEQE
61 CDKTAEECKA ELLERLRARQ VENGHRGPAG SDDELLQLAR QLYELLVPQA IGAKGDAQQI
121 ARKFLSPLAD KDAVGGLGIA KAGNKPRWVR MREAGEPGWE EEKEKAETRK SADRTADVLR
181 ALADFGLKPL MRVYTDSEMS SVEWKPLRKG QAVRTWDRDM FQQAIERMMS WESWNQRVGQ
241 EYAKLVEQKN RFEQKNFVGQ EHLVHLVNQL QQDMKEASPG LESKEQTAHY VTGRALRGSD
301 KVFEKWGKLA PDAPFDLYDA EIKNVQRRNT RRFGSHDLFA KLAEPEYQAL WREDASFLTR
361 YAVYNSILRK LNHAKMFATF TLPDATAHPI WTRFDKLGGN LHQYTFLFNE FGERRHAIRF
421 HKLLKVENGV AREVDDVTVP ISMSEQLDNL LPRDPNEPIA LYFRDYGAEQ HFTGEFGGAK
481 IQCRRDQLAH MHRRRGARDV YLNVSVRVQS QSEARGERRP PYAAVFRLVG DNHRAFVHFD
541 KLSDYLAEHP DDGKLGSEGL LSGLRVMSVD LGLRTSASIS VFRVARKDEL KPNSKGRVPF
601 FFPIKGNDNL VAVHERSQLL KLPGETESKD LRAIREERQR TLRQLRTQLA YLRLLVRCGS
661 EDVGRRERSW AKLIEQPVDA ANHMTPDWRE AFENELQKLK SLHGICSDKE WMDAVYESVR
721 RVWRHMGKQV RDWRKDVRSG ERPKIRGYAK DVVGGNSIEQ IEYLERQYKF LKSWSFFGKV
781 SGQVIRAEKG SRFAITLREH IDHAKEDRLK KLADRIIMEA LGYVYALDER GKGKWVAKYP
841 PCQLILLEEL SEYQFNNDRP PSENNQLMQW SHRGVFQELI NQAQVHDLLV GTMYAAFSSR
901 FDARTGAPGI RCRRVPARCT QEHNPEPFPW WLNKFVVEHT LDACPLRADD LIPTGEGEIF
961 VSPFSAEEGD FHQIHADLNA AQNLQQRLWS DFDISQIRLR CDWGEVDGEL VLIPRLTGKR
1021 TADSYSNKVF YTNTGVTYYE RERGKKRRKV FAQEKLSEEE AELLVEADEA REKSVVLMRD
1081 PSGIINRGNW TRQKEFWSMV NQRIEGYLVK QIRSRVPLQD SACENTGDI (SEQ ID NO:147)
Alicyclobacillus kakegawensis Cas12b 
1 MAVKSIKVKL RLSECPDILA GMWQLHRATN AGVRYYTEWV SLMRQEILYS RGPDGGQQCY
61 MTAEDCQREL LRRLRNRQLH NGRQDQPGTD ADLLAISRRL YEILVLQSIG KRGDAQQIAS
121 SFLSPLVDPN SKGGRGEAKS GRKPAWQKMR DQGDPRWVAA REKYEQRKAV DPSKEILNSL
181 DALGLRPLFA VFTETYRSGV DWKPLGKSQG VRTWDRDMFQ QALERLMSWE SWNRRVGEEY
241 ARLFQQKMKF EQEHFAEQSH LVKLARALEA DMRAASQGFE AKRGTAHQIT RRALRGADRV
301 FEIWKSIPEE ALFSQYDEVI RQVQAEKRRD FGSHDLFAKL AEPKYQPLWR ADETFLTRYA
361 LYNGVLRDLE KARQFATFTL PDACVNPIWT RFESSQGSNL HKYEFLFDHL GPGRHAVRFQ
 
421 RLLVVESEGA KERDSVVVPV APSGQLDKLV LREEEKSSVA LHLHDTARPD GFMAEWAGAK
481 LQYERSTLAR KARRDKQGMR SWRRQPSMLM SAAQMLEDAK QAGDVYLNIS VRVKSPSEVR
541 GQRRPPYAAL FRIDDKQRRV TVNYNKLSAY LEEHPDKQIP GAPGLLSGLR VMSVDLGLRT
601 SASISVFRVA KKEEVEALGD GRPPHYYPIH GTDDLVAVHE RSHLIQMPGE TETKQLRKLR
661 EERQAVLRPL FAQLALLRLL VRCGAADERI RTRSWQRLTK QGREFTKRLT PSWREALELE
721 LTRLEAYCGR VPDDEWSRIV DRTVIALWRR MGKQVRDWRK QVKSGAKVKV KGYQLDVVGG
781 NSLAQIDYLE QQYKFLRRWS FFARASGLVV RADRESHFAV ALRQHIENAK RDRLKKLADR
841 ILMEALGYVY EASGPREGQW TAQHPPCQLI ILEELSAYRF SDDRPPSENS KLMAWGHRGI
901 LEELVNQAQV HDVLVGTVYA AFSSRFDART GAPGVRCRRV PARFVGATVD DSLPLWLTEF
961 LDKHRLDKNL LRPDDVIPTG EGEFLVSPCG EEAARVRQVH ADINAAQNLQ RRLWQNFDIT
1021 ELRLRCDVKM GGEGTVLVPR VNNARAKQLF GKKVLVSQDG VTFFERSQTG GKPHSEKQTD
1081 LTDKELELIA EADEARAKSV VLFRDPSGHI GKGHWIRQRE FWSLVKQRIE SHTAERIRVR
1141 GVGSSLD (SEQ ID NO:148)
Alicyclobacillus macrosporangiidus Cas12b 
1 MAVKSIKVKL MLGHLPEIRE GLWHLHEAVN LGVRYYTEWL ALLRQGNLYR RGKDGAQECY
61 MTAEQCRQEL LVRLRDRQKR NGHTGDPGTD EELLGVARRL YELLVPQSVG KKGQAQMLAS
121 GFLSPLADPK SEGGKGTSKS GRKPAWMGMK EAGDSRWVEA KARYEANKAK DPTKQVIASL
181 EMYGLRPLFD VFTETYKTIR WMPLGKHQGV RAWDRDMFQQ SLERLMSWES WNERVGAEFA
241 RLVDRRDRFR EKHFTGQEHL VALAQRLEQE MKEASPGFES KSSQAHRITK RALRGADGII
301 DDWLKLSEGE PVDRFDEILR KRQAQNPRRF GSHDLFLKLA EPVFQPLWRE DPSFLSRWAS
361 YNEVLNKLED AKQFATFTLP SPCSNPVWAR FENAEGTNIF KYDFLFDHFG KGRHGVRFQR
421 MIVMRDGVPT EVEGIVVPIA PSRQLDALAP NDAASPIDVF VGDPAAPGAF RGQFGGAKIQ
481 YRRSALVRKG RREEKAYLCG FRLPSQRRTG TPADDAGEVF LNLSLRVESQ SEQAGRRNPP
541 YAAVFHISDQ TRRVIVRYGE IERYLAEHPD TGIPGSRGLT SGLRVMSVDL GLRTSAAISV
601 FRVAHRDELT PDAHGRQPFF FPIHGMDHLV ALHERSHLIR LPGETESKKV RSIREQRLDR
661 LNRLRSQMAS LRLLVRTGVL DEQKRDRNWE RLQSSMERGG ERMPSDWWDL FQAQVRYLAQ
721 HRDASGEAWG RMVQAAVRTL WRQLAKQVRD WRKEVRRNAD KVKIRGIARD VPGGHSLAQL
781 DYLERQYRFL RSWSAFSVQA GQVVRAERDS RFAVALREHI DNGKKDRLKK LADRILMEAL
841 GYVYVTDGRR AGQWQAVYPP CQLVLLEELS EYRFSNDRPP SENSQLMVWS HRGVLEELIH
901 QAQVHDVLVG TIPAAFSSRF DARTGAPGIR CRRVPSIPLK DAPSIPIWLS HYLKQTERDA
961 AALRPGELIP TGDGEFLVTP AGRGASGVRV VHADINAAHN LQRRLWENFD LSDIRVRCDR
1021 REGKDGTVVL IPRLTNQRVK ERYSGVIFTS EDGVSFTVGD AKTRRRSSAS QGEGDDLSDE
1081 EQELLAEADD ARERSVVLFR DPSGFVNGGR WTAQRAFWGM VHNRIETLLA ERFSVSGAAE
1141 KVRG (SEQ ID NO:149)
Alicyclobacillus hesperidum Cas12b 
1 MTVRSIRVKL AVGSPQYRDV RRGLWKTHEI MNQGVRYYCE WLVLMRQEPI YDEDEHGLTV
61 VQRTREDIQA ELLSRLRTLQ SAHQHSGDMG TDEELLSLMR QLYEQLVPSS VDKNKSGDAR
121 MIARNFFNPL TNPNSQGGLG ISNAGRKPKW LLKKLSGDPT WEEDYKKAME QKQESSVSFL
181 LLELRRFGLH PIFLPYTDTV LEVSWAPKKA RQWVRKWDYD LFQQSIERML SWESWTRRVK
 
241 ERFEKLVESE KKFYDENFAT DPEFIKLAET LEGELQASSQ GFVAVDEHAF QIRPRSMRGF
301 DRVADEWCKL ADDAPIEEYE AAIKRVQARL GRNFGSYVLF AHLAKPEYWS LWRSDPTKIL
361 RFARLRALQR AVARAKRHAR LTLPDAIHHP IWIRYDAKGK NIYSYRLLIP EKRSKRYYVE
421 FSSLIMPDGE NRWAEHRNIR VPLAFSRQWE RLHFSIMEDG SLCVQYRDPG VDEPLRAELG
481 GAKIQFDRRY LIRRSSTLSA GECGPVYLNV SVDVNPAHRP DVQVLQSAKL VSVSRDTNRI
541 YLRPENLSAY WKSQGDGTLP LRVMSVDLGV RSSAAVVICR LEHRDSVVSS GRRTATIYRI
601 AGTDEFVAVQ ERAFLLRLPG EGKGTNEDAP LRDVYAQLGT IRQGIQILRS LLRLCDTKTP
661 DERQEALHGL AQSLEPSGAW KDELHPHLVM LQGVVHDSVD NWKQKVISVH RQMERILGHA
721 VREWKVARKN AGKPPIRRGA GGLSLRRIRQ LEQERRTLVA WSNHAREPGQ VVRIKRGTQV
781 AQWLVERVNH LKEDRLKKLA DLLIMTALGY VYDETKPSGH KWDKRYPPCQ IILMEDLSRY
841 RFQSDRPPSE NSQLMAWSHR RLLEILKLQA DLHKLIVGTV FPAFSSRFDA QSGAPGVRCR
901 SVKKQDIENA AQGKGWLARE LQRLNWTLEW LQPNDLIPTG DGELFVTPAC CDRQKGIKIV
961 HADLNAAQNL QRRFWGGHAE SLCRVTCDVV ERDGRRYAVP RISNAFADSF YKVFGQGVFV
1021 STDEEDVYRW MVGEKISSRG RSRGRTSDEE AEAETWIDEA REQQGKVIAL FRDASGQIHG
1081 GDWLVAKVFW GWVERLVTAR LLSRMSEREA AAHKE (SEQ ID NO:150)
Sulfobacillus thermotolerans Cas12b 
1 MSARNIKVKI DTKGNPELRL GLWKTHQVTN EGVKYYTEWL IKLRQQDIYR QSREDASPRV
61 IISASDLKAD LLCHARQLQK ERLPRITGSD AEILGTLRQV YELIVPSSVG KSGDSKTLAR
121 KFLSPLTDPG SAGGRDQSAS GRKPTWMKMK SEGNPRWEET FRKWKDRKDN DPTPLVLNQI
181 ADYGLLPLIP LFTDVGENIF DPKSKSQFVR TWDRSMFQQA IERLMSWESW NQRVRREWEA
241 LNQKHSAFYR EQFTADPDAA LYRVAQSLEE EMRKEHQGFA SDAPEAFRIR RVALKGFDRL
301 LERWQKTLGK NGQSATLLDD IRRVQSDLGD KFGSAPLYQK LLDERWQRLW AVDPTFLQRY
361 AAFNDLTQRL QRAKRVANLT LPDAVAHPIW SRYEGANASS GNRYHIHLPT KGQPGSVTFD
421 RILWPDGNGG WYERKRVTVF LRPSHQVDRI HEAPTDSVVD NFPLVVEDQS ARTILRASWG
481 GAKLEYDRNR LPRQLKKGVP DSIYLSLTLN LDTNKPSGLF HTQQNGRVWI RKDVLMQYYN
541 ETPGDNVQFK PLYVMSVDLG IRSAAAVSIF SVQLKAGIEE HRLTYPVADC PGLVAVHERS
601 VLLTMPGERR EQWDRRYEQQ RQGLRELRTD MRGMNDLLRG AYMDGDRREE FLARLSKLEE
661 TSPELWGPVY RSLNDSKVAS ATEWERLVVY CHRQVEQSLS SRIQNLRSGR SAYRMSGGLS
721 LDHVQDLERI RGIIASWTNH PRIPGSVVRW QQGRSHTVAL GRHILELKRD RVKKVANYLI
781 MTTLGYAYDS KRARGEKWVR RYPACHLMVF EDLTRYRFRT DRPRSENRQL MRWTHQELIA
841 VTGIQAEPHG ISVGTMYAGF SSRFDAVTKA PGVRGATVRQ ILRTRGMVRL KEIAADVGID
901 INTLRPHDVL PTGDGEYLLS VVRHGESYRL KQVHADINAA HNLQRRLWTQ DEVFRVSCRL
961 ALNSGRVVAM PPPSYNKRYG KGFFEKGDNG VYIWKTGGKI KISDTLEEDM DIPEDTAELL
1021 RGNSVTLFRD PSGTIAGGNW LEAKEFWGRV NSLVNKGVRD KILGGIPVDN SSAHAE (SEQ ID
NO:151)
Cas12c (c2c3) sequences
   Cas12c1 (see Yan et al (2019) Science Vol. 363, Issue 6422, pp. 88-91) MQTKKTHLHLISAKASRKYRRTIACLSDTAKKDLERRKQSGAADPAQELSCLKT 
IKFKLEVPEGSKLPSFDRISQIYNALETIEKGSLSYLLFALILSGFRIFPNSSA
AKTFASSSCYKNDQFASQIKEIFGEMVKNFIPSELESILKKGRRKNNKDWTEEN
IKRVLNSEFGRKNSEGSSALFDSFLSKFSQELFRKFDSWNEVNKKYLEAAELLD
SMLASYGPFDSVCKMIGDSDSRNSLPDKSTIAFTNNAEITVDIESSVMPYMAIA
ALLREYRQSKSKAAPVAYVQSHLTTTNGNGLSWFFKFGLDLIRKAPVSSKQSTS
DGSKSLQELFSVPDDKLDGLKFIKEACEALPEASLLCGEKGELLGYQDFRTSFA
GHIDSWVANYVNRLFELIELVNQLPESIKLPSILTQKNHNLVASLGLQEAEVSH
SLELFEGLVKNVRQTLKKLAGIDISSSPNEQDIKEFYAFSDVLNRLGSIRNQIE
NAVQTAKKDKIDLESAIEWKEWKKLKKLPKLNGLGGGVPKQQELLDKALESVKQ
IRHYQRIDFERVIQWAVNEHCLETVPKFLVDAEKKKINKESSTDFAAKENAVRF
LLEGIGAAARGKTDSVSKAAYNWFVVNNFLAKKDLNRYFINCQGCIYKPPYSKR
RSLAFALRSDNKDTIEVVWEKFETFYKEISKEIEKFNIFSQEFQTFLHLENLRM
KLLLRRIQKPIPAEIAFFSLPQEYYDSLPPNVAFLALNQEITPSEYITQFNLYS
SFLNGNLILLRRSRSYLRAKFSWVGNSKLIYAAKEARLWKIPNAYWKSDEWKMI
LDSNVLVFDKAGNVLPAPTLKKVCEREGDLRLFYPLLRQLPHDWCYRNPFVKSV
GREKNVIEVNKEGEPKVASALPGSLFRLIGPAPFKSLLDDCFFNPLDKDLRECM
LIVDQEISQKVEAQKVEASLESCTYSIAVPIRYHLEEPKVSNQFENVLAIDQGE
AGLAYAVFSLKSIGEAETKPIAVGTIRIPSIRRLIHSVSTYRKKKQRLQNFKQN
YDSTAFIMRENVTGDVCAKIVGLMKEFNAFPVLEYDVKNLESGSRQLSAVYKAV
NSHFLYFKEPGRDALRKQLWYGGDSWTIDGIEIVTRERKEDGKEGVEKIVPLKV
FPGRSVSARFTSKTCSCCGRNVFDWLFTEKKAKTNKKFNVNSKGELTTADGVIQ
LFEADRSKGPKFYARRKERTPLTKPIAKGSYSLEEIERRVRTNLRRAPKSKQSR
DTSQSQYFCVYKDCALHFSGMQADENAAINIGRRFLTALRKNRRSDFPSNVKIS
DRLLDN** (SEQ ID NO:152)
Cas12c2 (see Yan et al (2019) Science Vol. 363, Issue 6422, pp. 88-91) 
MTKHSIPLHAFRNSGADARKWKGRIALLAKRGKETMRTLQFPLEMSEPEAAAIN
TTPFAVAYNAIEGTGKGTLFDYWAKLHLAGFRFFPSGGAATIFRQQAVFEDASW
NAAFCQQSGKDWPWLVPSKLYERFTKAPREVAKKDGSKKSIEFTQENVANESHV
SLVGASITDKTPEDQKEFFLKMAGALAEKFDSWKSANEDRIVAMKVIDEFLKSE
GLHLPSLENIAVKCSVETKPDNATVAWHDAPMSGVQNLAIGVFATCASRIDNIY
DLNGGKLSKLIQESATTPNVTALSWLFGKGLEYFRTTDIDTIMQDFNIPASAKE
SIKPLVESAQAIPTMTVLGKKNYAPFRPNFGGKIDSWIANYASRLMLLNDILEQ
IEPGFELPQALLDNETLMSGIDMTGDELKELIEAVYAWVDAAKQGLATLLGRGG
NVDDAVQTFEQFSAMMDTLNGTLNTISARYVRAVEMAGKDEARLEKLIECKFDI
PKWCKSVPKLVGISGGLPKVEEEIKVMNAAFKDVRARMFVRFEEIAAYVASKGA
GMDVYDALEKRELEQIKKLKSAVPERAHIQAYRAVLHRIGRAVQNCSEKTKQLF
SSKVIEMGVFKNPSHLNNFIFNQKGAIYRSPFDRSRHAPYQLHADKLLKNDWLE
LLAEISATLMASESTEQMEDALRLERTRLQLQLSGLPDWEYPASLAKPDIEVEI
QTALKMQLAKDTVTSDVLQRAFNLYSSVLSGLTFKLLRRSFSLKMRFSVADTTQ
LIYVPKVCDWAIPKQYLQAEGEIGIAARVVTESSPAKMVTEVEMKEPKALGHFM
 
QQAPHDWYFDASLGGTQVAGRIVEKGKEVGKERKLVGYRMRGNSAYKTVLDKSL
VGNTELSQCSMIIEIPYTQTVDADFRAQVQAGLPKVSINLPVKETITASNKDEQ
MLFDRFVAIDLGERGLGYAVFDAKTLELQESGHRPIKAITNLLNRTHHYEQRPN
QRQKFQAKFNVNLSELRENTVGDVCHQINRICAYYNAFPVLEYMVPDRLDKQLK
SVYESVTNRYIWSSTDAHKSARVQFWLGGETWEHPYLKSAKDKKPLVLSPGRGA
SGKGTSQTCSCCGRNPFDLIKDMKPRAKIAVVDGKAKLENSELKLFERNLESKD
DMLARRHRNERAGMEQPLTPGNYTVDEIKALLRANLRRAPKNRRTKDTTVSEYH
CVFSDCGKTMHADENAAVNIGGKFIADIEK** (SEQ IN NO:153)
Cas12c3 (see Yan et al (2019) Science Vol. 363, Issue 6422, pp. 88-91) 
MTKLRHRQKKLTHDWAGSKKREVLGSNGKLQNPLLMPVKKGQVTEFRKAFSAYA
RATKGEMTDGRKNMFTHSFEPFKTKPSLHQCELADKAYQSLHSYLPGSLAHFLL
SAHALGFRIFSKSGEATAFQASSKIEAYESKLASELACVDLSIQNLTISTLFNA
LTTSVRGKGEETSADPLIARFYTLLTGKPLSRDTQGPERDLAEVISRKIASSFG
TWKEMTANPLQSLQFFEEELHALDANVSLSPAFDVLIKMNDLQGDLKNRTIVFD
PDAPVFEYNAEDPADIIIKLTARYAKEAVIKNQNVGNYVKNAITTTNANGLGWL
LNKGLSLLPVSTDDELLEFIGVERSHPSCHALIELIAQLEAPELFEKNVFSDTR
SEVQGMIDSAVSNHIARLSSSRNSLSMDSEELERLIKSFQIHTPHCSLFIGAQS
LSQQLESLPEALQSGVNSADILLGSTQYMLTNSLVEESIATYQRTLNRINYLSG
VAGQINGAIKRKAIDGEKIHLPAAWSELISLPFIGQPVIDVESDLAHLKNQYQT
LSNEFDTLISALQKNFDLNFNKALLNRTQHFEAMCRSTKKNALSKPEIVSYRDL
LARLTSCLYRGSLVLRRAGIEVLKKHKIFESNSELREHVHERKHFVFVSPLDRK
AKKLLRLTDSRPDLLHVIDEILQHDNLENKDRESLWLVRSGYLLAGLPDQLSSS
FINLPIITQKGDRRLIDLIQYDQINRDAFVMLVTSAFKSNLSGLQYRANKQSFV
VTRTLSPYLGSKLVYVPKDKDWLVPSQMFEGRFADILQSDYMVWKDAGRLCVID
TAKHLSNIKKSVFSSEEVLAFLRELPHRTFIQTEVRGLGVNVDGIAFNNGDIPS
LKTFSNCVQVKVSRTNTSLVQTLNRWFEGGKVSPPSIQFERAYYKKDDQIHEDA
AKRKIRFQMPATELVHASDDAGWTPSYLLGIDPGEYGMGLSLVSINNGEVLDSG
FIHINSLINFASKKSNHQTKVVPRQQYKSPYANYLEQSKDSAAGDIAHILDRLI
YKLNALPVFEALSGNSQSAADQVWTKVLSFYTWGDNDAQNSIRKQHWFGASHWD
IKGMLRQPPTEKKPKPYIAFPGSQVSSYGNSQRCSCCGRNPIEQLREMAKDTSI
KELKIRNSEIQLFDGTIKLFNPDPSTVIERRRHNLGPSRIPVADRTFKNISPSS
LEFKELITIVSRSIRHSPEFIAKKRGIGSEYFCAYSDCNSSLNSEANAAANVAQ
KFQKQLFFEL** (SEQ ID NO:154)
Cas12d (CasY) sequences
   Candidatus Katanobacteria Cas12d (CasY.1) MOEH01000029 
MRKKLFKGYILHNKRLVYTGKAAIRSIKYPLVAPNKTALNNLSEKIIYDYEHLFGPLNVA
SYARNSNRYSLVDFWIDSLRAGVIWQSKSTSLIDLISKLEGSKSPSEKIFEQIDFELKNK
LDKEQFKDIILLNTGIRSSSNVRSLRGRFLKCFKEEFRDTEEVIACVDKWSKDLIVEGKS
 
ILVSKQFLYWEEEFGIKIFPHFKDNHDLPKLTFFVEPSLEFSPHLPLANCLERLKKFDIS
RESLLGLDNNFSAFSNYFNELFNLLSRGEIKKIVTAVLAVSKSWENEPELEKRLHFLSEK
AKLLGYPKLTSSWADYRMIIGGKIKSWHSNYTEQLIKVREDLKKHQIALDKLQEDLKKVV
DSSLREQIEAQREALLPLLDTMLKEKDFSDDLELYRFILSDFKSLLNGSYQRYIQTEEER
KEDRDVTKKYKDLYSNLRNIPRFFGESKKEQFNKFINKSLPTIDVGLKILEDIRNALETV
SVRKPPSITEEYVTKQLEKLSRKYKINAFNSNRFKQITEQVLRKYNNGELPKISEVFYRY
PRESHVAIRILPVKISNPRKDISYLLDKYQISPDWKNSNPGEVVDLIEIYKLTLGWLLSC
NKDFSMDFSSYDLKLFPEAASLIKNFGSCLSGYYLSKMIFNCITSEIKGMITLYTRDKFV
VRYVTQMIGSNQKFPLLCLVGEKQTKNFSRNWGVLIEEKGDLGEEKNQEKCLIFKDKTDF
AKAKEVEIFKNNIWRIRTSKYQIQFLNRLFKKTKEWDLMNLVLSEPSLVLEEEWGVSWDK
DKLLPLLKKEKSCEERLYYSLPLNLVPATDYKEQSAEIEQRNTYLGLDVGEFGVAYAVVR
IVRDRIELLSWGFLKDPALRKIRERVQDMKKKQVMAVFSSSSTAVARVREMAIHSLRNQI
HSIALAYKAKIIYEISISNFETGGNRMAKIYRSIKVSDVYRESGADTLVSEMIWGKKNKQ
MGNHISSYATSYTCCNCARTPFELVIDNDKEYEKGGDEFIFNVGDEKKVRGFLQKSLLGK
TIKGKEVLKSIKEYARPPIREVLLEGEDVEQLLKRRGNSYIYRCPFCGYKTDADIQAALN
IACRGYISDNAKDAVKEGERKLDYILEVRKLWEKNGAVLRSAKFL (SEQ ID NO:155)
Candidatus Vogelbacteria Cas12d (CasY.2) MOEJ01000028 
MQKVRKTLSEVHKNPYGTKVRNAKTGYSLQIERLSYTGKEGMRSFKIPLENKNKEVFDEF
VKKIRNDYISQVGLLNLSDWYEHYQEKQEHYSLADFWLDSLRAGVIFAHKETEIKNLISK
IRGDKSIVDKFNASIKKKHADLYALVDIKALYDFLTSDARRGLKTEEEFFNSKRNTLFPK
FRKKDNKAVDLWVKKFIGLDNKDKLNFTKKFIGFDPNPQIKYDHTFFFHQDINFDLERIT
TPKELISTYKKFLGKNKDLYGSDETTEDQLKMVLGFHNNHGAFSKYFNASLEAFRGRDNS
LVEQIINNSPYWNSHRKELEKRIIFLQVQSKKIKETELGKPHEYLASFGGKFESWVSNYL
RQEEEVKRQLFGYEENKKGQKKFIVGNKQELDKIIRGTDEYEIKAISKETIGLTQKCLKL
LEQLKDSVDDYTLSLYRQLIVELRIRLNVEFQETYPELIGKSEKDKEKDAKNKRADKRYP
QIFKDIKLIPNFLGETKQMVYKKFIRSADILYEGINFIDQIDKQITQNLLPCFKNDKERI
EFTEKQFETLRRKYYLMNSSRFHHVIEGIINNRKLIEMKKRENSELKTFSDSKFVLSKLF
LKKGKKYENEVYYTFYINPKARDQRRIKIVLDINGNNSVGILQDLVQKLKPKWDDIIKKN
DMGELIDAIEIEKVRLGILIALYCEHKFKIKKELLSLDLFASAYQYLELEDDPEELSGTN
LGRFLQSLVCSEIKGAINKISRTEYIERYTVQPMNTEKNYPLLINKEGKATWHIAAKDDL
SKKKGGGTVAMNQKIGKNFFGKQDYKTVFMLQDKRFDLLTSKYHLQFLSKTLDTGGGSWW
KNKNIDLNLSSYSFIFEQKVKVEWDLTNLDHPIKIKPSENSDDRRLFVSIPFVIKPKQTK
RKDLQTRVNYMGIDIGEYGLAWTIINIDLKNKKINKISKQGFIYEPLTHKVRDYVATIKD
NQVRGTFGMPDTKLARLRENAITSLRNQVHDIAMRYDAKPVYEFEISNFETGSNKVKVIY
DSVKRADIGRGQNNTEADNTEVNLVWGKTSKQFGSQIGAYATSYICSFCGYSPYYEFENS
KSGDEEGARDNLYQMKKLSRPSLEDFLQGNPVYKTFRDFDKYKNDQRLQKTGDKDGEWKT
HRGNTAIYACQKCRHISDADIQASYWIALKQVVRDFYKDKEMDGDLIQGDNKDKRKVNEL
NRLIGVHKDVPIINKNLITSLDINLL (SEQ ID NO:156)
Candidatus Vogelbacteria Cas12d (CasY.3) MOEK01000006 
MKAKKSFYNQKRKFGKRGYRLHDERIAYSGGIGSMRSIKYELKDSYGIAGLRNR
IADATISDNKWLYGNINLNDYLEWRSSKTDKQIEDGDRESSLLGFWLEALRLGFVFSKQS
HAPNDFNETALQDLFETLDDDLKHVLDRKKWCDFIKIGTPKTNDQGRLKKQIKNLLKGNK
REEIEKTLNESDDELKEKINRIADVFAKNKSDKYTIFKLDKPNTEKYPRINDVQVAFFCH
PDFEEITERDRTKTLDLIINRFNKRYEITENKKDDKTSNRMALYSLNQGYIPRVLNDLFL
FVKDNEDDFSQFLSDLENFFSFSNEQIKIIKERLKKLKKYAEPIPGKPQLADKWDDYASD
FGGKLESWYSNRIEKLKKIPESVSDLRNNLEKIRNVLKKQNNASKILELSQKIIEYIRDY
GVSFEKPEIIKFSWINKTKDGQKKVFYVAKMADREFIEKLDLWMADLRSQLNEYNQDNKV
SFKKKGKKIEELGVLDFALNKAKKNKSTKNENGWQQKLSESIQSAPLFFGEGNRVRNEEV
YNLKDLLFSEIKNVENILMSSEAEDLKNIKIEYKEDGAKKGNYVLNVLARFYARFNEDGY
GGWNKVKTVLENIAREAGTDFSKYGNNNNRNAGRFYLNGRERQVFTLIKFEKSITVEKIL
ELVKLPSLLDEAYRDLVNENKNHKLRDVIQLSKTIMALVLSHSDKEKQIGGNYIHSKLSG
YNALISKRDFISRYSVQTTNGTQCKLAIGKGKSKKGNEIDRYFYAFQFFKNDDSKINLKV
IKNNSHKNIDFNDNENKINALQVYSSNYQIQFLDWFFEKHQGKKTSLEVGGSFTIAEKSL
TIDWSGSNPRVGFKRSDTEEKRVFVSQPFTLIPDDEDKERRKERMIKTKNRFIGIDIGEY
GLAWSLIEVDNGDKNNRGIRQLESGFITDNQQQVLKKNVKSWRQNQIRQTFTSPDTKIAR
LRESLIGSYKNQLESLMVAKKANLSFEYEVSGFEVGGKRVAKIYDSIKRGSVRKKDNNSQ
NDQSWGKKGINEWSFETTAAGTSQFCTHCKRWSSLAIVDIEEYELKDYNDNLFKVKINDG
EVRLLGKKGWRSGEKIKGKELFGPVKDAMRPNVDGLGMKIVKRKYLKLDLRDWVSRYGNM
AIFICPYVDCHHISHADKQAAFNIAV (SEQ ID NO:157)
Candidatus Parcubacteria Cas12d (CasY.4) KY040242 
MSKRHPRISGVKGYRLHAQRLEYTGKSGAMRTIKYPLYSSPSGGRTVPREIVSAINDDYV
GLYGLSNFDDLYNAEKRNEEKVYSVLDFWYDCVQYGAVFSYTAPGLLKNVAEVRGGSYEL
TKTLKGSHLYDELQIDKVIKFLNKKEISRANGSLDKLKKDIIDCFKAEYRERHKDQCNKL
ADDIKNAKKDAGASLGERQKKLFRDFFGISEQSENDKPSFTNPLNLTCCLLPFDTVNNNR
NRGEVLFNKLKEYAQKLDKNEGSLEMWEYIGIGNSGTAFSNFLGEGFLGRLRENKITELK
KAMMDITDAWRGQEQEEELEKRLRILAALTIKLREPKFDNHWGGYRSDINGKLSSWLQNY
INQTVKIKEDLKGHKKDLKKAKEMINRFGESDTKEEAVVSSLLESIEKIVPDDSADDEKP
DIPAIAIYRRFLSDGRLTLNRFVQREDVQEALIKERLEAEKKKKPKKRKKKSDAEDEKET
IDFKELFPHLAKPLKLVPNFYGDSKRELYKKYKNAAIYTDALWKAVEKIYKSAFSSSLKN
SFFDTDFDKDFFIKRLQKIFSVYRRFNTDKWKPIVKNSFAPYCDIVSLAENEVLYKPKQS
RSRKSAAIDKNRVRLPSTENIAKAGIALARELSVAGFDWKDLLKKEEHEEYIDLIELHKT
ALALLLAVTETQLDISALDFVENGTVKDFMKTRDGNLVLEGRFLEMFSQSIVFSELRGLA
GLMSRKEFITRSAIQTMNGKQAELLYIPHEFQSAKITTPKEMSRAFLDLAPAEFATSLEP
ESLSEKSLLKLKQMRYYPHYFGYELTRTGQGIDGGVAENALRLEKSPVKKREIKCKQYKT
LGRGQNKIVLYVRSSYYQTQFLEWFLHRPKNVQTDVAVSGSFLIDEKKVKTRWNYDALTV
ALEPVSGSERVFVSQPFTIFPEKSAEEEGQRYLGIDIGEYGIAYTALEITGDSAKILDQN
FISDPQLKTLREEVKGLKLDQRRGTFAMPSTKIARIRESLVHSLRNRIHHLALKHKAKIV
YELEVSRFEEGKQKIKKVYATLKKADVYSEIDADKNLQTTVWGKLAVASEISASYTSQFC
GACKKLWRAEMQVDETITTQELIGTVRVIKGGTLIDAIKDFMRPPIFDENDTPFPKYRDF
 
CDKHHISKKMRGNSCLFICPFCRANADADIQASQTIALLRYVKEEKKVEDYFERFRKLKN
IKVLGQMKKI (SEQ ID NO:158)
Candidatus Komeilibacteria Cas12d (CasY.5) MOEI01000022 
MAESKQMQCRKCGASMKYEVIGLGKKSCRYMCPDCGNHTSARKIQNKKKRD
KKYGSASKAQSQRIAVAGALYPDKKVQTIKTYKYPADLNGEVHDSGVAEKIAQAIQEDEI
GLLGPSSEYACWIASQKQSEPYSVVDFWFDAVCAGGVFAYSGARLLSTVLQLSGEESVLR
AALASSPFVDDINLAQAEKFLAVSRRTGQDKLGKRIGECFAEGRLEALGIKDRMREFVQA
IDVAQTAGQRFAAKLKIFGISQMPEAKQWNNDSGLTVCILPDYYVPEENRADQLVVLLRR
LREIAYCMGIEDEAGFEHLGIDPGALSNFSNGNPKRGFLGRLLNNDIIALANNMSAMTPY
WEGRKGELIERLAWLKHRAEGLYLKEPHFGNSWADHRSRIFSRIAGWLSGCAGKLKIAKD
QISGVRTDLFLLKRLLDAVPQSAPSPDFIASISALDRFLEAAESSQDPAEQVRALYAFHL
NAPAVRSIANKAVQRSDSQEWLIKELDAVDHLEFNKAFPFFSDTGKKKKKGANSNGAPSE
EEYTETESIQQPEDAEQEVNGQEGNGASKNQKKFQRIPRFFGEGSRSEYRILTEAPQYFD
MFCNNMRAIFMQLESQPRKAPRDFKCFLQNRLQKLYKQTFLNARSNKCRALLESVLISWG
EFYTYGANEKKFRLRHEASERSSDPDYVVQQALEIARRLFLFGFEWRDCSAGERVDLVEI
HKKAISFLLAITQAEVSVGSYNWLGNSTVSRYLSVAGTDTLYGTQLEEFLNATVLSQMRG
LAIRLSSQELKDGFDVQLESSCQDNLQHLLVYRASRDLAACKRATCPAELDPKILVLPVG
AFIASVMKMIERGDEPLAGAYLRHRPHSFGWQIRVRGVAEVGMDQGTALAFQKPTESEPF
KIKPFSAQYGPVLWLNSSSYSQSQYLDGFLSQPKNWSMRVLPQAGSVRVEQRVALIWNLQ
AGKMRLERSGARAFFMPVPFSFRPSGSGDEAVLAPNRYLGLFPHSGGIEYAVVDVLDSAG
FKILERGTIAVNGFSQKRGERQEEAHREKQRRGISDIGRKKPVQAEVDAANELHRKYTDV
ATRLGCRIVVQWAPQPKPGTAPTAQTVYARAVRTEAPRSGNQEDHARMKSSWGYTWGTYW
EKRKPEDILGISTQVYWTGGIGESCPAVAVALLGHIRATSTQTEWEKEEVVFGRLKKFFP
S (SEQ ID NO:159)
Candidatus Kerfeldbacteria Cas12d (CasY.6) MHKD01000036 
MAESKQMQCRKCGASMKYEVIGLGKKSCRYMCPDCGNHTSARKIQNKKKRD
KKYGSASKAQSQRIAVAGALYPDKKVQTIKTYKYPADLNGEVHDRGVAEKIEQAIQEDEI
GLLGPSSEYACWIASQKQSEPYSVVDFWFDAVCAGGVFAYSGARLLSTVLQLSGEESVLR
AALASSPFVDDINLAQAEKFLAVSRRTGQDKLGKRIGECFAEGRLEALGIKDRMREFVQA
IDVAQTAGQRFAAKLKIFGISQMPEAKQWNNDSGLTVCILPDYYVPEENRADQLVVLLRR
LREIAYCMGIEDEAGFEHLGIDPGALSNFSNGNPKRGFLGRLLNNDIIALANNMSAMTPY
WEGRKGELIERLAWLKHRAEGLYLKEPHFGNSWADHRSRIFSRIAGWLSGCAGKLKIAKD
QISGVRTDLFLLKRLLDAVPQSAPSPDFIASISALDRFLEAAESSQDPAEQVRALYAFHL
NAPAVRSIANKAVQRSDSQEWLIKELDAVDHLEFNKAFPFFSDTGKKKKKGANSNGAPSE
EEYTETESIQQPEDAEQEVNGQEGNGASKNQKKFQRIPRFFGEGSRSEYRILTEAPQYFD
MFCNNMRAIFMQLESQPRKAPRDFKCFLQNRLQKLYKQTFLNARSNKCRALLESVLISWG
EFYTYGANEKKFRLRHEASERSSDPDYVVQQALEIARRLFLFGFEWRDCSAGERVDLVEI
HKKAISFLLAITQAEVSVGSYNWLGNSTVSRYLSVAGTDTLYGTQLEEFLNATVLSQMRG
LAIRLSSQELKDGFDVQLESSCQDNLQHLLVYRASRDLAACKRATCPAELDPKILVLPAG
 
AFIASVMKMIERGDEPLAGAYLRHRPHSFGWQIRVRGVAEVGMDQGTALAFQKPTESEPF
KIKPFSAQYGPVLWLNSSSYSQSQYLDGFLSQPKNWSMRVLPQAGSVRVEQRVALIWNLQ
AGKMRLERSGARAFFMPVPFSFRPSGSGDEAVLAPNRYLGLFPHSGGIEYAVVDVLDSAG
FKILERGTIAVNGFSQKRGERQEEAHREKQRRGISDIGRKKPVQAEVDAANELHRKYTDV
ATRLGCRIVVQWAPQPKPGTAPTAQTVYARAVRTEAPRSGNQEDHARMKSSWGYTWSTYW
EKRKPEDILGISTQVYWTGGIGESCPAVAVALLGHIRATSTQTEWEKEEVVFGRLKKFFP
S (SEQ ID NO:160)
Cas12e (CasX)
   > Deltaproteobacteria bacterium GWA2 43 19 Cas12e1 (CasX) 
MEKRINKIRKKLSADNATKPVSRSGPMKTLLVRVMTDDLKKRLEKRRKKPEVMPQVISNNAANN
LRMLLDDYTKMKEAILQVYWQEFKDDHVGLMCKFAQPASKKIDQNKLKPEMDEKGNLTTAGFAC
SQCGQPLFVYKLEQVSEKGKAYTNYFGRCNVAEHEKLILLAQLKPEKDSDEAVTYSLGKFGQRA
LDFYSIHVTKESTHPVKPLAQIAGNRYASGPVGKALSDACMGTIASFLSKYQDIIIEHQKVVKG
NQKRLESLRELAGKENLEYPSVTLPPQPHTKEGVDAYNEVIARVRMWVNLNLWQKLKLSRDDAK
PLLRLKGFPSFPVVERRENEVDWWNTINEVKKLIDAKRDMGRVFWSGVTAEKRNTILEGYNYLP
NENDHKKREGSLENPKKPAKRQFGDLLLYLEKKYAGDWGKVFDEAWERIDKKIAGLTSHIEREE
ARNAEDAQSKAVLTDWLRAKASFVLERLKEMDEKEFYACEIQLQKWYGDLRGNPFAVEAENRVV
DISGFSIGSDGHSIQYRNLLAWKYLENGKREFYLLMNYGKKGRIRFTDGTDIKKSGKWQGLLYG
GGKAKVIDLTFDPDDEQLIIILPLAFGTRQGREFIWNDLLSLETGLIKLANGRVIEKTIYNKKIG
RDEPALFVALTFERREVVDPSNIKPVNLIGVDRGENIPAVIALTDPEGCPLPEFKDSSGGPTDI
LRIGEGYKEKQRAIQAAKEVEQRRAGGYSRKFASKSRNLADDMVRNSARDLFYHAVTHDAVLVF
ENLSRGFGRQGKRTFMTERQYTKMEDWLTAKLAYEGLTSKTYLSKTLAQYTSKTCSNCGFTITT
ADYDGMLVRLKKTSDGWATTLNNKELKAEGQITYYNRYKRQTVEKELSAELDRLSEESGNNDIS
KWTKGRRDEALFLLKKRFSHRPVQEQFVCLDCGHEVHADEQAALNIARSWLFLNSNSTEFKSYK
SGKQPFVGAWQAFYKRRLKEVWKPNA (SEQ ID NO:161)
   > Plantomycetes bacterium Cas12e (CasX2) 
MQEIKRINKIRRRLVKDSNTKKAGKTGPMKTLLVRVMTPDLRERLENLRKKPENIPQPISNTSR
ANLNKLLTDYTEMKKAILHVYWEEFQKDPVGLMSRVAQPAPKNIDQRKLIPVKDGNERLTSSGF
ACSQCCQPLYVYKLEQVNDKGKPHTNYFGRCNVSEHERLILLSPHKPEANDELVTYSLGKFGQR
ALDFYSIHVTRESNHPVKPLEQIGGNSCASGPVGKALSDACMGAVASFLTKYQDIILEHQKVIK
KNEKRLANLKDIASANGLAFPKITLPPQPHTKEGIEAYNNVVAQIVIWVNLNLWQKLKIGRDEA
KPLQRLKGFPSFPLVERQANEVDWWDMVCNVKKLINEKKEDGKVFWQNLAGYKRQEALLPYLSS
EEDRKKGKKFARYQFGDLLLHLEKKHGEDWGKVYDEAWERIDKKVEGLSKHIKLEEERRSEDAQ
SKAALTDWLRAKASFVIEGLKEADKDEFCRCELKLQKWYGDLRGKPFAIEAENSILDISGFSKQ
YNCAFIWQKDGVKKLNLYLIINYFKGGKLRFKKIKPEAFEANRFYTVINKKSGEIVPMEVNFNF
DDPNLIILPLAFGKRQGREFIWNDLLSLETGSLKLANGRVIEKTLYNRRTRQDEPALFVALTFE
RREVLDSSNIKPMNLIGIDRGENIPAVIALTDPEGCPLSRFKDSLGNPTHILRIGESYKEKQRT
IQAAKEVEQRRAGGYSRKYASKAKNLADDMVRNTARDLLYYAVTQDAMLIFENLSRGFGRQGKR
 
TFMAERQYTRMEDWLTAKLAYEGLPSKTYLSKTLAQYTSKTCSNCGFTITSADYDRVLEKLKKT
ATGWMTTINGKELKVEGQITYYNRYKRQNVVKDLSVELDRLSEESVNNDISSWTKGRSGEALSL
LKKRFSHRPVQEKFVCLNCGFETHADEQAALNIARSWLFLRSQEYKKYQTNKTTGNTDKRAFVE
TWQSFYRKKLKEVWKPAV (SEQ ID NO:162)
   >Cas12e3 (CasX3) 
MDNANKPSTKSLVNTTRISDHFGVTPGQVTRVFSFGIIPTKRQYAIIERWFAAVEAARERLYGM
LYAHFQENPPAYLKEKFSYETFFKGRPVLNGLRDIDPTIMTSAVFTALRHKAEGAMAAFHTNHR
RLFEEARKKMREYAECLKANEALLRGAADIDWDKIVNALRTRLNTCLAPEYDAVIADFGALCAF
RALIAETNALKGAYNHALNQMLPALVKVDEPEEAEESPRLRFFNGRINDLPKFPVAERETPPDT
ETIIRQLEDMARVIPDTAEILGYIHRIRHKAARRKPGSAVPLPQRVALYCAIRMERNPEEDPST
VAGHFLGEIDRVCEKRRQGLVRTPFDSQIRARYMDIISFRATLAHPDRWTEIQFLRSNAASRRV
RAETISAPFEGFSWTSNRTNPAPQYGMALAKDANAPADAPELCICLSPSSAAFSVREKGGDLIY
MRPTGGRRGKDNPGKEITWVPGSFDEYPASGVALKLRLYFGRSQARRMLTNKTWGLLSDNPRVF
AANAELVGKKRNPQDRWKLFFHMVISGPPPVEYLDFSSDVRSRARTVIGINRGEVNPLAYAVVS
VEDGQVLEEGLLGKKEYIDQLIETRRRISEYQSREQTPPRDLRQRVRHLQDTVLGSARAKIHSL
IAFWKGILAIERLDDQFHGREQKIIPKKTYLANKTGFMNALSFSGAVRVDKKGNPWGGMIEIYP
GGISRTCTQCGTVWLARRPKNPGHRDAMVVIPDIVDDAAATGFDNVDCDAGTVDYGELFTLSRE
WVRLTPRYSRVMRGTLGDLERAIRQGDDRKSRQMLELALEPQPQWGQFFCHRCGFNGQSDVLAA
TNLARRAISLIRRLPDTDTPPTP (SEQ ID NO:163)
Cas12J
   Cas12J 1947455 
MADTPTLFTQFLRHHLPGQRFRKDILKQAGRILANKGEDATIAFLRGKSEESPPDFQPP
VKCPIIACSRPLTEWPIYQASVAIQGYVYGQSLAEFEASDPGCSKDGLLGWFDKTGVCTDYFSV
QGLNLIFQNARKRYIGVQTKVTNRNEKRHKKLKRINAKRIAEGLPELTSDEPESALDETGHLIDP
PGLNTNIYCYQQVSPKPLALSEVNQLPTAYAGYSTSGDDPIQPMVTKDRLSISKGQPGYIPEHQ
RALLSQKKHRRMRGYGLKARALLVIVRIQDDWAVIDLRSLLRNAYWRRIVQTKEPSTITKLLKLV
TGDPVLDATRMVATFTYKPGIVQVRSAKCLKNKQGSKLFSERYLNETVSVTSIDLGSNNLVAVA
TYRLVNGNTPELLQRFTLPSHLVKDFERYKQAHDTLEDSIQKTAVASLPQGQQTEIRMWSMYG
FREAQERVCQELGLADGSIPWNVMTATSTILTDLFLARGGDPKKCMFTSEPKKKKNSKQVLYKI
RDRAWAKMYRTLLSKETREAWNKALWGLKRGSPDYARLSKRKEELARRCVNYTISTAEKRAQ
CGRTIVALEDLNIGFFHGRGKQEPGWVGLFTRKKENRWLMQALHKAFLELAHHRGYHVIEVNP
AYTSQTCPVCRHCDPDNRDQHNREAFHCIGCGFRGNADLDVATHNIAMVAITGESLKRARGS
VASKTPQPLAAE* (SEQ ID NO:164)
Cas12J 2071242 
MPKPAVESEFSKVLKKHFPGERFRSSYMKRGGKILAAQGEEAVVAYLQGKSEEEPPNF
QPPAKCHVVTKSRDFAEWPIMKASEAIQRYIYALSTTERAACKPGKSSESHAAWFAATGVSNH
GYSHVQGLNLIFDHTLGRYDGVLKKVQLRNEKARARLESINASRADEGLPEIKAEEEEVATNET
 
GHLLQPPGINPSFYVYQTISPQAYRPRDEIVLPPEYAGYVRDPNAPIPLGVVRNRCDIQKGCPG
YIPEWQREAGTAISPKTGKAVTVPGLSPKKNKRMRRYWRSEKEKAQDALLVTVRIGTDWVVID
VRGLLRNARWRTIAPKDISLNALLDLFTGDPVIDVRRNIVTFTYTLDACGTYARKWTLKGKQTKA
TLDKLTATQTVALVAIDLGQTNPISAGISRVTQENGALQCEPLDRFTLPDDLLKDISAYRIAWDRN
EEELRARSVEALPEAQQAEVRALDGVSKETARTQLCADFGLDPKRLPWDKMSSNTTFISEALL
SNSVSRDQVFFTPAPKKGAKKKAPVEVMRKDRTWARAYKPRLSVEAQKLKNEALWALKRTSP
EYLKLSRRKEELCRRSINYVIEKTRRRTQCQIVIPVIEDLNVRFFHGSGKRLPGWDNFFTAKKEN
RWFIQGLHKAFSDLRTHRSFYVFEVRPERTSITCPKCGHCEVGNRDGEAFQCLSCGKTCNADL
DVATHNLTQVALTGKTMPKREEPRDAQGTAPARKTKKASKSKAPPAEREDQTPAQEPSQTS (SEQ ID NO:165)
Cas12J_1973640 
MYILEMADLKSEPSLLAKLLRDRFPGKYWLPKYWKLAEKKRLTGGEEAACEYMADKQL
DSPPPNFRPPARCVILAKSRPFEDWPVHRVASKAQSFVIGLSEQGFAALRAAPPSTADARRDW
LRSHGASEDDLMALEAQLLETIMGNAISLHGGVLKKIDNANVKAAKRLSGRNEARLNKGLQELP
PEQEGSAYGADGLLVNPPGLNLNIYCRKSCCPKPVKNTARFVGHYPGYLRDSDSILISGTMDRL
TIIEGMPGHIPAWQREQGLVKPGGRRRRLSGSESNMRQKVDPSTGPRRSTRSGTVNRSNQRT
GRNGDPLLVEIRMKEDWVLLDARGLLRNLRWRESKRGLSCDHEDLSLSGLLALFSGDPVIDPV
RNEVVFLYGEGIIPVRSTKPVGTRQSKKLLERQASMGPLTLISCDLGQTNLIAGRASAISLTHGS
LGVRSSVRIELDPEIIKSFERLRKDADRLETEILTAAKETLSDEQRGEVNSHEKDSPQTAKASLC
RELGLHPPSLPWGQMGPSTTFIADMLISHGRDDDAFLSHGEFPTLEKRKKFDKRFCLESRPLLS
SETRKALNESLWEVKRTSSEYARLSQRKKEMARRAVNFVVEISRRKTGLSNVIVNIEDLNVRIFH
GGGKQAPGWDGFFRPKSENRWFIQAIHKAFSDLAAHHGIPVIESDPQRTSMTCPECGHCDSK
NRNGVRFLCKGCGASMDADFDAACRNLERVALTGKPMPKPSTSCERLLSATTGKVCSDHSLS
HDAIEKAS* (SEQ ID NO:166)
Cas12J 3339380 
MEKEITELTKIRREFPNKKFSSTDMKKAGKLLKAEGPDAVRDFLNSCQEIIGDFKPPVKT
NIVSISRPFEEWPVSMVGRAIQEYYFSLTKEELESVHPGTSSEDHKSFFNITGLSNYNYTSVQGL
NLIFKNAKAIYDGTLVKANNKNKKLEKKFNEINHKRSLEGLPIITPDFEEPFDENGHLNNPPGINR
NIYGYQGCAAKVFVPSKHKMVSLPKEYEGYNRDPNLSLAGFRNRLEIPEGEPGHVPWFQRMDI
PEGQIGHVNKIQRFNFVHGKNSGKVKFSDKTGRVKRYHHSKYKDATKPYKFLEESKKVSALDSI
LAIITIGDDWVVFDIRGLYRNVFYRELAQKGLTAVQLLDLFTGDPVIDPKKGVVTFSYKEGVVPVF
SQKIVPRFKSRDTLEKLTSQGPVALLSVDLGQNEPVAARVCSLKNINDKITLDNSCRISFLDDYK
KQIKDYRDSLDELEIKIRLEAINSLETNQQVEIRDLDVFSADRAKANTVDMFDIDPNLISWDSMSD
ARVSTQISDLYLKNGGDESRVYFEINNKRIKRSDYNISQLVRPKLSDSTRKNLNDSIWKLKRTSE
EYLKLSKRKLELSRAVVNYTIRQSKLLSGINDIVIILEDLDVKKKFNGRGIRDIGWDNFFSSRKEN
RWFIPAFHKAFSELSSNRGLCVIEVNPAWTSATCPDCGFCSKENRDGINFTCRKCGVSYHADI
DVATLNIARVAVLGKPMSGPADRERLGDTKKPRVARSRKTMKRKDISNSTVEAMVTA* (SEQ ID NO:167)
Cas12J 10037042 3 MDMLDTETNYATETPAQQQDYSPKPPKKAQRAPKGFSKKARPEKKPPKPITLFTQKHF 
SGVRFLKRVIRDASKILKLSESRTITFLEQAIERDGSAPPDVTPPVHNTIMAVTRPFEEWPEVILS
KALQKHCYALTKKIKIKTWPKKGPGKKCLAAWSARTKIPLIPGQVQATNGLFDRIGSIYDGVEKK
VTNRNANKKLEYDEAIKEGRNPAVPEYETAYNIDGTLINKPGYNPNLYITQSRTPRLITEADRPLV
EKILWQMVEKKTQSRNQARRARLEKAAHLQGLPVPKFVPEKVDRSQKIEIRIIDPLDKIEPYMPQ
DRMAIKASQDGHVPYWQRPFLSKRRNRRVRAGWGKQVSSIQAWLTGALLVIVRLGNEAFLADI
RGALRNAQWRKLLKPDATYQSLFNLFTGDPVVNTRTNHLTMAYREGVVNIVKSRSFKGRQTR
EHLLTLLGQGKTVAGVSFDLGQKHAAGLLAAHFGLGEDGNPVFTPIQACFLPQRYLDSLTNYR
NRYDALTLDMRRQSLLALTPAQQQEFADAQRDPGGQAKRACCLKLNLNPDEIRWDLVSGIST
MISDLYIERGGDPRDVHQQVETKPKGKRKSEIRILKIRDGKWAYDFRPKIADETRKAQREQLWK
LQKASSEFERLSRYKINIARAIANWALQWGRELSGCDIVIPVLEDLNVGSKFFDGKGKWLLGWD
NRFTPKKENRWFIKVLHKAVAELAPHRGVPVYEVMPHRTSMTCPACHYCHPTNREGDRFECQ
SCHVVKNTDRDVAPYNILRVAVEGKTLDRWQAEKKPQAEPDRPMILIDNQES* (SEQ ID NO:168)
Cas12J 10020921 9 
MDMLDTETNYATETPAQQQDYSPKPPKKAQRAPKGFSKKARPEKKPPKPITLFTQKHF
SGVRFLKRVIRDASKILKLSESRTITFLEQAIERDGSAPPDVTPPVHNTIMAVTRPFEEWPEVILS
KALQKHCYALTKKIKIKTWPKKGPGKKCLAAWSARTKIPLIPGQVQATNGLFDRIGSIYDGVEKK
VTNRNANKKLEYDEAIKEGRNPAVPEYETAYNIDGTLINKPGYNPNLYITQSRTPRLITEADRPLV
EKILWQMVEKKTQSRNQARRARLEKAAHLQGLPVPKFVPEKVDRSQKIEIRIIDPLDKIEPYMPQ
DRMAIKASQDGHVPYWQRPFLSKRRNRRVRAGWGKQVSSIQAWLTGALLVIVRLGNEAFLADI
RGALRNAQWRKLLKPDATYQSLFNLFTGDPVVNTRTNHLTMAYREGVVDIVKSRSFKGRQTR
EHLLTLLGQGKTVAGVSFDLGQKHAAGLLAAHFGLGEDGNPVFTPIQACFLPQRYLDSLTNYR
NRYDALTLDMRRQSLLALTPAQQQEFADAQRDPGGQAKRACCLKLNLNPDEIRWDLVSGIST
MISDLYIERGGDPRDVHQQVETKPKGKRKSEIRILKIRDGKWAYDFRPKIADETRKAQREQLWK
LQKASSEFERLSRYKINIARAIANWALQWGRELSGCDIVIPVLEDLNVGSKFFDGKGKWLLGWD
NRFTPKKENRWFIKVLHKAVAELAPHKGVPVYEVMPHRTSMTCPACHYCHPTNREGDRFECQ
SCHVVKNTDRDVAPYNILRVAVEGKTLDRWQAEKKPQAEPDRPMILIDNQES*(SEQ ID NO:169)
Cas12J_10000002_47 
MSSLPTPLELLKQKHADLFKGLQFSSKDNKMAGKVLKKDGEEAALAFLSERGVSRGEL
PNFRPPAKTLVVAQSRPFEEFPIYRVSEAIQLYVYSLSVKELETVPSGSSTKKEHQRFFQDSSV
PDFGYTSVQGLNKIFGLARGIYLGVITRGENQLQKAKSKHEALNKKRRASGEAETEFDPTPYEY
MTPERKLAKPPGVNHSIMCYVDISVDEFDFRNPDGIVLPSEYAGYCREINTAIEKGTVDRLGHLK
GGPGYIPGHQRKESTTEGPKINFRKGRIRRSYTALYAKRDSRRVRQGKLALPSYRHHMMRLNS
NAESAILAVIFFGKDWVVFDLRGLLRNVRWRNLFVDGSTPSTLLGMFGDPVIDPKRGVVAFCYK
EQIVPVVSKSITKMVKAPELLNKLYLKSEDPLVLVAIDLGQTNPVGVGVYRVMNASLDYEVVTRF
ALESELLREIESYRQRTNAFEAQIRAETFDAMTSEEQEEITRVRAFSASKAKENVCHRFGMPVD
AVDWATMGSNTIHIAKWVMRHGDPSLVEVLEYRKDNEIKLDKNGVPKKVKLTDKRIANLTSIRL
RFSQETSKHYNDTMWELRRKHPVYQKLSKSKADFSRRVVNSIIRRVNHLVPRARIVFIIEDLKNL
GKVFHGSGKRELGWDSYFEPKSENRWFIQVLHKAFSETGKHKGYYIIECWPNWTSCTCPKCS
CCDSENRHGEVFRCLACGYTCNTDFGTAPDNLVKIATTGKGLPGPKKRCKGSSKGKNPKIARS
 SETGVSVTESGAPKVKKSSPTQTSQSSSQSAP* (SEQ ID NO:170)
Cas12J_10100763_4 
MNKIEKEKTPLAKLMNENFAGLRFPFAIIKQAGKKLLKEGELKTIEYMTGKGSIEPLPNFK
PPVKCLIVAKRRDLKYFPICKASCEIQSYVYSLNYKDFMDYFSTPMTSQKQHEEFFKKSGLNIEY
QNVAGLNLIFNNVKNTYNGVILKVKNRNEKLKKKAIKNNYEFEEIKTFNDDGCLINKPGINNVIYC
FQSISPKILKNITHLPKEYNDYDCSVDRNIIQKYVSRLDIPESQPGHVPEWQRKLPEFNNTNNPR
RRRKWYSNGRNISKGYSVDQVNQAKIEDSLLAQIKIGEDWIILDIRGLLRDLNRRELISYKNKLTI
KDVLGFFSDYPIIDIKKNLVTFCYKEGVIQVVSQKSIGNKKSKQLLEKLIENKPIALVSIDLGQTNP
VSVKISKLNKINNKISIESFTYRFLNEEILKEIEKYRKDYDKLELKLINEA (SEQ ID NO:171)
Cas12J 10004149 10 
MDMLDTETNYATETPSQQQDYSPKPPKKDRRAPKGFSKKARPEKKPPKPITLFTQKHF
SGVRFLKRVIRDASKILKLSESRTITFLEQAIERDGSAPPDVTPPVHNTIMAVTRPFEEWPEVILS
KALQKHCYALTKKIKIKTWPKKGPGKKCLAAWSARTKIPLIPGQVQATNGLFDRIGSIYDGVEKK
VTNRNANKKLEYDEAIKEGRNPAVPEYETAYNIDGTLINKPGYNPNLYITQSRTPRLITEADRPLV
EKILWQMVEKKTQSRNQARRARLEKAAHLQGLPVPKFVPEKVDRSQKIEIRIIDPLDKIEPYMPQ
DRMAIKASQDGHVPYWQRPFLSKRRNRRVRAGWGKQVSSIQAWLTGALLVIVRLGNEAFLADI
RGALRNAQWRKLLKPDATYQSLFNLFTGDPVVNTRTNHLTMAYREGVVDIVKSRSFKGRQTR
EHLLTLLGQGKTVAGVSFDLGQKHAAGLLAAHFGLGEDGNPVFTPIQACFLPQRYLDSLTNYR
NRYDALTLDMRRQSLLALTPAQQQEFADAQRDPGGQAKRACCLKLNLNPDEIRWDLVSGIST
MISDLYIERGGDPRDVHQQVETKPKGKRKSEIRILKIRDGKWAYDFRPKIADETRKAQREQLWK
LQKASSEFERLSRYKINIARAIANWALQWGRELSGCDIVIPVLEDLNVGSKFFDGKGKWLLGWD
NRFTPKKENRWFIKVLHKAVAELAPHRGVPVYEVMPHRTSMTCPACHYCHPTNREGDRFECQ
SCHVVKNTDRDVAPYNILRVAVEGKTLDRWQAEKKPQAEPDRPMILIDNQES* (SEQ ID NO:172)
Cas12J_10000724_71 
MDMLDTETNYATETPSQQQDYSPKPPKKDRRAPKGFSKKARPEKKPPKPITLFTQKHF
SGVRFLKRVIRDASKILKLSESRTITFLEQAIERDGSAPPDVTPPVHNTIMAVTRPFEEWPEVILS
KALQKHCYALTKKIKIKTWPKKGPGKKCLAAWSARTKIPLIPGQVQATNGLFDRIGSIYDGVEKK
VTNRNANKKLEYDEAIKEGRNPAVPEYETAYNIDGTLINKPGYNPNLYITQSRTPRLITEADRPLV
EKILWQMVEKKTQSRNQARRARLEKAAHLQGLPVPKFVPEKVDRSQKIEIRIIDPLDKIEPYMPQ
DRMAIKASQDGHVPYWQRPFLSKRRNRRVRAGWGKQVSSIQAWLTGALLVIVRLGNEAFLADI
RGALRNAQWRKLLKPDATYQSLFNLFTGDPVVNTRTNHLTMAYREGVVNIVKSRSFKGRQTR
EHLLTLLGQGKTVAGVSFDLGQKHAAGLLAAHFGLGEDGNPVFTPIQACFLPQRYLDSLTNYR
NRYDALTLDMRRQSLLALTPAQQQEFADAQRDPGGQAKRACCLKLNLNPDEIRWDLVSGIST
MISDLYIERGGDPRDVHQQVETKPKGKRKSEIRILKIRDGKWAYDFRPKIADETRKAQREQLWK
LQKASSEFERLSRYKINIARAIANWALQWGRELSGCDIVIPVLEDLNVGSKFFDGKGKWLLGWD
NRFTPKKENRWFIKVLHKAVAELAPHRGVPVYEVMPHRTSMTCPACHYCHPTNREGDRFECQ
SCHVVKNTDRDVAPYNILRVAVEGKTLDRWQAEKKPQAEPDRPMILIDNQES* (SEQ ID NO:173)
Cas12J_1000001_267 
MSNTAVSTREHMSNKTTPPSPLSLLLRAHFPGLKFESQDYKIAGKKLRDGGPEAVISYL
TGKGQAKLKDVKPPAKAFVIAQSRPFIEWDLVRVSRQIQEKIFGIPATKGRPKQDGLSETAFNEA
VASLEVDGKSKLNEETRAAFYEVLGLDAPSLHAQAQNALIKSAISIREGVLKKVENRNEKNLSKT
KRRKEAGEEATFVEEKAHDERGYLIHPPGVNQTIPGYQAVVIKSCPSDFIGLPSGCLAKESAEA
LTDYLPHDRMTIPKGQPGYVPEWQHPLLNRRKNRRRRDWYSASLNKPKATCSKRSGTPNRK
NSRTDQIQSGRFKGAIPVLMRFQDEWVIIDIRGLLRNARYRKLLKEKSTIPDLLSLFTGDPSIDMR
QGVCTFIYKAGQACSAKMVKTKNAPEILSELTKSGPVVLVSIDLGQTNPIAAKVSRVTQLSDGQL
SHETLLRELLSNDSSDGKEIARYRVASDRLRDKLANLAVERLSPEHKSEILRAKNDTPALCKARV
CAALGLNPEMIAWDKMTPYTEFLATAYLEKGGDRKVATLKPKNRPEMLRRDIKFKGTEGVRIEV
SPEAAEAYREAQWDLQRTSPEYLRLSTWKQELTKRILNQLRHKAAKSSQCEVVVMAFEDLNIK
MMHGNGKWADGGWDAFFIKKRENRWFMQAFHKSLTELGAHKGVPTIEVTPHRTSITCTKCGH
CDKANRDGERFACQKCGFVAHADLEIATDNIERVALTGKPMPKPESERSGDAKKSVGARKAAF
KPEEDAEAAE* (SEQ ID NO:174)
Cas12J 10000286 53 
MIKPTVSQFLTPGFKLIRNHSRTAGLKLKNEGEEACKKFVRENEIPKDECPNFQGGPAIA
NIIAKSREFTEWEIYQSSLAIQEVIFTLPKDKLPEPILKEEWRAQWLSEHGLDTVPYKEAAGLNLII
KNAVNTYKGVQVKVDNKNKNNLAKINRKNEIAKLNGEQEISFEEIKAFDDKGYLLQKPSPNKSIY
CYQSVSPKPFITSKYHNVNLPEEYIGYYRKSNEPIVSPYQFDRLRIPIGEPGYVPKWQYTFLSKK
ENKRRKLSKRIKNVSPILGIICIKKDWCVFDMRGLLRTNHWKKYHKPTDSINDLFDYFTGDPVIDT
KANVVRFRYKMENGIVNYKPVREKKGKELLENICDQNGSCKLATVDVGQNNPVAIGLFELKKV
NGELTKTLISRHPTPIDFCNKITAYRERYDKLESSIKLDAIKQLTSEQKIEVDNYNNNFTPQNTKQI
VCSKLNINPNDLPWDKMISGTHFISEKAQVSNKSEIYFTSTDKGKTKDVMKSDYKWFQDYKPKL
SKEVRDALSDIEWRLRRESLEFNKLSKSREQDARQLANWISSMCDVIGIENLVKKNNFFGGSG
KREPGWDNFYKPKKENRWWINAIHKALTELSQNKGKRVILLPAMRTSITCPKCKYCDSKNRNG
EKFNCLKCGIELNADIDVATENLATVAITAQSMPKPTCERSGDAKKPVRARKAKAPEFHDKLAP
SYTVVLREAV* (SEQ ID NO:175)
Cas12J 10001283 7 
MRSSREIGDKILMRQPAEKTAFQVFRQEVIGTQKLSGGDAKTAGRLYKQGKMEAARE
WLLKGARDDVPPNFQPPAKCLVVAVSHPFEEWDISKTNHDVQAYIYAQPLQAEGHLNGLSEK
WEDTSADQHKLWFEKTGVPDRGLPVQAINKIAKAAVNRAFGVVRKVENRNEKRRSRDNRIAE
HNRENGLTEVVREAPEVATNADGFLLHPPGIDPSILSYASVSPVPYNSSKHSFVRLPEEYQAYN
VEPDAPIPQFVVEDRFAIPPGQPGYVPEWQRLKCSTNKHRRMRQWSNQDYKPKAGRRAKPL
EFQAHLTRERAKGALLVVMRIKEDWVVFDVRGLLRNVEWRKVLSEEAREKLTLKGLLDLFTGD
PVIDTKRGIVTFLYKAEITKILSKRTVKTKNARDLLLRLTEPGEDGLRREVGLVAVDLGQTHPIAA
AIYRIGRTSAGALESTVLHRQGLREDQKEKLKEYRKRHTALDSRLRKEAFETLSVEQQKEIVTVS
GSGAQITKDKVCNYLGVDPSTLPWEKMGSYTHFISDDFLRRGGDPNIVHFDRQPKKGKVSKKS
QRIKRSDSQWVGRMRPRLSQETAKARMEADWAAQNENEEYKRLARSKQELARWCVNTLLQN
TRCITQCDEIVVVIEDLNVKSLHGKGAREPGWDNFFTPKTENRWFIQILHKTFSELPKHRGEHVI
 
EGCPLRTSITCPACSYCDKNSRNGEKFVCVACGATFHADFEVATYNLVRLATTGMPMPKSLER
QGGGEKAGGARKARKKAKQVEKIVVQANANVTMNGASLHSP* (SEQ ID NO:176)
Cas12J 1000002 112 
MSSLPTPLELLKQKHADLFKGLQFSSKDNKMAGKVLKKDGEEAALAFLSERGVSRGEL
PNFRPPAKTLVVAQSRPFEEFPIYRVSEAIQLYVYSLSVKELETVPSGSSTKKEHQRFFQDSSV
PDFGYTSVQGLNKIFGLARGIYLGVITRGENQLQKAKSKHEALNKKRRASGEAETEFDPTPYEY
MTPERKLAKPPGVNHSIMCYVDISVDEFDFRNPDGIVLPSEYAGYCREINTAIEKGTVDRLGHLK
GGPGYIPGHQRKESTTEGPKINFRKGRIRRSYTALYAKRDSRRVRQGKLALPSYRHHMMRLNS
NAESAILAVIFFGKDWVVFDLRGLLRNVRWRNLFVDGSTPSTLLGMFGDPVIDPKRGVVAFCYK
EQIVPVVSKSITKMVKAPELLNKLYLKSEDPLVLVAIDLGQTNPVGVGVYRVMNASLDYEVVTRF
ALESELLREIESYRQRTNAFEAQIRAETFDAMTSEEQEEITRVRAFSASKAKENVCHRFGMPVD
AVDWATMGSNTIHIAKWVMRHGDPSLVEVLEYRKDNEIKLDKNGVPKKVKLTDKRIANLTSIRL
RFSQETSKHYNDTMWELRRKHPVYQKLSKSKADFSRRVVNSIIRRVNHLVPRARIVFIIEDLKNL
GKVFHGSGKRELGWDSYFEPKSENRWFIQVLHKAFSETGKHKGYYIIECWPNWTSCTCPKCS
CCDSENRHGEVFRCLACGYTCNTDFGTAPDNLVKIATTGKGLPGPKKRCKGSSKGKNPKIARS
SETGVSVTESGAPKVKKSSPTQTSQSSSQSAP* (SEQ ID NO:177)
Cas12J_10000506_8 
MIKPTVSQFLTPGFKLIRNHSRTAGLKLKNEGEEACKKFVRENEIPKDECPNFQGGPAIA
NIIAKSREFTEWEIYQSSLAIQEVIFTLPKDKLPEPILKEEWRAQWLSEHGLDTVPYKEAAGLNLII
KNAVNTYKGVQVKVDNKNKNNLAKINRKNEIAKLNGEQEISFEEIKAFDDKGYLLQKPSPNKSIY
CYQSVSPKPFITSKYHNVNLPEEYIGYYRKSNEPIVSPYQFDRLRIPIGEPGYVPKWQYTFLSKK
ENKRRKLSKRIKNVSPILGIICIKKDWCVFDMRGLLRTNHWKKYHKPTDSINDLFDYFTGDPVIDT
KANVVRFRYKMENGIVNYKPVREKKGKELLENICDQNGSCKLATVDVGQNNPVAIGLFELKKV
NGELTKTLISRHPTPIDFCNKITAYRERYDKLESSIKLDAIKQLTSEQKIEVDNYNNNFTPQNTKQI
VCSKLNINPNDLPWDKMISGTHFISEKAQVSNKSEIYFTSTDKGKTKDVMKSDYKWFQDYKPKL
SKEVRDALSDIEWRLRRESLEFNKLSKSREQDARQLANWISSMCDVIGIENLVKKNNFFGGSG
KREPGWDNFYKPKKENRWWINAIHKALTELSQNKGKRVILLPAMRTSITCPKCKYCDSKNRNG
EKFNCLKCGIELNADIDVATENLATVAITAQSMPKPTCERSGDAKKPVRARKAKAPEFHDKLAP
SYTVVLREAV* (SEQ ID NO:178)
Cas12J_1000007_143 
MSNTAVSTREHMSNKTTPPSPLSLLLRAHFPGLKFESQDYKIAGKKLRDGGPEAVISYL
TGKGQAKLKDVKPPAKAFVIAQSRPFIEWDLVRVSRQIQEKIFGIPATKGRPKQDGLSETAFNEA
VASLEVDGKSKLNEETRAAFYEVLGLDAPSLHAQAQNALIKSAISIREGVLKKVENRNEKNLSKT
KRRKEAGEEATFVEEKAHDERGYLIHPPGVNQTIPGYQAVVIKSCPSDFIGLPSGCLAKESAEA
LTDYLPHDRMTIPKGQPGYVPEWQHPLLNRRKNRRRRDWYSASLNKPKATCSKRSGTPNRK
NSRTDQIQSGRFKGAIPVLMRFQDEWVIIDIRGLLRNARYRKLLKEKSTIPDLLSLFTGDPSIDMR
QGVCTFIYKAGQACSAKMVKTKNAPEILSELTKSGPVVLVSIDLGQTNPIAAKVSRVTQLSDGQL
SHETLLRELLSNDSSDGKEIARYRVASDRLRDKLANLAVERLSPEHKSEILRAKNDTPALCKARV
 
CAALGLNPEMIAWDKMTPYTEFLATAYLEKGGDRKVATLKPKNRPEMLRRDIKFKGTEGVRIEV
SPEAAEAYREAQWDLQRTSPEYLRLSTWKQELTKRILNQLRHKAAKSSQCEVVVMAFEDLNIK
MMHGNGKWADGGWDAFFIKKRENRWFMQAFHKSLTELGAHKGVPTIEVTPHRTSITCTKCGH
CDKANRDGERFACQKCGFVAHADLEIATDNIERVALTGKPMPKPESERSGDAKKSVGARKAAF
KPEEDAEAAE* (SQ ID NO:179)
Cas12J_3877103_16 
MYSLEMADLKSEPSLLAKLLRDRFPGKYWLPKYWKLAEKKRLTGGEEAACEYMADKQ
LDSPPPNFRPPARCVILAKSRPFEDWPVHRVASKAQSFVIGLSEQGFAALRAAPPSTADARRD
WLRSHGASEDDLMALEAQLLETIMGNAISLHGGVLKKIDNANVKAAKRLSGRNEARLNKGLQEL
PPEQEGSAYGADGLLVNPPGLNLNIYCRKSCCPKPVKNTARFVGHYPGYLRDSDSILISGTMD
RLTIIEGMPGHIPAWQREQGLVKPGGRRRRLSGSESNMRQKVDPSTGPRRSTRSGTVNRSNQ
RTGRNGDPLLVEIRMKEDWVLLDARGLLRNLRWRESKRGLSCDHEDLSLSGLLALFSGDPVID
PVRNEVVFLYGEGIIPVRSTKPVGTRQSKKLLERQASMGPLTLISCDLGQTNLIAGRASAISLTH
GSLGVRSSVRIELDPEIIKSFERLRKDADRLETEILTAAKETLSDEQRGEVNSHEKDSPQTAKAS
LCRELGLHPPSLPWGQMGPSTTFIADMLISHGRDDDAFLSHGEFPTLEKRKKFDKRFCLESRP
LLSSETRKALNESLWEVKRTSSEYARLSQRKKEMARRAVNFVVEISRRKTGLSNVIVNIEDLNV
RIFHGGGKQAPGWDGFFRPKSENRWFIQAIHKAFSDLAAHHGIPVIESDPQRTSMTCPECGHC
DSKNRNGVRFLCKGCGASMDADFDAACRNLERVALTGKPMPKPSTSCERLLSATTGKVCSDH
SLSHDAIEKAS* (SEQ ID NO:180)
Cas12J 877636 12 
MEKEITELTKIRREFPNKKFSSTDMKKAGKLLKAEGPDAVRDFLNSCQEIIGDFKPPVKT
NIVSISRPFEEWPVSMVGRAIQEYYFSLTKEELESVHPGTSSEDHKSFFNITGLSNYNYTSVQGL
NLIFKNAKAIYDGTLVKANNKNKKLEKKFNEINHKRSLEGLPIITPDFEEPFDENGHLNNPPGINR
NIYGYQGCAAKVFVPSKHKMVSLPKEYEGYNRDPNLSLAGFRNRLEIPEGEPGHVPWFQRMDI
PEGQIGHVNKIQRFNFVHGKNSGKVKFSDKTGRVKRYHHSKYKDATKPYKFLEESKKVSALDSI
LAIITIGDDWVVFDIRGLYRNVFYRELAQKGLTAVQLLDLFTGDPVIDPKKGVVTFSYKEGVVPVF
SQKIVPRFKSRDTLEKLTSQGPVALLSVDLGQNEPVAARVCSLKNINDKITLDNSCRISFLDDYK
KQIKDYRDSLDELEIKIRLEAINSLETNQQVEIRDLDVFSADRAKANTVDMFDIDPNLISWDSMSD
ARVSTQISDLYLKNGGDESRVYFEINNKRIKRSDYNISQLVRPKLSDSTRKNLNDSIWKLKRTSE
EYLKLSKRKLELSRAVVNYTIRQSKLLSGINDIVIILEDLDVKKKFNGRGIRDIGWDNFFSSRKEN
RWFIPAFHKTFSELSSNRGLCVIEVNPAWTSATCPDCGFCSKENRDGINFTCRKCGVSYHADID
VATLNIARVAVLGKPMSGPADRERLGDTKKPRVARSRKTMKRKDISNSTVEAMVTA* (SEQ ID NO:181)
Cas12L Sequences
   >Cas12L 1 257905508 
MASHKKTESNQIIKTFSFKIKNANGLSLDVLNDAITEYQNYYNICSDWIKDHLTMKISELYKYIPNEKKN
SGYALTLISDEWKDKPMYMMFKKGYPANNRDNAIYETLNTCNTEHYTGNILNFSDTYYRRFGYVASAISN
 
YVTKISKMSTGSRSKNISNDSDVDTIMEQVIYEMEHNGWTSVKDWENQMEYLESKTDSNPNFVYRMTTLY
EFYKSHIDEVNSKMETMSIDSLIKFGGCRRKDSKKSMYIMGGSNTPFDITQIGGNSLNIKFSKNLNVDVF
GRYDVIKDNTLLVDIINGHGASFVLKIINDEIYIDINVSVPFDKKIATTNKVVGIDVNIKHMLLATNILD
DGNVKGYVNIYKEVINDSDFKKVCNSTVMQYFTDFSKFVTFCPLEFDFLFSRVCNQKGIYNDNSAMEKSF
SDVLNKLKWNFIETGDNTKRIYIENVMKLRSQMKAYAIVKNAYYKQQSEYDFGKSEEFIQEHPFSNTDKG
IEILNKLDNISKKILGCRNNIIQYSYNLFEINGYDMVSLEKLTSSQFKKKPFPTVNSLLKYHKILGCTQE
EMEKKDIYSVIKKGYYDIIFDNDVVTDAKLSAKGELSKFKDDFFNLMIKSIHFADIKDYFITLSNNGTAG
VSLVPSYFTSQMDSIDHKIYFVQDNKSGKLKLANKHKVRSSQEKHINGLNADYNAARNIAYIMENTDCRN
MFMKQSRTDKSLYNKPSYETFIKTQGSAVAKLKKEGFVKILDEASV* (SEQ ID NO:182)
   >Cas12L_2_196848753 
MAHKKNVGAEIVKTYSFKVKNTNGITMEKLMNAIDEFQSYYNLCSDWICKNLTTMTIGDLDQYIPEKAKGNTYATVLLD
EAWKNQPLYKIFGKKYSSNNRNNALYCALSSVIDMTKENVLGFSKTHYIRNDYILNVISNYASKLSKLNTGVKSRAIKE
TSDEATIIEQVIYEMEHNKWESIEDWKNQIEYLNSKTDYNPTYMERMKTLSAYYSTHKSEVDAKMQEMAVENLVKFGGC
RRNNSKKSMFIMGSNTTNYTISYIGGNSFNINFANILNFDVYGRRDVVKNGEVLVDIMANHGDSIVLKIVNGELYADVP
CSVTLNKVESNFDKVVGIDVNMKHMLLSTSITDNGSSDFLNIYKEMSNNAEFMALCPEEDRKYYKDISKYVTFAPLELD
LLFSRISKQGKVKMEKVYSEILEALKWKFFANGDNKNRIYVESIQKIRQQIKALCVIKNAYYEQQSAYDIDKTQEYIET
HPFSLTEKGMSIKSKMDKICQTIIGCRNNIIDYAYSFFERNGYSIIGLEKLTSSQFEKTKSMPTCKSLLNFHKVLGHTL
SELETLPINDVVKKGYYTFTTDNEGKITDASLSEKGKVRKMKDDFFNQAIKAIHFADVKDYFATLSNNGQTGIFFVPSQ
FTSQMDSNTHNLYFENAKNGGLKLAPKYKVRQTQEYHLNGLPADYNAARNIAYIGLDETMRNTFLKKANSNKSLYNQPI
YDTGIKKTAGVFSRMKKLKRYEII*. (SEQ ID NO:183)
   >Cas12L_3_66741167 
MRISPHLFYIFFKKIWKCHFFVLSLYQLNQYIMASHEKTESNQIIKTFSFKIKNANGLSLDVLNDAITEYQNYYNICSD
WIKDHLTMKIGELYKYIPDEKKNSGYALTLISDEWKDKPMYMMFKKGYPANNRDNAIYETLNTCNTEHYTGNILNFSDT
YYRRFGYVASAISNYVTKISKMSTGSRSKNISNDSDVDTIMEQVIYEMEHNGWTSVKDWENQMEYLESKTDSNPNFVYR
MTTLYEFYKSHIDEVNSKMETMSIDSLIKFGGCRRKDSKKSMYIMGGSNTPFDITQIGGNSLNIKFSKNLNVDVFGRYD
VIKDNTLLVDIINGHGASFVLKIINGEIYIDINVSVPFDKKIATTNKVVGIDVNIKHMLLATNILDDGNVNGYVNIYKE
VINDSDFKKVCNSTVMKYFTDFSKFVTFCPLEFDFLFSRVCNQKGIYNDNSAMEKSFSDVLNKLKWNFIETGDNTKRIY
IENVMKLRSQMKAYAIVKNAYYKQQSEYDFGKSEEFIQEHPFSNTDKGIEILNKLDNISKKILGCRNNIIQYSYNLFEI
NGYDMISLEKLTSSQFKKKPFPTVNSLLKYHKILGCTQEEMEKKDIYSVIKKGYYDIIFDNDVVTDAKLSTKGELSKFK
DDFFNLMIKSIHFADIKDYFITLSNNGTAGVSLVPSYFTSQMDSIDHKIYFVQDNKSGKLKLANKHKVRSSQEKHINGL
NADYNAARNIAYIMENTECRNMFMKQSRTDKSLYNKPSYETFIKTQGSAVAKLKKEGFVKILDEASV* (SEQ ID
NO:184)
   >Cas12L 4 67031163 
MRISPHLFYIFFKKIWKSHFFVLSLYQLNQYIMASHKKTESNQIIKTFSFKIKNANGLSLDVLNDAITEYQNYYNICSD
WIKDHLTMKIGELYKYIPDEKKNSGYALTLISDEWKDKPMYMMFKKGYPANNRDNAIYETLNTCNTEHYTGNILNFPDT
YYRRFGYVASTISNYVTKISKMSTGSRSKNISNDSDVDTIMEQVIYEMEHNGWTSVKDWENQMEYLESKTDSNPNFVYR
MTTLYEFYKSHIDEVNSKMETMSIDSLIKFGGCRRKDSKKSMYIMGGSNTPFDITQIGGNSLNIKFSKNLNVDVFGRYD
VIKDNTLLVDIINGHGASFVLKIINGEIYIDINVSVPFDKKIATTNKVVGIDVNIKHMLLATNILDDGNVNGYVNIYKE
 
VINDSDFKKVCNSTVMKYFTDFSKFVTFCPLEFDFLFSRVCNQKGIYNDNSAMEKSFSDVLNKLKWNFIETGDNTKRIY
IENVMKLRSQMKAYAIVKNAYYKQQSEYDFGKSEEFIQEHPFSNTDKGIEILNKLDNISKKILGCRNNIIQYSYNLFEI
NGYDMISLEKLTSSQFKKNPFPTVNSLLKYHKILGCTQEEMEKKDIYSVIKKGYYDIIFDNDVVTDAKLSTKGELSKFK
DDFFNLMIKSIHFADIKDYFITLSNNGTAGVSLVPSYFTSQMDSIDHKIYFVQDNKSGKLKLANKHKVRSSQEKHINGL
NADYNAARNIAYIMENTECRNMFMKQSRTDKSLYNKPSYETFIKTQGSAVAKLKKEGFVKILDEASV* (SEQ ID
NO:185)
   >Cas12L_5_67793351 
MRISPHLFYIFFKKIWKCHIFVLSLYQLNQYIMASHKKTESNQIIKTFSFKIKNANGLSLDVLNDAITEYQNYYNICSD
WIKDHLTMKIGELYKYIPDEKKNSGYALTLISDEWKDKPMYMMFKKGYPANNRDNAIYETLNTCNTEHYTGNILNFSDT
YYRRFGYVASTISNYVTKISKMSTGSRSKNISNDSDVDT[?]MEQVIYEMEHNGWTSVKDWENQMEYLESKTDSNPNFVYR
MTTLYEFYKSHIDEVNSKMETMSIDSLIKFGGCRRKDSKKSMYIMGGSNTPFDITQIGGNSLNIKFSKNLNVDVFGRYD
VIKDNTLLVDIINGHGASFVLKIINGEIYIDINVSVPFDKKIATTNKVVGVDVNIKHMLLATNILDDGNVNGYVNIYKE
VINDSDFKKVCNSTVMKYFTDFSKFVTFCPLEFDFLFSRVCNQKGIYNDNSAMEKSFSDVLNKLKWNFIETGDNTKRIY
IENVMKLRSQMKAYAIVKNAYYKQQSEYDFGKSEEFIQEHPFSNTDKGIEILNKLDNISKKILGCRNNIIQYSYNLFEI
NGYDMISLEKLTSSQFKKKPFPTVNSLLKYHKILGCTQEEMEKKDIYSVIKKGYYDIIFDNDVVTDAKLSTKGELSKFK
DDFFNLMIKSIHFADIKDYFITLSNNGTAGVSLVPSYFTSQMDSIDHKIYFVQDNKSGKLKLANKHKVRSSQEKHINGL
NADYNAARNIAYIMENTDCRNMFMKQSRTDKSLYNKPSYETFIKTQGSAVAKLKKEGFVKILDEASV* (SEQ ID
NO:186)
   >Cas12L_9_68454124 
MASHKKTESNQIIKTFSFKIKNANGLSLDVLNDAITEYQNYYNICSDWIKDHLTMKIGELYKYIPDEKKNSGYALTLIS
DEWKDKPMYMMFKKGYPANNRDNAIYETLNTCNTEHYTGNILNFSDTYYRRFGYVASAISNYVTKISKMSTGSRSKNIS
NDSDVDTIMEQVIYEMEHNGWTSVKDWENQMEYLESKTDSNPNFVYRMTTLYEFYKSHIDEVNSKMETMSIDSLIKFGG
CRRKDSKKSMYIMGGSNTPFDITQIGGNSLNIKFSKNLNVDVFGRYDVIKDNTLLVDIINGHGASFVLKIINGEIYIDI
NVSVPFDKKIATTNKVVGVDVNIKHMLLATNILDDGNVNGYVNIYKEVINDSDFKKVCNSTVMKYFTDFSKFVTFCPLE
FDFLFSRVCNQKGIYNDNSAMEKSFSDVLNKLKWNFIETGDNTKRIYIENVMKLRSQMKAYAIVKNAYYKQQSEYDFGK
SEEFIQEHPFSNTDKGIEILNKLDNISKKILGCRNNIIQYSYNLFEINGYDMISLEKLTSSQFKKKPFPTVNSLLKYHK
ILGCTQEEMEKKDIYSVIKKGYYDIIFDNDVVTDAKLSAKGELSKFKDDFFNLMIKSIHFADIKDYFITLSNNGTAGVS
LVPSYFTSQMDSIDHKIYFVQDNKSGKLKLANKHKVRSSQEKHINGLNADYNAARNIAYIMENTECRNMFMKQSRTDKS
LYNKPSYETFIKTQGSAVSKLKKDGFVKILDEASV* (SEQ ID NO:187)
   >Cas12L_10_68605313 
MMKKMRTNPHLFYICFKKIWKCHFFALSLYQLNQYIMASHKKTESNQIIKTFSFKIKNANGLSLDVLNDAITEYQNYYN
ICSDWIKDHLTMKIGELYKYIPDEKKNSGYALTLISDEWKDKPMYMMFKKGYPANSRDNAIYEALNTCNTEHYTGNILN
FSDTYYRRFGYVASTISNYVTKISKMSTGSRSKNISNDSDVDTIMEQVIYEMEHNGWTSVKDWENQMEYLESKTDSNPN
FVYRMTTLYEFYKSHIDEVNSKMETMSIDSLIKFGGCRRKDSKKSMYIMGGSNTPFDITQIGGNSLNIKFSKNLNVDVF
GRYDVIKDNTLLVDIINEHGASFVLKIINDEIYIDINVSVPFDKKIATTNKVVGVDVNIKHMLLATNILDDGNVNGYVN
IYKEVINDSDFKKVCNSTVMKYFTDFSKFVTFCPLEFDFLFSRVCNQKGIYNDNSAMEKSFSDVLNKLKWNFIETGDNT
KRIYIENVMKLRSQMKAYAIVKNAYYKQQSEYDFGKSEEFIQEHPFSNTDKGIEILNKLDNISKKILGCRNNIIQYSYN
LFEINGYDMISLEKLTSSQFKKKPFPTVNSLLKYHKILGCTQEEMEKKDIYSVIKKGYYDIIFDNDVVTDAKLSTKGEL
 
SKFKDDFFNLMIKSIHFADIKDYFITLSNNGTAGVSLVPSYFTSQMDSIDHKIYFVQDNKSGKLKLANKHKVRSSQEKH
INGLNADYNAARNIAYIMENTECRNMFMKQSRTDKSLYNKPSYETFIKTQGSAVAKLKKEGFVKILDEASA* (SEQ
ID NO:188)
   >Cas12L_13_69733214 
MASHKKTESNQIIKTFSFKIKNANGLSLDVLNDAITEYQNYYNICSDWIKDHLTMKIGELYKYIPDEKKNSGYALTLIS
DEWKDKPMYMMFKKGYPANSRDNAIYEALNTCNTEHYTGNILNFSDTYYRRFGYVASTISNYVTKISKMSTGSRSKNIS
NDSDVDTIMEQVIYEMEHNGWTSVKDWENQMEYLESKTDSNPNFVYRMTTLYEFYKSHIDEVNSKMETMSIDSLIKFGG
CRRKDSKKSMYIMGGSNTPFDITQIGGNSLNIKFSKNLNVDVFGRYDVIKDNTLLVDIINGHGASFVLKIINGEIYIDI
NVSVPFDKKIATTNKVVGVDVNIKHMLLATNILDDGNVNGYVNIYKEVINDSDFKKVCNSTVMKYFTDFSKFVTFCPLE
FDFLFSRVCNQKGIYNDNSAMEKSFSDVLNKLKWNFIETGDNTKRIYIENVMKLRSQMKAYAIVKNAYYKQQSEYDFGK
SEEFIQEHPFSNTDKGIEILNKLDNISKKILGCRNNIIQYSYNLFEINGYDMISLEKLTSSQFEKKPFPTVNSLLKYHK
ILGCTQEEMEKKDIYSVIKKGYYDIIFDNDVVTDAKLSTKGELSKFKDDFFNLMIKSIHFADIKDYFITLSNNGTAGVS
LVPSYFTSQMDSIDHKIYFVQDNKSGKLKLANKHKVRSSQEKHINGLNADYNAARNIAYIMENTDCRNMFMKQSRTDKS
LYNKPSYETFIKTQGSAVAKLKKEGFVKILDEASV* (SEQ ID NO:189)
   >Cas12L_15_70724743 
MASHKKTESNQIIKTFSFKIKNANGLSLDVLNDAITEYQNYYNICSDWIKDHLTMKIGELYKYIPDEKKNSGYALTLIS
DEWKDKPMYMMFKKGYPANNRDNAIYETLNTCNTEHYTGNILNFSDTYYRRFGYVASAISNYVTKISKMSTGSRSKNIS
NDSDVDTIMEQVIYEMEHNGWTSVKDWENQMEYLESKTDSNPNFVYRMTTLYEFYKSHIDEVNSKMETMSIDSLIKFGG
CRRKDSKKSMYIMGGSNTPFDITQIGGNSLNIKFSKNLNVDVFGRYDVIKDNTLLVDIINGHGASFVLKIINGEIYIDI
NVSVPFDKKIATTNKVVGVDVNIKHMLLATNILDDGNVNGYVNIYKEVINDSDFKKVCNSTVMKYFTDFSKFVTFCPLE
FDFLFSRVCNQKGIYNDNSAMEKSFSDVLNKLKWNFIETGDNTKRIYIENVMKLRSQMKAYAIVKNAYYKQQSEYDFGK
SEEFIQEHPFSNTDKGIEILNKLDNISKKILGCRNNIIQYSYNLFEINGYDMISLEKLTSSQFKKKPFPTVNSLLKYHK
ILGCTQEEMEKKDIYSVIKKGYYDIIFDNDVVTDAKLSTKGELSKFKDDFFNLMIKSIHFADIKDYFITLSNNGTAGVS
LVPSYFTSQMDSIDHKIYFVQDNKSGKLKLANKHKVRSSQEKHINGLNADYNAARNIAYIMENTECRNMFMKQSRTDKS
LYNKPSYETFIKTQGSAVAKLKKEGFVKILDEASV* (SEQ ID NO:190)
   >Cas12L_16_70731038 
MAHKKNIGAEIVKTYSFKVKNTNGITMEKLMNAIDEYQSYYNLCSDWICKNLTTMTIGDLDRYIPEKAKDNIYATVLLD
EVWKNQPLYKIFGKKYSSNNRINALYCTLSSVIDINKKNILGLSQTYYARNGYILNVISNYASKLSKLNTGVKRHTIKE
TSDEATIVEQVIYEMEHNKWESIEDWKNQIEYLNSKTDYNPTYMERMKTLSAYYSEHKSEVDAKMQEMAVENLVKFGGC
RRNNSKKSMFIMGSSKTTYTISYIGDNCFNINFANILNFDVYGRRDVVKNGEVLVDIMANHGDSIVLKIVNGELYADVP
CSTTLNKVESTFDKVAGIDVNMKHMLLSTSVTDNGNSDFVNIYKEMSNNAEFMALCPEEDRKYYKDISQYVTFAPLELD
LLFSRISKQGKVKMEKAYSEILEALKWKFFANGDNKNRIYVENIQKIRQQIKALCVIKNAYYEQQSAYDIDKTQEYIEA
HPFSLTEKGMSIKSKMDNICRTIIGCRNNIIDYAYSFFERNDYSIIGLEKLTSSQFEKTKSLPTCKSLLNFHKVLGHTL
SELETLPINDVVKKGYYTFTTDNEGRITDASLSEKGKVRKMKDDFFNQAIKAIHFADVKDYFATLSNNGQTGIFFVPSQ
FTSQMDSNTHTLYFENAKNGGLKLASKYKVRKSQEYHLNGLPADYNAARNIAYIGLDEIMRNTFLKKANSNKSLYNQPI
YDTGIKKTAGVFSRMKKLKKYKVI* (SEQ ID NO:191)
   >Cas12L_17_70959391 
MASHKKTESNQIIKTFSFKIKNANGLSLDVLNDAITEYQNYYNICSDWIKDHLTMKIGELYKYIPDEKKNSGYALTLIS
DEWKDKPMYMMFKKGYPANSRDNAIYEALNTCNTEHYTGNILNFSDTYYRRFGYVASTISNYVTKISKMSTGSRSKNIS
NDSDVDTIMEQVIYEMEHNGWTSVKDWENQMEYLESKTDSNPNFVYRMTTLYEFYKSHIDEVNSKMETMSIDSLIKFGG
CRRKDSKKSMYIMGGSNTPFDITQIGGNSLNIKFSKNLNVDVFGRYDVIKDNTLLVDIINGHGASFVLKIINGEIYIDI
NVSVPFDKKIATTNKVVGVDVNIKHMLLATNILDDGNVNGYVNIYKEVINDSDFKKVCNSTVMQYFTDFSKFVTFCPLE
FDFLFSRVCNQKGIYNDNSAMEKSFSDVLNKLKWNFIETGDNTKRIYIENVMKLRSQMKAYAIVKNAYYKQQSEYDFGK
SEEFIQEHPFSNTDKGIEILNKLDNISKKILGCRNNIIQYSYNLFEINGYDMISLEKLTSSQFKKKPFPTVNSLLKYHK
ILGCTQEEMEKKDIYSVIKKGYYDIIFDNDVVTDAKLSTKGELSKFKDDFFNLMIKSIHFADIKDYFITLSNNGTAGVS
LVPSYFTSQMDSIDHKIYFVQDNKSGKLKLANKHKVRSSQEKHINGLNADYNAARNIAYIMENTECRNMFMKQSRTDKS
LYNKPSYETFIKTQGSAVAKLKKEGFVKIIDEASV* (SEQ ID NO:192)
   >Cas12L 18 71078086 
MASHKKTESNQIIKTFSFKIKNANGLSLDVLNDAITEYQNYYNICSDWIKDHLTMKIGELYKYIPDEKKNSGYALTLIS
DEWKDKPMYMMFKKGYPANNRDNAIYEALNTCNTEHYTGNILNFSDTYYRRFGYVASTISNYVTKISKMSTGSRSKNIS
NDSDVDTIMEQVIYEMEHNGWTSVKDWENQMEYLESKTDSNPNFVYRMTTLYEFYKSHIDEVNSKMETMSIDSLIKFGG
CRRKDSKKSMYIMGGSNTPFDITQIGGNSLNIKFSKNLNVDVFGRYDVIKDNTLLVDIINGHGASFVLKIINGEIYIDI
NVSVPFDKKIATTNKVVGVDVNIKHMLLATNILDDGNVNGYVNIYKEVINDSDFKKVCNSTVMKYFTDFSKFVTFCPLE
FDFLFSRVCNQKGIYNDNSAMEKSFSDVLNKLKWNFIETGDNTKRIYIENVMKLRSQMKAYAIVKNAYYKQQSEYDFGK
SEEFIQEHPFSNTDKGIEILNKLDNISKKILGCRNNIIQYSYNLFEINGYDMISLEKLTSSQFKKKPFPTVNSLLKYHK
ILGCTQEEMEKKDIYSVIKKGYYDIIFDNGVVTDAKLSTKGELSKFKDDFFNLMIKSIHFADIKDYFITLSNNGTAGVS
LVPSYFTSQMDSIDHKIYFVQDNKSGKLKLANKHKVRSSQEKHINGLNADYNAARNIAYIMENTECRNMFMKQSRTDKS
LYNKPSYETFIKTQGSAVAKLKKEGFVKILDEASV* (SEQ ID NO:193)
   >Cas12L 22 71456687 
MASHKKTESNQIIKTFSFKIKNANGLSLDVLNDAITEYQNYYNICSDWIKDHLTMKIGELYKYIPDEKKNSGYALTLIS
DEWKDKPMYMMFKKGYPANSRDNAIYEALNTCNTEHYTGNILNFSDTYYRRFGYVASAISNYVTKISKMSTGSRSKNIS
NDSDVDTIMEQVIYEMEHNGWTSVKDWENQMEYLESKTDSNPNFVYRMTTLYEFYKSHIDEVNSKMETMSIDSLIKFGG
CRRKDSKKSMYIMGGSNTPFDITQIGGNSLNIKFSKNLNVDVFGRYDVIKDNTLLVDIINEHGASFVLKIINDEIYIDI
NVSVPFDKKIATTNKVVGVDVNIKHMLLATNILDDGNVNGYVNIYKEVINDSDFKKVCNSTVMKYFTDFSKFVTFCPLE
FDFLFSRVCNQKGIYNDNSAMEKSFSDVLNKLKWNFIETGDNTKRIYIENVMKLRSQMKAYAIVKNAYYKQQSEYDFGK
SEEFIQEHPFSNTDKGIEILNKLDNISKKILGCRNNIIQYSYNLFEINGYDMISLEKLTSSQFEKKPFPTVNSLLKYHK
ILGCTQEEMEKKDIYSVIKKGYYDIIFDNDVVTDAKLSTKGELSKFKDDFFNLMIKSIHFADIKDYFITLSNNGTAGVS
LVPSYFTSQMDSIDHKIYFVQDNKSGKLKLANKHKVRSSQEKHINGLNADYNAARNIAYIMENTECRNMFMKQSRTDKS
LYNKPSYETFIKTQGSAVAKLKKEGFVKILDEASV* (SEQ ID NO:194)
   >Cas12L 23 71708971 
DVDTIMEQVIYEMEHNGWTSVKDWENQMEYLESKTDSNPNFVYRMTTLYEFYKSHIDEVNSKMETMSIDSLIKFGGCRR
KDSKKSMYIMGGSNTPFDITQIGGNSLNIKFSKNLNVDVFGRYDVIKDNTLLVDIINEHGASFVLKIINDEIYIDINVS
VPFDKKIATTNKVVGVDVNIKHMLLATNILDDGNVNGYVNIYKEVINDSDFKKVCNSTVMKYFTDFSKFVTFCPLEFDF
LFSRVCNQKGIYNDNSAMEKSFSDVLNKLKWNFIETGDNTKRIYIENVMKLRSQMKAYAIVKNAYYKQQSEYDFGKSEE
FIQEHPFSNTDKGIEILNKLDNISKKILGCRNNIIQYSYNLFEINGYDMISLEKLTSSQFKKKPFPTVDSLLKYHKILG
 
CTQEEMEKKDIYSVIKKGYYDIIFDNDVVTDAKLSTKGELSKFKDDFFNLMIKSIHFADIKDYFITLSNNGTAGVSLVP
SYFTSQMDSIDHKIYFVQDNKSGKLKLANKHKVRSSQEKHINGLNADYNAARNIAYIMENTDCRNMFMKQSRTDKSLYN
KPSYETFIKTQGSAVAKLKKEGFVKILDEASV* (SEQ ID NO:195)
Cas14a sequences
   >Cas14a.1|rifcsphigho2 02 scaffold 2167 curated|30296..31798|revcom 
MEVQKTVMKTLSLRILRPLYSQEIEKEIKEEKERRKQAGGTGELDGGFYKKLEKKHSEMF
SFDRLNLLLNQLQREIAKVYNHAISELYIATIAQGNKSNKHYISSIVYNRAYGYFYNAYI
ALGICSKVEANFRSNELLTQQSALPTAKSDNFPIVLHKQKGAEGEDGGFRISTEGSDLIF
EIPIPFYEYNGENRKEPYKWVKKGGQKPVLKLILSTFRRQRNKGWAKDEGTDAEIRKVTE
GKYQVSQIEINRGKKLGEHQKWFANFSIEQPIYERKPNRSIVGGLDVGIRSPLVCAINNS
FSRYSVDSNDVFKFSKQVFAFRRRLLSKNSLKRKGHGAAHKLEPITEMTEKNDKFRKKII
ERWAKEVTNFFVKNQVGIVQIEDLSTMKDREDHFFNQYLRGFWPYYQMQTLIENKLKEYG
IEVKRVQAKYTSQLCSNPNCRYWNNYFNFEYRKVNKFPKFKCEKCNLEISADYNAARNLS
TPDIEKFVAKATKGINLPEK (SEQ ID NO:196)
   >Cas14a.2|gwa2 scaffold _18027 curated|7105..8628 
MEEAKTVSKTLSLRILRPLYSAEIEKEIKEEKERRKQGGKSGELDSGFYKKLEKKHTQMF
GWDKLNLMLSQLQRQIARVFNQSISELYIETVIQGKKSNKHYTSKIVYNRAYSVFYNAYL
ALGITSKVEANFRSTELLMQKSSLPTAKSDNFPILLHKQKGVEGEEGGFKISADGNDLIF
EIPIPFYEYDSANKKEPFKWIKKGGQKPTIKLILSTFRRQRNKGWAKDEGTDAEIRKVIE
GKYQVSHIEINRGKKLGDHQKWFVNFTIEQPIYERKLDKNIIGGIDVGIKSPLVCAVNNS
FARYSVDSNDVLKFSKQAFAFRRRLLSKNSLKRSGHGSKNKLDPITRMTEKNDRFRKKII
ERWAKEVTNFFIKNQVGTVQIEDLSTMKDRQDNFFNQYLRGFWPYYQMQNLIENKLKEYG
IETKRIKARYTSQLCSNPSCRHWNSYFSFDHRKTNNFPKFKCEKCALEISADYNAARNIS
TPDIEKFVAKATKGINLPDKNENVILE. (SEQ ID NO:197)
   >Cas14a.3|gwa1_scaffold _1795_curated|25635..27224|revcom 
MAKNTITKTLKLRIVRPYNSAEVEKIVADEKNNREKIALEKNKDVKEACSKHLKVAAYC
TTQVERNACLFCKARKLDDKFYQKLRGQFPDAVFWQEISEIFRQLQKQAAEIYNQSLIEL
YYEIFIKGKGIANASSVEHYLSDVCYTRAAELFKNAAIASGLRSKIKSNFRLKELKNMKS
GLPTTKSDNFPIPLVKQKGGQYTGFEISNHNSDFIIKIPFGRWQVKKEIDKYRPWEKFDF
EQVQKSPKPISLLLSTQRRKRNKGWSKDEGTEAEIKKVMNGDYQTSYIEVKRGSKIGEKS
AWMLNLSIDVPKIDKGVDPSIIGGIDVGVKSPLVCAINNAFSRYSISDNDLFHFNKKMFA
RRRILLKKNRHKRAGHGAKNKLKPITILTEKSERFRKKLIERWACEIADFFIKNKVGTVQ
MENLESMKRKEDSYFNIRLRGFWPYAEMQNKIEFKLKQYGIEIRKVAPNNTSKTCSKCGH
LNNYFNFEYRKKNKFPHFKCEKCNFKENADYNAALNISNPKLKSTKEEP (SEQ ID NO:198)
   >Cas14a.4|CG10 big fil rev 8 21 14 0.10 scaffold 20906 curated|649..2829 MERQKVPQIRKIVRVVPLRILRPKYSDVIENALKKFKEKGDDTNTNDFWRAIRDRDTEFF 
RKELNFSEDEINQLERDTLFRVGLDNRVLFSYFDFLQEKLMKDYNKIISKLFINRQSKSS
FENDLTDEEVEELIEKDVTPFYGAYIGKGIKSVIKSNLGGKFIKSVKIDRETKKVTKLTA
INIGLMGLPVAKSDTFPIKIIKTNPDYITFQKSTKENLQKIEDYETGIEYGDLLVQITIP
WFKNENKDFSLIKTKEAIEYYKLNGVGKKDLLNINLVLTTYHIRKKKSWQIDGSSQSLVR
EMANGELEEKWKSFFDTFIKKYGDEGKSALVKRRVNKKSRAKGEKGRELNLDERIKRLYD
SIKAKSFPSEINLIPENYKWKLHFSIEIPPMVNDIDSNLYGGIDFGEQNIATLCVKNIEK
DDYDFLTIYGNDLLKHAQASYARRRIMRVQDEYKARGHGKSRKTKAQEDYSERMQKLRQK
ITERLVKQISDFFLWRNKFHMAVCSLRYEDLNTLYKGESVKAKRMRQFINKQQLFNGIER
KLKDYNSEIYVNSRYPHYTSRLCSKCGKLNLYFDFLKFRTKNIIIRKNPDGSEIKYMPFF
ICEFCGWKQAGDKNASANIADKDYQDKLNKEKEFCNIRKPKSKKEDIGEENEEERDYSRR
FNRNSFIYNSLKKDNKLNQEKLFDEWKNQLKRKIDGRNKFEPKEYKDRFSYLFAYYQEII
KNESES (SEQ ID NO:199)
   >Cas14a.5|rifcsplowo2_01_scaffold_34461 curated|4968..6521 
MVPTELITKTLQLRVIRPLYFEEIEKELAELKEQKEKEFEETNSLLLESKKIDAKSLKKL
KRKARSSAAVEFWKIAKEKYPDILTKPEMEFIFSEMQKMMARFYNKSMTNIFIEMNNDEK
VNPLSLISKASTEANQVIKCSSISSGLNRKIAGSINKTKFKQVRDGLISLPTARTETFPI
SFYKSTANKDEIPISKINLPSEEEADLTITLPFPFFEIKKEKKGQKAYSYFNIIEKSGRS
NNKIDLLLSTHRRQRRKGWKEEGGTSAEIRRLMEGEFDKEWEIYLGEAEKSEKAKNDLIK
NMTRGKLSKDIKEQLEDIQVKYFSDNNVESWNDLSKEQKQELSKLRKKKVEELKDWKHVK
EILKTRAKIGWVELKRGKRQRDRNKWFVNITITRPPFINKELDDTKFGGIDLGVKVPFVC
AVHGSPARLIIKENEILQFNKMVSARNRQITKDSEQRKGRGKKNKFIKKEIFNERNELFR
KKIIERWANQIVKFFEDQKCATVQIENLESFDRTSYK (SEQ ID NO:200)
   >Cas14a.6|3300012359.a|Ga0137385_10000156|41289..42734 
MKSDTKDKKIIIHQTKTLSLRIVKPQSIPMEEFTDLVRYHQMIIFPVYNNGAIDLYKKLF
KAKIQKGNEARAIKYFMNKIVYAPIANTVKNSYIALGYSTKMQSSFSGKRLWDLRFGEAT
PPTIKADFPLPFYNQSGFKVSSENGEFIIGIPFGQYTKKTVSDIEKKTSFAWDKFTLEDT
TKKTLIELLLSTKTRKMNEGWKNNEGTEAEIKRVMDGTYQVTSLEILQRDDSWFVNFNIA
YDSLKKQPDRDKIAGIHMGITRPLTAVIYNNKYRALSIYPNTVMHLTQKQLARIKEQRTN
SKYATGGHGRNAKVTGTDTLSEAYRQRRKKIIEDWIASIVKFAINNEIGTIYLEDISNTN
SFFAAREQKLIYLEDISNTNSFLSTYKYPISAISDTLQHKLEEKAIQVIRKKAYYVNQIC
SLCGHYNKGFTYQFRRKNKFPKMKCQGCLEATSTEFNAAANVANPDYEKLLIKHGLLQLK
K (SEQ ID NO:201)
Cas14b sequences
   >Cas14b.10|CG08_land_8_20_14_0.20_scaffold_1609_curated|6134..7975 
MISLKLKLLPDEEQKKLLDEMFWKWASICTRVGFGRADKEDLKPPKDAEGVWFSLTQLNQ
ANTDINDLREAMKHQKHRLEYEKNRLEAQRDDTQDALKNPDRREISTKRKDLFRPKASVE
 
KGFLKLKYHQERYWVRRLKEINKLIERKTKTLIKIEKGRIKFKATRITLHQGSFKIRFGD
KPAFLIKALSGKNQIDAPFVVVPEQPICGSVVNSKKYLDEITTNFLAYSVNAMLFGLSRS
EEMLLKAKRPEKIKKKEEKLAKKQSAFENKKKELQKLLGRELTQQEEAIIEETRNQFFQD
FEVKITKQYSELLSKIANELKQKNDFLKVNKYPILLRKPLKKAKSKKINNLSPSEWKYYL
QFGVKPLLKQKSRRKSRNVLGIDRGLKHLLAVTVLEPDKKTFVWNKLYPNPITGWKWRRR
KLLRSLKRLKRRIKSQKHETIHENQTRKKLKSLQGRIDDLLHNISRKIVETAKEYDAVIV
VEDLQSMRQHGRSKGNRLKTLNYALSLFDYANVMQLIKYKAGIEGIQIYDVKPAGTSQNC
AYCLLAQRDSHEYKRSQENSKIGVCLNPNCQNHKKQIDADLNAARVIASCYALKINDSQP
FGTRKRFKKRTTN (SEQ ID NO:202)
   >Cas14b.11|CG_4_10_14_0.8_um_filter_scaffold_20762_curated|1372..3219 
METLSLKLKLNPSKEQLLVLDKMFWKWASICTRLGLKKAEMSDLEPPKDAEGVWFSKTQL
NQANTDVNDLRKAMQHQGKRIEYELDKVENRRNEIQEMLEKPDRRDISPNRKDLFRPKAA
VEKGYLKLKYHKLGYWSKELKTANKLIERKRKTLAKIDAGKMKFKPTRISLHTNSFRIKF
GEEPKIALSTTSKHEKIELPLITSLQRPLKTSCAKKSKTYLDAAILNFLAYSTNAALFGL
SRSEEMLLKAKKPEKIEKRDRKLATKRESFDKKLKTLEKLLERKLSEKEKSVFKRKQTEF
FDKFCITLDETYVEALHRIAEELVSKNKYLEIKKYPVLLRKPESRLRSKKLKNLKPEDWT
YYIQFGFQPLLDTPKPIKTKTVLGIDRGVRHLLAVSIFDPRTKTFTFNRLYSNPIVDWKW
RRRKLLRSIKRLKRRLKSEKHVHLHENQFKAKLRSLEGRIEDHFHNLSKEIVDLAKENNS
VIVVENLGGMRQHGRGRGKWLKALNYALSHFDYAKVMQLIKYKAELAGVFVYDVAPAGTS
INCAYCLLNDKDASNYTRGKVINGKKNTKIGECKTCKKEFDADLNAARVIALCYEKRLND
PQPFGTRKQFKPKKP. (SEQ ID NO:203)
   >Cas14b.12|CG22_combo_CG10-13_8_21_14_all_scaffold_2003_curated|553..2880|revcom 
MKALKLQLIPTRKQYKILDEMFWKWASLANRVSQKGESKETLAPKKDIQKIQFNATQLNQ
IEKDIKDLRGAMKEQQKQKERLLLQIQERRSTISEMLNDDNNKERDPHRPLNFRPKGWRK
FHTSKHWVGELSKILRQEDRVKKTIERIVAGKISFKPKRIGIWSSNYKINFFKRKISINP
LNSKGFELTLMTEPTQDLIGKNGGKSVLNNKRYLDDSIKSLLMFALHSRFFGLNNTDTYL
LGGKINPSLVKYYKKNQDMGEFGREIVEKFERKLKQEINEQQKKIIMSQIKEQYSNRDSA
FNKDYLGLINEFSEVFNQRKSERAEYLLDSFEDKIKQIKQEIGESLNISDWDFLIDEAKK
AYGYEEGFTEYVYSKRYLEILNKIVKAVLITDIYFDLRKYPILLRKPLDKIKKISNLKPD
EWSYYIQFGYDSINPVQLMSTDKFLGIDRGLTHLLAYSVFDKEKKEFIINQLEPNPIMGW
KWKLRKVKRSLQHLERRIRAQKMVKLPENQMKKKLKSIEPKIEVHYHNISRKIVNLAKDY
NASIVVESLEGGGLKQHGRKKNARNRSLNYALSLFDYGKIASLIKYKADLEGVPMYEVLP
AYTSQQCAKCVLEKGSFVDPEIIGYVEDIGIKGSLLDSLFEGTELSSIQVLKKIKNKIEL
SARDNHNKEINLILKYNFKGLVIVRGQDKEEIAEHPIKEINGKFAILDFVYKRGKEKVGK
KGNQKVRYTGNKKVGYCSKHGQVDADLNASRVIALCKYLDINDPILFGEQRKSFK. (SEQI DNO:204)
   >Cas14b.13|rifcsphigho2_01_scaffold_82367_curated|curated|1523..3856|revcom MVTRAIKLKLDPTKNQYKLLNEMFWKWASLANRFSQKGASKETLAPKDGTQKIQFNATQL 
NQIKKDVDDLRGAMEKQGKQKERLLIQIQERLLTISEILRDDSKKEKDPHRPQNFRPFGW
RRFHTSAYWSSEASKLTRQVDRVRRTIERIKAGKINFKPKRIGLWSSTYKINFLKKKINI
SPLKSKSFELDLITEPQQKIIGKEGGKSVANSKKYLDDSIKSLLIFAIKSRLFGLNNKDK
PLFENIITPNLVRYHKKGQEQENFKKEVIKKFENKLKKEISQKQKEIIFSQIERQYENRD
ATFSEDYLRAISEFSEIFNQRKKERAKELLNSFNEKIRQLKKEVNGNISEEDLKILEVEA
EKAYNYENGFIEWEYSEQFLGVLEKIARAVLISDNYFDLKKYPILIRKPTNKSKKITNLK
PEEWDYYIQFGYGLINSPMKIETKNFMGIDRGLTHLLAYSIFDRDSEKFTINQLELNPIK
GWKWKLRKVKRSLQHLERRMRAQKGVKLPENQMKKRLKSIEPKIESYYHNLSRKIVNLAK
ANNASIVVESLEGGGLKQHGRKKNSRHRALNYALSLFDYGKIASLIKYKSDLEGVPMYEV
LPAYTSQQCAKCVLKKGSFVEPEIIGYIEEIGFKENLLTLLFEDTGLSSVQVLKKSKNKM
TLSARDKEGKMVDLVLKYNFKGLVISQEKKKEEIVEFPIKEIDGKFAVLDSAYKRGKERI
SKKGNQKLVYTGNKKVGYCSVHGQVDADLNASRVIALCKYLGINEPIVFGEQRKSFK (SEQ ID NO:205)
   >Cas14b.14|gwc1_scaffold_8732_curated|2705..4537 
LDLITEPIQPHKSSSLRSKEFLEYQISDFLNFSLHSLFFGLASNEGPLVDFKIYDKIVIP
KPEERFPKKESEEGKKLDSFDKRVEEYYSDKLEKKIERKLNTEEKNVIDREKTRIWGEVN
KLEEIRSIIDEINEIKKQKHISEKSKLLGEKWKKVNNIQETLLSQEYVSLISNLSDELTN
KKKELLAKKYSKFDDKIKKIKEDYGLEFDENTIKKEGEKAFLNPDKFSKYQFSSSYLKLI
GEIARSLITYKGFLDLNKYPIIFRKPINKVKKIHNLEPDEWKYYIQFGYEQINNPKLETE
NILGIDRGLTHILAYSVFEPRSSKFILNKLEPNPIEGWKWKLRKLRRSIQNLERRWRAQD
NVKLPENQMKKNLRSIEDKVENLYHNLSRKIVDLAKEKNACIVFEKLEGQGMKQHGRKKS
DRLRGLNYKLSLFDYGKIAKLIKYKAEIEGIPIYRIDSAYTSQNCAKCVLESRRFAQPEE
ISCLDDFKEGDNLDKRILEGTGLVEAKIYKKLLKEKKEDFEIEEDIAMFDTKKVIKENKE
KTVILDYVYTRRKEIIGTNHKKNIKGIAKYTGNTKIGYCMKHGQVDADLNASRTIALCKN
FDINNPEIWK. (SEQ ID NO:206)
   >Cas14b.15|3300010293.a|Ga0116204_100857412134..4032 
MSDESLVSSEDKLAIKIKIVPNAEQAKMLDEMFKKWSSICNRISRGKEDIETLRPDEGKE
LQFNSTQLNSATMDVSDLKKAMARQGERLEAEVSKLRGRYETIDASLRDPSRRHTNPQKP
SSFYPSDWDISGRLTPRFHTARHYSTELRKLKAKEDKMLKTINKIKNGKIVFKPKRITLW
PSSVNMAFKGSRLLLKPFANGFEMELPIVISPQKTADGKSQKASAEYMRNALLGLAGYSI
NQLLFGMNRSQKMLANAKKPEKVEKFLEQMKNKDANFDKKIKALEGKWLLDRKLKESEKS
SIAVVRTKFFKSGKVELNEDYLKLLKHMANEILERDGFVNLNKYPILSRKPMKRYKQKNI
DNLKPNMWKYYIQFGYEPIFERKASGKPKNIMGIDRGLTHLLAVAVFSPDQQKFLFNHLE
SNPIMHWKWKLRKIRRSIQHMERRIRAEKNKHIHEAQLKKRLGSIEEKTEQHYHIVSSKI
INWAIEYEAAIVLESLSHMKQRGGKKSVRTRALNYALSLFDYEKVARLITYKARIRGIPV
YDVLPGMTSKTCATCLLNGSQGAYVRGLETTKAAGKATKRKNMKIGKCMVCNSSENSMID
ADLNAARVIAICKYKNLNDPQPAGSRKVFKRF. (SEQ ID NO:207)
   >Cas14b.16|3300005573.a|Ga0078972_1001015a|33750..35627 MLALKLKIMPTEKQAEILDAMFWKWASICSRIAKMKKKVSVKENKKELSKKIPSNSDIWF 
SKTQLCQAEVDVGDHKKALKNFEKRQESLLDELKYKVKAINEVINDESKREIDPNNPSKF
RIKDSTKKGNLNSPKFFTLKKWQKILQENEKRIKKKESTIEKLRGNIFFNPTKISLHEE
EYSINFGSSKLLLNCFYKYNKKSGINSDQLENKFNEFQNGLNIICSPLQPIRGSSKRSFE
FIRNSIINFLMYSLYAKLFGIPRSVKALMKSNKDENKLKLEEKLKKKKSSFNKTVKEFEK
MIGRKLSDNESKILNDESKKFFEIIKSNNKYIPSEEYLKLLKDISEEIYNSNIDFKPYKY
SILIRKPLSKFKSKKLYNLKPTDYKYYLQLSYEPFSKQLIATKTILGIDRGLKHLLAVSV
FDPSQNKFVYNKLIKNPVFKWKKRYHDLKRSIRNRERRIRALTGVHIHENQLIKKLKSMK
NKINVLYHNVSKNIVDLAKKYESTIVLERLENLKQHGRSKGKRYKKLNYVLSNFDYKKIE
SLISYKAKKEGVPVSNINPKYTSKTCAKCLLEVNQLSELKNEYNRDSKNSKIGICNIHGQ
IDADLNAARVIALCYSKNLNEPHFK. (SEQ ID NO:208)
   >Cas14b.11rifcsplowo201_scaffold_239_curated|54653..56257 
LKLSEQENITTGVKFKLKLDKETSEGLNDYFDEYGKAINFAIKVIQKELAEDRFAGKVRL
DENKKPLLNEDGKKIWDFPNEFCSCGKQVNRYVNGKSLCQECYKNKFTEYGIRKRMYSAK
GRKAEQDINIKNSTNKISKTHFNYAIREAFILDKSIKKQRKERFRRLREMKKKLQEFIEI
RDGNKILCPKIEKQRVERYIHPSWINKEKKLEDFRGYSMSNVLGKIKILDRNIKREEKSL
KEKGQINFKARRLMLDKSVKFLNDNKISFTISKNLPKEYELDLPEKEKRLNWLKEKIKII
KNQKPKYAYLLRKDDNFYLQYTLETEFNLKEDYSGIVGIDRGVSHIAVYTFVHNNGKNER
PLFLNSSEILRLKNLQKERDRFLRRKHNKKRKKSNMRNIEKKIQLILHNYSKQIVDFAKN
KNAFIVFEKLEKPKKNRSKMSKKSQYKLSQFTFKKLSDLVDYKAKREGIKVLYISPEYTS
KECSHCGEKVNTQRPFNGNSSLFKCNKCGVELNADYNASINIAKKGLNILNSTN. (SEQ ID NO:209)
   >Cas14b.2|rifcsplowo2_01_scaffold_282_curated|77370..78983 
MEESIITGVKFKLRIDKETTKKLNEYFDEYGKAINFAVKIIQKELADDRFAGKAKLDQNK
NPILDENGKKIYEFPDEFCSCGKQVNKYVNNKPFCQECYKIRFTENGIRKRMYSAKGRKA
EHKINILNSTNKISKTHFNYAIREAFILDKSIKKQRKKRNERLRESKKRLQQFIDMRDGK
REICPTIKGQKVDRFIHPSWITKDKKLEDFRGYTLSIINSKIKILDRNIKREEKSLKEKG
QIIFKAKRLMLDKSIRFVGDRKVLFTISKTLPKEYELDLPSKEKRLNWLKEKIEIIKNQK
PKYAYLLRKNIESEKKPNYEYYLQYTLEIKPELKDFYDGAIGIDRGINHIAVCTFISNDG
KVTPPKFFSSGEILRLKNLQKERDRFLLRKHNKNRKKGNMRVIENKINLILHRYSKQIVD
MAKKLNASIVFEELGRIGKSRTKMKKSQRYKLSLFIFKKLSDLVDYKSRREGIRVTYVPP
EYTSKECSHCGEKVNTQRPFNGNYSLFKCNKCGIQLNSDYNASINIAKKGLKIPNST (SEQ ID NO:210)
   >Cas14b.3|rifcsphigho2_01_scaffold_36781_curated|2592..4217 
LWTIVIGDFIEMPKQDLVTTGIKFKLDVDKETRKKLDDYFDEYGKAINFAVKIIQKNLKE
DRFAGKIALGEDKKPLLDKDGKKIYNYPNESCSCGNQVRRYVNAKPFCVDCYKLKFTENG
IRKRMYSARGRKADSDINIKNSTNKISKTHFNYAIREGFILDKSLKKQRSKRIKKLLELK
RKLQEFIDIRQGQMVLCPKIKNQRVDKFIHPSWLKRDKKLEEFRGYSLSVVEGKIKIFNR
NILREEDSLRQRGHVNFKANRIMLDKSVRFLDGGKVNFNLNKGLPKEYLLDLPKKENKLS
WLNEKISLIKLQKPKYAYLLRREGSFFIQYTIENVPKTFSDYLGAIGIDRGISHIAVCTF
VSKNGVNKAPVFFSSGEILKLKSLQKQRDLFLRGKHNKIRKKSNMRNIDNKINLILHKYS
 
RNIVNLAKSEKAFIVFEKLEKIKKSRFKMSKSLQYKLSQFTFKKLSDLVEYKAKIEGIKV
DYVPPEYTSKECSHCGEKVDTQRPFNGNSSLFKCNKCRVQLNADYNASINIAKKSLNISN
N. (SEQ ID NO:211)
   >Cas14b.4|cg1_0.2_scaffold_785_c_curated|32521..34155 
MSKTTISVKLKIIDLSSEKKEFLDNYFNEYAKATTFCQLRIRRLLRNTHWLGKKEKSSKK
WIFESGICDLCGENKELVNEDRNSGEPAKICKRCYNGRYGNQMIRKLFVSTKKREVQENM
DIRRVAKLNNTHYHRIPEEAFDMIKAADTAEKRRKKNVEYDKKRQMEFIEMFNDEKKRAA
RPKKPNERETRYVHISKLESPSKGYTLNGIKRKIDGMGKKIERAEKGLSRKKIFGYQGNR
IKLDSNWVRFDLAESEITIPSLFKEMKLRITGPTNVHSKSGQIYFAEWFERINKQPNNYC
YLIRKTSSNGKYEYYLQYTYEAEVEANKEYAGCLGVDIGCSKLAAAVYYDSKNKKAQKPI
EIFTNPIKKIKMRREKLIKLLSRVKVRHRRRKLMQLSKTEPIIDYTCHKTARKIVEMANT
AKAFISMENLETGIKQKQQARETKKQKFYRNMFLFRKLSKLIEYKALLKGIKIVYVKPDY
TSQTCSSCGADKEKTERPSQAIFRCLNPTCRYYQRDINADFNAAVNIAKKALNNTEVVTT
LL. (SEQ ID NO:212)
   >Cas14b.5|rifcsphigho2_02_scaffold_55589_curated|1904..3598 
MARAKNQPYQKLTTTTGIKFKLDLSEEEGKRFDEYFSEYAKAVNFCAKVIYQLRKNLKFA
GKKELAAKEWKFEISNCDFCNKQKEIYYKNIANGQKVCKGCHRTNFSDNAIRKKMIPVKG
RKVESKFNIHNTTKKISGTHRHWAFEDAADIIESMDKQRKEKQKRLRREKRKLSYFFELF
GDPAKRYELPKVGKQRVPRYLHKIIDKDSLTKKRGYSLSYIKNKIKISERNIERDEKSLR
KASPIAFGARKIKMSKLDPKRAFDLENNVFKIPGKVIKGQYKFFGTNVANEHGKKFYKDR
ISKILAGKPKYFYLLRKKVAESDGNPIFEYYVQWSIDTETPAITSYDNILGIDAGITNLA
TTVLIPKNLSAEHCSHCGNNHVKPIFTKFFSGKELKAIKIKSRKQKYFLRGKHNKLVKIK
RIRPIEQKVDGYCHVVSKQIVEMAKERNSCIALEKLEKPKKSKFRQRRREKYAVSMFVFK
KLATFIKYKAAREGIEIIPVEPEGTSYTCSHCKNAQNNQRPYFKPNSKKSWTSMFKCGKC
GIELNSDYNAAFNIAQKALNMTSA. (SEQ ID NO:213)
   >Cas14b.6|CG03_land_8_20_14_0.80_scaffold_2214_curated|6634..8466|revcom 
MDEKHFFCSYCNKELKISKNLINKISKGSIREDEAVSKAISIHNKKEHSLILGIKFKLFI
ENKLDKKKLNEYFDNYSKAVTFAARIFDKIRSPYKFIGLKDKNTKKWTFPKAKCVFCLEE
KEVAYANEKDNSKICTECYLKEFGENGIRKKIYSTRGRKVEPKYNIFNSTKELSSTHYNY
AIRDAFQLLDALKKQRQKKLKSIFNQKLRLKEFEDIFSDPQKRIELSLKPHQREKRYIHL
SKSGQESINRGYTLRFVRGKIKSLTRNIEREEKSLRKKTPIHFKGNRLMIFPAGIKFDFA
SNKVKISISKNLPNEFNFSGTNVKNEHGKSFFKSRIELIKTQKPKYAYVLRKIKREYSKL
RNYEIEKIRLENPNADLCDFYLQYTIETESRNNEEINGIIGIDRGITNLACLVLLKKGDK
KPSGVKFYKGNKILGMKIAYRKHLYLLKGKRNKLRKQRQIRAIEPKINLILHQISKDIVK
IAKEKNFAIALEQLEKPKKARFAQRKKEKYKLALFTFKNLSTLIEYKSKREGIPVIYVPP
EKTSQMCSHCAINGDEHVDTQRPYKKPNAQKPSYSLFKCNKCGIELNADYNAAFNIAQKG
LKTLMLNHSH. (SEQ ID NO:214)
   >Cas14b.7|3300013125.a|Ga0172369_10000737|994..2652|revcom 
MDEEPDSAEPNLAPISVKLKLVKLDGEKLAALNDYFNEYAKAVNFCELKMQKIRKNLVNI
RGTYLKEKKAWINQTGECCICKKIDELRCEDKNPDINGKICKKCYNGRYGNQMIRKLFVS
TNKRAVPKSLDIRKVARLHNTHYHRIPPEAADIIKAIETAERKRRNRILFDERRYNELKD
ALENEEKRVARPKKPKEREVRYVPISKKDTPSKGYTMNALVRKVSGMAKKIERAKRNLNK
RKKIEYLGRRILLDKNWVRFDFDKSEISIPTMKEFFGEMRFEITGPSNVMSPNGREYFTK
WFDRIKAQPDNYCYLLRKESEDETDFYLQYTWRPDAHPKKDYTGCLGIDIGGSKLASAVY
FDADKNRAKQPIQIFSNPIGKWKTKRQKVIKVLSKAAVRHKTKKLESLRNIEPRIDVHCH
RIARKIVGMALAANAFISMENLEGGIREKQKAKETKKQKFSRNMFVFRKLSKLIEYKALM
EGVKVVYIVPDYTSQLCSSCGTNNTKRPKQAIFMCQNTECRYFGKNINADFNAAINIAKK
ALNRKDIVRELS. (SEQ ID NO:215)
   >Cas14b.8|3300013125.a|Ga0172369_10010464|885..2489|revcom 
MEKNNSEQTSITTGIKFKLKLDKETKEKLNNYFDEYGKAINFAVRIIQMQLNDDRLAGKY
KRDEKGKPILGEDGKKILEIPNDFCSCGNQVNHYVNGVSFCQECYKKRFSENGIRKRMYS
AKGRKAEQDINIKNSTNKISKTHFNYAIREAFNLDKSIKKQREKRFKKLKDMKRKLQEFL
EIRDGKRVICPKIEKQKVERYIHPSWINKEKKLEEFRGYSLSIVNSKIKSFDRNIQREEK
SLKEKGQINFKAQRLMLDKSVKFLKDNKVSFTISKELPKTFELDLPKKEKKLNWLNEKLE
IIKNQKPKYAYLLRKENNIFLQYTLDSIPEIHSEYSGAVGIDRGVSHIAVYTFLDKDGKN
ERPFFLSSSGILRLKNLQKERDKFLRKKHNKIRKKGNMRNIEQKINLILHEYSKQIVNFA
KDKNAFIVFELLEKPKKSRERMSKKIQYKLSQFTFKKLSDLVDYKAKREGIKVIYVEPAY
TSKDCSHCGERVNTQRPFNGNFSLFKCNKCGIVLNSDYNASLNIARKGLNISAN. (SEQ ID NO:216)
   >Cas14b.9|3300013127.a|Ga0172365_10004421|633..2366|revcom 
MAEEKFFFCEKCNKDIKIPKNYINKQGAEEKARAKHEHRVHALILGIKFKIYPKKEDISK
LNDYFDEYAKAVTFTAKIVDKLKAPFLFAGKRDKDTSKKKWVFPVDKCSFCKEKTEINYR
TKQGKNICNSCYLTEFGEQGLLEKIYATKGRKVSSSFNLFNSTKKLTGTHNNYVVKESLQ
LLDALKKQRSKRLKKLSNTRRKLKQFEEMFEKEDKRFQLPLKEKQRELRFIHVSQKDRAT
EFKGYTMNKIKSKIKVLRRNIEREQRSLNRKSPVFFRGTRIRLSPSVQFDDKDNKIKLTL
SKELPKEYSFSGLNVANEHGRKFFAEKLKLIKENKSKYAYLLRRQVNKNNKKPIYDYYLQ
YTVEFLPNIITNYNGILGIDRGINTLACIVLLENKKEKPSFVKFFSGKGILNLKNKRRKQ
LYFLKGVHNKYRKQQKIRPIEPRIDQILHDISKQIIDLAKEKRVAISLEQLEKPQKPKFR
QSRKAKYKLSQFNFKTLSNYIDYKAKKEGIRVIYIAPEMTSQNCSRCAMKNDLHVNTQRP
YKNTSSLFKCNKCGVELNADYNAAFNIAQKGLKILNS. (SEQ ID NO:217)
Cas14c Sequences
   >Cas14c.1|CG10_big_fil_rev_8_21_14_0.10_scaffold_4477_curated|19327..20880|revc om 
VINLFGYKFALYPNKTQEELLNKHLGECGWLYNKAIEQNEYYKADSNIEEAQKKFELLPD
KNSDEAKVLRGNISKDNYVYRTLVKKKKSEINVQIRKAVVLRPAETIRNLAKVKKKGLSV
 
GRLKFIPIREWDVLPFKQSDQIRLEENYLILEPYGRLKFKMHRPLLGKPKTFCIKRTATD
RWTISFSTEYDDSNMRKNDGGQVGIDVGLKTHLRLSNENPDEDPRYPNPKIWKRYDRRLT
ILQRRISKSKKLGKNRTRLRLRLSRLWEKIRNSRADLIQNETYEILSENKLIAIEDLNVK
GMQEKKDKKGRKGRTRAQEKGLHRSISDAAFSEFRRVLEYKAKRFGSEVKPVSAIDSSKE
CHNCGNKKGMPLESRIYECPKCGLKIDRDLNSAKVILARATGVRPGSNARADTKISATAG
ASVQTEGTVSEDFRQQMETSDQKPMQGEGSKEPPMNPEHKSSGRGSKHVNIGCKNKVGLY
NEDENSRSTEKQIMDENRSTTEDMVEIGALHSPVLTT. (SEQ ID NO:218)
   >Cas14c.2|3300001245.a|JGI12048J13642_1020128614257..5489|revcom 
MIASIDYEAVSQALIVFEFKAKGKDSQYQAIDEAIRSYRFIRNSCLRYWMDNKKVGKYDL
NKYCKVLAKQYPFANKLNSQARQSAAECSWSAISRFYDNCKRKVSGKKGFPKFKKHARSV
EYKTSGWKLSENRKAITFTDKNGIGKLKLKGTYDLHFSQLEDMKRVRLVRRADGYYVQFC
ISVDVKVETEPTGKAIGLDVGIKYFLADSSGNTIENPQFYRKAEKKLNRANRRKSKKYIR
GVKPQSKNYHKARCRYARKHLRVSRQRKEYCKRVAYCVIHSNDVVAYEDLNVKGMVKNRH
LAKSISDVAWSTFRHWLEYFAIKYGKLTIPVAPHNTSQNCSNCDKKVPKSLSTRTHICHH
CGYSEDRDVNAAKNILKKALSTVGQTGSLKLGEIEPLLVLEQSCTRKFDL  (SEQ ID NO:219)
Cas14d sequences
   >Cas14d.1|RIFCSPEIGE02_01_FULL_CPR_46_36_rifcsphigho2_01_scaffold_646_curated|4 9808..51616|revcom 
MSQSLLKWHDMAGRDKDASRSLQKSAVEGVLLHLTASHRVALEMLEKSVSQTVAVTMEAA
QQRLVIVLEDDPTKATSRKRVISADLQFTREEFGSLPNWAQKLASTCPEIATKYADKHIN
SIRIAWGVAKESTNGDAVEQKLQWQIRLLDVTMFLQQLVLQLADKALLEQIPSSIRGGIG
QEVAQQVTSHIQLLDSGTVLKAELPTISDRNSELARKQWEDAIQTVCTYALPFSRERARI
LDPGKYAAEDPRGDRLINIDPMWARVLKGPTVKSLPLLFVSGSSIRIVKLTLPRKHAAGH
KHTFTATYLVLPVSREWINSLPGTVQEKVQWWKKPDVLATQELLVGKGALKKSANTLVIP
ISAGKKRFFNHILPALQRGFPLQWQRIVGRSYRRPATHRKWFAQLTIGYTNPSSLPEMAL
GIHFGMKDILWWALADKQGNILKDGSIPGNSILDFSLQEKGKIERQQKAGKNVAGKKYGK
SLLNATYRVVNGVLEFSKGISAEHASQPIGLGLETIRFVDKASGSSPVNARHSNWNYGQL
SGIFANKAGPAGFSVTEITLKKAQRDLSDAEQARVLAIEATKRFASRIKRLATKRKDDTL
FV. (SEQ ID NO:220)
   >Cas14d.2|rifcsphigho2_01_scaffold_10981_curated|5762..7246|revcom 
VEPVEKERFYYRTYTFRLDGQPRTQNLTTQSGWGLLTKAVLDNTKHYWEIVHHARIANQP
IVFENPVIDEQGNPKLNKLGQPRFWKRPISDIVNQLRALFENQNPYQLGSSLIQGTYWDV
AENLASWYALNKEYLAGTATWGEPSFPEPHPLTEINQWMPLTFSSGKVVRLLKNASGRYF
IGLPILGENNPCYRMRTIEKLIPCDGKGRVTSGSLILFPLVGIYAQQHRRMTDICESIRT
EKGKLAWAQVSIDYVREVDKRRRMRRTRKSQGWIQGPWQEVFILRLVLAHKAPKLYKPRC
FAGISLGPKTLASCVILDQDERVVEKQQWSGSELLSLIHQGEERLRSLREQSKPTWNAAY
RKQLKSLINTQVFTIVTFLRERGAAVRLESIARVRKSTPAPPVNFLLSHWAYRQITERLK
 
DLAIRNGMPLTHSNGSYGVRFTCSQCGATNQGIKDPTKYKVDIESETFLCSICSHREIAA
VNTATNLAKQLLDE. (SEQ ID NO:221)
   >Cas14d.3|RIFCSPLOW02_01_FULL_OD1_45_34b_rifcsplowo2_01_scaffold_3495_curated|2 5656..27605|revcom 
MNDTETSETLTSHRTVCAHLHVVGETGSLPRLVEAALAELITLNGRATQALLSLAKNGLV
LRRDKEENLIAAELTLPCRKNKYADVAAKAGEPILATRINNKGKLVTKKWYGEGNSYHIV
RFTPETGMFTVRVFDRYAFDEELLHLHSEVVFGSDLPKGIKAKTDSLPANFLQAVFTSFL
ELPFQGFPDIVVKPAMKQAAEQLLSYVQLEAGENQQAEYPDTNERDPELRLVEWQKSLHE
LSVRTEPFEFVRARDIDYYAETDRRGNRFVNITPEWTKFAESPFARRLPLKIPPEFCILL
RRKTEGHAKIPNRIYLGLQIFDGVTPDSTLGVLATAEDGKLFWWHDHLDEFSNLEGKPEP
KLKNKPQLLMVSLEYDREQRFEESVGGDRKICLVTLKETRNFRRGRHGHTRTDRLPAGNT
LWRADFATSAEVAAPKWNGRILGIHFQHNPVITWALMDHDAEVLEKGFIEGNAFLGKALD
KQALNEYLQKGGKWVGDRSFGNKLKGITHTLASLIVRLAREKDAWIALEEISWVQKQSAD
SVANRRFSMWNYSRLATLIEWLGTDIATRDCGTAAPLAHKVSDYLTHFTCPECGACRKAG
QKKEIADTVRAGDILTCRKCGFSGPIPDNFIAEFVAKKALERMLKKKPV. (SEQ ID NO:222)
Cas14e sequences
   >Cas14e.1|rifcsphigho2 01 scaffold 566 curated|113069..114313 
MAKRNFGEKSEALYRAVRFEVRPSKEELSILLAVSEVLRMLFNSALAERQQVFTEFIASL
YAELKSASVPEEISEIRKKLREAYKEHSISLFDQINALTARRVEDEAFASVTRNWQEETL
DALDGAYKSFLSLRRKGDYDAHSPRSRDSGFFQKIPGRSGFKIGEGRIALSCGAGRKLSF
PIPDYQQGRLAETTKLKKFELYRDQPNLAKSGRFWISVVYELPKPEATTCQSEQVAFVAL
GASSIGVVSQRGEEVIALWRSDKHWVPKIEAVEERMKRRVKGSRGWLRLLNSGKRRMHMI
SSRQHVQDEREIVDYLVRNHGSHFVVTELVVRSKEGKLADSSKPERGGSLGLNWAAQNTG
SLSRLVRQLEEKVKEHGGSVRKHKLTLTEAPPARGAENKLWMARKLRESFLKEV. (SEQ ID NO:223)
   >Cas14e.2|rifcsplowo2_01_scaffold_81231_curated|976..2217 
LAKNDEKELLYQSVKFEIYPDESKIRVLTRVSNILVLVWNSALGERRARFELYIAPLYEE
LKKFPRKSAESNALRQKIREGYKEHIPTFFDQLKKLLTPMRKEDPALLGSVPRAYQEETL
NTLNGSFVSFMTLRRNNDMDAKPPKGRAEDRFHEISGRSGFKIDGSEFVLSTKEQKLRFP
IPNYQLEKLKEAKQIKKFTLYQSRDRRFWISIAYEIELPDQRPFNPEEVIYIAFGASSIG
VISPEGEKVIDFWRPDKHWKPKIKEVENRMRSCKKGSRAWKKRAAARRKMYAMTQRQQKL
NHREIVASLLRLGFHFVVTEYTVRSKPGKLADGSNPKRGGAPQGFNWSAQNTGSFGEFIL
WLKQKVKEQGGTVQTFRLVLGQSERPEKRGRDNKIEMVRLLREKYLESQTIVV. (SEQ ID NO:224)
   >Cas14e.3|rifcsphigho2_01_scaffold_4702_curated|82881..84230|revcom 
MAKGKKKEGKPLYRAVRFEIFPTSDQITLFLRVSKNLQQVWNEAWQERQSCYEQFFGSIY
ERIGQAKKRAQEAGFSEVWENEAKKGLNKKLRQQEISMQLVSEKESLLQELSIAFQEHGV
TLYDQINGLTARRIIGEFALIPRNWQEETLDSLDGSFKSFLALRKNGDPDAKPPRQRVSE
 
NSFYKIPGRSGFKVSNGQIYLSFGKIGQTLTSVIPEFQLKRLETAIKLKKFELCRDERDM
AKPGRFWISVAYEIPKPEKVPVVSKQITYLAIGASRLGVVSPKGEFCLNLPRSDYHWKPQ
INALQERLEGVVKGSRKWKKRMAACTRMFAKLGHQQKQHGQYEVVKKLLRHGVHFVVTEL
KVRSKPGALADASKSDRKGSPTGPNWSAQNTGNIARLIQKLTDKASEHGGTVIKRNPPLL
SLEERQLPDAQRKIFIAKKLREEFLADQK. (SEQ ID NO:225)
Cas14f sequences
   >Cas14f.1|rifcsp13_1_sub10_scaffold_3_curated|38906..41041 
MAKREKKDDVVLRGTKMRIYPTDRQVTLMDMWRRRCISLWNLLLNLETAAYGAKNTRSKL
GWRSIWARVVEENHAKALIVYQHGKCKKDGSFVLKRDGTVKHPPRERFPGDRKILLGLFD
ALRHTLDKGAKCKCNVNQPYALTRAWLDETGHGARTADIIAWLKDFKGECDCTAISTAAK
YCPAPPTAELLTKIKRAAPADDLPVDQAILLDLFGALRGGLKQKECDHTHARTVAYFEKH
ELAGRAEDILAWLIAHGGTCDCKIVEEAANHCPGPRLFIWEHELAMIMARLKAEPRTEWI
GDLPSHAAQTVVKDLVKALQTMLKERAKAAAGDESARKTGFPKFKKQAYAAGSVYFPNTT
MFFDVAAGRVQLPNGCGSMRCEIPRQLVAELLERNLKPGLVIGAQLGLLGGRIWRQGDRW
YLSCQWERPQPTLLPKTGRTAGVKIAASIVFTTYDNRGQTKEYPMPPADKKLTAVHLVAG
KQNSRALEAQKEKEKKLKARKERLRLGKLEKGHDPNALKPLKRPRVRRSKLFYKSAARLA
ACEAIERDRRDGFLHRVTNEIVHKFDAVSVQKMSVAPMMRRQKQKEKQIESKKNEAKKED
NGAAKKPRNLKPVRKLLRHVAMARGRQFLEYKYNDLRGPGSVLIADRLEPEVQECSRCGT
KNPQMKDGRRLLRCIGVLPDGTDCDAVLPRNRNAARNAEKRLRKHREAHNA. (SEQ ID NO:226)
   >Cas14f.2|3300009991.a|Ga0105042_10014011624..3348 
MNEVLPIPAVGEDAADTIMRGSKMRIYPSVRQAATMDLWRRRCIQLWNLLLELEQAAYSG
ENRRTQIGWRSIWATVVEDSHAEAVRVAREGKKRKDGTFRKAPSGKEIPPLDPAMLAKIQ
RQMNGAVDVDPKTGEVTPAQPRLFMWEHELQKIMARLKQAPRTHWIDDLPSHAAQSVVKD
LIKALQAMLRERKKRASGIGGRDTGFPKFKKNRYAAGSVYFANTQLRFEAKRGKAGDPDA
VRGEFARVKLPNGVGWMECRMPRHINAAHAYAQATLMGGRIWRQGENWYLSCQWKMPKPA
PLPRAGRTAAIKIAAAIPITTVDNRGQTREYAMPPIDRERIAAHAAAGRAQSRALEARKR
RAKKREAYAKKRHAKKLERGIAAKPPGRARIKLSPGFYAAAAKLAKLEAEDANAREAWLH
EITTQIVRNFDVIAVPRMEVAKLMKKPEPPEEKEEQVKAPWQGKRRSLKAARVMMRRTAM
ALIQTTLKYKAVDLRGPQAYEEIAPLDVTAAACSGCGVLKPEWKMARAKGREIMRCQEPL
PGGKTCNTVLTYTRNSARVIGRELAVRLAERQKA (SEQ ID NO:227)
Cas14g sequences
   >Cas14g.1|RBG_13_scaffold_1401_curated|15949..18180 
MSVRSFQARVECDKQTMEHLWRTHKVFNERLPEIIKILFKMKRGECGQNDKQKSLYKSIS
QSILEANAQNADYLLNSVSIKGWKPGTAKKYRNASFTWADDAAKLSSQGIHVYDKKQVLG
DLPGMMSQMVCRQSVEAISGHIELTKKWEKEHNEWLKEKEKWESEDEHKKYLDLREKFEQ
FEQSIGGKITKRRGRWHLYLKWLSDNPDFAAWRGNKAVINPLSEKAQIRINKAKPNKKNS
 
VERDEFFKANPEMKALDNLHGYYERNFVRRRKTKKNPDGFDHKPTFTLPHPTIHPRWFVF
NKPKTNPEGYRKLILPKKAGDLGSLEMRLLTGEKNKGNYPDDWISVKFKADPRLSLIRPV
KGRRVVRKGKEQGQTKETDSYEFFDKHLKKWRPAKLSGVKLIFPDKTPKAAYLYFTCDIP
DEPLTETAKKIQWLETGDVTKKGKKRKKKVLPHGLVSCAVDLSMRRGTTGFATLCRYENG
KIHILRSRNLWVGYKEGKGCHPYRWTEGPDLGHIAKHKREIRILRSKRGKPVKGEESHID
LQKHIDYMGEDRFKKAARTIVNFALNTENAASKNGFYPRADVLLLENLEGLIPDAEKERG
INRALAGWNRRHLVERVIEMAKDAGFKRRVFEIPPYGTSQVCSKCGALGRRYSIIRENNR
REIRFGYVEKLFACPNCGYCANADHNASVNLNRRFLIEDSFKSYYDWKRLSEKKQKEEIE
TIESKLMDKLCAMHKISRGSISK. (SEQ ID NO:228)
   >Cas14g.2|3300009652.a|Ga0123330_1010394|2814..5123 
MHLWRTHCVFNQRLPALLKRLFAMRRGEVGGNEAQRQVYQRVAQFVLARDAKDSVDLLNA
VSLRKRSANSAFKKKATISCNGQAREVTGEEVFAEAVALASKGVFAYDKDDMRAGLPDSL
FQPLTRDAVACMRSHEELVATWKKEYREWRDRKSEWEAEPEHALYLNLRPKFEEGEAARG
GRFRKRAERDHAYLDWLEANPQLAAWRRKAPPAVVPIDEAGKRRIARAKAWKQASVRAEE
FWKRNPELHALHKIHVQYLREFVRPRRTRRNKRREGFKQRPTFTMPDPVRHPRWCLFNAP
QTSPQGYRLLRLPQSRRTVGSVELRLLTGPSDGAGFPDAWVNVRFKADPRLAQLRPVKVP
RTVTRGKNKGAKVEADGFRYYDDQLLIERDAQVSGVKLLFRDIRMAPFADKPIEDRLLSA
TPYLVFAVEIKDEARTERAKAIRFDETSELTKSGKKRKTLPAGLVSVAVDLDTRGVGFLT
RAVIGVPEIQQTHHGVRLLQSRYVAVGQVEARASGEAEWSPGPDLAHIARHKREIRRLRQ
LRGKPVKGERSHVRLQAHIDRMGEDRFKKAARKIVNEALRGSNPAAGDPYTRADVLLYES
LETLLPDAERERGINRALLRWNRAKLIEHLKRMCDDAGIRHFPVSPFGTSQVCSKCGALG
RRYSLARENGRAVIRFGWVERLFACPNPECPGRRPDRPDRPFTCNSDHNASVNLHRVFAL
GDQAVAAFRALAPRDSPARTLAVKRVEDTLRPQLMRVHKLADAGVDSPF (SEQ ID NO:229)
Cas14h sequences
   >Cas14h.1|3300005602.a|Ga0070762_10001740|7377..9071|revcom 
MSRVELHRAYKFRLYPTPAQVAELAEWERQLRRLYNLAHSQRLAAMQRHVRPKSPGVLKS
ECLSCGAVAVAEIGTDGKAKKTVKHAVGCSVLECRSCGGSPDAEGRTAHTAACSFVDYYR
QGREMTQLLEEDDQLARVVCSARQETLRDLEKAWQRWHKMPGFGKPHFKKRIDSCRIYFS
TPKSWAVDLGYLSFTGVASSVGRIKIRQDRVWPGDAKFSSCHVVRDVDEWYAVFPLTFTK
EIEKPKGGAVGINRGAVHAIADSTGRVVDSPKFYARSLGVIRHRARLLDRKVPFGRAVKP
SPTKYHGLPKADIDAAAARVNASPGRLVYEARARGSIAAAEAHLAALVLPAPRQTSQLPS
EGRNRERARRFLALAHQRVRRQREWFLHNESAHYAQSYTKIAIEDWSTKEMTSSEPRDAE
EMKRVTRARNRSILDVGWYELGRQIAYKSEATGAEFAKVDPGLRETETHVPEAIVRERDV
DVSGMLRGEAGISGTCSRCGGLLRASASGHADAECEVCLHVEVGDVNAAVNVLKRAMFPG
AAPPSKEKAKVTIGIKGRKKKRAA. (SEQ ID NO:230)
   >Cas14h.2|3300005921.a|Ga0070766_10011912|384..2081 MSRVELHRAYKFRLYPTPVQVAELSEWERQLRRLYNLGHEQRLLTLTRHLRPKSPGVLKG 
ECLSCDSTQVQEVGADGRPKTTVRHAEQCPTLACRSCGALRDAEGRTAHTVACAFVDYYR
QGREMTELLAADDQLARVVCSARQEVLRDLDKAWQRWRKMPGFGKPRFKRRTDSCRIYFS
TPKAWKLEGGHLSFTGAATTVGAIKMRQDRNWPASVQFSSCHVVRDVDEWYAVFPLTFVA
EVARPKGGAVGINRGAVHAIADSTGRVVDSPRYYARALGVIRHRARLFDRKVPSGHAVKP
SPTKYRGLSAIEVDRVARATGFTPGRVVTEALNRGGVAYAECALAAIAVLGHGPERPLTS
DGRNREKARKFLALAHQRVRRQREWFLHNESAHYARTYSKIAIEDWSTKEMTASEPQGEE
TRRVTRSRNRSILDVGWYELGRQLAYKTEATGAEFAQVDPGLKETETNVPKAIADARDVD
VSGMLRGEAGISGTCSKCGGLLRAPASGHADAECEICLNVEVGDVNAAVNVLKRAMFPGD
APPASGEKPKVSIGIKGRQKKKKAA. (SEQ ID NO:231)
   >Cas14h.3|3300009698.a|Ga0116216_10000905|8005..9504 
MEAIATGMSPERRVELGILPGSVELKRAYKFRLYPMKVQQAELSEWERQLRRLYNLAHEQ
RLAALLRYRDWDFQKGACPSCRVAVPGVHTAACDHVDYFRQAREMTQLLEVDAQLSRVIC
CARQEVLRDLDKAWQRWRKKLGGRPRFKRRTDSCRIYLSTPKHWEIAGRYLRLSGLASSV
GEIRIEQDRAFPEGALLSSCSIVRDVDEWYACLPLTFTQPIERAPHRSVGLNRGVVHALA
DSDGRVVDSPKFFERALATVQKRSRDLARKVSGSRNAHKARIKLAKAHQRVRRQRAAFLH
QESAYYSKGFDLVALEDMSVRKMTATAGEAPEMGRGAQRDLNRGILDVGWYELARQIDYK
RLAHGGELLRVDPGQTTPLACVTEEQPARGISSACAVCGIPLARPASGNARMRCTACGSS
QVGDVNAAENVLTRALSSAPSGPKSPKASIKIKGRQKRLGTPANRAGEASGGDPPVRGPV
EGGTLAYVVEPVSESQSDT. (SEQ ID NO:232)
Cas14i sequences
   >Ga0066868 100162752 
MTRNYPYKFRLEPTEEQKTRLKHYGFTCRFIYNLALDQRNLSRDPKPLPTLLEMWEKRVADKLAGVKPERKER
NFEEERKQEVVHKNINYGFQSPQMTVLRREVEWMQDVPFSCLQETLRSLQTAFKNFFDRVKKGQRVSDGR
NPYGYPVYRSRYRLSIPFKPANVSIKKVSERAGGEEGAYFSELKVPLMGSLIRFRQDRPVLGTPKTPTLKLEGDG
KWYVVILTEQEVEDPQTPEAEVGIDLGVAKMITLSDGTIYPLTKKQQQTFTNIDTTEKRIRKLQAACDRRKTKF
SKNWIKVKRQVVKLKHRQKRSRESLHHEITHLITSGFGRVAVENLNIKGMTPSASGTEEEPGTNVAQKSGLN
REILKRGWGLLVSQLEYKAKWRGGEVIKVDPKYTSQTCSKCGHVEKANRATQATFLCQKCGHKENADVNAA
KNILTRAEKQ*. (SEQ ID NO:233)
   >PhageCas14_SR-VP_2-4_scaffold_141_2548329_92 
MAKQAPGKRTDESKERKAFSFRLYPTPEQERYLARVVGSCRYIYNALVREHERRMKYMRTFGAWPKPIGFKT
SKKKQSLAEDYKLEASLYEIQTALHEPGGPAPWLEDVAGNIRNHAVAMFGAAQTNWMSGRTGPPNFKQRR
PAGSFRFQDTRVASITGGPDRQPGFDFIRIPLPHGIEIDSWICFRRHRRLRGQPKTATIRRAAGIWYVSILCEW
DKPAKLPVHRAPNAKVGVDLNVRNLCALSDGTIIDGRSADLARLEKSINRLKHRESKLRLREKAASAPRSKRHF
RLQCRIARLQDRQANLRNEVTNQVAHAVALKHAFVGLEGLDIKGMTASAKGTVDAPGLNVRAKAGLNRAIL
NRGWGKLREKIESKVKIYGGQTVRVPPQYTSQTCAKCGHIAAENRDGVIFHCVKCGFTAHADVNAATNILEK
ALRLSAQESPGSGSLDGERPTELGSTTRQRVRKQKDTKTLGAPKATSRKGATAPRSTIPSLHVDMQVTSARVV
PAPQEALATEIAQQMKALAKSEVDAAPRQKINRRRRSQTEVEVPTGSVE* (SEQ ID NO:234)
   >PhageCas14_SR-VP_4-6_scaffold_141_3640E89_5 
MAKQAPGKRTDESKERKAFSFRLYPTPEQERYLARVVGSCRYIYNALVREHERRMKYMRTFGAWPKPIGFKT
SKKKQSLAEDYKLEASLYEIQTALHEPGGPAPWLEDVAGNIRNHAVALFGAAQTNWMSGRTGPPNFKQRR
PAGSFRFQDTRVASITGGPDRQPGFDFIRIPLPHGIEIDSWICFRRHRRLRGQPKTATIRRAAGIWYVSILCEW
DKPAKLPVHRAPNAKVGVDLNVRYLCALSDGTIIDGRSADLARLEKSINRLKHRESKLRLREKAASAPRSKRHF
RLQCRIARLQDRQANLRNEVTNQVAHAVALKHAFVGLEGLDIKGMTASAKGTVDAPGLNVRAKAGLNRAIL
NRGWGKLREKIESKVKIYGGQTVRVPPQYTSQTCAKCGHIAAENRDGVIFHCVKCGFTAHADVNAATNILEK
ALRLSAQESPGSGSLDGERPTELGSTTRQRVRKQKDTKTLGAPKATSRKGATAPRSTIRSLHVDMQVTSARVV
PAPQEALATEIAQQMKALAKSEVDAAPRQKINRRRRSQTEVEVPTGSVE* (SEQ ID NO:235)
Cas14J sequences
   >PhageCas14J_k87_9374247_16 
MIESKAFKFRVYPTDKQKELIHNSVRASNFIFNFSLRQQIDISDKMNEMGIIEKGERKKYMKDNDLYFNKYTM
SRQLTVMGNTEEFSFLKEIDATSKSYALRRIDNAFKNMVKMGAGFPKFKNINKSTYSFTGQIQYQNDRIKNLR
VIKTKNPKIVHLNLSKLKNLKCVCH[?]PMFIENWSNMDTIKINSYTISRKGNNYYISFQVEHNQPLISEPIKREIKYE
TTIGIDMGVERPITTSDEADFNLKLFNERFNILKKHRKELHKLSAILNKKRDYHKKNESEIKFYETATYKRILKKM
RGLYHKITNIRENLQHNITSNLVNKENIDTFILEELNLKN[?]TKRSGKGKSNNKSNLNRVLLDVGMHGIKSKLEY
KAEKMGKNVETINPRFTSQKCSDCGHINKLNRKSQAVFKCVKCGYTLNADLNAAINIKNNFFGKNT* (SEQ IN
NO:236)
   >PhageCas14J_LacPavin_0818_WC40_scaffold_407201_205 
MEDIIEISEKKKKTKISGTGKGFSIRIYPDKKQIEYIRDSFRVNNFIYNYFLSKQEKIVSELKEMGLEGKALKSHMK
LNNLYFDYNSSRDLLYEMKKTPEYSFLGNASALSYHYALMRLKNAFDNMWKMNTGFPNYRKRHINKSFSGQ
ILFNTKADKYSPFEIQTINDKWCEITLTKITELKCVVHNNELLDFWNDRSYMHLKSYTITETPSGEFYLAITADIIS
KPMLEKRIVNEETSIGIDMGVARPITTSDEELFNDKQLSDKFNLIKEYKSEVERLSQILAKKREGNKNWKESKKY
ERIKKRLAKLHSKIANIRKYLQHNITSKLINSKYDTIIIEDLDVKNMMKKSAKGKSNNKRGLNRVLSDTGLGEIKR
QLVYKSNWCGKNIVTVDPKYTSQMCSNCGHTHRDNRKKQDEFICVSCGHNENADLNAAKNIKNKFFKKLAE
LKN* (SEQ ID NO:237)
   >PhageCas14J_BML_08042016_6_5m_scaffold_18_prodigal-single_54 
MITKAYKFRIYPTKVQEETINNCFRVNDFIYNFFLGLEQETYDVLYMYGLRNGEKKEDKHLNKWRTENKLWF
NRFDASRLLTKMAKLEKYKFLKTYPSTSRTYSLKSLESGMKSFMKGGGFPKFKNKKSNKSFTIQTQKDLKIIHKN
GKWHSINLPSALDFPIKKLDIKIHNELFLSPNIKTNSCTVSKRGNQYFISFQVELPGELPRKREIKKETSVGVDFG
VKKIITISSDEENPYSCETRFLKNSMNELKRLQKALSQKKKGSVKYNNIKEKINKLHIKISNQRKNLQHNISSFLV
NLNADTIIMEDLNLKGMTKTPNPIESNGTFLPNGKSRKSGLNASILDVGIGEIKTQVQYKSDFCGKNVVLVNP
QYTSQKCNNCGFTHKENRISQSEFECKNCGHKDNADKNASKNIKQKYFDN* (SEQ ID NO:238)
   >Ga0194119_1000113823 VKQNKAYKYRIYPTEKQIEYFEGAFKAGRYVYNVSLDCEKQIYQLGGKSNLSHFGLNYHIKNYRVKAPFLNEYD 
VNIYCNEMKALSKAYKNFFKNKGGYPKFKKESDTTQSFTTRPSTKQNSKNLYITYDGYLKIPKVEKLIKIKYHRPI
EGKIKTVTISKKHNKYYVSIMVEYTNNFKKVEVKKSVGIDLGVKAFVVTSDNEVIENPKHLTKNQEHLTVLQRK
LARAKKGSNNYKKIKKNISKIHENVANTRENFLHNESKKLVDNYDLICMEDLNVKGMTKSSKGTKENPGKNV
KQKSGLNRSIIDVGFGKFKTMIGYKTKNSGKYLVEIGRFEPTSKKCNCCGTINKNLELKDRIWKCENCGEILNRD
LNAALNIRDLGTKKFFDSLKK (SEQ ID NO:239)
   >Ga0116197_10005458 
MLKAYKYRIYPTKEQITLIEKHFGSTRFLYNYFLEYRQKAYAKGNQKVGYMVTQAELTKLKKLKEYEWLNECGS
QSLQMALRDLDSAYSRFFKKQGGYPKFKSKKHTSQSFTAPQNIKLASNRVYLPKFTKDGIKVKLHREIPQDAVL
KQATVSRQNNQYFVSILIDDNNAIPKPIKAKNAVGLDMGLTDLIITSDFTKYPNNKYFVKSQQKLKKLQRRHSK
KQKGSNNRQKAKLRVQKLHTKVSNQRKDTLHKISNEITNQYDIICLETLNVRGMQKNRRLAKGIADVAWSEF
MRQLAYKAQWKGKTVLKIDQWFPSSQICSNCGASSKKKELHVRKWECPECHAKHDRDINASINIKNYGLGQ
IDNRNTVGTIGI* (SEQ ID NO:240)
   >Ga0116179_10426881 
MKIINKTYRFRLFPTKEQEVLLNKHFGCCRWVYNHFLNERKEQYQANKKSDNYYKQAATLAKLKNEEDTKWL
KEVNSQSLQFALRSLDTAFLNFFRGKAQFPKFKSKKHKNTFTIPQFGKLEDGKIVIPKFKEGIKVKLHREVKGKI
GKMSITKTPTGKYYVSIFTEQEVEELPKTNKQVGIDLGLKDFVITSDNKKFKNNRYVKKYEKQLKKAQQHLSRK
QKGSKGFEKQKLKVAKIHEKIANCRLDILHKVSTELVKNYDLIAVEDLNVKGMTKNHKLSKHIADASWGKFVT
LLQYKCDWYGKKLVKVNRFYPSSKTCSECGWINQELKLSDREWTCNSCGAIHDRDLNASKNILKEGLKIISAG
AVDYTDGDLNDASVKKRKSVKSEAQPIAFGVGG* (SEQ ID NO:241)
   >Ga0268285_10062095 
MIKAFKYRIYPTQDQKELLSNIFGQVRFVYNLGLETKISAYTGNKKHLSCFDLNKQITQLKNECPWLKESPSQA
LQQSIRNLDVAYTNFFRGAGFPKFKNKYTKQSFQLPQGVFLSDDKKQIFIPKLKFTDIDLHKEFKGEVKTVTVSK
TTTNKYYISILVDDKKPIPEKRQIKLESTVGIDLGIKDFAITSDGKKFKNHDFFKSAMNELRIQQRSLARKQKGSN
HYIKQKMKVSLLHEHIKNQREDYLHKISKYLVYNYDTICIENLGVSNMMKNHKLSRVIGDMGWHKFKSMLEY
KCEWYGKNLSVIGRFDPSSKTCSSCGSINKELTLNDREWTCKCGTKHDRDINAAINIRNFGLRNQPSVTQSE
WLHCACDVETHQSLADV (SEQ ID NO:242)
Cas14K sequences
   >PhageCas14_RifSed 
MTTQKTYNFCFYDQRFFELSKEAGEVYSRSLEEFWKIYDETGVWLSKFDLQKHMRNKLERKLLHSDSFLGAM
QQVHANLASWKQAKKVVPDACPPRKPKFLQAILFKKSQIKYKNGFLRLTLGTEKEFLYLKWDINIPLPIYGSVT
YSKTRGWKINLCLETEVEQKNLSENKYLSIDLGVKRVATIFDGENTITLSGKKFMGLMHYRNKLNGKTQSRLS
HKKKGSNNYKKIQRAKRKTTDRLLNIQKEMLHKYSSFIVNYAIRNDIGNIIIGDNSSTHDSPNMRGKTNQKISQ
NPEQKLKNYIKYKFESISGRVDIVPEPYTSRKCPHCKNIKKSSPKGRTYKCKKCGFIFDRDGVGAINIYNENVSFG
QIISPGRIRSLTEPIGMKFHNEIYFKSYVAA (SEQ ID NO:243)
   >PhageCas14_16ft_4_scaffold_2_465_16ft_4 Phage 29 13 
VITKKTYNFSLYDPRFFELAKEAGDVYSRSLEEFWKVYDETGVWLSKFDLQKHMRNKLERKLLHSDSFIGAMQ
QVHANLASWKQAKKVVKDACPPRKPKFLQAILFKKSQIKYKNGFLKLTLGIGNEYLNLKWNQEIPLPIYGSVTY
SKTRGWKINLCLETDVEQKNLDNNKFLSIDLGVKRIATIFDGENTITLSGKKFMGLMHYRNKLNGKTQSRLSH
KKKGSNNYKKIQRAKRRTTDKILNIQKDMLHKYSSFVVNYAIKNNIGNIIIGDNSSTHDSPNMRGKTNQKISQ
NPEQKLKNYIKYKFEGISGQVNIVPEPYTSRKCPCCKNIKKSSPRGRTYKCKKCDFVFDRDGVGAINIYNENVSF
GTCLNLDSGRIRFLTEPIGMKFHNEVYFKSYVAVA* (SEQ ID NO:244)
   >Ga0116179 10109322 
LKELYKTYILPVKQQELARKLSRESGRIYSKVVSKVFDIYKRKGFWLNEFDMKKYIRLYAKNIGLHSQTKQGIVE
QYYIALDSFFKAYKNHRNPKPPYKRRKYNVVMYKDSAIKLKNGILKLSNGKGNEPLMVKANKLGKKPKYAELV
YHHNKRKYFLHITVEMKGVQRVYEKDRAIAVDLGQIHPMVTYDSKRSIIFNGGVLNSFIRFRNKQLSKLQQK
MSMCKKYSKRWKKLNGAKKKLLNKSKNKVNDVLQKYTSYLVGYCIEQGIGTIVIGDIKSIRENINYGVKTNQKL
HNSWLFRKMTNIIEHKANNVGIKVEYINEAYTSQTCPVCNKKHKPGNRNFTCKCGFKYHRDAVGAINIHKKY
TSSLSARLEGDLTPPVGYRYRYNQRCLAGWNTSIFDAGYFSDLPTKKVA* (SEQ ID NO:245)
   >Ga0116179 10465782 
MSRYVVRTYKVAVPKELYPLCAELNKTAARIYNKTMSLVKKIKYKKGFWLSPNNTQKYILRWACSINVHTHSK
QAIIQQYFQALDSYFNAVKTKPDLNPPYKRKRFMPFIWKDTAIKLLPDGKLKLSMGSNREPIVIQTTLLADTKIR
QAKLVYEEGKYYLHLVIEGKNVARKPQNGKIMAVDLGILRPITCFDGTEVISYHGGILNSLIRYRNKELAKFQQ
MLSRCKKGSKRYRKLVKAKKKMLRRTRHQIKDILHKITSNFLKMCLQKGIGTIALGDVTNIRERVEGNDSANQ
KLHQWCFRKMVDMITYKAELLGMDVKLVPEEYTSQTCPMCGSRNHSNNRNYKCQNCGFKYHRDGVGAIN
IYVRYLGKKSQVVAGLAPVRGVRYKPHLCGHGVRNAPWKAA* (SEQ ID NO:246)
   >Ga0134101 10165752 
MPGYVVRTYKVPVPEELYPLCAELNKTAARIYNKTMSLVKKIKRKKGIWLSSNNAQKYILRWACGINVHTHSK
QAMVQQYFQALDSYFNAVKAKPDLRPPYKKKRFMPFIWKDAAIKLLPDGKLRLSMGNNQKPVVIQTTLPAD
TKIRQAKLVYEDGKYYLHLATEVKNEVQKQQGKKVMAVDLGILRPITCFDGIEVISYHGGILNSLIRYRNKELAK
FQQMLSRCKKGSKRYRKLVKAKKKMLRRIRHQIKDILHKITSNFLKMCLQKGIKTIAVGDITNIRERVQGNDN
ANQKLHQWCFRKMIDMLTYKVHPLGIDVKLVPENYTSQTCPACGSRNHPTDRNYECQNCGFKYHRDGVG
AINIYARYLGKKSQVVAGLAPVRGVRYKPHLCGHGV (SEQ ID NO:247)
   >Ga0066665 100815632 
MYQVRRVNIGKTAQLDELARECGRLYSQTLASFWRTVRHKGIWLKPKHLMRWHTSEKLHAHTADACVQAF
FASLKSWRERRKLGDPDAHPPRKRKWYFRIEYKSTAMHHKDSVLTLSNGKGNTPLVLEWPWETPKTVVIHW
TGTQYEAIATYKIEAQGQPQGNKVAGIDLGEIHMAVSHDGTETHILNGRLLRSKRQYQNKLKAELSTMIDVK
KKGSLRRKKLIRSKQKQLKKLQHQVNDIEHKQSSRLISTLHAKGVQTVVIGDVRDIRQDLDVGSKNNQKLHQ
WSHGSIRHKLTYKAEWLGMEVALQDEHYTSRTCPMCQHVRKSKVQGRVFRCPTCHWTYHRDGVGAINIR
QKYLGSLPVIGDMAPPIGMRFRPHTSVARWEKTYQ* (SEQ ID NO:248)
   >Ga0224523 10070512 MYNVRKLKIDQTEQLDVLATASGELYSRTLVSFWRTVRKHGLWLKPSSMMRWQNSGELHAHSADAVVQS 
FYASLKSWRALRKVDPDAKPPKRRKHFFKVQWKNSAIRLKDGCLVLSNGKGNEPLIIPWNWTLPTLVELGW
NGTGYELRVIYSTTPTGVPLGVKVAGVDMGEIHLAVTHDGDDCHIYNGRYLRSVKRYQNKKKAEISARLDRM
KKGSRRSKYLKHNKARTLKKLDNQINDILHKQTTKLVSTLHEAGVKTVVIGDVRDIRKGLDYGAKANQKIHQW
HLGKTRWLVSYKAERLGMEVVLQDEAYTSQTCPACGKRHKPKDRNYRCSCGFQYHRDGIGAYNIRAKYLGE
LETPHVVGAMMSPTGVRVLQRCSHLARKNPLPLGMG (SEQ ID NO:249)
   >Ga0247839_10583994 
MNIAHQDAIWEASKESASIYNDAIKLNQDGIPKAQAMKSLSIQSKHTKYLQSQSSQAPYQNFFIDLSSYFASLK
RYQKSKRGYKNEPKPPHKIKTLHAITFKKSAIRVQNGYLLLSLRKPNKPIKLKWSLSKPIWVLINFDIRTGWKMN
CVMEQEVQQHQLDKTKILAIDLGNKRIAASFDGKRCVTYSGKILKSLTRLQNKCSARSKASTSSLIKNSKKYKRV
MRARRKITARINNQKRDILHKTSRAIVNYAIENNIDKIVFGDCSSIHDGTTLGKENTQQVQQGCEQKLRKYVE
YKFRNVGGTTELVSERYSSQECPICDHRYEPRGRTYKCSACGYVYDRDGVGSINIYTNVSSGLTLDVVGGLMP
PRGWKFHSQLPCTTLRNSYFSMLYCGEPNDL (SEQ ID NO:250)
Cas14u sequences
   >Cas14u9|PhageCas14|LacPavin_0818_WC55_scaffold_56344_prodigal-single_16 
VRKIAESKGYFTKAVSVELVGHSKEDTVWLLDILNRGYPLANKMYLLYRWYYEGLFPT
EIELNKLETYVYHKAREDSRFTDIPSNIIACTNRTILQKIKYDIKSGAKSGKRSWSQFKKG
QPLYFVQHNYLEKTDDGYNYNFIFGHKFKLKFGRHNEGEQLIEKLMDSESQFKLNANA
AFKVIKRRLFLLLSYEIPDKIENKPNPDNIMGIDFGMANFATCYLANDRKFKIVRDHKYLK
KRLLLQRKIKNLQSELSMHHAGLGRARKTRKIEDYRNKEKNLTKTEISQILSSIVRLAQA
NNIGTIKIEYLTIDQKTQLEDKYVYRNWAVMMTIDMLREKAKYVGINVVTIDPYHTSQKC
STCGTIGTRDGRIFSCENPSCKSFHKVVNADKNAAINIANSTQFVDDVKDTEYYKQKQEFFKTLREKKETNIT* (SEQ
ID NO:251)
   >Cas14u10|Ga0153798_100522201 
MAKKNIDDTKKVTLCEKVKLTQIYSPVVDWKEFHKIFKILQKETILASNKIISICNIFNSFNN
KEEQKDWLIKKYQSEKLRNVLYDVARKYCYYSYSRNANAISNDIYYKYFKGPNSYKVKI
QKGIGNPPMTFTESIPLYITVQRHKIECTNNVRHYYTIEVPLLSNNCKSGIQITDTEQTQV
NNNALRFGINAAGNKRLIEILDNIIYGKYEFCDSKLKRVKSKKRSHRYDYYFLLSYKKPVI
EIKSLKPENVLGVDLGMTVPAYCAVNYCDYKKKAVGDSRIIRFNLIQEKINKRIQRNIKYN
LRDGHGRKYKLDGYDGASNKIAKRNSTFNFNLASEIIQLAIKWQCGTIHLEDLTKIHEINP
QNRFLKNWTYYDLQKKIENKAKEYGIVVKYINPYYTSQICSNCGHFESGQRISQSQFQC
KSCGYSANADYNAARNIALYKF* (SEQ ID NO:252)
   >Cas14u_VU_u11|rifcsplowo2_12_scaffold_23_prodigal-single_23 
MSTMVFEYYLRSPEKEQEQIVIQQLRASYEYYNTLIRIEQNRRNQFRAIQSQDPKIAQLE
LEISSLDTEIDLHLTSIQNTRSTNRKNVLDKKDVDRVKSLKADRKLKRDELKIAKKSFCD
NLIFQKACEDINLFAKNESKAARKATPSYWGSYLLIENAIDAAKKSKTDPKRKYWDWTG
RLGVQVQGGMSVSELFGNDTRIQIDPVSLDAWYHPIRGKRKYAQRQPKLRFRINSDDK
 
GKPIFVEFPMIMHRPLPQNACIKQANVIVTNRDRKLCYVLQLTVNIPEPVASPCTNGVGI
DLGWRLMDSGDIRVAYGYDQKGTKIDLRLPKSITSLFQKAESIRAIRDKEFEDHRKIMIP
LIQGVTFPNINTTNIGLSKSFRRFHSLYLGWKANRQDGDQIAFDALETWHRKDRHLEQ
YEVGCRKRAMNYRREEYRKFAKQMTSTYGYLALENWNISKVALRPEIEDGTREQSEP
QHQRVMACVSMLRQILINTAKREGVSIISVPAAYTTLECAACHKINTWDTSKNVCQTCE
NCDTVWDQDENAARNLLASGTVLKNTAPLPEEANIANTEKKSRWSKRKAEVVIDEKVDRSQIAS*  (SEQ ID
NO:253)
   >Cas14u_VU_u12ISR-VP_4-6_scaffold_141_2630357_509 
MKVYKYGLLPPIKNQTLVFEQLNKAYQYKKQLIDLVNQEKALLKKEEDNIFQRLNPALIS
KKETTQQTVEELLALMKQQRSKNRSKQDNIELKQQFKIAKENAKQAKKDYFTELSRIKT
LEEVKTSKEKIKTNFKQLHKEARKKCGVYWGTYLLIEEAVEQSKKTSFKKDFIFYGRRD
NERLGNQIQTSKDDSGSKIMGMLSSHLFNEKNSQIYIEPVADTAWIGVYRKDRRRTAKT
ILHWRIASDEKLKPIWAEFPMIMHRPLPKDSKIKSATISRRFYGPHQEWTLEITIDDNLSP
TKELGNGVVALDIGWRKLNDKIRVATLYDGEFHKELVISTYQLDKANELKSLRDDLFNQ
VKNQITEWNKEKFPEWILKELEFVSKWKSQARLVRLVKNWKKERWQDDNIYFELVEA
WRYKDQHLWQWECGSRRSGLRERIIIATLPPNLERNITVLYWKTLIFQRWQNYQNFRQ
KKI* (SEQ ID NO:254)
   >Cas14u_VU_u13|gwd1_scaffold_1554_3 
MPVKAVKFQIIKPLNATWDVLGKTLRDLNYHTTLMCNRAIQLYWEYGNFRSQYKAEHG
KYPIDKDIYGCSYRNHVYRQLRLMYPLMASSNTSQTNQFALKRWQTDVPDIRKLAKSIP
SFKLGTPIQVANQNFDLRFNDDTFSVDVTLLGRESEVGRFSILLDTGDKSKRVIFQRILD
RTYKQGSMQIVYSKKKGKWFCVIAYDSPIKVNELDIDKVMGIDLGIVNAVYWAFNSGHN
RGCISGGEIDTFRKQIEVRRRQILRTPRKDGHGRKRNMQAADILGEKISNFRDTVNHKY
SKKIIDIAIANKCGVIQMEDLTGISKDSFFLRNWTYRDLQDKIVYKALQEGIIVKLIDPRNT
SKTCSVCGHLDAENREDQATFICKNPECGSNMNADHNAAKNISVWSKVSKEFGL* (SEQ ID NO:255)
   >Cas14u_VU_u14|pig_F100_scaffold_13388_4 
MNKVMRYQIIKPIDIDWKTFGDILNKLRQEVRFTKNKTIALYNDWLTYCFQYKNEHNEY
PKLVDYCGYKVFSGYAYDKFKTEVVFSNTANYTTSVREACSAYDAHKTDILKGNCSIPS
MGANQPIDLHNKSLSVDINEFGDYIATISLLSNRGKKEFGLKSGQIKIVLKAGDKSSRDIL
QRCVSKEYKICGSKIIYKDKKTFINLCYGFEPVTSELDKSKVMGIDLGVSVPAYMAFNFD
KYKRDSIKDNRIMATKWMMDRQLSIAKQSCKYLSDGNCGHGRKKKMVCYDKYSNKS
RNLSQTINHGWSKYIVDVAFRNGCGTIQMEDLSGVTSEKDKFLKNWTFYDLQQKIEYK
AKERGINVVKINPKYTSQRCCECGCICKRNRPDQKTFKCISCGYSANADFNAAKNIATI
GIEDIIANTEVIE* (SEQ ID NO:256)
   >Cas14u_VU_u15|pig_ID_3640_F65_scaffold_73762_2 
MRIEIMVKKKGINMNKIMKYQILKPTNIGWEDFGNILYNLRSEVRKIKNRTIALYHEWTGY
TLECHDRTGEWPKPKDVYNYGTIGGYIYDRLKGEVKYSNSVNFSSSVRDAMSKYDTH
 
KKDILAGKASVPSMGDGQPIDIYNKNIVLHHLDNEKKDYAATLSLLNNGAKTELGLLSG
RVDVILTIKNETQTAILDRCLSGEYRVCGSQLVYEAAGKEKKGKKDKPKVWLYLCYGFE
PEAPELDDSRIMGIDLGMKLPAVMAFNFNDKKYEVIDDNRILDRKIRLDKMLSISKHQCQ
WRCDGNSGHGRKKKVDVYERYSHKSHNLSMHINHQWSKYIVDTAVKNKCGVIQMED
LSGIKASRQNFLGNWTYYDLQQKITYKAEEKGVKVIKVDPSYTSQMCPVCGYINKRNR
STQADFECLECGHIANADYNAARNIATPDIANIIKNRLAQQKKEGKPIE* (SEQ ID NO:257)
   >Cas14u_VU_u16|pig_ID_1851_F40_2_scaffold_55126_1 
MPMSSYRKTHYTNTCELREIYMRIEIMVKKKGINMNKIMKYQILKPTNISWEDFGNILYN
LRSEVRKIKNRTIALYHEWTNYTLECHDKTGEWPKPKDVYNYGTMSGYIYDRLKGEVR
YSNSVNFNSSVRDAMSKYDTHKKDILAGKVSVPSMGDGQPIDIYNKNIVLHHLDNEKK
DYAATLSLLNNGAKAELGLLSGRVDVILTIKNETQTAILDRCLSGEYRICGSQLIYEGGK
EKKGKKDKPKVWLYLCYGFEPEAPELDDSRIMGIDLGMKLPAVMAFNFNDKKYEVIDD
NRILDRKIRLDKMLSMSKHQCQWRCDGNSGHGRNKKVDVYERYSHKSHNLSMDINH
QWSKYIVDTAVKNKCGVIQMEDLSGIKASRQNFLGNWTYYDLQQKITYKAEEKGIKVIK
VDPCYTSQMCPVCGYINKRNRSTQADFECLECGHIANADYNAARNIATPDIANIIKNRL
AQQKKEGKPIE*. (SEQ ID NO:258)
   >Cas14u_VU_u17|pig_ID_3784_F96_scaffold_13509_10 
MNKIMKYQIIKPLNIDWETFGNILFNLRKESRQVKNRAIAIYHEWVLYSMAYYDECGKW
PKIIDVYPPYKTADGYIYDKLKNEMGHMLSNNFNATIRNALSKYDTHKKDIMAGKVSVP
SMDAGQPIDVYAKGITLHHIDGDKGDYVATLSLLNSKAKATLNLPSGRIDMVLKMNDKT
QTAILDRCLSGEYRICGSQLVYEAAGKEKKGKKDKPKVWLYLCYGFEPEAPELDDSRI
MGIDLGMKLPAVMAFNFNDKKYEVIDDNRILDRKIRLDKMLSISKHQCQWRCDGNSGH
GRKKKVDVYERYSHKSHNLSMDINHQWSKYIVETAVKNKCGVIQVEDLSGIKASRQNF
LGNWTYYDLQQKITYKAEEKGIKVIKVDPSYTSQMCPVCGYINKRNRSTQADFECLEC
GHIANADYNAARNIATPDIANIIKNRLAQQKKEGKPIE* (SEQ ID NO:259)
   >Cas14u_VU_u18|SRR1747065_scaffold_28 
MNKVMKYQIIKPLNIDWEDFGNILFNLRKESRQIKNRAIAIYHEWVQYSMSYYDEYGKW
PKVIDVYPPYKTVDGYIYDRLKNEMGHTSSNNFNATIRNALSKYDTHKKDIMAGKVSVP
SMDAGQPIDVYAKGITLHHIDGDKDDYVATLSLLNSKAKATLNLPSGRIDMVLKMNDKT
QTAILDRCLSGEYRICGSQLIYEAAGKEKKGKKDKPKVWLYLCYGFEPEAPELDDSRIM
GIDLGMKLPAVMAFNFNDKKYEVIDDNRILGQKIRLDKMLSISKHQCQWRCDGNSGHG
RKKKVDVYEKCSHRSHNLSMDINHQWSKYIVETAIKNKCGVIQMEDLSGIKASRQNFL
GNWTYYDLQQKITYKAEGKGIKVIKIDPHYTSQMCPICGYINKRNRSTQADFECLECGH
IANADYNAARNIATPDIANIIKNRVKQQEKEGKSID (SEQ ID NO:260)
   >Cas14u.1|3300009029.a|Ga0066793_10010091|37..1113|revcom 
MSTITRQVRLSPTPEQSRLLMAHCQQYISTVNVLVAAFDSEVLTGKVSTKDFRAALPSAV
KNQALRDAQSVFKRSVELGCLPVLKKPHCQWNNQNWRVEGDQLILPICKDGKTQQERFRC
 
AAVALEGKAGILRIKKKRGKWIADLTVTQEDAPESSGSAIMGVDLGIKVPAVAHIGGKGT
RFFGNGRSQRSMRRRFYARRKTLQKAKKLRAVRKSKGKEARWMKTINHQLSRQIVNHAHA
LGVGTIKIEALQGIRKGTTRKSRGAAARKNNRMTNTWSFSQLTLFITYKAQRQGITVEQV
DPAYTSQDCPACRARNGAQDRTYVCSECGWRGHRDTVGAINISRRAGLSGHRRGATGA (SEQ ID NO:261)
   >Cas14u.2|3300002172.a|JGI24730J26740_1002785|496..1605|revcom 
MLQTLLVKLDPSKEQYKMLYETMERFNEACNQIAETVFAIHSANKIEVQKTVYYPIREKF
GLSAQLTILAIRKVCEAYKRDKSIKPEFRLDGALVYDQRVLSWKGLDKVSLVTLQGRQII
PIKFGDYQKARMDRIRGQADLILVKGVFYLCVVVEVSEESPYDPKGVLGVDLGIKNLAVD
SDGEVHSGEQTTNTRERLDSLKARLQSKGTKSAKRHLKKLSGRMAKFSKDVNHCISKKLV
AKAKGTLMSIALEDLQGIRDRVTVRKAQRRNLHTWNFGLLRMFVDYKAKIAGVPLVFVDP
RNTSRTCPSCGHVAKANRPTRDEFRCVSCGFAGAADHIAAMNIAFRAEVSQPIVTRFFVQ
SQAPSFRVG (SEQ ID NO:262)
   >Cas14u.3|19ft-_2_nophage_noknown_scaffold_0_curated|508188..509648 
LAEENTLHLTLAMSLPLNDLPENRTRSELWRRQWLPQKKLSLLLGVNQSVRKAAADCLRW
FEPYQELLWWEPTDPDGKKLLDKEGRPIKRTAGHMRVLRKLEEIAPFRGYQLGSAVKNGL
RHKVADLLLSYAKRKLDPQFTDKTSYPSIGDQFPIVWTGAFVCYEQSITGQLYLYLPLFP
RGSHQEDITNNYDPDRGPALQVFGEKEIARLSRSTSGLLLPLQFDKWGEATFIRGENNPP
TWKATHRRSDKKWLSEVLLREKDFQPKRVELLVRNGRIFVNVACEIPTKPLLEVENFMGV
SFGLEHLVTVVVINRDGNVVHQRQEPARRYEKTYFARLERLRRRGGPFSQELETFHYRQV
AQIVEEALRFKSVPAVEQVGNIPKGRYNPRLNLRLSYWPFGKLADLTSYKAVKEGLPKPY
SVYSATAKMLCSTCGAANKEGDQPISLKGPTVYCGNCGTRHNTGFNTALNLARRAQELFV
KGVVAR. (SEQ ID NO:263)
   >Cas14u.4|rifcsp2_19_4_full_scaffold_168_curated|84455..85657 
MTTQKTYNFCFYDQRFFELSKEAGEVYSRSLEEFWKIYDETGVWLSKFDLQKHMRNKLER
KLLHSDSFLGAMQQVHANLASWKQAKKVVPDACPPRKPKFLQAILFKKSQIKYKNGFLRL
TLGTEKEFLYLKWDINIPLPIYGSVTYSKTRGWKINLCLETEVEQKNLSENKYLSIDLGV
KRVATIFDGENTITLSGKKFMGLMHYRNKLNGKTQSRLSHKKKGSNNYKKIQRAKRKTTD
RLLNIQKEMLHKYSSFIVNYAIRNDIGNIIIGDNSSTHDSPNMRGKTNQKISQNPEQKLK
NYIKYKFESISGRVDIVPEPYTSRKCPHCKNIKKSSPKGRTYKCKKCGFIFDRDGVGAIN
IYNENVSFGQIISPGRIRSLTEPIGMKFHNEIYFKSYVAA. (SEQ ID NO:264)
   >Cas14u.5|3300012532.a|Ga0137373_10000316|3286..5286 
MATLVYRYGVRAHGSARQQDAVVSDPAMLEQLRLGHELRNALVGVQHRYEDGKRAVWSGF
ASVAAADHRVTTGETAVAELEKQARAEHSADRTAATRQGTAESLKAARAAVKQARADRKA
AMAAVAEQAKPKIQALGDDRDAEIKDLYRRFCQDGVLLPRCGRCAGDLRSDGDCTDCGAA
HEPRKLYWATYNAIREDHQTAVKLVEAKRKAGQPARLRFRRWTGDGTLTVQLQRMHGPAC
RCVTCAEKLTRRARKTDPQAPAVAADPAYPPTDPPRDPALLASGQGKWRNVLQLGTWIPP
GEWSAMSRAERRRVGRSHIGWQLGGGRQLTLPVQLHRQMPADADVAMAQLTRVRVGGRHR
 
MSVALTAKLPDPPQVQGLPPVALHLGWRQRPDGSLRVATWACPQPLDLPPAVADVVVSHG
GRWGEVIMPARWLADAEVPPRLLGRRDKAMEPVLEALADWLEAHTEACTARMTPALVRRW
RSQGRLAGLTNRWRGQPPTGSAEILTYLEAWRIQDKLLWERESHLRRRLAARRDDAWRRV
ASWLARHAGVLVVDDADIAELRRRDDPADTDPTMPASAAQAARARAALAAPGRLRHLATI
TATRDGLGVHTVASAGLTRLHRKCGHQAQPDPRYAASAVVTCPGCGNGYDQDYNAAMLML
DRQQQP (SEQ ID NO:265)
   >Cas14u.6|3300006028.a|Ga0070717_10000077|54519..56201|revcom 
MTVRTYKYRAYPTPEQAEALTSWLRFASQLYNAALEHRKNAWGRHDAHGRGFRFWDGDAA
PRKKSDPPGRWVYRGGGGAHISKNDQGKLLTEFRREHAELLPPGMPALVQHEVLARLERS
MAAFFQRATKGQKAGYPRWRSEHRYDSLTFGLTSPSKERFDPETGESLGRGKTVGAGTYH
NGDLRLTGLGELRILEHRRIPMGAIPKSVIVRRSGKRWFVSIAMEMPSVEPAASGRPAVG
LDMGVVTWGTAFTADTSAAAALVADLRRMATDPSDCRRLEELEREAAQLSEVLAHCRARG
LDPARPRRCPKELTKLYRRSLHRLGELDRACARIRRRLQAAHDIAEPVPDEAGSAVLIEG
SNAGMRHARRVARTQRRVARRTRAGHAHSNRRKKAVQAYARAKERERSARGDHRHKVSRA
LVRQFEEISVEALDIKQLTVAPEHNPDPQPDLPAHVQRRRNRGELDAAWGAFFAALDYKA
ADAGGRVARKPAPHTTQECARCGTLVPKPISLRVHRCPACGYTAPRTVNSARNVLQRPLE
EPGRAGPSGANGRGVPHAVA. (SEQ ID NO:266)
   >Cas14u.7|330000|256.a|JGI12210J13797_10004690|5792..7006 
MNCRYRYRIYPTPGQRQSLARLFGCVRVVWNDALFLCRQSEKLPKNSELQKLCITQAKKT
EARGWLGQVSAIPLQQSVADLGVAFKNFFQSRSGKRKGKKVNPPRVKRRNNRQGARFTRG
GFKVKTSKVYLARIGDIKIKWSRPLPSEPSSVTVIKDCAGQYFLSFVVEVKPEIKPPKNP
SIGIDLGLKTFASCSNGEKIDSPDYSRLYRKLKRCQRRLAKRQRGSKRRERMRVKVAKLN
AQIRDKRKDFLHKLSTKVVNENQVIALEDLNVGGMLKNRKLSRAISQAGWYEFRSLCEGK
AEKHNRDFRVISRWEPTSQVCSECGYRWGKIDLSVRSIVCINCGVEHDRDDNASVNIEQA
GLKVGVGHTHDSKRTGSACKTSNGAVCVEPSTHREYVQLTLFDW. (SEQ ID NO:267)
   >Cas14u.8|3300005660.a|Ga0073904_10021651|765..1943

MKSRWTFRCYPTPEQEQHLARTFGCVRFVWNWALRARTDAFRAGERIGYPATDKALTLLK
QQPETVWLNEVSSVCLQQALRDLQVAFSNFFDKRAAHPSFKRKEARQSANYTERGFSFDH
ERRILKLAKIGAIKVKWSRKAIPHPSSIRLIRTASGKYFVSLVVETQPAPMPETGESVGV
DFGVARLATLSNGERISNPKHGAKWQRRLAFYQKRLARATKGSKRRMRIKRHVARIHEKI
GNSRSDTLHKLSTDLVTRFDLICVEDLNLRGMVKNHSLARSLHDASIGSAIRMIEEKAER
YGKNVVKIDRWFPSSKTCSDCGHIVEQLPLNVREWTCPECGTTHDRDANAAANILAVGQT
VSAHGGTVRRSRAKASERKSQRSANRQGVNRA. (SEQ ID NO:268)

## Claims

1. A nucleic acid detection system comprising:
i) a reporter molecule comprising a detectable label, wherein the detectable label is released for detection upon cleavage of the reporter molecule;
ii) a primary activator complex comprising a first Cas-effector enzyme programmed with a first guide RNA, wherein the first guide RNA recognizes one or more target sequences in the sample, wherein upon hybridization of the first guide RNA to the one or more target sequences, the primary activator complex is activated and is a non-specific nuclease that cleaves the reporter molecule and releases the detectable label; and
iii) an inactive second recognizing complex comprising a second Cas-effector enzyme, one or more activator sequences, and a second guide RNA, wherein activation of the primary activator complex results in the activation of one or more activator sequences, wherein the one or more activator sequences are recognized by the inactive second recognizing complex comprising the second Cas-effector enzyme and the second guide RNA, wherein upon hybridization of the second guide RNA of the inactive second recognizing complex to the one or more activated activator sequences, the second recognizing complex is activated and is a non-specific nuclease that cleaves the reporter molecule and releases the detectable label.

2. The nucleic acid detection system of claim 1, wherein the first and/or second Cas-effector enzymes comprise a non-specific RNase and/or a DNase when activated.

3. The nucleic acid detection system of any one of the preceding claims, wherein the first and/or second Cas-effector enzymes comprise one or more Cas13 proteins, one or more Cas12 proteins, one or more Cas14 proteins, one or more Csm6 proteins, and/or one or more Csx1 proteins, optionally one or more proteins as shown in any one of SEQ ID NOs: 115 through 268.

4. The nucleic acid detection system of any one of the preceding claims, wherein the reporter molecule comprises:
a) a quencher operably linked to the detectable label, optionally wherein the detectable label comprises one or more fluorescent molecule;
b) an oligonucleotide linking the quencher and the fluorophore, optionally wherein the oligonucleotide comprises a caged structure; and/or
c) a detectable label comprising one or more fluorescent dyes.

5. The nucleic acid detection system of any one of the preceding claims, wherein the one or more activator sequences comprise:
a) modified nucleotide bases; and/or
b) both RNA and DNA bases.

6. The nucleic acid detection system of any one of the preceding claims, wherein the first guide RNA, the second guide RNA, and/or one or more of the activator sequences are caged, optionally comprising or causing a stem-loop structure.

7. The nucleic acid detection system of claim 6, wherein the cage comprises or causes one or more structures such as a loop structure and/or a modification to one or more of the activator sequences comprising one or more locked nucleic acid (LNA) or moieties and/or 2'-OMe RNA.

8. The nucleic acid detection system of any one of the preceding claimswherein one or more of the activator sequences comprise caging structures on their 3' and/or 5' ends.

9. The nucleic acid detection system of any one of the preceding claims further comprising trans caging molecules.

10. The nucleic acid detection system of any one of the preceding claims, wherein the one or more activator sequences comprise poly U and/or poly A sequences, optionally A₄-Uₙ, A₅-Uₙ and A₆-Uₙ sequences.

11. The nucleic acid detection system of any one of the preceding claims, wherein the target sequence is DNA or RNA from one or more mammals, viruses, bacteria, or fungi.

12. A method of detecting a target sequence in a sample, the method comprising
(a) contacting a sample suspected of including the target sequence with:
(i) a nucleic acid detection system of any of the preceding claims, and
(b) measuring a detectable signal from the detectable label, thereby detecting the target sequence.

## Patentansprüche

1. Nukleinsäurenachweissystem, das umfasst:
i) ein Reportermolekül, das eine nachweisbare Markierung umfasst, wobei die nachweisbare Markierung nach Spaltung des Reportermoleküls für den Nachweis freigegeben wird;
ii) einen primären Aktivatorkomplex, der ein erstes Cas-Effektor-Enzym umfasst, das mit einer ersten Leit-RNA programmiert ist, wobei die erste Leit-RNA eine oder mehrere Zielsequenzen in der Probe erkennt, wobei bei der Hybridisierung der ersten Leit-RNA an die eine oder die mehreren Zielsequenzen der primäre Aktivatorkomplex aktiviert wird und eine unspezifische Nuklease ist, die das Reportermolekül spaltet und die nachweisbare Markierung freigibt; und
iii) einen inaktiven zweiten Erkennungskomplex, der ein zweites Cas-Effektor-Enzym, eine oder mehrere Aktivatorsequenzen und eine zweite Leit-RNA umfasst, wobei die Aktivierung des primären Aktivatorkomplexes zu der Aktivierung einer oder mehrerer Aktivatorsequenzen führt, wobei die eine oder die mehreren Aktivatorsequenzen von dem inaktiven zweiten Erkennungskomplex erkannt werden, der das zweite Cas-Effektor-Enzym und die zweite Leit-RNA umfasst, wobei bei der Hybridisierung der zweiten Leit-RNA des inaktiven zweiten Erkennungskomplexes an die eine oder die mehreren aktivierten Aktivatorsequenzen der zweite Erkennungskomplex aktiviert wird und eine unspezifische Nuklease ist, die das Reportermolekül spaltet und die nachweisbare Markierung freigibt.

2. Nukleinsäurenachweissystem nach Anspruch 1, wobei das erste und/oder zweite Cas-Effektor-Enzym bei Aktivierung eine unspezifische RNase und/oder eine DNase umfasst.

3. Nukleinsäurenachweissystem nach einem der vorstehenden Ansprüche, wobei das erste und/oder das zweite Cas-Effektor-Enzym ein oder mehrere Cas13-Proteine, ein oder mehrere Cas12-Proteine, ein oder mehrere Cas14-Proteine, ein oder mehrere Csm6-Proteine und/oder ein oder mehrere Csx1-Proteine, optional ein oder mehrere Proteine umfasst, wie in einer der SEQ ID NO: 115 bis 268 gezeigt.

4. Nukleinsäurenachweissystem nach einem der vorstehenden Ansprüche, wobei das Reportermolekül umfasst:
a) einen Quencher, der mit der nachweisbaren Markierung wirkverbunden ist, wobei die nachweisbare Markierung optional ein oder mehrere fluoreszierende Moleküle umfasst;
b) ein Oligonukleotid, das den Quencher und das Fluorophor verbindet, wobei das Oligonukleotid optional eine Käfigstruktur umfasst; und/oder
c) eine nachweisbare Markierung, die einen oder mehrere Fluoreszenzfarbstoffe umfasst.

5. Nukleinsäurenachweissystem nach einem der vorstehenden Ansprüche, wobei die eine oder die mehreren Aktivatorsequenzen umfasst/umfassen:
a) modifizierte Nukleotidbasen; und/oder
b) sowohl RNA- als auch DNA-Basen.

6. Nukleinsäurenachweissystem nach einem der vorstehenden Ansprüche, wobei die erste Leit-RNA, die zweite Leit-RNA und/oder eine oder mehrere der Aktivatorsequenzen eingekapselt sind und optional eine Stamm-Schleifen-Struktur umfassen oder bewirken.

7. Nukleinsäurenachweissystem nach Anspruch 6, wobei der Käfig eine oder mehrere Strukturen wie eine Schleifenstruktur und/oder eine Modifikation an einer oder mehreren der Aktivatorsequenzen umfasst oder bewirkt, die eine oder mehrere Locked Nucleic Acid (LNA-) Einheiten und/oder 2'-O-Me-RNA umfasst/umfassen.

8. Nukleinsäurenachweissystem nach einem der vorstehenden Ansprüche, wobei eine oder mehrere der Aktivatorsequenzen an ihren 3'- und/oder 5'-Enden Käfigstrukturen umfassen.

9. Nukleinsäurenachweissystem nach einem der vorstehenden Ansprüche, das ferner trans-Käfigmoleküle umfasst.

10. Nukleinsäurenachweissystem nach einem der vorstehenden Ansprüche, wobei die eine oder die mehreren Aktivatorsequenzen Poly-U- und/oder Poly-A-Sequenzen umfassen, optional A₄-Uₙ-, A₅-Uₙ- und A₆-Uₙ-Sequenzen.

11. Nukleinsäurenachweissystem nach einem der vorstehenden Ansprüche, wobei die Zielsequenz DNA oder RNA von einem oder mehreren Säugetieren, Viren, Bakterien oder Pilzen ist.

12. Verfahren zum Nachweisen einer Zielsequenz in einer Probe, wobei das Verfahren umfasst
(a) das Inberührungbringen einer Probe, die die Zielsequenz beinhalten könnte, mit:
(i) einem Nukleinsäurenachweissystem nach einem der vorstehenden Ansprüche, und
(b) das Messen eines nachweisbaren Signals von der nachweisbaren Markierung, wobei dadurch die Zielsequenz nachgewiesen wird.

## Revendications

1. Système de détection d'acide nucléique comprenant :
i) une molécule rapporteur comprenant un marqueur détectable, le marqueur détectable étant libéré en vue de sa détection lors du clivage de la molécule rapporteur,
ii) un complexe activateur primaire comprenant une première enzyme effectrice Cas programmée avec un premier ARN guide, le premier ARN guide reconnaissant une ou plusieurs séquences cibles dans l'échantillon, ledit complexe activateur primaire étant activé lors de l'hybridation du premier ARN guide avec la ou les séquences cibles, ledit complexe activateur consistant en une nucléase non spécifique qui clive la molécule rapporteur et libère le marqueur détectable, et
iii) un deuxième complexe de reconnaissance inactif comprenant une deuxième enzyme effectrice Cas, une ou plusieurs séquences activatrices et un deuxième ARN guide, l'activation du complexe activateur primaire entraînant l'activation d'une ou plusieurs séquences activatrices, la ou les séquences activatrices étant reconnues par le deuxième complexe de reconnaissance inactif comprenant ladite deuxième enzyme effectrice Cas et ledit deuxième ARN guide, ledit deuxième complexe de reconnaissance étant activé lors de l'hybridation du deuxième ARN guide du deuxième complexe de reconnaissance inactif avec la ou les séquences activatrices activées, ledit deuxième complexe de reconnaissance consistant en une nucléase non spécifique qui clive la molécule rapporteur et libère le marqueur détectable.

2. Système de détection d'acide nucléique selon la revendication 1, dans lequel les première et/ou deuxième enzymes effectrices Cas comprennent une RNase non spécifique et/ou une DNase en cas d'activation.

3. Système de détection d'acide nucléique selon l'une quelconque des revendications précédentes, dans lequel les première et/ou deuxième enzymes effectrices Cas comprennent une ou plusieurs protéines Cas13, une ou plusieurs protéines Cas12, une ou plusieurs protéines Cas14, une ou plusieurs protéines Csm6 et/ou une ou plusieurs protéines Csx1, éventuellement une ou plusieurs protéines telles que celles représentées dans l'une quelconque des SEQ ID NO 115 à 268.

4. Système de détection d'acide nucléique selon l'une quelconque des revendications précédentes, dans lequel la molécule rapporteur comprend :
a) un extincteur lié de manière fonctionnelle au marqueur détectable, le marqueur détectable comprenant éventuellement une ou plusieurs molécules fluorescentes,
b) un oligonucléotide reliant l'extincteur et le fluorophore, l'oligonucléotide comprenant éventuellement une structure en cage, et/ou
c) un marqueur détectable comprenant un ou plusieurs colorants fluorescents.

5. Système de détection d'acide nucléique selon l'une quelconque des revendications précédentes, dans lequel la ou les séquences activatrices comprennent :
a) des bases nucléotidiques modifiées, et/ou
b) à la fois des bases ARN et ADN.

6. Système de détection d'acide nucléique selon l'une quelconque des revendications précédentes, dans lequel le premier ARN guide, le deuxième ARN guide et/ou une ou plusieurs séquences activatrices sont en cage et comprennent ou provoquent éventuellement une structure tige-boucle.

7. Système de détection d'acide nucléique selon la revendication 6, dans lequel la cage comprend ou provoque une ou plusieurs structures telles qu'une structure en boucle et/ou une modification d'une ou plusieurs des séquences activatrices comprenant un ou plusieurs acides nucléiques verrouillés (LNA, locked nucleic acid) ou des fractions et/ou un ARN 2'-OMe.

8. Système de détection d'acide nucléique selon l'une quelconque des revendications précédentes, dans lequel une ou plusieurs des séquences activatrices comprennent des structures en cage à leurs extrémités 3' et/ou 5'.

9. Système de détection d'acide nucléique selon l'une quelconque des revendications précédentes, comprenant en outre des cages moléculaires trans.

10. Système de détection d'acide nucléique selon l'une quelconque des revendications précédentes, dans lequel la ou les séquences activatrices comprennent des séquences poly U et/ou poly A, éventuellement des séquences A₄-Uₙ, A₅-Uₙ et A₆-Uₙ.

11. Système de détection d'acide nucléique selon l'une quelconque des revendications précédentes, dans lequel la séquence cible est de l'ADN ou de l'ARN issu d'un ou plusieurs mammifères, virus, bactéries ou champignons.

12. Procédé de détection d'une séquence cible dans un échantillon, le procédé comprenant
(a) la mise en contact d'un échantillon soupçonné de contenir la séquence avec :
(i) un système de détection d'acide nucléique selon l'une quelconque des revendications précédentes, et
(b) la mesure d'un signal détectable en provenance du marqueur détectable, permettant ainsi la détection de la séquence cible.
